(19) Europäisches Patentamt
European Patent Office
Office européen des brevets

(11) EP 4 263 492 B1

(12) EUROPEAN PATENT SPECIFICATION

(45) Date of publication and mention of the grant of the patent:
**09.04.2025 Bulletin 2025/15**

(21) Application number: **21835811.7**

(22) Date of filing: **17.12.2021**

(51) International Patent Classification (IPC):
***C07C 235/38*** (2006.01) ***C07D 213/56*** (2006.01)
***C07D 213/64*** (2006.01) ***C07D 213/74*** (2006.01)
***C07D 215/08*** (2006.01) ***C07D 239/34*** (2006.01)
***C07D 239/42*** (2006.01) ***C07D 295/135*** (2006.01)
***C07D 317/58*** (2006.01) ***C07D 401/12*** (2006.01)
***A61K 31/4402*** (2006.01) ***A61K 31/5375*** (2006.01)
***A61K 31/505*** (2006.01) ***A61P 25/00*** (2006.01)

(52) Cooperative Patent Classification (CPC):
**C07D 213/56; A61P 25/00; C07C 235/22; C07C 235/24; C07C 235/40; C07C 237/42; C07C 255/54; C07C 317/32; C07D 213/64; C07D 213/74; C07D 215/08; C07D 239/34; C07D 239/42; C07D 295/135; C07D 317/58;**
(Cont.)

(86) International application number:
**PCT/GB2021/053346**

(87) International publication number:
**WO 2022/129933 (23.06.2022 Gazette 2022/25)**

(54) **MODULATORS OF G PROTEIN-COUPLED RECEPTOR 88**

MODULATOREN DES G-PROTEIN-GEKOPPELTEN REZEPTORS 88

MODULATEURS DU RÉCEPTEUR 88 COUPLÉ À UNE PROTÉINE G

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2020 GB 202020037**
**14.06.2021 GB 202108483**

(43) Date of publication of application:
**25.10.2023 Bulletin 2023/43**

(73) Proprietor: **ACADIA Pharmaceuticals Inc.**
**San Diego, CA 92130 (US)**

(72) Inventors:
- **QUIBELL, Martin**
  **Cambridge Cambridgeshire CB22 3AT (GB)**
- **SCHULZ-UTERMOEHL, Tim**
  **Cambridge Cambridgeshire CB22 3AT (GB)**
- **MURRAY, Fraser**
  **Cambridge Cambridgeshire CB22 3AT (GB)**
- **PICHOWICZ, Mark**
  **Nottingham NG1 1GR (GB)**

(74) Representative: **Maiwald GmbH**
**Grünstraße 25**
**40212 Düsseldorf (DE)**

(56) References cited:
**WO-A1-2009/075874 WO-A1-2011/044212**

- **DATABASE CAPLUS [online] 4 January 2018 (2018-01-04), - -: "-", XP055902775, Database accession no. 2018:1845616; 2016:369053; 2013:1658469;**
- **BI YINGZHI ET AL: "The discovery of potent agonists for GPR88, an orphan GPCR, for the potential treatment of CNS disorders", BIOORGANIC & MEDICINAL CHEMISTRY LETTERS, ELSEVIER, AMSTERDAM, NL, vol. 25, no. 7, 21 February 2015 (2015-02-21), pages 1443 - 1447, XP029148591, ISSN: 0960-894X, DOI: 10.1016/J.BMCL.2015.02.038**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 263 492 B1

- **JIN CHUNYANG ET AL: "Discovery of a Potent, Selective, and Brain-Penetrant Small Molecule that Activates the Orphan Receptor GPR88 and Reduces Alcohol Intake", JOURNAL OF MEDICINAL CHEMISTRY, vol. 61, no. 15, 16 July 2018 (2018-07-16), US, pages 6748 - 6758, XP055902551, ISSN: 0022-2623, DOI: 10.1021/acs.jmedchem.8b00566**
- **JIN CHUNYANG ET AL: "Synthesis, Pharmacological Characterization, and Structure-Activity Relationship Studies of Small Molecular Agonists for the Orphan GPR88 Receptor", ACS CHEMICAL NEUROSCIENCE, vol. 5, no. 7, 14 May 2014 (2014-05-14), US, pages 576 - 587, XP055902763, ISSN: 1948-7193, DOI: 10.1021/cn500082p**
- **JIN CHUNYANG ET AL: "Effect of Substitution on the Aniline Moiety of the GPR88 Agonist 2-PCCA: Synthesis, Structure-Activity Relationships, and Molecular Modeling Studies", ACS CHEMICAL NEUROSCIENCE, vol. 7, no. 10, 16 August 2016 (2016-08-16), US, pages 1418 - 1432, XP055902764, ISSN: 1948-7193, DOI: 10.1021/acschemneuro.6b00182**
- **JIN CHUNYANG ET AL: "Design, synthesis and pharmacological evaluation of 4-hydroxyphenylglycine and 4-hydroxyphenylglycinol derivatives as GPR88 agonists", BIOORG. & MED. CHEM. LETT., vol. 25, no. 2, 1 December 2016 (2016-12-01), pages 805 - 812, XP029862896, ISSN: 0968-0896, DOI: 10.1016/J.BMC.2016.11.058**


(52) Cooperative Patent Classification (CPC): (Cont.)
**C07D 401/12;** C07C 2601/02; C07C 2601/04; C07C 2603/62

## Description

## FIELD OF INVENTION

**[0001]** The present disclosure is generally directed to compounds which can modulate G-protein coupled receptor 88, compositions comprising such compounds, methods for modulating G-protein coupled receptor 88 and compounds for use in such methods.

## BACKGROUND

**[0002]** GPR88 is an orphan member of the G protein coupled receptor (GPCR) superfamily and a member of the class A rhodopsin family of GPCRs. The receptor exhibits high expression in the central nervous system (CNS) with limited expression in the periphery.

**[0003]** Within the CNS, the mRNA for the GPR88 receptor is localised primarily to selective areas of the brain, namely the striatum (Mizushima et al., 2000; Vassilatis et al., 2002; Massart et al., 2009). It is also present at lower expression levels in the frontal cortex and thalamus (Thompson et al., 2020). Striatal expression is on GABAergic medium spiny neurons (MSN)Data from rodents suggest that GPR88 displays the highest mRNA expression levels compared to other knowns GPCRs in the striatum (Komatsu et al., 2014).

**[0004]** The striatum regulates various aspects of cognition, motivation and reward as well as movement and motor learning and has been implicated in neuropsychiatric diseases such as Tourette's Syndrome, Huntington's Disease (HD), Addiction, Parkinson's Disease (PD), Schizophrenia, and Attention Deficit Hyperactivity Disorder (ADHD) (Ena et al., 2011). The selective GPR88 expression profile in striatal output neurons, led to the discovery that the GPR88 receptor modulates the function of several cortico-striato-thalamic loops via striatal MSNs influencing both direct and indirect pathways and subsequently influencing cortical transmission. The receptor also regulates monoamine neurotransmission (Quintana et al., 2012; Meirsman et al., 2016), influences neural connectivity (Arefin et al., 2017), and thus suggest its possible relevance as a target for motor symptoms in CNS diseases (van Waes et al., 2011) as well as its previously suggested roles in cognitive and reward pathways.

**[0005]** In GPR88$^{Cre/Cre}$ knockout (KO) mice, MSNs have increased glutamatergic excitation resulting from enhanced phosphorylation of the AMPA-type glutamate receptor subunit GluR1, reduced tonic GABAergic inhibition resulting from low level of b3 protein (a GABA-A subunit) that together promote enhanced firing rates *in vivo*, resulting in hyperactivity, poor motor-coordination, and impaired cue-based learning in mice (Quintana et al., 2012). Furthermore, GPR88$^{-/-}$ knockout mice display impaired striatal dependent behaviours (Meirsman et al., 2016). GPR88 deletion impaired motor coordination and motor learning in the accelerating rotarod test. GPR88 knockout mice travelled a longer distance in the open field as compared to controls and this hyperactivity failed to habituate over sessions. In a separate study (Thompson et al., 2020), Gpr88 KO mice showed impaired correct responding in an N-back task, suggesting a role for GPR88 receptors in working memory. In a touchscreen task, performance was impaired at the reversal learning stage, suggesting cognitive inflexibility. Evidence for a role of GPR88 in reward processing was demonstrated in a touchscreen-based equivalent of the lowa gambling task.

**[0006]** In post-mortem brains from HD patients, it has been shown that GPR88 mRNA is significantly downregulated (Hodges et al., 2006). Additionally, in aged BACHD and R6/1 murine models of HD, a significant decrease in GPR88 mRNA has also been detected (Desplats et al., 2006; Rocher et al., 2015).

**[0007]** Rare mutations in humans suggest a role in cognition and motor function. A recent molecular investigation of patients from a consanguineous family (non-HD patients) who presented in childhood with choreiform movements, speech delay, and learning disabilities indicated a GPR88 deficiency due to a homozygous deleterious mutation in GPR88 (Alkufri et al., 2017). This clinical data is consistent with the reported abundant expression of GPR88 in the striatum and the hyperkinetic activity and learning impairment observed in GPR88 knockout mice as highlighted previously.

**[0008]** The therapeutic potential of GPR88 modulators in PD has been demonstrated by studies showing that the knockdown of GPR88 in the striatum reduces psychiatric symptoms in a translational male rat model of Parkinson disease (Galet et al., 2019; 2020) and further studies showing that genetic deletion of GPR88 promotes L-DOPA-induced rotation and spontaneous locomotion yet suppresses the induction of LIDs and also reduces tremor (Mantas et al., 2020). Transcriptional profiling studies have also revealed that GPR88 expression is altered by treatments or conditions related to bipolar disorder (Ogden et al., 2004) and depression (Brandish et al., 2005; Boehm et al., 2006). Furthermore, GPR88 receptors have been implicated in addiction (Hamida et al., 2018) and affective disorders (Watkins & Orlandi, 2020).

**[0009]** Based on these data, compounds that modulate GPR88 activity (agonists, antagonists, or modulators) are predicted to have therapeutic utility in the treatment of Huntington's Disease (HD) and other hyperkinetic movement disorders characterised by chorea and/or dystonia, psychosis, cognitive deficits in schizophrenia, affective disorders, atten,tion deficit hyperactivity disorders (ADHD), Tourette's Syndrome, bipolar disorder, addiction, Alzheimer's disease (AD) Parkinson's disease (PD), and other basal ganglia disorders.

**[0010]** GPR88 demonstrates GPCR activity in several assays including GTPgS binding, calcium influx, and cAMP inhibition assays.

**[0011]** Bi et al Bioorganic & Medicinal Chemistry Letters 25 (2015) 1443-1447 describe a class of GPR88 agonists. The authors modified the amino alkyl side chain in the linker portion (shown in the below structure by the box) to determine the effect on potency:

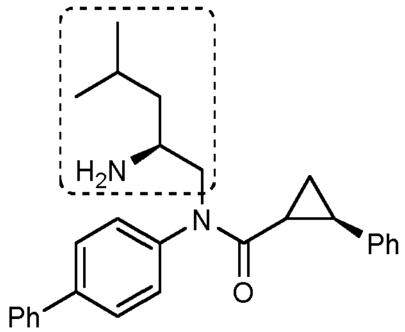

The authors concluded from the SAR in this region that there was little tolerance for modification. Their data showed that the primary amino group was important for potency. They found that, without the group highlighted in the box above, the compounds were completely inactive.

**[0012]** Jin et al, ACS Chem. Neurosci. 2014, 5(7), 576-587 and Jin et al, ACS Chem Neurosci. 2016 Oct 19; 7(10):1418-1432 are two papers by the same authors that describe various GPR88 modulators. In the earier paper, the authors produced compounds having various different subsitutents on the phenyl ring of the aniline portion and different substituents on the ethylamine portion of the linker. In the later paper, the authors concentrated on producing compounds having various different subsitutents on the phenyl ring of the aniline portion. Again, the compounds in these papers have an amino alkyl side chain in the linker portion.

**[0013]** Similar to Bi et al and each Jin et al paper, WO2011044212 provides various GPR88 modulators that require an amino alkyl side chain in the linker portion.

**[0014]** It has now been found that the prior art GPR88 modulators exhibit one or more suboptimal pharmacokinetic properties and/or exhibit off target activity.

## AIMS OF THE INVENTION

**[0015]** The present invention is directed towards the identification of a novel class of GPR88 modulators having improved pharmacokinetic properties and/or reduced off target activity relative to prior art GPR88 modulators.

**[0016]** It is an aim of certain embodiments of this invention to provide compounds having GPR88 modulating activitiy.

**[0017]** It is an aim of certain embodiments of this invention to provide compounds having GPR88 modulating activitiy and improved pharmacokinetic properties relative to prior art GPR88 modulators.

**[0018]** It is an aim of certain embodiments of this invention to provide compounds having GPR88 modulating activitiy and reduced off target activity relative to prior art GPR88 modulators.

**[0019]** It is an aim of certain embodiments of this invention to provide compounds having GPR88 modulating activitiy, improved pharmacokinetic properties relative to prior art GPR88 modulators and reduced off target activity relative to prior art GPR88 modulators.

**[0020]** Certain embodiments of the present invention satisfy some or all of the above aims.

## BRIEF SUMMARY OF THE DISCLOSURE

**[0021]** According to a first aspect, there is provided a compound of Formula (I) or a pharmaceutically acceptable salt thereof:

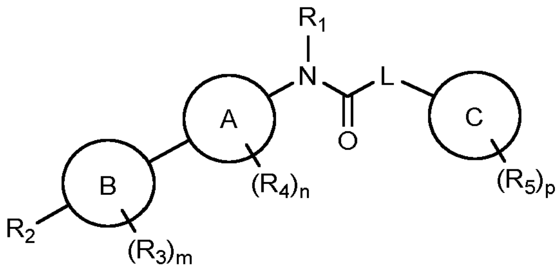

(I)

wherein:

-L- is selected from the group consisting of

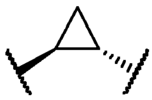
,
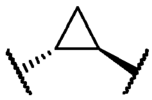
,
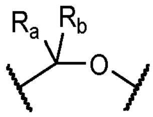

and

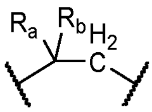
;

Ring A is selected from the group consisting of: a 5-10 membered cycloalkyl, a 5-10 membered cycloalkenyl, a 5-6 membered aromatic ring system, a 5-6 membered heteroaromatic ring system, an 8-10 membered bicyclic heterocyclic ring system and an 8-10 membered bicyclic heteroaromatic ring system;

Ring B is selected from the group consisting of: a 5-8 membered cycloalkyl, a 5-6 membered aromatic and a 5-6 membered heteroaromatic ring;

Ring C is selected from the group consisting of: a 5-6 membered aromatic and a 5-6 membered heteroaromatic ring;

m is 0, 1 or 2;

n is 0, 1 or 2;

p is 0, 1 or 2;

$R_1$ is selected from the group consisting of: hydrogen, substituted or unsubstituted $C_1$-$C_4$ alkyl, substituted or unsubstituted $C_3$-$C_4$ cycloalkyl and substituted or unsubstituted $C_3$-$C_4$ cycloalkyl $C_1$-$C_2$ alkyl; wherein each substituent is independently selected from the group consisting of -$OR_c$, nitrile or halo; wherein each $R_c$ is independently selected from the group consisting of H, Me, Et and cyclopropyl;

$R_2$ is selected from the group consisting of: substituted or unsubstituted $C_1$-$C_6$ alkyl, substituted or unsubstituted $C_2$-$C_6$ alkenyl, substituted or unsubstituted $C_2$-$C_6$ alkynyl; substituted or unsubstituted $C_3$-$C_6$ cycloalkyl; substituted or unsubstituted $C_1$-$C_6$ alkoxy, substituted or unsubstituted $C_3$-$C_6$ cycloalkoxy; substituted or unsubstituted $C_1$-$C_6$ alkoxy-$C_1$-$C_6$ alkyl; substituted or unsubstituted $C_3$-$C_6$ cycloalkoxy-$C_1$-$C_6$ alkyl; substituted or unsubstituted $C_1$-$C_6$ haloalkyl, substituted or unsubstituted $C_2$-$C_6$ haloalkenyl, substituted or unsubstituted $C_2$-$C_6$ haloalkynyl; substituted or unsubstituted $C_3$-$C_6$ cyclohaloalkyl; substituted or unsubstituted $C_1$-$C_6$ haloalkoxy, substituted or unsubstituted $C_3$-$C_6$ cyclohaloalkoxy; substituted or unsubstituted $C_1$-$C_6$ haloalkoxy-$C_1$-$C_6$ alkyl, substituted or unsubstituted $C_3$-$C_6$ cyclohaloalkoxy-$C_1$-$C_6$ alkyl, substituted or unsubstituted $C_1$-$C_6$ alkoxy-$C_1$-$C_6$ haloalkyl, substituted or unsubstituted $C_3$-$C_6$ cycloalkoxy-$C_1$-$C_6$ haloalkyl, substituted or unsubstituted $C_1$-$C_6$ haloalkoxy-$C_1$-$C_6$ haloalkyl, substituted or unsubstituted $C_3$-$C_6$ cyclohaloalkoxy-$C_1$-$C_6$ haloalkyl $SO_2R_d$, and -$NR_eR_e$;

wherein $R_d$ is $C_{1-4}$ alkyl;

wherein each $R_e$ is independently selected from the group consisting of H and $C_{1-4}$ alkyl;

wherein each substituent is independently selected from the group consisting of $-OR_e$, =O, $-NR_eR_e$, nitrile, halo, $C_1$-$C_4$ alkyl, $C_1$-$C_6$ alkoxy, and a substituted or unsubstituted monocyclic, bicyclic or bridged 3-8 membered cycloalkyl or heterocycloalkyl ring, wherein each $R_e$ is independently selected from the group consisting of H and $C_{1-4}$ alkyl;
wherein each substituent of the monocyclic, bicyclic or bridged 3-8 membered cycloalkyl or heterocycloalkyl ring is independently selected from the group consisting of -OH, nitrile, halo, methyl or methoxy;

each $R_3$ is independently selected from the group consisting of: halogen, $C_1$-$C_3$ alkyl, $C_1$-$C_3$ haloalkyl, $C_1$-$C_3$ alkoxy, $C_1$-$C_3$ haloalkoxy or -CN;

each $R_4$ is independently selected from the group consisting of: halogen, $C_1$-$C_3$ alkyl, $C_1$-$C_3$ haloalkyl, $C_1$-$C_3$ alkoxy, $C_1$-$C_3$ haloalkoxy or -CN;

each $R_5$ is independently selected from the group consisting of: halogen, $C_1$-$C_3$ alkyl, $C_1$-$C_3$ haloalkyl, $C_1$-$C_3$ alkoxy, $C_1$-$C_3$ haloalkoxy or -CN;

$R_a$ and $R_b$ are each independently selected from the group consisting of: hydrogen, halogen, $C_1$-$C_6$ alkyl, and $C_1$-$C_6$ alkoxy; or wherein $R_a$ and $R_b$, together with the atom to which they are bonded, form a 3-membered to 6-membered cycloalkyl or heterocycloalkyl ring comprising -O- or $-NR_f$-, wherein $R_f$ is H or methyl;

provided that the compound is not:

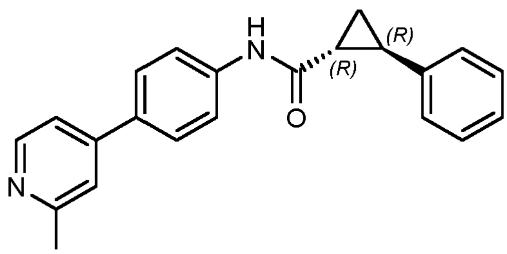

or

-L-, Ring A, Ring B, Ring C, m, p, $R_2$, $R_3$, $R_5$, $R_a$, $R_b$, $R_c$, $R_d$, $R_e$ and $R_f$ are as defined above,

$R_1$ is a $C_1$ or $C_2$ alkylenyl residue,

n is 1 or 2, and

one $R_4$ moiety is a $C_1$ alkylenyl residue and, when present, the other $R_4$ moiety is as defined above,
wherein the $C_1$ alkylenyl $R_4$ moeity is bonded to a carbon atom in Ring A that is ortho to the carbon atom of Ring A having the amide residue, and, together with the atoms to which they are bonded, the $R_1$ and $R_4$ moieties form a 5- or 6-membered ring;
and/or

-L-, Ring A, Ring B, Ring C, n, p, $R_1$, $R_4$, $R_5$, $R_a$, $R_b$, $R_c$ and $R_f$ are as defined above,

m is 1 or 2;

$R_2$ is selected from the group consisting of: substituted or unsubstituted $-O-C_{1-2}$ alkylenyl, wherein the oxygen atom is connected to Ring B;
wherein each substituent is independently selected from the group consisting of -OH, nitrile, halo, methyl or methoxy;

one $R_3$ moiety is an -O- residue and, when present, the other $R_3$ moiety is as defined above;
wherein, together with the atoms to which they are bonded, the $R_2$ moiety and $R_3$ moiety, which is an -O- residue, form a 5- or 6-membered ring.

**[0022]** In an embodiment, Ring A is selected from the group consisting of: a 5-8 membered cycloalkyl, a 5-8 membered cycloalkenyl, a 5-6 membered aromatic, a 5-6 membered heteroaromatic ring, an 8-10 membered bicyclic heterocyclic ring system and an 8-10 membered bicyclic heteroaromatic ring system.

**[0023]** In an embodiment, Ring A is selected from the group consisting of: a 5-8 membered cycloalkyl, a 5-8 membered cycloalkenyl, a 5-6 membered aromatic and a 5-6 membered heteroaromatic ring.

**[0024]** In an embodiment, Ring A is selected from the group consisting of: a 6 membered cycloalkyl, cycloalkenyl, aromatic and heteroaromatic ring.

**[0025]** In an embodiment, Ring A is selected from the group consisting of: a 6 membered aromatic and heteroaromatic ring.

**[0026]** In an embodiment, Ring A is phenyl.

**[0027]** In an embodiment, Ring A is a 5 or 8 membered cycloalkyl ring system. In an embodiment, Ring A is a 5 membered cycloalkyl ring system. In an embodiment, Ring A is an 8 membered cycloalkyl ring system. In an embodiment, Ring A is bicyclo-[1.1.1]-pentyl. In an embodiment, Ring A is bicyclo-[2.2.2]-octyl.

**[0028]** In an embodiment, Ring B is selected from the group consisting of: a 5 or 6 membered cycloalkyl, a 5 or 6 membered aromatic and a 5 or 6 membered heteroaromatic ring.

**[0029]** In an embodiment, Ring B is selected from the group consisting of: a 6 membered cycloalkyl, a 6 membered aromatic and a 6 membered heteroaromatic ring.

**[0030]** In an embodiment, Ring B is phenyl. In an embodiment, Ring B is pyridinyl. In an embodiment, Ring B is pyrimidinyl.

**[0031]** In an embodiment, Ring C is selected from the group consisting of: a 6 membered aromatic and a 6 membered heteroaromatic ring.

**[0032]** In an embodiment, Ring C is phenyl. In an embodiment, Ring C is pyridinyl.

**[0033]** In an embodiment, Ring A is selected from the group consisting of: a 5-8 membered cycloalkyl, a 5-8 membered cycloalkenyl, a 5-6 membered aromatic and a 5-6 membered heteroaromatic ring and Ring B is selected from the group consisting of: a 5 or 6 membered cycloalkyl, a 5 or 6 membered aromatic and a 5 or 6 membered heteroaromatic ring.

**[0034]** In an embodiment, Ring A is selected from the group consisting of: a 6 membered aromatic and heteroaromatic ring and Ring B is selected from the group consisting of: a 6 membered cycloalkyl, a 6 membered aromatic and a 6 membered heteroaromatic ring.

**[0035]** In an embodiment, Ring A is a 5 or 8 membered cycloalkyl ring system and Ring B is selected from the group consisting of: a 6 membered cycloalkyl, a 6 membered aromatic and a 6 membered heteroaromatic ring.

**[0036]** In an embodiment, Ring A is a 5 membered cycloalkyl ring system and Ring B is selected from the group consisting of: a 6 membered cycloalkyl, a 6 membered aromatic and a 6 membered heteroaromatic ring.

**[0037]** In an embodiment, Ring A is phenyl and Ring B is phenyl. In an embodiment, Ring A is phenyl and Ring B is pyridinyl. In an embodiment, Ring A is phenyl and Ring B is pyrimidinyl.

**[0038]** In an embodiment, Ring A is bicyclo-[1.1.1]-pentyl and Ring B is phenyl. In an embodiment, Ring A is bicyclo-[1.1.1]-pentyl and Ring B is pyridinyl. In an embodiment, Ring A is bicyclo-[1.1.1]-pentyl and Ring B is pyrimidinyl.

**[0039]** In an embodiment, Ring A is selected from the group consisting of: a 5-8 membered cycloalkyl, a 5-8 membered cycloalkenyl, a 5-6 membered aromatic and a 5-6 membered heteroaromatic ring and Ring C is selected from the group consisting of: a 6 membered aromatic and a 6 membered heteroaromatic ring.

**[0040]** In an embodiment, Ring A is selected from the group consisting of: a 6 membered aromatic and heteroaromatic ring and Ring C is selected from the group consisting of: a 6 membered aromatic and a 6 membered heteroaromatic ring.

**[0041]** In an embodiment, Ring A is a 5 or 8 membered cycloalkyl ring system and Ring C is selected from the group consisting of: a 6 membered aromatic and a 6 membered heteroaromatic ring.

**[0042]** In an embodiment, Ring A is a 5 membered cycloalkyl ring system and Ring C is selected from the group consisting of: a 6 membered aromatic and a 6 membered heteroaromatic ring.

**[0043]** In an embodiment, Ring A is phenyl and Ring C is phenyl. In an embodiment, Ring A is phenyl and Ring C is pyridinyl.

**[0044]** In an embodiment, Ring A is bicyclo-[1.1.1]-pentyl and Ring C is phenyl. In an embodiment, Ring A is bicyclo-[1.1.1]-pentyl and Ring C is pyridinyl.

**[0045]** In an embodiment, Ring B is selected from the group consisting of: a 5 or 6 membered cycloalkyl, a 5 or 6 membered aromatic and a 5 or 6 membered heteroaromatic ring and Ring C is selected from the group consisting of: a 6 membered aromatic and a 6 membered heteroaromatic ring.

**[0046]** In an embodiment, Ring B is selected from the group consisting of: a 6 membered cycloalkyl, a 6 membered aromatic and a 6 membered heteroaromatic ring and Ring C is selected from the group consisting of: a 6 membered aromatic and a 6 membered heteroaromatic ring.

**[0047]** In an embodiment, Ring B is phenyl and Ring C is phenyl. In an embodiment, Ring B is phenyl and Ring C is pyridinyl.

**[0048]** In an embodiment, Ring B is pyridinyl and Ring C is phenyl. In an embodiment, Ring B is pyridinyl and Ring C is

pyridinyl.

**[0049]** In an embodiment, Ring A is selected from the group consisting of: a 5-8 membered cycloalkyl, a 5-8 membered cycloalkenyl, a 5-6 membered aromatic and a 5-6 membered heteroaromatic ring, Ring B is selected from the group consisting of: a 5 or 6 membered cycloalkyl, a 5 or 6 membered aromatic and a 5 or 6 membered heteroaromatic ring and Ring C is selected from the group consisting of: a 6 membered aromatic and a 6 membered heteroaromatic ring.

**[0050]** In an embodiment, Ring A is selected from the group consisting of: a 6 membered aromatic and heteroaromatic ring, Ring B is selected from the group consisting of: a 6 membered cycloalkyl, a 6 membered aromatic and a 6 membered heteroaromatic ring and Ring C is selected from the group consisting of: a 6 membered aromatic and a 6 membered heteroaromatic ring.

**[0051]** In an embodiment, Ring A is a 5 or 8 membered cycloalkyl ring system, Ring B is selected from the group consisting of: a 6 membered cycloalkyl, a 6 membered aromatic and a 6 membered heteroaromatic ring and Ring C is selected from the group consisting of: a 6 membered aromatic and a 6 membered heteroaromatic ring.

**[0052]** In an embodiment, Ring A is a 5 membered cycloalkyl ring system, Ring B is selected from the group consisting of: a 6 membered cycloalkyl, a 6 membered aromatic and a 6 membered heteroaromatic ring and Ring C is selected from the group consisting of: a 6 membered aromatic and a 6 membered heteroaromatic ring.

**[0053]** In an embodiment, Ring A is phenyl, Ring B is phenyl and Ring C is phenyl. In an embodiment, Ring A is phenyl, Ring B is pyridinyl and Ring C is phenyl. In an embodiment, Ring A is phenyl, Ring B is pyrimidinyl and Ring C is phenyl.

**[0054]** In an embodiment, Ring A is phenyl, Ring B is phenyl and Ring C is pyridinyl. In an embodiment, Ring A is phenyl, Ring B is pyridinyl and Ring C is pyridinyl. In an embodiment, Ring A is phenyl, Ring B is pyrimidinyl and Ring C is pyridinyl.

**[0055]** In an embodiment, Ring A is bicyclo-[1.1.1]-pentyl, Ring B is phenyl and Ring C is phenyl. In an embodiment, Ring A is bicyclo-[1.1.1]-pentyl, Ring B is pyridinyl and Ring C is phenyl. In an embodiment, Ring A is bicyclo-[1.1.1]-pentyl, Ring B is pyrimidinyl and Ring C is phenyl.

**[0056]** In an embodiment, Ring A is bicyclo-[1.1.1]-pentyl, Ring B is phenyl and Ring C is pyridinyl. In an embodiment, Ring A is bicyclo-[1.1.1]-pentyl, Ring B is pyridinyl and Ring C is pyridinyl. In an embodiment, Ring A is bicyclo-[1.1.1]-pentyl, Ring B is pyrimidinyl and Ring C is pyridinyl.

**[0057]** In an embodiment, -L- is selected from the group consisting of

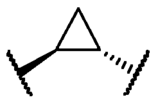
;

and

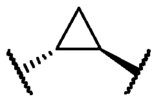
.

In an embodiment, -L- is selected from the group consisting of

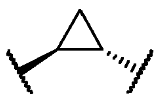

and

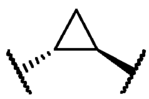

and Ring

**[0058]** In an embodiment, -L- is A, Ring B and Ring C are as defined in any of paragraphs [0021] to [0056].

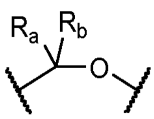
.

In an embodiment, -L- is

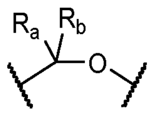

and Ring A, Ring B and Ring C are as defined in any of paragraphs [0021] to [0056].

**[0059]** In an embodiment, -L- is

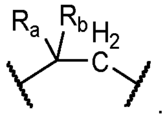

In an embodiment, -L- is

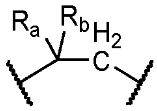

and Ring A, Ring B and Ring C are as defined in any of paragraphs [0021] to [0056].

**[0060]** In an embodiment, $R_a$ and $R_b$ are each independently selected from the group consisting of: hydrogen, halogen, $C_1$-$C_4$ alkyl, and $C_1$-$C_4$ alkoxy; or wherein $R_a$ and $R_b$, together with the atom to which they are bonded, form a 3-membered to 4-membered cycloalkyl or heterocycloalkyl ring comprising -O- or -$NR_f$-, wherein $R_f$ is H or methyl.

**[0061]** In an embodiment, $R_a$ and $R_b$ are each independently selected from the group consisting of: hydrogen, halogen, $C_1$-$C_6$ alkyl, and $C_1$-$C_6$ alkoxy.

**[0062]** In an embodiment, $R_a$ and $R_b$ are each independently selected from the group consisting of: hydrogen, halogen, $C_1$-$C_4$ alkyl, and $C_1$-$C_4$ alkoxy.

**[0063]** In an embodiment, $R_a$ and $R_b$ are each independently selected from the group consisting of: hydrogen and $C_1$-$C_6$ alkyl.

**[0064]** In an embodiment, $R_a$ and $R_b$ are each independently selected from the group consisting of: hydrogen and $C_1$-$C_3$ alkyl.

**[0065]** In an embodiment, $R_a$ and $R_b$ are each independently selected from the group consisting of: hydrogen and methyl.

**[0066]** In an embodiment, $R_a$ is H and $R_b$ is methyl. In an embodiment, $R_a$ is methyl and $R_b$ is methyl. In an embodiment, $R_a$ is H and $R_b$ is H.

**[0067]** In an embodiment, $R_a$ and $R_b$ are as defined in any of paragraphs [0060] to [0066] and -L-, Ring A, Ring B and Ring C are as defined in any of paragraphs [0021] to [0059].

**[0068]** In an embodiment, $R_1$ is selected from the group consisting of: hydrogen, substituted or unsubstituted $C_1$-$C_4$ alkyl, substituted or unsubstituted $C_3$-$C_4$ cycloalkyl and substituted or unsubstituted $C_3$-$C_4$ cycloalkyl $C_1$-$C_2$ alkyl; wherein each substituent is independently -$OR_c$; and wherein each $R_c$ is independently selected from the group consisting of H, Me, Et and cyclopropyl.

**[0069]** In an embodiment, $R_1$ is selected from the group consisting of: hydrogen, substituted or unsubstituted $C_1$-$C_3$ alkyl, substituted or unsubstituted $C_3$ cycloalkyl and substituted or unsubstituted $C_3$ cycloalkyl $C_1$ alkyl; wherein each substituent is independently -$OR_c$; and wherein each $R_c$ is independently selected from the group consisting of H, Me, Et and cyclopropyl.

**[0070]** In an embodiment, $R_1$ is selected from the group consisting of: hydrogen, substituted or unsubstituted $C_1$-$C_4$ alkyl and substituted or unsubstituted $C_3$-$C_4$ cycloalkyl $C_1$-$C_2$ alkyl; wherein each substituent is independently -$OR_c$; and wherein each $R_c$ is independently selected from the group consisting of H, Me, Et and cyclopropyl.

**[0071]** In an embodiment, $R_1$ is selected from the group consisting of: hydrogen, substituted or unsubstituted $C_1$-$C_4$ alkyl and substituted or unsubstituted $C_3$ cycloalkyl $C_1$ alkyl; wherein each substituent is independently -$OR_c$; and wherein each $R_c$ is independently selected from the group consisting of H, Me, Et and cyclopropyl.

**[0072]** In an embodiment, $R_1$ is selected from the group consisting of: hydrogen and substituted or unsubstituted $C_1$-$C_3$ alkyl; wherein each substituent is independently -$OR_c$; and wherein each $R_c$ is independently selected from the group consisting of H, Me, Et and cyclopropyl.

**[0073]** In an embodiment, $R_1$ is selected from the group consisting of: hydrogen and substituted or unsubstituted $C_1$-$C_2$ alkyl; wherein each substituent is independently -$OR_c$; and wherein each $R_c$ is independently selected from the group

consisting of H, Me, Et and cyclopropyl.

**[0074]** In an embodiment, $R_1$ is substituted or unsubstituted $C_3$-$C_4$ cycloalkyl $C_1$-$C_2$ alkyl; wherein each substituent is independently -$OR_c$; and wherein each $R_c$ is independently selected from the group consisting of H, Me, Et and cyclopropyl.

**[0075]** In an embodiment, $R_1$ is selected from the group consisting of: hydrogen, substituted or unsubstituted $C_1$-$C_4$ alkyl, substituted or unsubstituted $C_3$-$C_4$ cycloalkyl and substituted or unsubstituted $C_3$-$C_4$ cycloalkyl $C_1$-$C_2$ alkyl; wherein each substituent is independently -$OR_c$; and wherein each $R_c$ is independently selected from the group consisting of H and Me.

**[0076]** In an embodiment, $R_1$ is selected from the group consisting of: hydrogen, substituted or unsubstituted $C_1$-$C_3$ alkyl, substituted or unsubstituted $C_3$ cycloalkyl and substituted or unsubstituted $C_3$ cycloalkyl $C_1$ alkyl; wherein each substituent is independently -$OR_c$; and wherein each $R_c$ is independently selected from the group consisting of H and Me.

**[0077]** In an embodiment, $R_1$ is selected from the group consisting of: hydrogen and substituted or unsubstituted $C_1$-$C_4$ alkyl and substituted or unsubstituted $C_3$-$C_4$ cycloalkyl $C_1$-$C_2$ alkyl; wherein each substituent is independently -$OR_c$; and wherein each $R_c$ is independently selected from the group consisting of H and Me.

**[0078]** In an embodiment, $R_1$ is selected from the group consisting of: hydrogen and substituted or unsubstituted $C_1$-$C_4$ alkyl and substituted or unsubstituted $C_3$ cycloalkyl $C_1$ alkyl; wherein each substituent is independently -$OR_c$; and wherein each $R_c$ is independently selected from the group consisting of H and Me.

**[0079]** In an embodiment, $R_1$ is selected from the group consisting of: hydrogen, substituted or unsubstituted $C_1$-$C_3$ alkyl; wherein each substituent is independently -$OR_c$; and wherein each $R_c$ is independently selected from the group consisting of H and Me.

**[0080]** In an embodiment, $R_1$ is selected from the group consisting of: hydrogen, substituted or unsubstituted $C_1$-$C_2$ alkyl; wherein each substituent is independently -$OR_c$; and wherein each $R_c$ is independently selected from the group consisting of H and Me.

**[0081]** In an embodiment, $R_1$ is substituted or unsubstituted $C_3$-$C_4$ cycloalkyl $C_1$-$C_2$ alkyl; wherein each substituent is independently -$OR_c$; and wherein each $R_c$ is independently selected from the group consisting of H and Me.

**[0082]** In an embodiment, $R_1$ is selected from the group consisting of: hydrogen, substituted or unsubstituted $C_1$-$C_4$ alkyl, substituted or unsubstituted $C_3$-$C_4$ cycloalkyl and substituted or unsubstituted $C_3$-$C_4$ cycloalkyl $C_1$-$C_2$ alkyl; wherein each substituent is nitrile.

**[0083]** In an embodiment, $R_1$ is selected from the group consisting of: hydrogen, substituted or unsubstituted $C_1$-$C_3$ alkyl, substituted or unsubstituted $C_3$ cycloalkyl and substituted or unsubstituted $C_3$ cycloalkyl $C_1$ alkyl; wherein each substituent is nitrile.

**[0084]** In an embodiment, $R_1$ is selected from the group consisting of: hydrogen, substituted or unsubstituted $C_1$-$C_4$ alkyl and substituted or unsubstituted $C_3$-$C_4$ cycloalkyl $C_1$-$C_2$ alkyl; wherein each substituent is nitrile.

**[0085]** In an embodiment, $R_1$ is selected from the group consisting of: hydrogen, substituted or unsubstituted $C_1$-$C_4$ alkyl and substituted or unsubstituted $C_3$ cycloalkyl $C_1$ alkyl; wherein each substituent is nitrile.

**[0086]** In an embodiment, $R_1$ is selected from the group consisting of: hydrogen and substituted or unsubstituted $C_1$-$C_3$ alkyl; wherein each substituent is nitrile.

**[0087]** In an embodiment, $R_1$ is selected from the group consisting of: hydrogen and substituted or unsubstituted $C_1$-$C_2$ alkyl; wherein each substituent is nitrile.

**[0088]** In an embodiment, $R_1$ is substituted or unsubstituted $C_3$-$C_4$ cycloalkyl $C_1$-$C_2$ alkyl; wherein each substituent is nitrile.

**[0089]** In an embodiment, $R_1$ is selected from the group consisting of: hydrogen, substituted or unsubstituted $C_1$-$C_4$ alkyl, substituted or unsubstituted $C_3$-$C_4$ cycloalkyl and substituted or unsubstituted $C_3$-$C_4$ cycloalkyl $C_1$-$C_2$ alkyl; wherein each substituent is halo.

**[0090]** In an embodiment, $R_1$ is selected from the group consisting of: hydrogen, substituted or unsubstituted $C_1$-$C_3$ alkyl, substituted or unsubstituted $C_3$ cycloalkyl and substituted or unsubstituted $C_3$ cycloalkyl $C_1$ alkyl; wherein each substituent is halo.

**[0091]** In an embodiment, $R_1$ is selected from the group consisting of: hydrogen, substituted or unsubstituted $C_1$-$C_4$ alkyl and substituted or unsubstituted $C_3$-$C_4$ cycloalkyl $C_1$-$C_2$ alkyl; wherein each substituent is halo.

**[0092]** In an embodiment, $R_1$ is selected from the group consisting of: hydrogen, substituted or unsubstituted $C_1$-$C_4$ alkyl and substituted or unsubstituted $C_3$ cycloalkyl $C_1$ alkyl; wherein each substituent is halo.

**[0093]** In an embodiment, $R_1$ is selected from the group consisting of: hydrogen and substituted or unsubstituted $C_1$-$C_3$ alkyl; wherein each substituent is halo.

**[0094]** In an embodiment, $R_1$ is selected from the group consisting of: hydrogen and substituted or unsubstituted $C_1$-$C_2$ alkyl; wherein each substituent is halo.

**[0095]** In an embodiment, $R_1$ is substituted or unsubstituted $C_3$-$C_4$ cycloalkyl $C_1$-$C_2$ alkyl; wherein each substituent is halo.

**[0096]** In an embodiment, $R_1$ is selected from the group consisting of hydrogen, $C_1$-$C_4$ alkyl and $C_3$ cycloalkyl $C_1$ alkyl.

**[0097]** In an embodiment, $R_1$ is selected from the group consisting of hydrogen, $C_1$-$C_3$ alkyl and $C_3$ cycloalkyl $C_1$ alkyl.

**[0098]** In an embodiment, $R_1$ is selected from the group consisting of hydrogen, $C_1$-$C_2$ alkyl and $C_3$ cycloalkyl $C_1$ alkyl.

**[0099]** In an embodiment, $R_1$ is selected from the group consisting of hydrogen, $C_1$ alkyl and $C_3$ cycloalkyl $C_1$ alkyl.

**[0100]** In an embodiment, $R_1$ is selected from the group consisting of hydrogen and $C_1$-$C_4$ alkyl.

**[0101]** In an embodiment, $R_1$ is selected from the group consisting of hydrogen and $C_1$-$C_3$ alkyl.

**[0102]** In an embodiment, $R_1$ is selected from the group consisting of hydrogen and $C_1$-$C_2$ alkyl.

**[0103]** In an embodiment, $R_1$ is hydrogen.

**[0104]** In an embodiment, $R_1$ is $C_1$ alkyl.

**[0105]** In an embodiment, $R_1$ is $C_2$ alkyl.

**[0106]** In an embodiment, $R_1$ is $C_3$ alkyl, e.g. n-propyl or i-propyl.

**[0107]** In an embodiment, $R_1$ is $C_4$ alkyl, e.g. n-butyl, s-butyl, i-butyl or t-butyl.

**[0108]** In an embodiment, $R_1$ is substituted or unsubstituted $C_3$ cycloalkyl $C_1$ alkyl.

**[0109]** In an embodiment, $R_1$ is selected from the group consisting of:

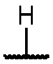 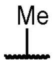 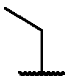 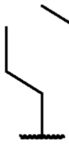 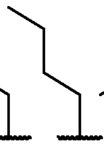 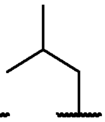 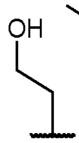 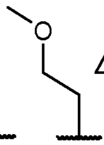 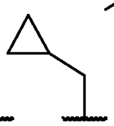 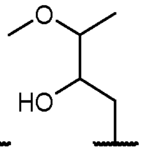 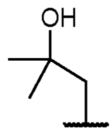.

**[0110]** In an embodiment, $R_1$ is a $C_1$ alkylenyl residue, and one $R_4$ moiety is a $C_1$ alkylenyl residue, wherein the $C_1$ alkylenyl $R_4$ moeity is bonded to a carbon atom in Ring A that is ortho to the carbon atom of Ring A having the amide residue, and, together with the atoms to which they are bonded, the $R_1$ and $R_4$ moieties form a 5-membered ring.

**[0111]** In an embodiment, $R_1$ is a $C_2$ alkylenyl residue, and one $R_4$ moiety is a $C_1$ alkylenyl residue, wherein the $C_1$ alkylenyl $R_4$ moeity is bonded to a carbon atom in Ring A that is ortho to the carbon atom of Ring A having the amide residue, and, together with the atoms to which they are bonded, the $R_1$ and $R_4$ moieties form a 6-membered ring.

**[0112]** For the avoidance of doubt, the following sub-structure of formula I

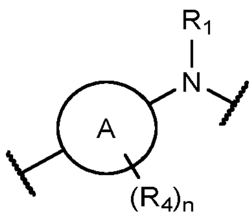,

has the structure

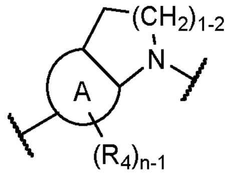.

**[0113]** In an embodiment, $R_2$ is selected from the group consisting of: substituted or unsubstituted $C_1$-$C_6$ alkyl, substituted or unsubstituted $C_2$-$C_6$ alkynyl; substituted or unsubstituted $C_3$-$C_6$ cycloalkyl; substituted or unsubstituted $C_1$-$C_6$ alkoxy, substituted or unsubstituted $C_3$-$C_6$ cycloalkoxy; substituted or unsubstituted $C_1$-$C_6$ alkoxy-$C_1$-$C_6$ alkyl; substituted or unsubstituted $C_3$-$C_6$ cycloalkoxy-$C_1$-$C_6$ alkyl; substituted or unsubstituted $C_1$-$C_6$ haloalkyl, substituted or unsubstituted $C_2$-$C_6$ haloalkynyl; substituted or unsubstituted $C_3$-$C_6$ cyclohaloalkyl; substituted or unsubstituted $C_1$-$C_6$ haloalkoxy, substituted or unsubstituted $C_3$-$C_6$ cyclohaloalkoxy; substituted or unsubstituted $C_1$-$C_6$ haloalkoxy-$C_1$-$C_6$ alkyl; substituted or unsubstituted $C_3$-$C_6$ cyclohaloalkoxy-$C_1$-$C_6$ alkyl; substituted or unsubstituted $C_1$-$C_6$ alkoxy-$C_1$-$C_6$ haloalkyl; substituted or unsubstituted $C_3$-$C_6$ cycloalkoxy-$C_1$-$C_6$ haloalkyl; substituted or unsubstituted $C_1$-$C_6$ haloalkoxy-$C_1$-$C_6$ haloalkyl; substituted or unsubstituted $C_3$-$C_6$ cyclohaloalkoxy-$C_1$-$C_6$ haloalkyl; $SO_2R_d$; and $-NR_eR_e$.

**[0114]** In an embodiment, $R_2$ is selected from the group consisting of: substituted or unsubstituted $C_1$-$C_6$ alkyl; substituted or unsubstituted $C_3$-$C_6$ cycloalkyl; substituted or unsubstituted $C_1$-$C_6$ alkoxy, substituted or unsubstituted $C_3$-$C_6$ cycloalkoxy; substituted or unsubstituted $C_1$-$C_6$ alkoxy-$C_1$-$C_6$ alkyl; substituted or unsubstituted $C_3$-$C_6$ cycloalkoxy-$C_1$-$C_6$ alkyl; substituted or unsubstituted $C_1$-$C_6$ haloalkyl; substituted or unsubstituted $C_3$-$C_6$ cyclohaloalkyl; substituted or

unsubstituted $C_1$-$C_6$ haloalkoxy, substituted or unsubstituted $C_3$-$C_6$ cyclohaloalkoxy; substituted or unsubstituted $C_1$-$C_6$ haloalkoxy-$C_1$-$C_6$ alkyl; substituted or unsubstituted $C_3$-$C_6$ cyclohaloalkoxy-$C_1$-$C_6$ alkyl; substituted or unsubstituted $C_1$-$C_6$ alkoxy-$C_1$-$C_6$ haloalkyl; substituted or unsubstituted $C_3$-$C_6$ cycloalkoxy-$C_1$-$C_6$ haloalkyl; substituted or unsubstituted $C_1$-$C_6$ haloalkoxy-$C_1$-$C_6$ haloalkyl; substituted or unsubstituted $C_3$-$C_6$ cyclohaloalkoxy-$C_1$-$C_6$ haloalkyl; $SO_2R_d$; and -$NR_eR_e$.

**[0115]** In an embodiment, $R_2$ is selected from the group consisting of: substituted or unsubstituted $C_1$-$C_6$ alkyl; substituted or unsubstituted $C_3$-$C_6$ cycloalkyl; substituted or unsubstituted $C_1$-$C_6$ alkoxy, substituted or unsubstituted $C_3$-$C_6$ cycloalkoxy; substituted or unsubstituted $C_1$-$C_6$ alkoxy-$C_1$-$C_6$ alkyl; substituted or unsubstituted $C_3$-$C_6$ cycloalkoxy-$C_1$-$C_6$ alkyl; substituted or unsubstituted $C_1$-$C_6$ haloalkyl; substituted or unsubstituted $C_3$-$C_6$ cyclohaloalkyl; substituted or unsubstituted $C_1$-$C_6$ haloalkoxy; substituted or unsubstituted $C_3$-$C_6$ cyclohaloalkoxy; substituted or unsubstituted $C_1$-$C_6$ haloalkoxy-$C_1$-$C_6$ alkyl; substituted or unsubstituted $C_3$-$C_6$ cyclohaloalkoxy-$C_1$-$C_6$ alkyl; $SO_2R_d$; and -$NR_eR_e$.

**[0116]** In an embodiment, $R_2$ is selected from the group consisting of: substituted or unsubstituted $C_1$-$C_6$ alkyl; substituted or unsubstituted $C_3$-$C_6$ cycloalkyl; substituted or unsubstituted $C_1$-$C_6$ alkoxy, substituted or unsubstituted $C_3$-$C_6$ cycloalkoxy; substituted or unsubstituted $C_1$-$C_6$ alkoxy-$C_1$-$C_6$ alkyl; substituted or unsubstituted $C_3$-$C_6$ cycloalkoxy-$C_1$-$C_6$ alkyl; substituted or unsubstituted $C_1$-$C_6$ haloalkyl; substituted or unsubstituted $C_3$-$C_6$ cyclohaloalkyl; substituted or unsubstituted $C_1$-$C_6$ haloalkoxy; substituted or unsubstituted $C_3$-$C_6$ cyclohaloalkoxy; $SO_2R_d$; and -$NR_eR_e$.

**[0117]** In an embodiment, $R_2$ is selected from the group consisting of: substituted or unsubstituted $C_1$-$C_6$ alkyl; substituted or unsubstituted $C_3$-$C_6$ cycloalkyl; substituted or unsubstituted $C_1$-$C_6$ alkoxy, substituted or unsubstituted $C_3$-$C_6$ cycloalkoxy; substituted or unsubstituted $C_1$-$C_6$ alkoxy-$C_1$-$C_6$ alkyl; substituted or unsubstituted $C_1$-$C_6$ haloalkyl; substituted or unsubstituted $C_1$-$C_6$ haloalkoxy; $SO_2R_d$; and - $NR_eR_e$.

**[0118]** In an embodiment, $R_2$ is selected from the group consisting of: substituted or unsubstituted $C_1$-$C_6$ alkyl; substituted or unsubstituted $C_1$-$C_6$ alkoxy, substituted or unsubstituted $C_3$-$C_6$ cycloalkoxy; substituted or unsubstituted $C_1$-$C_6$ alkoxy-$C_1$-$C_6$ alkyl; substituted or unsubstituted $C_1$-$C_6$ haloalkyl; substituted or unsubstituted $C_1$-$C_6$ haloalkoxy; $SO_2R_d$; and -$NR_eR_e$.

**[0119]** In an embodiment, $R_2$ is selected from the group consisting of: substituted or unsubstituted $C_1$-$C_4$ alkyl, substituted or unsubstituted $C_2$-$C_4$ alkynyl; substituted or unsubstituted $C_3$-$C_4$ cycloalkyl substituted or unsubstituted $C_1$-$C_4$ alkoxy; substituted or unsubstituted $C_3$-$C_4$ cycloalkoxy; substituted or unsubstituted $C_1$-$C_4$ alkoxy-$C_1$-$C_4$ alkyl; substituted or unsubstituted $C_3$-$C_4$ cycloalkoxy-$C_1$-$C_4$ alkyl; substituted or unsubstituted $C_1$-$C_4$ haloalkyl; substituted or unsubstituted $C_2$-$C_4$ haloalkynyl; substituted or unsubstituted $C_3$-$C_4$ cyclohaloalkyl; substituted or unsubstituted $C_1$-$C_4$ haloalkoxy; substituted or unsubstituted $C_3$-$C_4$ cyclohaloalkoxy; substituted or unsubstituted $C_1$-$C_4$ haloalkoxy-$C_1$-$C_4$ alkyl; substituted or unsubstituted $C_3$-$C_4$ cyclohaloalkoxy-$C_1$-$C_4$ alkyl; substituted or unsubstituted $C_1$-$C_4$ alkoxy-$C_1$-$C_4$ haloalkyl; substituted or unsubstituted $C_3$-$C_4$ cycloalkoxy-$C_1$-$C_4$ haloalkyl; substituted or unsubstituted $C_1$-$C_4$ haloalkoxy-$C_1$-$C_4$ haloalkyl; substituted or unsubstituted $C_3$-$C_4$ cyclohaloalkoxy-$C_1$-$C_4$ haloalkyl; $SO_2R_d$; and -$NR_eR_e$.

**[0120]** In an embodiment, $R_2$ is selected from the group consisting of: substituted or unsubstituted $C_1$-$C_4$ alkyl; substituted or unsubstituted $C_3$-$C_4$ cycloalkyl; substituted or unsubstituted $C_1$-$C_4$ alkoxy; substituted or unsubstituted $C_3$-$C_4$ cycloalkoxy; substituted or unsubstituted $C_1$-$C_4$ alkoxy-$C_1$-$C_4$ alkyl; substituted or unsubstituted $C_3$-$C_4$ cycloalkoxy-$C_1$-$C_4$ alkyl; substituted or unsubstituted $C_1$-$C_4$ haloalkyl; substituted or unsubstituted $C_3$-$C_4$ cyclohaloalkyl; substituted or unsubstituted $C_1$-$C_4$ haloalkoxy; substituted or unsubstituted $C_3$-$C_4$ cyclohaloalkoxy; substituted or unsubstituted $C_1$-$C_4$ haloalkoxy-$C_1$-$C_4$ alkyl; substituted or unsubstituted $C_3$-$C_4$ cyclohaloalkoxy-$C_1$-$C_4$ alkyl; substituted or unsubstituted $C_1$-$C_4$ alkoxy-$C_1$-$C_4$ haloalkyl; substituted or unsubstituted $C_3$-$C_4$ cycloalkoxy-$C_1$-$C_4$ haloalkyl; substituted or unsubstituted $C_1$-$C_4$ haloalkoxy-$C_1$-$C_4$ haloalkyl; substituted or unsubstituted $C_3$-$C_4$ cyclohaloalkoxy-$C_1$-$C_4$ haloalkyl; $SO_2R_d$; and -$NR_eR_e$.

**[0121]** In an embodiment, $R_2$ is selected from the group consisting of: substituted or unsubstituted $C_1$-$C_4$ alkyl; substituted or unsubstituted $C_3$-$C_4$ cycloalkyl; substituted or unsubstituted $C_1$-$C_4$ alkoxy; substituted or unsubstituted $C_3$-$C_4$ cycloalkoxy; substituted or unsubstituted $C_1$-$C_4$ alkoxy-$C_1$-$C_4$ alkyl; substituted or unsubstituted $C_3$-$C_4$ cycloalkoxy-$C_1$-$C_4$ alkyl; substituted or unsubstituted $C_1$-$C_4$ haloalkyl; substituted or unsubstituted $C_3$-$C_4$ cyclohaloalkyl; substituted or unsubstituted $C_1$-$C_4$ haloalkoxy; substituted or unsubstituted $C_3$-$C_4$ cyclohaloalkoxy; substituted or unsubstituted $C_1$-$C_4$ haloalkoxy-$C_1$-$C_4$ alkyl; substituted or unsubstituted $C_3$-$C_4$ cyclohaloalkoxy-$C_1$-$C_4$ alkyl; $SO_2R_d$; and -$NR_eR_e$.

**[0122]** In an embodiment, $R_2$ is selected from the group consisting of: substituted or unsubstituted $C_1$-$C_4$ alkyl; substituted or unsubstituted $C_3$-$C_4$ cycloalkyl; substituted or unsubstituted $C_1$-$C_4$ alkoxy; substituted or unsubstituted $C_3$-$C_4$ cycloalkoxy; substituted or unsubstituted $C_1$-$C_4$ alkoxy-$C_1$-$C_4$ alkyl; substituted or unsubstituted $C_3$-$C_4$ cycloalkoxy-$C_1$-$C_4$ alkyl; substituted or unsubstituted $C_1$-$C_4$ haloalkyl; substituted or unsubstituted $C_3$-$C_4$ cyclohaloalkyl; substituted or unsubstituted $C_1$-$C_4$ haloalkoxy; substituted or unsubstituted $C_3$-$C_4$ cyclohaloalkoxy; $SO_2R_d$; and -$NR_eR_e$.

**[0123]** In an embodiment, $R_2$ is selected from the group consisting of: substituted or unsubstituted $C_1$-$C_4$ alkyl; substituted or unsubstituted $C_3$-$C_4$ cycloalkyl; substituted or unsubstituted $C_1$-$C_4$ alkoxy, substituted or unsubstituted $C_3$-$C_4$ cycloalkoxy; substituted or unsubstituted $C_1$-$C_4$ alkoxy-$C_1$-$C_4$ alkyl; substituted or unsubstituted $C_1$-$C_4$ haloalkyl; substituted or unsubstituted $C_1$-$C_4$ haloalkoxy; $SO_2R_d$; and - $NR_eR_e$.

**[0124]** In an embodiment, $R_2$ is selected from the group consisting of: substituted or unsubstituted $C_1$-$C_4$ alkyl;

substituted or unsubstituted $C_1$-$C_4$ alkoxy, substituted or unsubstituted $C_3$-$C_4$ cycloalkoxy; substituted or unsubstituted $C_1$-$C_4$ alkoxy-$C_1$-$C_4$ alkyl; substituted or unsubstituted $C_1$-$C_4$ haloalkyl; substituted or unsubstituted $C_1$-$C_4$ haloalkoxy; $SO_2R_d$; and $-NR_eR_e$.

**[0125]** In an embodiment, $R_2$ is a substituted or unsubstituted $C_1$-$C_4$ alkoxy-$C_1$-$C_4$ alkyl. Optionally, $R_2$ is a substituted or unsubstituted $C_1$-$C_3$ alkoxy-$C_1$-$C_3$ alkyl. Further optionally, $R_2$ is a substituted or unsubstituted $C_1$-$C_2$ alkoxy-$C_1$-$C_2$ alkyl. Still further optionally, $R_2$ is a substituted or unsubstituted $C_1$ alkoxy-$C_1$ alkyl.

**[0126]** In an embodiment, $R_2$ is a substituted or unsubstituted $C_1$-$C_4$ alkyl. Optionally, $R_2$ is a substituted or unsubstituted $C_1$-$C_3$ alkyl. Further optionally, $R_2$ is a substituted or unsubstituted $C_1$-$C_2$ alkyl. Still further optionally, $R_2$ is a substituted or unsubstituted $C_1$ alkyl.

**[0127]** In an embodiment, $R_2$ is a substituted or unsubstituted $C_1$-$C_4$ haloalkyl. Optionally, $R_2$ is a substituted or unsubstituted $C_1$-$C_3$ haloalkyl. Further optionally, $R_2$ is a substituted or unsubstituted $C_1$-$C_2$ haloalkyl. Still further optionally, $R_2$ is a substituted or unsubstituted $C_1$ haloalkyl.

**[0128]** In an embodiment, $R_2$ is a substituted or unsubstituted $C_1$-$C_4$ alkoxy. Optionally, $R_2$ is a substituted or unsubstituted $C_1$-$C_3$ alkoxy. Further optionally, $R_2$ is a substituted or unsubstituted $C_1$-$C_2$ alkoxy. Still further optionally, $R_2$ is a substituted or unsubstituted $C_1$ alkoxy.

**[0129]** In an embodiment, $R_2$ is a substituted or unsubstituted $C_1$-$C_4$ haloalkoxy. Optionally, $R_2$ is a substituted or unsubstituted $C_1$-$C_3$ haloalkoxy. Further optionally, $R_2$ is a substituted or unsubstituted $C_1$-$C_2$ haloalkoxy. Still further optionally, $R_2$ is a substituted or unsubstituted $C_1$ haloalkoxy.

**[0130]** In an embodiment, $R_2$ is a substituted or unsubstituted $C_3$-$C_6$ cycloalkyl. Optionally, $R_2$ is a substituted or unsubstituted $C_3$-$C_4$ cycloalkyl. Further optionally, $R_2$ is a substituted or unsubstituted $C_3$ cycloalkyl.

**[0131]** In an embodiment, $R_2$ is a substituted or unsubstituted $C_3$-$C_6$ cycloalkoxy. Optionally, $R_2$ is a substituted or unsubstituted $C_3$-$C_4$ cycloalkoxy. Further optionally, $R_2$ is a substituted or unsubstituted $C_3$ cycloalkoxy.

**[0132]** In an embodiment, $R_2$ is $SO_2R_d$.

**[0133]** In an embodiment, $R_d$ is $C_{1-3}$ alkyl. In an embodiment, $R_d$ is $C_{1-2}$ alkyl. In an embodiment, $R_d$ is methyl.

**[0134]** In an embodiment, $R_2$ is $-NR_eR_e$.

**[0135]** In an embodiment, each $R_e$ is independently selected from the group consisting of H and $C_{1-3}$ alkyl. In an embodiment, each $R_e$ is independently selected from the group consisting of H and $C_{1-2}$ alkyl. In an embodiment, each $R_e$ is independently selected from the group consisting of H and methyl.

**[0136]** In an embodiment, the $R_2$ substituent is independently selected from the group consisting of - $OR_e$, =O, $-NR_eR_e$, and a substituted or unsubstituted monocyclic, bicyclic or bridged 3-8 membered cycloalkyl or heterocycloalkyl ring, wherein each $R_e$ is independently selected from the group consisting of H and $C_{1-4}$ alkyl.

**[0137]** In an embodiment, the substituent on $R_2$ is independently selected from the group consisting of =O, $-NR_eR_e$, and a substituted or unsubstituted monocyclic, bicyclic or bridged 3-8 membered cycloalkyl or heterocycloalkyl ring, wherein each $R_e$ is independently selected from the group consisting of H and $C_1$-$_4$ alkyl.

**[0138]** In an embodiment, the substituent on $R_2$ is independently selected from the group consisting of =O, $-NR_eR_e$, and a substituted or unsubstituted monocyclic 3-8 membered cycloalkyl or heterocycloalkyl ring, wherein each $R_e$ is independently selected from the group consisting of H and $C_{1-4}$ alkyl.

**[0139]** In an embodiment, the substituent on $R_2$ is independently selected from the group consisting of =O, $-NR_eR_e$, and a substituted or unsubstituted monocyclic 3-8 membered heterocycloalkyl ring, wherein each $R_e$ is independently selected from the group consisting of H and $C_{1-4}$ alkyl.

**[0140]** In an embodiment, the substituent on $R_2$ is independently selected from the group consisting of =O, $-NR_eR_e$, and a substituted or unsubstituted monocyclic 6 membered heterocycloalkyl ring, wherein each $R_e$ is independently selected from the group consisting of H and $C_{1-4}$ alkyl.

**[0141]** In an embodiment, the substituent on $R_2$ is independently selected from the group consisting of =O, $-NMe_2$, and a substituted or unsubstituted monocyclic 6 membered heterocycloalkyl ring.

**[0142]** In an embodiment, $R_2$ is selected from the group consisting of:

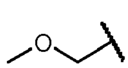 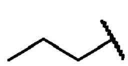 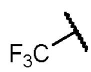 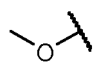 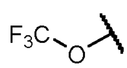

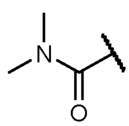 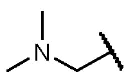 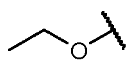 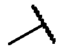 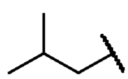

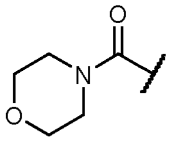 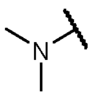 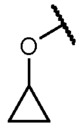 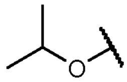 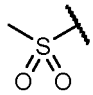

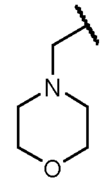 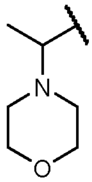 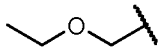 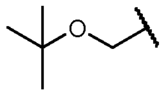 .

**[0143]** In an embodiment, m is 0 or 1.

**[0144]** In an embodiment, $R_2$ is a substituted or unsubstituted -O-$C_1$ alkylenyl residue, wherein the oxygen atom is connected to Ring B; and one $R_3$ moiety is an -O- residue, wherein together with the atoms to which they are bonded, the -O-$C_1$ alkylenyl and -O- residues form a 5-membered ring. Optionally, the substituent on the -O-$C_1$ alkylenyl residue, when present, is selected from -OH, nitrile, halo, methyl or methoxy.

**[0145]** In an embodiment, $R_2$, one $R_3$ moiety, and Ring B form the following substructure

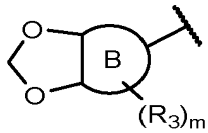 ,

wherein m is 0 or 1.

**[0146]** In an embodiment, $R_2$, one $R_3$ moiety, and Ring B form the following substructure

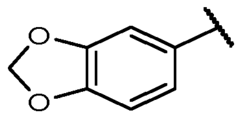 .

**[0147]** In an embodiment, $R_2$ is a substituted or unsubstituted -O-$C_2$ alkylenyl residue, wherein the oxygen atom is connected to Ring B; and one $R_3$ moiety is an -O- residue, wherein together with the atoms to which they are bonded, the -O-$C_2$ alkylenyl and -O- residues form a 6-membered ring. Optionally, the substituent on the -O-$C_2$ alkylenyl residue, when present, is selected from -OH, nitrile, halo, methyl or methoxy.

**[0148]** In an embodiment, $R_2$, one $R_3$ moiety, and Ring B form the following substructure

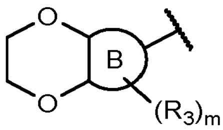 ,

wherein m is 0 or 1.

**[0149]** In an embodiment, $R_2$, one $R_3$ moiety, and Ring B form the following substructure

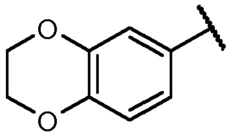 .

**[0150]** In an embodiment, each $R_3$ is independently selected from the group consisting of: halogen, $C_1$-$C_3$ alkyl, and $C_1$-$C_3$ alkoxy. In an embodiment, each $R_3$ is independently selected from the group consisting of: halogen, $C_1$-$C_2$ alkyl, and $C_1$-$C_2$ alkoxy.

**[0151]** In an embodiment, $R_3$ is methyl.

**[0152]** In an embodiment, $R_3$ is halo, preferably fluoro.

**[0153]** In an embodiment, m is 0.

**[0154]** In an embodiment, n is 0 or 1.

**[0155]** In an embodiment, each $R_4$ is independently selected from the group consisting of: halogen, $C_1$-$C_3$ alkyl, and $C_1$-$C_3$ alkoxy. In an embodiment, each $R_4$ is independently selected from the group consisting of: halogen, $C_1$-$C_2$ alkyl, and $C_1$-$C_2$ alkoxy.

**[0156]** In an embodiment, $R_4$ is methyl.

**[0157]** In an embodiment, n is 0.

**[0158]** In an embodiment, p is 0. In an embodiment, p is 1. In an embodiment, p is 2.

**[0159]** In an embodiment, each $R_5$ is independently selected from the group consisting of: halogen, $C_1$-$C_3$ alkyl, $C_1$-$C_3$ haloalkyl, $C_1$-$C_3$ alkoxy or -CN.

**[0160]** In an embodiment, each $R_5$ is independently selected from the group consisting of: halogen, $C_1$-$C_2$ alkyl, $C_1$-$C_2$ haloalkyl, $C_1$-$C_2$ alkoxy or -CN.

**[0161]** In an embodiment, each $R_5$ is independently selected from the group consisting of: halogen, $C_1$-$C_2$ alkyl, $C_1$ haloalkyl, $C_1$-$C_2$ alkoxy or -CN.

**[0162]** In an embodiment, $R_5$ is $C_1$-$C_3$ alkyl, e.g. methyl, ethyl or propyl.

**[0163]** In an embodiment, $R_5$ is halogen, e.g fluoro.

**[0164]** In an embodiment, the compound of Formula (I) is selected from:

| | | | |
|---|---|---|---|
| (1) | 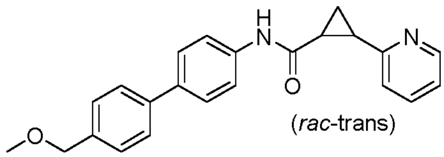 (*rac*-trans) | (2) | 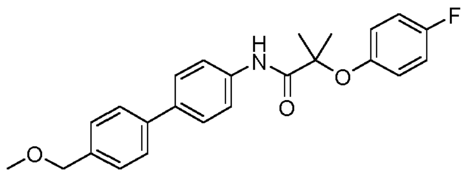 |
| (3) | 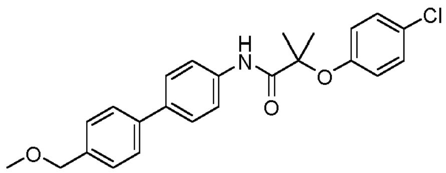 | (4) | 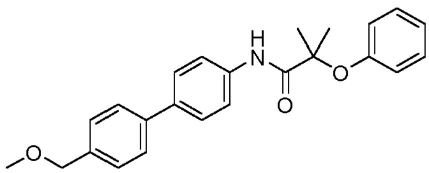 |
| (5) | 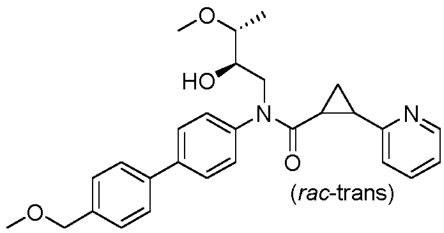 (*rac*-trans) | (6) | 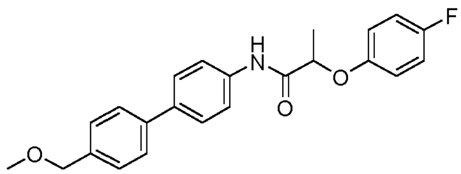 |
| (7) | 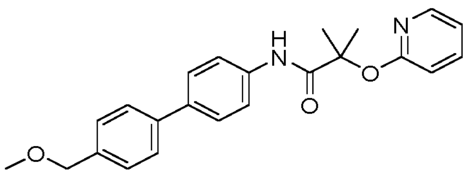 | (8) | 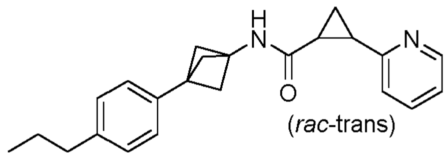 (*rac*-trans) |
| (9) | 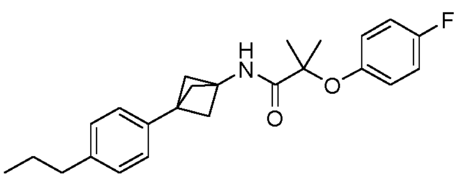 | (10) | 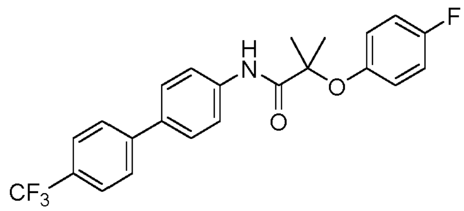 |

(continued)

| | | | |
|---|---|---|---|
| (11) | 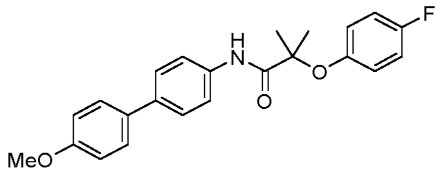 | (12) | 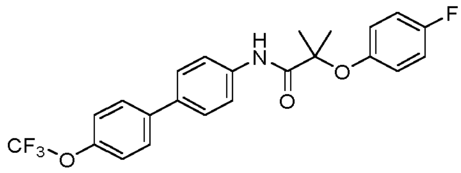 |
| (13) | 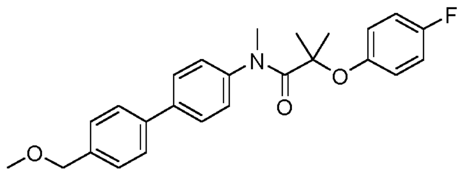 | (14) | 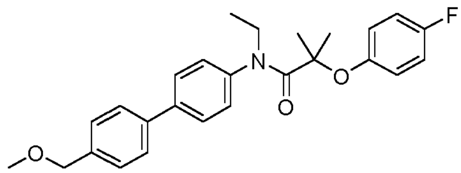 |
| (15) | 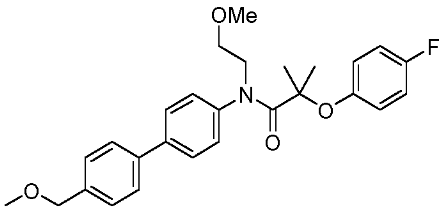 | (16) | 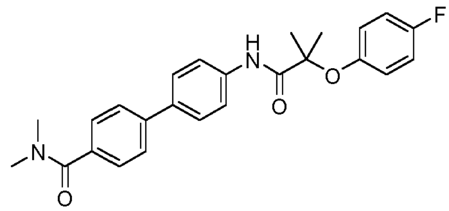 |
| (17) | 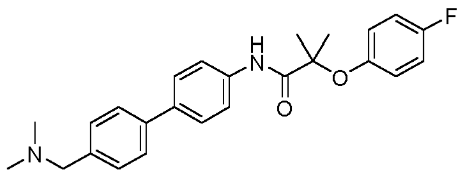 | (18) D1 | 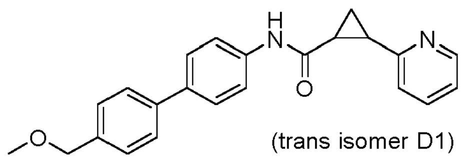 (trans isomer D1) |
| (19) D2 | 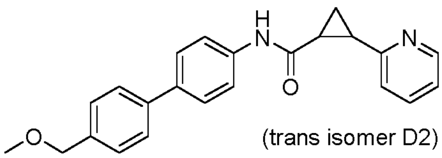 (trans isomer D2) | (20) | 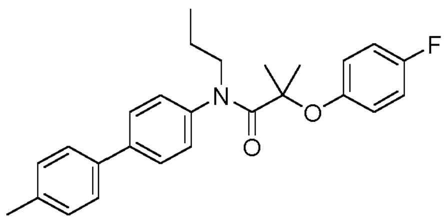 |
| (21) | 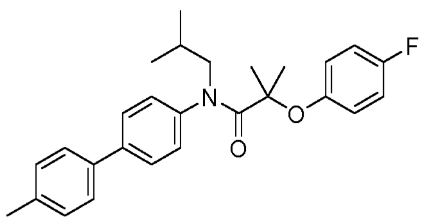 | (22) | 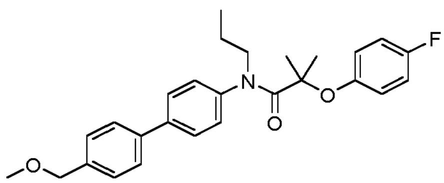 |
| (23) | 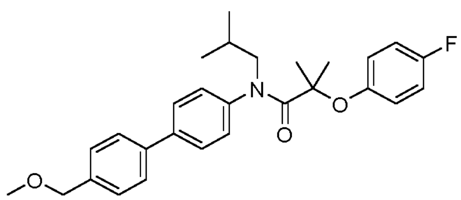 | (24) | 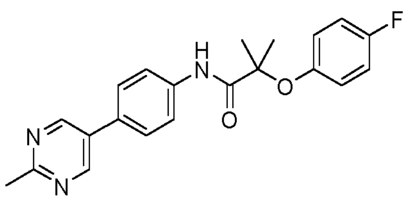 |
| (25) | 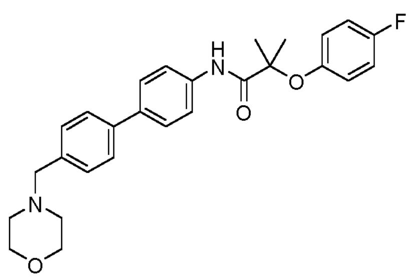 | (26) | 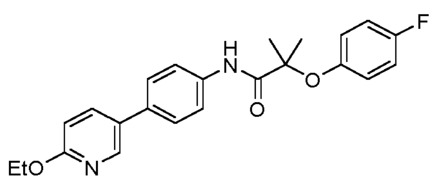 |

(continued)

| | | | |
|---|---|---|---|
| (27) | 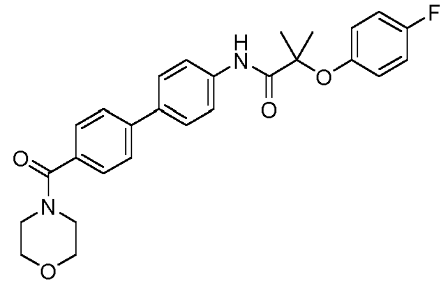 | (28) | 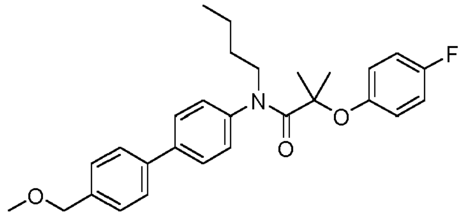 |
| (29) | 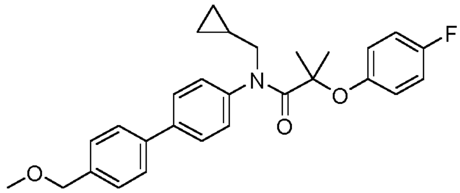 | (30) | 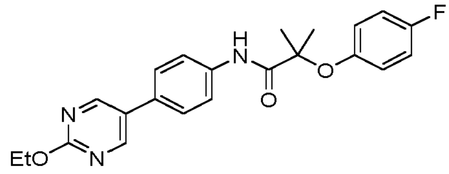 |
| (31) | 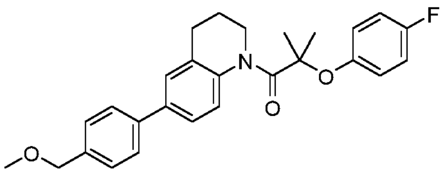 | (32) | 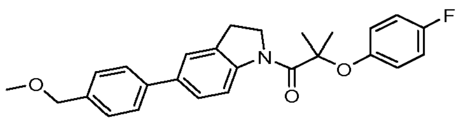 |
| (33) | 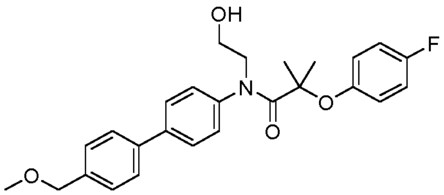 | (34) D1 | 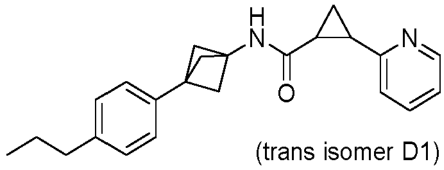 (trans isomer D1) |
| (35) D2 | 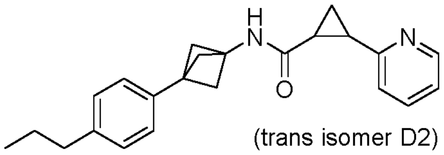 (trans isomer D2) | (36) | 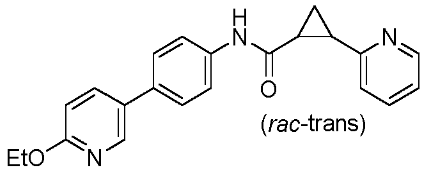 (*rac*-trans) |
| (37) | 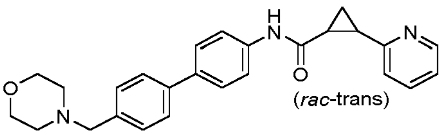 (*rac*-trans) | (38) | 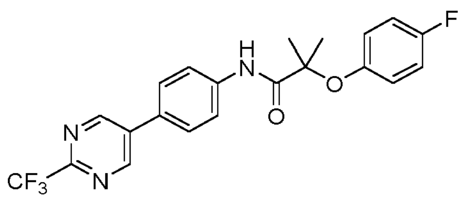 |
| (39) | 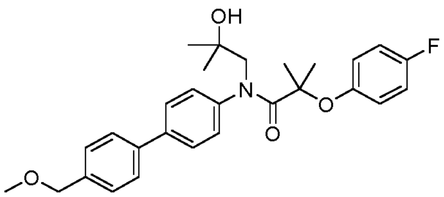 | (40) | 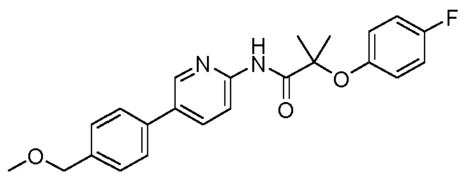 |
| (41) | 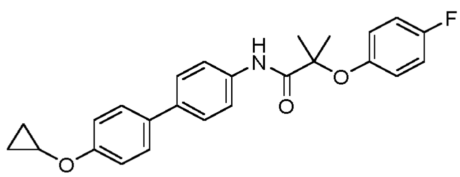 | (42) | 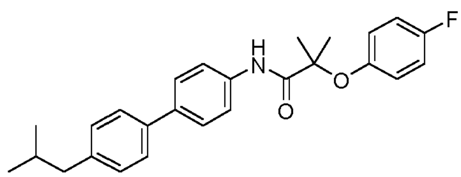 |

(continued)

| | | | |
|---|---|---|---|
| (43) | 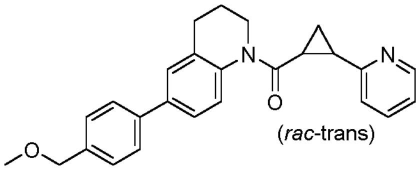 (*rac*-trans) | (44) | 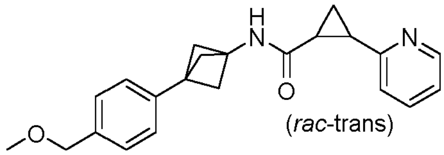 (*rac*-trans) |
| (45) | 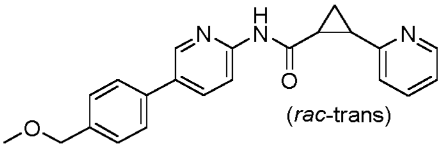 (*rac*-trans) | (46) | 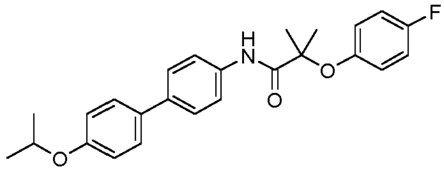 |
| (47) | 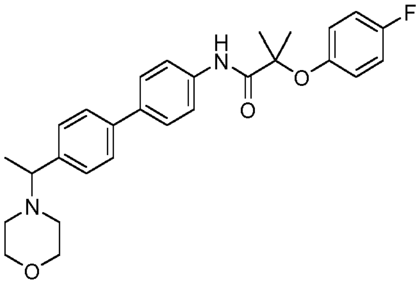 | (48) | 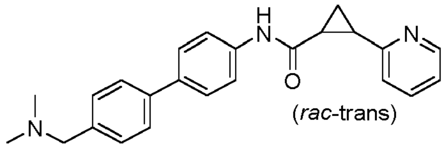 (*rac*-trans) |
| (49) | 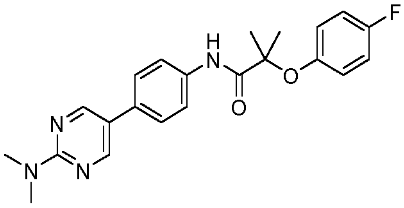 | (50) | 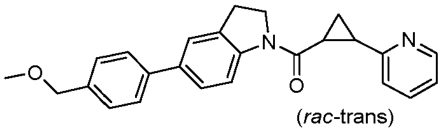 (*rac*-trans) |
| (51) | 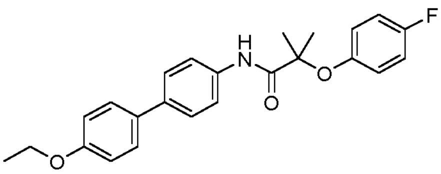 | (52) | 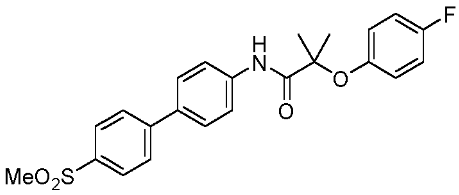 |
| (53) | 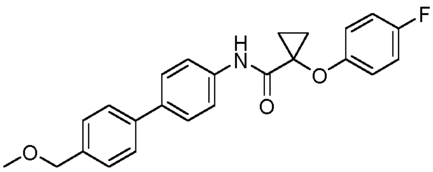 | (54) | 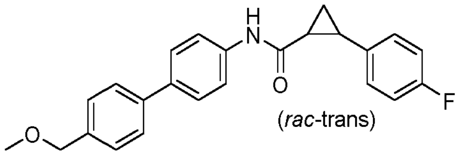 (*rac*-trans) |
| (55) | 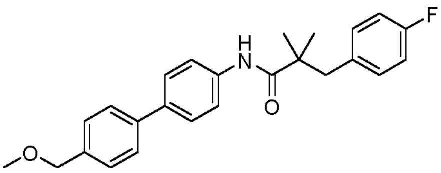 | (56) | 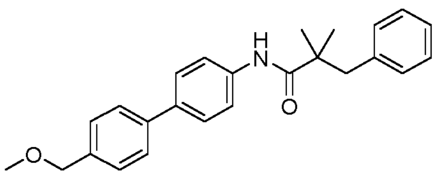 |
| (57) | 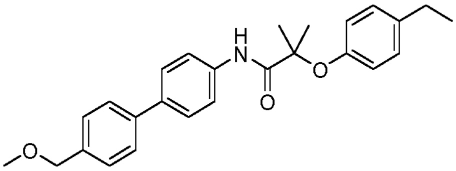 | (58) | 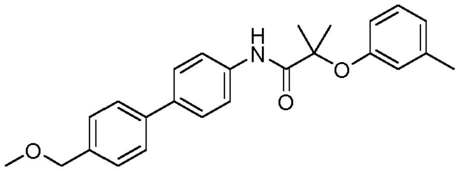 |

(continued)

| | | | |
|---|---|---|---|
| (59) | 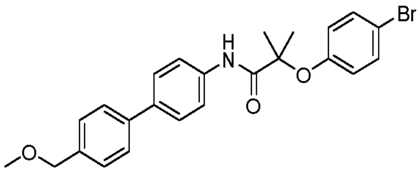 | (60) | 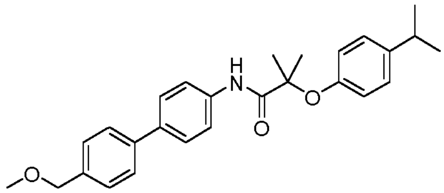 |
| (61) | 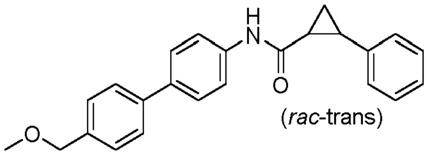 (*rac*-trans) | (62) | 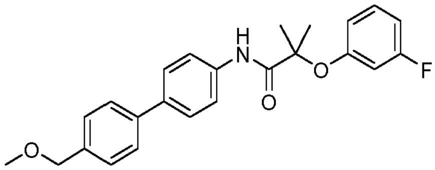 |
| (63) | 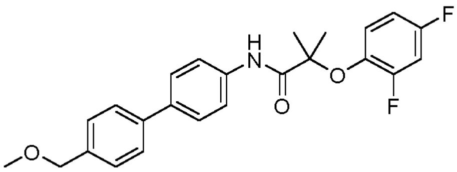 | (64) | 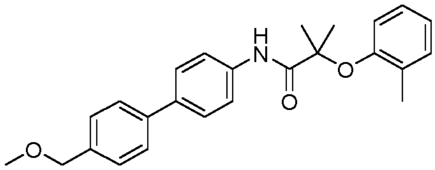 |
| (65) | 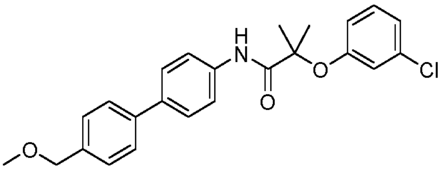 | (66) | 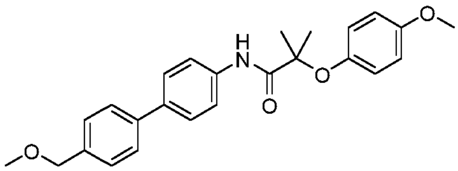 |
| (67) | 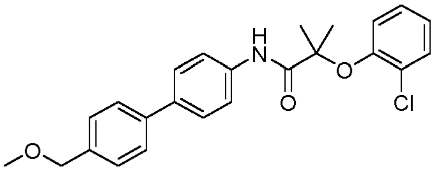 | (68) | 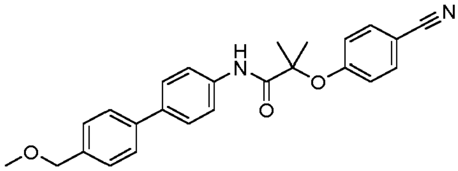 |
| (69) | 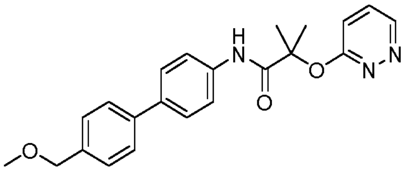 | (70) | 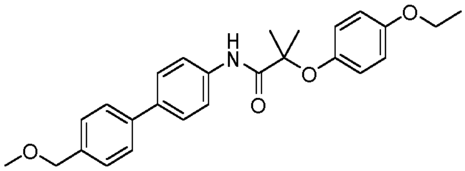 |
| (71) | 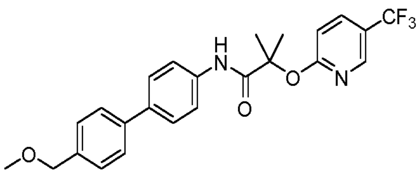 | (72) | 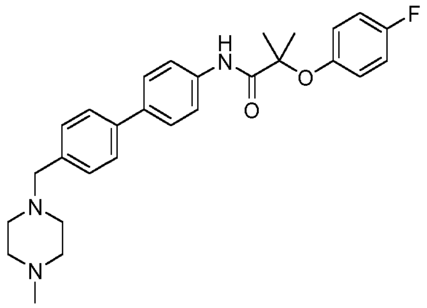 |
| (73)<br>D1 | 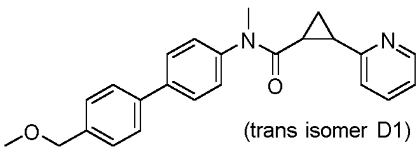 (trans isomer D1) | (74)<br>D2 | 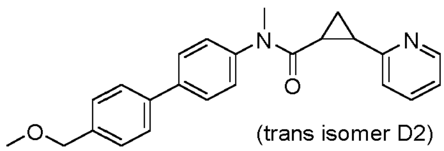 (trans isomer D2) |

(continued)

| | | | |
|---|---|---|---|
| (75)<br>D1 | 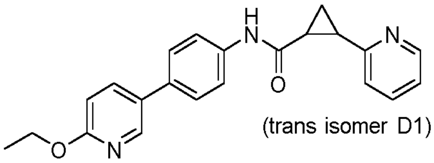<br>(trans isomer D1) | (76)<br>D2 | 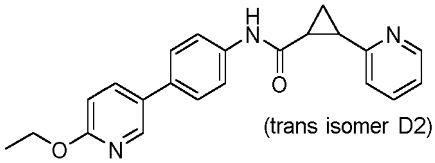<br>(trans isomer D2) |
| (77)<br>D1 | 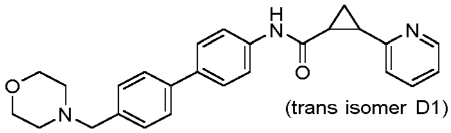<br>(trans isomer D1) | (78)<br>D2 | 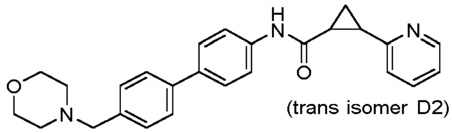<br>(trans isomer D2) |
| (79) | 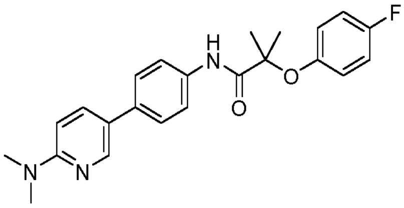 | (80) | 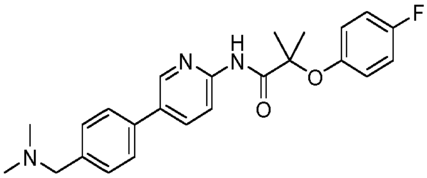 |
| (81) | 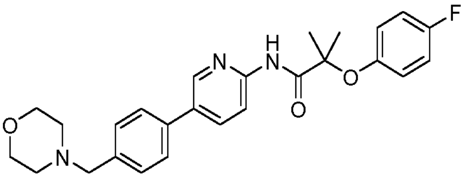 | (82) | 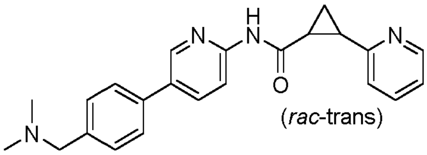<br>(*rac*-trans) |
| (83) | 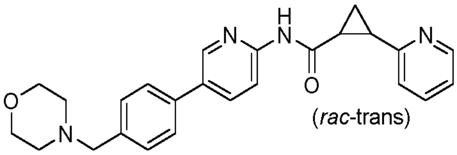<br>(*rac*-trans) | (84) | 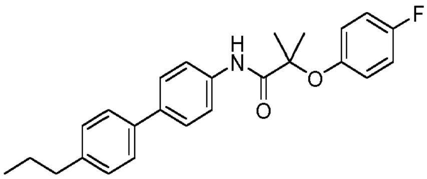 |
| (85) | 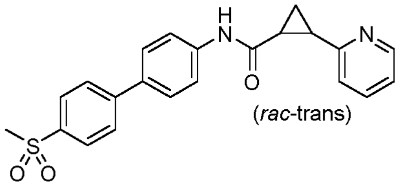<br>(*rac*-trans) | (86) | 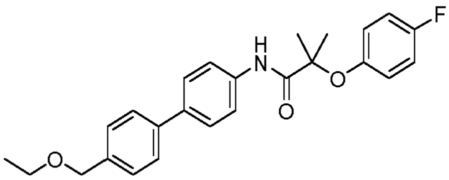 |
| (87) | 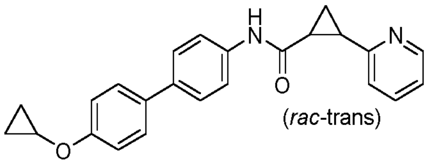<br>(*rac*-trans) | (88) | 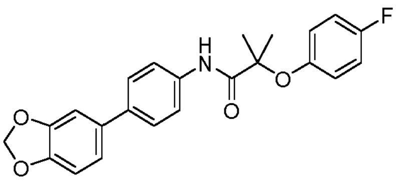 |
| (89) | 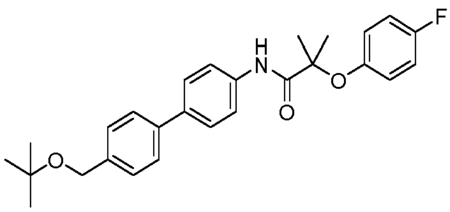 | (90) | 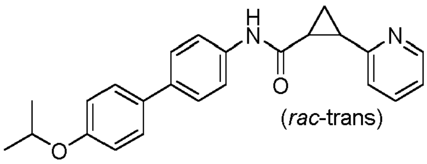<br>(*rac*-trans) |

(continued)

| | | | |
|---|---|---|---|
| (91) | 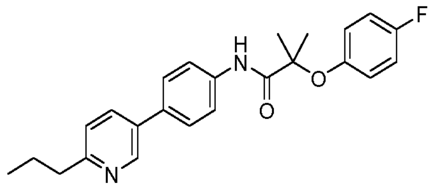 | (92) | 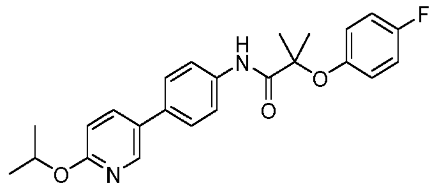 |
| (93) | 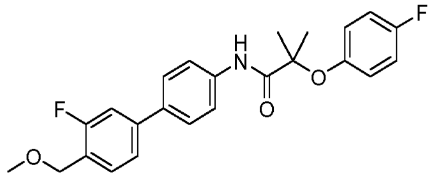 | | |

or a pharmaceutically acceptable salt thereof.

**[0165]** Also provided is a compound selected from the compounds recited in the examples below or a pharmaceutically acceptable salt thereof.

## Definitions

**[0166]** Unless otherwise stated, the following terms used in the specification and claims have the meanings set out below.

**[0167]** It is to be appreciated that references to "treating" or "treatment" include prophylaxis as well as the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, i.e., causing regression of the state, disorder or condition or at least one of its clinical or subclinical symptoms.

**[0168]** A "therapeutically effective amount" includes the amount of a compound that, when administered to a mammal for treating a disease, is sufficient to affect such treatment for the disease. The "therapeutically effective amount" will vary depending on the compound, the disease and its severity and the age, weight, etc., of the mammal to be treated.

**[0169]** The term "halo" or "halogen" includes to one of the halogens, group 17 of the periodic table. In particular the term includes fluorine, chlorine, bromine and iodine.

**[0170]** The term "$C_1$-$C_6$ alkyl" includes a linear or branched hydrocarbon chain containing 1, 2, 3, 4, 5 or 6 carbon atoms, for example methyl, ethyl, n-propyl, iso-propyl, n-butyl, sec-butyl, tert-butyl, n-pentyl and n-hexyl. The term "$C_1$-$C_4$ alkyl" includes such groups containing up to 4 carbon atoms. Alkylene groups include divalent alkyl groups and may likewise be linear or branched and have two points of attachment to the remainder of the molecule. Furthermore, an alkylene group may, for example, correspond to one of those alkyl groups listed in this paragraph. The alkyl and alkylene groups may be unsubstituted or substituted by one or more substituents. Possible substituents are described below. Substituents for the alkyl group may be halogen, e.g. fluorine, chlorine, bromine and iodine, OH, $C_1$-$C_4$ alkoxy. Other substituents for the alkyl group may alternatively be used.

**[0171]** The term "$C_1$-$C_6$ haloalkyl", e.g. "$C_1$-$C_4$ haloalkyl", includes a hydrocarbon chain substituted with at least one halogen atom independently chosen at each occurrence, for example, from fluorine, chlorine, bromine and iodine. The halogen atom may be present at any position on the hydrocarbon chain. For example, $C_1$-$C_6$ haloalkyl may refer to chloromethyl, fluoromethyl, trifluoromethyl, chloroethyl e.g. 1-chloromethyl and 2-chloroethyl, trichloroethyl e.g. 1,2,2-trichloroethyl, 2,2,2-trichloroethyl, fluoroethyl e.g. 1-fluoromethyl and 2-fluoroethyl, trifluoroethyl e.g. 1,2,2-trifluoroethyl and 2,2,2-trifluoroethyl, chloropropyl, trichloropropyl, fluoropropyl or trifluoropropyl.

**[0172]** The term "heteroalkyl", includes an alkyl group in which the hydrocarbon chain has at least one heteroatom selected from nitrogen, oxygen and/or sulfur atom interrupting the hydrocarbon chain. The heteroatom may be present at any position in the hydrocarbon chain. For example, $C_1$-$C_6$ heteroalkyl may refer to an ether, thioether or amine compound such as $CH_3CH_2OCH_2CH_3$, $CH_3NHCH_2CH_3$ or $CH_3SCH_3$. A heteroalkylene group includes divalent heteroalkyl group having two points of attachment to the remainder of the molecule. The groups $-CH_2CH_2OCH_2CH_2-$, $-CH_2NHCH_2CH_2-$ or $-CH_2SCH_2-$ are examples of heteroalkylene groups. The heteroalkyl and heteroalkylene groups may be unsubstituted or substituted by one or more substituents. Possible substituents are described below. Substituents for the alkyl group may

be halogen, e.g. fluorine, chlorine, bromine and iodine, OH, $C_1$-$C_4$ alkoxy. Other substituents for the heteroalkyl group may alternatively be used.

**[0173]** The term "$C_2$-$C_6$ alkenyl" includes a branched or linear hydrocarbon chain containing at least one double bond and having 2, 3, 4, 5 or 6 carbon atoms. The double bond(s) may be present as the E or Z isomer. The double bond may be at any possible position of the hydrocarbon chain. For example, the "$C_{2-6}$ alkenyl" may be ethenyl, propenyl, butenyl, butadienyl, pentenyl, pentadienyl, hexenyl and hexadienyl.

**[0174]** The term "$C_2$-$C_6$ alkynyl" includes a branched or linear hydrocarbon chain containing at least one triple bond and having 2, 3, 4, 5 or 6 carbon atoms. The triple bond may be at any possible position of the hydrocarbon chain. For example, the "$C_2$-$C_6$ alkynyl" may be ethynyl, propynyl, butynyl, pentynyl and hexynyl.

**[0175]** The term "$C_3$-$C_6$ cycloalkyl" includes a saturated hydrocarbon ring system containing 3, 4, 5 or 6 carbon atoms. For example, the "$C_3$-$C_6$ cycloalkyl" may be cyclopropyl, cyclobutyl, cyclopentyl, or cyclohexyl.

**[0176]** The term "5-10 membered cycloalkyl" includes a saturated hydrocarbon ring system containing 5, 6, 7, 8, 9, or 10 carbon atoms. The term "5-10 membered cycloalkyl" includes bicyclic saturated hydrocarbon ring systems, for example bicyclo-[1.1.1]-pentyl, bicyclo-[2.2.2]-octyl, bicyclo[2.1.1]hexyl or a residue of pentacyclo[4.2.0.$0^{2,5}$.$0^{3,8}$.$0^{4,7}$]octyl (namely a cubane).

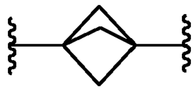
bicyclo[1.1.1]pentyl

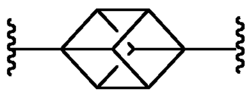
pentacyclo[4.2.0.$0^{2,5}$.$0^{3,8}$.$0^{4,7}$]octyl "cubane"

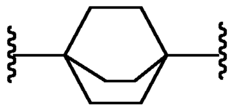
bicyclo[2.2.2]octyl

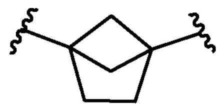
bicyclo[2.1.1]hexyl

**[0177]** The term "heterocyclyl", "heterocyclic" or "heterocycle" includes a non-aromatic saturated or partially saturated monocyclic or fused, bridged, or spiro bicyclic heterocyclic ring system(s). Monocyclic heterocyclic rings may contain from about 3 to 12 (suitably from 3 to 7) ring atoms, with from 1 to 5 (suitably 1, 2 or 3) heteroatoms selected from nitrogen, oxygen or sulfur in the ring. Bicyclic heterocycles may contain from 7 to 17 member atoms, suitably 7 to 12 member atoms, in the ring. Bicyclic heterocyclic(s) rings may be fused, spiro, or bridged ring systems. Examples of heterocyclic groups include cyclic ethers such as oxiranyl, oxetanyl, tetrahydrofuranyl, dioxanyl, and substituted cyclic ethers. Heterocycles comprising at least one nitrogen in a ring position include, for example, azetidinyl, pyrrolidinyl, piperidinyl, piperazinyl, morpholinyl, thiomorpholinyl, tetrahydrotriazinyl, tetrahydropyrazolyl, tetrahydropyridinyl, homopiperidinyl, homopiperazinyl, 3,8-diaza-bicyclo[3.2.1]octanyl, 8-aza-bicyclo[3.2.1]octanyl, 2,5-Diazabicyclo[2.2.1]heptanyl and the like. Typical sulfur containing heterocycles include tetrahydrothienyl, dihydro-1,3-dithiol, tetrahydro-2H-thiopyran, and hexahydrothiepine. Other heterocycles include dihydro oxathiolyl, tetrahydro oxazolyl, tetrahydro-oxadiazolyl, tetrahydrodioxazolyl, tetrahydrooxathiazolyl, hexahydrotriazinyl, tetrahydro oxazinyl, tetrahydropyrimidinyl, dioxolinyl, octahydrobenzofuranyl, octahydrobenzimidazolyl, and octahydrobenzothiazolyl. For heterocycles containing sulfur, the oxidized sulfur heterocycles containing SO or $SO_2$ groups are also included. Examples include the sulfoxide and sulfone forms of tetrahydrothienyl and thiomorpholinyl such as tetrahydrothiene 1,1-dioxide and thiomorpholinyl 1,1-dioxide. A suitable value for a heterocyclyl group which bears 1 or 2 oxo (=O), for example, 2 oxopyrrolidinyl, 2-oxoimidazolidinyl, 2-oxopiperidinyl, 2,5-dioxopyrrolidinyl, 2,5-dioxoimidazolidinyl or 2,6-dioxopiperidinyl. Particular heterocyclyl groups are saturated monocyclic 3 to 7 membered heterocyclyls containing 1, 2 or 3 heteroatoms selected from nitrogen, oxygen or sulfur, for example azetidinyl, tetrahydrofuranyl, tetrahydropyranyl, pyrrolidinyl, morpholinyl, tetrahydrothienyl, tetrahydrothienyl 1,1-dioxide, thiomorpholinyl, thiomorpholinyl 1,1-dioxide, piperidinyl, homopiperidinyl, piperazinyl or homopiperazinyl. As the skilled person would appreciate, any heterocycle may be linked to another group via any suitable atom, such as via a carbon or nitrogen atom. For example, the term "piperidino" or "morpholino" refers to a piperidin-1-yl or morpholin-4-yl ring that is linked via the ring nitrogen.

**[0178]** The term "bridged ring systems" includes ring systems in which two rings share more than two atoms, see for example Advanced Organic Chemistry, by Jerry March, 4th Edition, Wiley Interscience, pages 131-133, 1992. Examples of bridged heterocyclyl ring systems include, aza-bicyclo[2.2.1]heptane, 2-oxa-5-azabicyclo[2.2.1]heptane, aza-bicyclo [2.2.2]octane, aza-bicyclo[3.2.1]octane, and quinuclidine.

**[0179]** The term "spiro bi-cyclic ring systems" includes ring systems in which two ring systems share one common spiro carbon atom, i.e. the heterocyclic ring is linked to a further carbocyclic or heterocyclic ring through a single common spiro carbon atom. Examples of spiro ring systems include 3,8-diazabicyclo[3.2.1]octane, 2,5-Diaza-bicyclo[2.2.1]heptane, 6-azaspiro[3.4]octane, 2-oxa-6-azaspiro[3.4]octane, 2-azaspiro[3.3]heptane, 2-oxa-6-azaspiro[3.3]heptane, 6-oxa-2-azaspiro[3.4]octane, 2,7-diaza-spiro[4.4]nonane, 2-azaspiro[3.5]nonane, 2-oxa-7-azaspiro[3.5]nonane and 2-oxa-6-azaspiro[3.5]nonane.

**[0180]** The term "aromatic" when applied to a substituent as a whole includes a single ring or polycyclic ring system with 4n + 2 electrons in a conjugated $\pi$ (pi) system within the ring or ring system where all atoms contributing to the conjugated $\pi$

(pi) system are in the same plane.

**[0181]** The term "aryl" includes an aromatic hydrocarbon ring system. The ring system has 4n + 2 electrons in a conjugated π (pi) system within a ring where all atoms contributing to the conjugated π (pi) system are in the same plane. For example, the "aryl" may be phenyl and naphthyl. The aryl system itself may be substituted with other groups.

**[0182]** The term "heteroaryl" includes an aromatic mono- or bicyclic ring incorporating one or more (for example 1-4, particularly 1, 2 or 3) heteroatoms selected from nitrogen, oxygen or sulfur. The ring or ring system has 4n + 2 electrons in a conjugated π (pi) system where all atoms contributing to the conjugated π (pi) system are in the same plane.

**[0183]** Examples of heteroaryl groups are monocyclic and bicyclic groups containing from five to twelve ring members, and more usually from five to ten ring members. The heteroaryl group can be, for example, a 5- or 6-membered monocyclic ring or a 9- or 10-membered bicyclic ring, for example a bicyclic structure formed from fused five and six membered rings or two fused six membered rings. Each ring may contain up to about four heteroatoms typically selected from nitrogen, sulfur and oxygen. Typically, the heteroaryl ring will contain up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general, the number of basic nitrogen atoms present in the heteroaryl group, including any amino group substituents of the ring, will be less than five.

**[0184]** Examples of heteroaryl include furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazenyl, benzofuranyl, indolyl, isoindolyl, benzothienyl, benzoxazolyl, benzimidazolyl, benzothiazolyl, benzothiazolyl, indazolyl, purinyl, benzofurazanyl, quinolyl, isoquinolyl, quinazolinyl, quinoxalinyl, cinnolinyl, pteridinyl, naphthyridinyl, carbazolyl, phenazinyl, benzisoquinolinyl, pyridopyrazinyl, thieno[2,3-b]furanyl, 2H-furo[3,2-b]-pyranyl, 5H-pyrido[2,3-d]-o-oxazinyl, 1H-pyrazolo[4,3-d]-oxazolyl, 4H-imidazo[4,5-d]thiazolyl, pyrazino[2,3-d]pyridazinyl, imidazo[2,1-b]thiazolyl and imidazo [1,2-b][1,2,4]triazinyl. Examples of heteroaryl groups comprising at least one nitrogen in a ring position include pyrrolyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazenyl, indolyl, isoindolyl, benzoxazolyl, benzimidazolyl, benzothiazolyl, benzothiazolyl, indazolyl, purinyl, benzofurazanyl, quinolyl, isoquinolyl, quinazolinyl, quinoxalinyl, cinnolinyl and pteridinyl. "Heteroaryl" also covers partially aromatic bi- or polycyclic ring systems wherein at least one ring is an aromatic ring and one or more of the other ring(s) is a non-aromatic, saturated or partially saturated ring, provided at least one ring contains one or more heteroatoms selected from nitrogen, oxygen or sulfur. Examples of partially aromatic heteroaryl groups include for example, tetrahydroisoquinolinyl, tetrahydroquinolinyl, 2-oxo-1,2,3,4-tetrahydroquinolinyl, dihydrobenzthienyl, dihydrobenzfuranyl, 2,3-dihydro-benzo[1,4]dioxinyl, benzo[1,3]dioxolyl, 2,2-dioxo-1,3-dihydro-2-benzothienyl, 4,5,6,7-tetrahydrobenzofuranyl, indolinyl, 1,2,3,4-tetrahydro-1,8-naphthyridinyl, 1,2,3,4-tetrahydropyrido[2,3-b]pyrazinyl and 3,4-dihydro-2*H*-pyrido[3,2-*b*][1,4]oxazinyl.

**[0185]** Examples of five membered heteroaryl groups include but are not limited to pyrrolyl, furanyl, thienyl, imidazolyl, furazanyl, oxazolyl, oxadiazolyl, oxatriazolyl, isoxazolyl, thiazolyl, isothiazolyl, pyrazolyl, triazolyl and tetrazolyl groups.

**[0186]** Examples of six membered heteroaryl groups include but are not limited to pyridyl, pyrazinyl, pyridazinyl, pyrimidinyl and triazinyl.

**[0187]** Particular examples of bicyclic heteroaryl groups containing a six membered ring fused to a five membered ring include but are not limited to benzofuranyl, benzothiophenyl, benzimidazolyl, benzoxazolyl, benzisoxazolyl, benzothiazolyl, benzisothiazolyl, isobenzofuranyl, indolyl, isoindolyl, indolizinyl, indolinyl, isoindolinyl, purinyl (e.g., adeninyl, guaninyl), indazolyl, benzodioxolyl, pyrrolopyridine, and pyrazolopyridinyl groups.

**[0188]** Particular examples of bicyclic heteroaryl groups containing two fused six membered rings include but are not limited to quinolinyl, isoquinolinyl, chromanyl, thiochromanyl, chromenyl, isochromenyl, chromanyl, isochromanyl, benzodioxanyl, quinolizinyl, benzoxazinyl, benzodiazinyl, pyridopyridinyl, quinoxalinyl, quinazolinyl, cinnolinyl, phthalazinyl, naphthyridinyl and pteridinyl groups.

**[0189]** The term "optionally substituted" includes either groups, structures, or molecules that are substituted and those that are not substituted.

**[0190]** Where optional substituents are chosen from "one or more" groups it is to be understood that this definition includes all substituents being chosen from one of the specified groups or the substituents being chosen from two or more of the specified groups.

**[0191]** The phrase "compound of the invention" means those compounds which are disclosed herein, both generically and specifically.

**[0192]** A bond terminating in a

“ 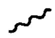 ”

represents that the bond is connected to another atom that is not shown in the structure. A bond terminating inside a cyclic structure and not terminating at an atom of the ring structure represents that the bond may be connected to any of the atoms

in the ring structure where allowed by valency.

**[0193]** Where a moiety is substituted, it may be substituted at any point on the moiety where chemically possible and consistent with atomic valency requirements. The moiety may be substituted by one or more substituents, e.g. 1, 2, 3 or 4 substituents; optionally there are 1 or 2 substituents on a group. Where there are two or more substituents, the substituents may be the same or different.

**[0194]** Substituents are only present at positions where they are chemically possible, the person skilled in the art being able to decide (either experimentally or theoretically) without undue effort which substitutions are chemically possible and which are not.

**[0195]** Ortho, meta and para substitution are well understood terms in the art. For the absence of doubt, "ortho" substitution is a substitution pattern where adjacent carbons possess a substituent, whether a simple group, for example the fluoro group in the example below, or other portions of the molecule, as indicated by the bond ending in

“ 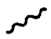 ”.

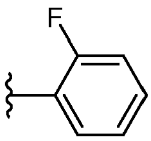 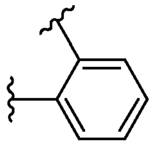 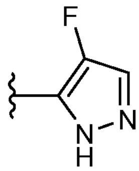 .

**[0196]** "Meta" substitution is a substitution pattern where two substituents are on carbons one carbon removed from each other, i.e. with a single carbon atom between the substituted carbons. In other words there is a substituent on the second atom away from the atom with another substituent. For example the groups below are meta substituted.

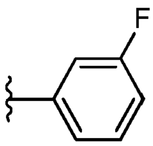 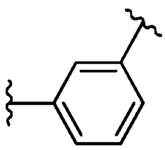 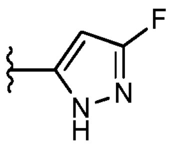 .

**[0197]** "Para" substitution is a substitution pattern where two substituents are on carbons two carbons removed from each other, i.e. with two carbon atoms between the substituted carbons. In other words there is a substituent on the third atom away from the atom with another substituent. For example the groups below are para substituted.

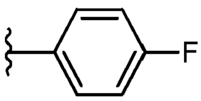 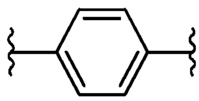 .

**[0198]** The term "acyl" includes an organic radical derived from, for example, an organic acid by the removal of the hydroxyl group, e.g. a radical having the formula R-C(O)-, where R may be selected from H, $C_{1-6}$ alkyl, $C_{3-8}$ cycloalkyl, phenyl, benzyl or phenethyl group, e.g. R is H or $C_{1-3}$ alkyl. In one embodiment acyl is alkyl-carbonyl. Examples of acyl groups include, but are not limited to, formyl, acetyl, propionyl and butyryl. A particular acyl group is acetyl (also represented as Ac).

**[0199]** Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

**[0200]** Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract

and drawings), or to any novel one, or any novel combination, of the steps of any method or process so disclosed.

**[0201]** The reader's attention is directed to all papers and documents which are filed concurrently with or previous to this specification in connection with this application and which are open to public inspection with this specification, and the contents of all such papers and documents are incorporated herein by reference.

**[0202]** The various functional groups and substituents making up the compounds of the present invention are typically chosen such that the molecular weight of the compound does not exceed 1000. More usually, the molecular weight of the compound will be less than 750, for example less than 700, or less than 650, or less than 600, or less than 550. More preferably, the molecular weight is less than 525.

**[0203]** Suitable or preferred features of any compounds of the present invention may also be suitable features of any other aspect.

**Methods and uses of the compounds:**

**[0204]** In accordance with a second aspect, the present invention also provides a pharmaceutical formulation comprising a compound of formula (I) above, or a pharmaceutically acceptable salt thereof, and a pharmaceutically acceptable excipient.

**[0205]** In accordance with a third aspect, the present invention provides a compound of the invention, or a pharmaceutically acceptable salt thereof, for use as a medicament.

**[0206]** In accordance with a fourth aspect, the present invention also provides the compounds of the present invention, or a pharmaceutically acceptable salt thereof, for use in the treatment of a disease mediated by GPR88.

**[0207]** In a fifth aspect, the present invention provides a compound for use in the treatment of, Tourette's Syndrome, Huntington's Disease (HD), Addiction, Parkinson's Disease (PD), Schizophrenia, and Attention Deficit Hyperactivity Disorder (ADHD), choreiform movements, speech delay, learning disabilities, depression, hyperkinetic movement disorders characterised by chorea and/or dystonia, psychosis, cognitive deficits in schizophrenia, affective disorders, bipolar disorder, Alzheimer's disease and basal ganglia disorders.

**[0208]** In an embodiment, the invention provides a compound for use in the treatment of, Tourette's Syndrome, Huntington's Disease (HD), Addiction, Parkinson's Disease (PD), Schizophrenia, Alzheimer's disease, and Attention Deficit Hyperactivity Disorder (ADHD).

**[0209]** In an embodiment, the invention provides a compound for use in the treatment of Huntington's Disease (HD).

**[0210]** Thus, the invention contemplates a method of treating a disease mediated by GPR88, or any specific disease recited above, wherein the method comprises administering to a patient in need thereof a therapeutically effective amount of a compound of the invention.

**[0211]** The embodiments relating to the first aspect are also applicable to all other aspects of the invention, including the second, third, fourth and fifth aspects above.

**Pharmaceutical compositions:**

**[0212]** A compound of the invention, or pharmaceutically acceptable salt thereof, may be used on their own but will generally be administered in the form of a pharmaceutical composition in which the compounds of the invention, or pharmaceutically acceptable salt thereof, is in association with a pharmaceutically acceptable adjuvant, diluent or carrier.

**[0213]** Conventional procedures for the selection and preparation of suitable pharmaceutical formulations are described in, for example, "Pharmaceuticals - The Science of Dosage Form Designs", M. E. Aulton, Churchill Livingstone, 1988.

**[0214]** Depending on the mode of administration of the compounds of the invention, the pharmaceutical composition which is used to administer the compounds of the invention will preferably comprise from 0.05 to 99 % w/w compounds of the invention, more preferably from 0.05 to 80 % w/w compounds of the invention, still more preferably from 0.10 to 70 % w/w compounds of the invention, and even more preferably from 0.10 to 50 % w/w compounds of the invention (all percentages by weight being based on total composition).

**[0215]** The pharmaceutical compositions may be administered topically (e.g. to the skin) in the form, e.g., of creams, ointments, gels, lotions, solutions, suspensions; or systemically, e.g. by oral administration in the form of tablets, lozenges, hard or soft capsules, aqueous or oily suspensions, emulsions, dispersible powders or granules, syrups or elixirs; or by parenteral administration in the form of a sterile aqueous or oily solution, suspension or emulsion for injection (including intravenous, intracoronary, subcutaneous, intramyocardial, intraperitoneal, intramuscular, intravascular or infusion); by rectal administration in the form of suppositories or enemas; by inhalation for example as a finely divided powder or a liquid aerosol; or for administration by insufflation (for example as a finely divided powder).

**[0216]** For oral administration the compounds of the invention may be admixed with an adjuvant or a carrier, for example, lactose, saccharose, sorbitol, mannitol; a starch, for example, potato starch, corn starch or amylopectin; a cellulose derivative; a binder, for example, gelatine or polyvinylpyrrolidone; and/or a lubricant, for example, magnesium stearate,

calcium stearate, polyethylene glycol, a wax, paraffin, and the like, and then compressed into tablets. If coated tablets are required, the cores, prepared as described above, may be coated with a concentrated sugar solution which may contain, for example, gum arabic, gelatine, talcum and titanium dioxide. Alternatively, the tablet may be coated with a suitable polymer dissolved in a readily volatile organic solvent. Thus, compositions intended for oral use may contain, for example, one or more colouring, sweetening, flavouring and/or preservative agents.

**[0217]** For the preparation of soft gelatine capsules, the compounds of the invention may be admixed with, for example, a vegetable oil or polyethylene glycol. Hard gelatine capsules may contain granules of the compound using either the above-mentioned excipients for tablets. Also liquid or semisolid formulations of the compound of the invention may be filled into hard gelatine capsules. Liquid preparations for oral application may be in the form of syrups or suspensions, for example, solutions containing the compound of the invention, the balance being sugar and a mixture of ethanol, water, glycerol and propylene glycol. Optionally such liquid preparations may contain colouring agents, flavouring agents, sweetening agents (such as saccharine), preservative agents and/or carboxymethylcellulose as a thickening agent or other excipients known to those skilled in art.

**[0218]** For intravenous (parenteral) administration the compounds of the invention may be administered as a sterile aqueous or oily solution.

**[0219]** The size of the dose for therapeutic or prophylactic purposes of a compound of the invention will naturally vary according to the nature and severity of the conditions, the concentration of the compound required for effectiveness in isolated cells, the concentration of the compound required for effectiveness in experimental animals, the age and sex of the animal or patient and the route of administration, according to well known principles of medicine.

**[0220]** Dosage levels, dose frequency, and treatment durations of compounds of the invention are expected to differ depending on the formulation and clinical indication, age, and co-morbid medical conditions of the patient.

**[0221]** An effective amount of a compound of the present invention for use in therapy of a condition is an amount sufficient to achieve symptomatic relief in a warm-blooded animal, particularly a human of the symptoms of the condition, to mitigate the physical manifestations of the condition, or to slow the progression of the condition.

**[0222]** The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

**[0223]** For the above-mentioned compounds of the invention the dosage administered will, of course, vary with the compound employed, the mode of administration, the treatment desired and the disorder indicated. In using a compound of the invention for therapeutic or prophylactic purposes it will generally be administered so that a daily dose in the range, for example, a daily dose selected from 0.1 mg/kg to 100 mg/kg, 1 mg/kg to 75mg/kg, 1 mg/kg to 50 mg/kg, 1 mg/kg to 20 mg/kg or 5 mg/kg to 10 mg/kg body weight is received, given if required in divided doses. In general lower doses will be administered when a parenteral route is employed. Thus, for example, for intravenous or intraperitoneal administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Suitably the compound of the invention is admistered orally, for example in the form of a tablet, or capsule doasage form. The daily dose administered orally may be, for example a total daily dose selected from 1 mg to 1000 mg, 5 mg to 1000 mg, 10 mg to 750 mg or 25 mg to 500 mg. Typically, unit dosage forms will contain about 0.5 mg to 0.5 g of a compound of this invention.

## ***Examples:***

### General Schemes

### Abbreviations

**[0224]** app: apparent; aq: aqueous; br: broad; *ca.: circa;* d: doublet; DCM: dichloromethane; dioxane: 1,4-dioxane; DIPEA: diisopropylethylamine; DMF: dimethylformamide; $Et_3N$: triethylamine; EtOAc: ethyl acetate; EtOH: ethanol; h: hours; HATU: 2-(3*H*-[1,2,3]triazolo[4,5-b]pyridin-3-yl)-1,1,3,3-tetramethyl isouronium hexafluorophosphate(V); HPLC: high performance liquid chromatography; IPA, isopropanol; LC: liquid chromatography; m: multiplet; M: molar, molecular ion; MeCN: acetonitrile; MeOH: methanol; min: minutes; MS: mass spectrometry; NMR: nuclear magnetic resonance; PDA: photodiode array; q: quartet; RT: room temperature (ca. 20 °C); $R_T$: retention time; s: singlet, solid; t: triplet; TBAF: tetrabutylammonium fluoride; TBME: tert-butyl methyl ether; TFA: trifluoroacetic acid; THF: tetrahydrofuran; UPLC: ultra performance liquid chromatography; UV: ultraviolet.

**[0225]** Other abbreviations are intended to convey their generally accepted meaning.

**[0226]** Examples of the invention were prepared following one of the general Schemes below, using the appropriate reagents for the target compound.

Scheme 1

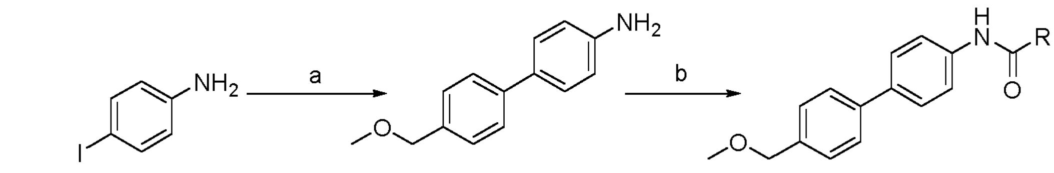


I-1 I-2 I-3

**Reagents:** (a) $Pd(dppf)Cl_2$, $K_3PO_4$, $ArB(OH)_2$, 1,4-dioxane-water 100 °C; (b) Carboxylic acid, HATU, DIPEA, MeCN RT or 50 °C

**[0227]** Suzuki coupling of I-1 produced I-2. This was reacted with carboxylic acid in the presence of HATU to afford amide I-3.

Scheme 2

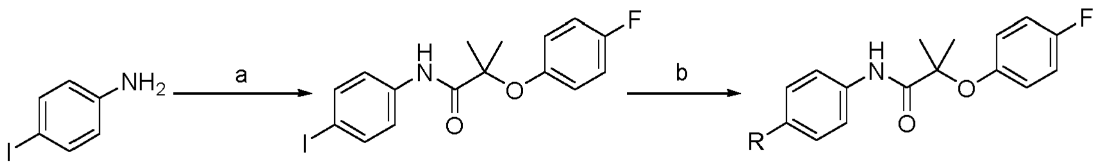


I-1 I-4 I-5

**Reagents:** (a) Carboxylic acid, HATU, DIPEA, MeCN RT (b) $Pd(dppf)Cl_2$, $K_3PO_4$, $ArB(OH)_2$, 1,4-dioxane-water 100 °C.

**[0228]** I-1 was reacted with 2-(4-fluorophenoxy)-2-methylpropanoic acid in the presence of HATU to produce amide I-4. This was coupled with an arylboronic acid or arylboronic ester under Pd catalysis to afford I-5.

Scheme 3

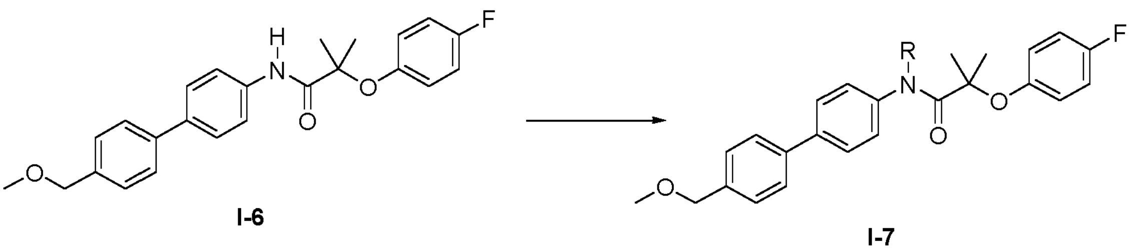

I-6 I-7

**Reagents:** alkyl halide, sodium hydride, THF 0 °C - RT.

**[0229]** Intermediate I-6 was alkylated with an alkyl halide in the presence of sodium hydride to afford I-7.

Scheme 4

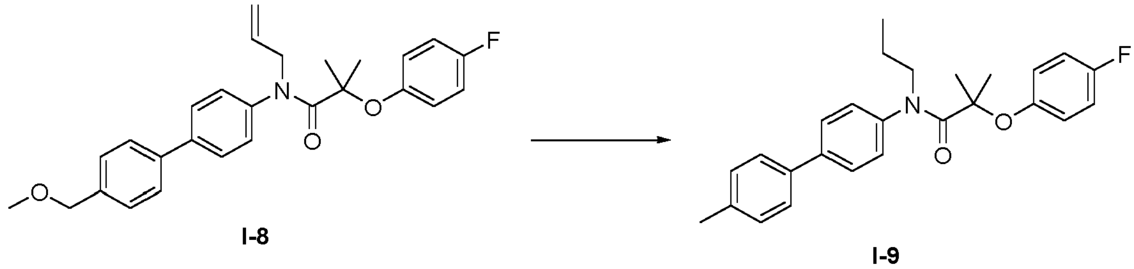

I-8

I-9

**Reagents:** Pd/C, $H_2$, EtOH

**[0230]** Intermediate I-8 was hydrogenated to afford I-9.

Scheme 5

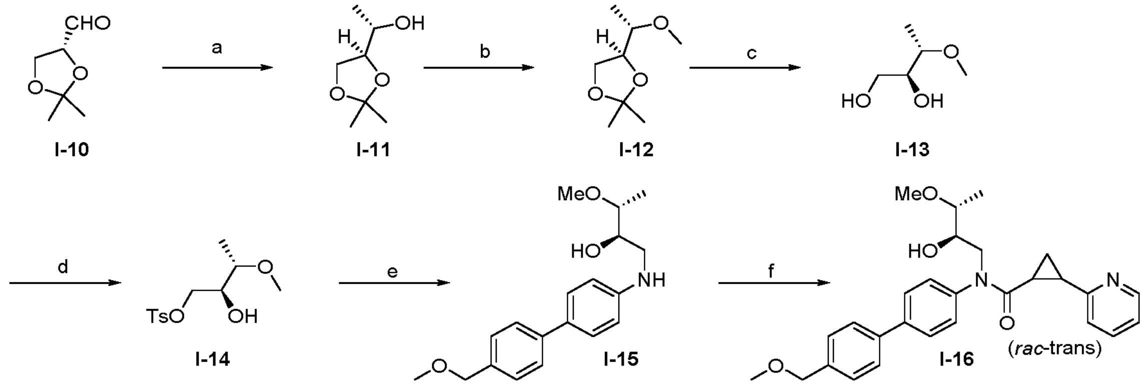


I-10 I-11 I-12 I-13

I-14 I-15 I-16

**Reagents:** (a) MeMgBr, $Et_2O$ (b) NaH, MeI, THF (c) AcOH-$H_2O$ (d) TsCl, pyridine, DCM (e) $ArNH_2$, KI, $NEt_3$, MeCN (f) $RCO_2H$, HATU, DIPEA, MeCN

**[0231]** Grignard addition to aldehyde I-10 gave alcohol I-11 which was alkylated to give ether I-12. Acetal deprotection gave diol I-13 which was tosylated to give I-14. Nucleophilic substitution with aniline I-2 afforded amine I-15 which underwent amide coupling to furnish I-16.

Scheme 6

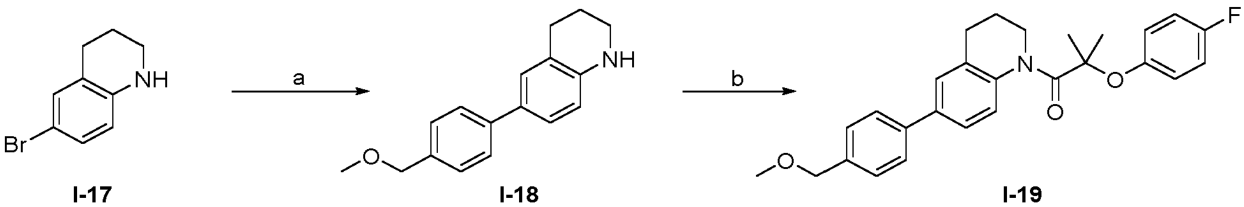


I-17 I-18 I-19

**Reagents:** a) $Pd(dppf)Cl_2$, $ArB(OH)_2$, $K_3PO_4$, dioxane-water (b) oxalyl chloride, DMF, $RCO_2H$.

**[0232]** Intermediate I-17 was coupled to aryl boronic ester to give intermediate I-18 which was coupled with an acid chloride to furnish I-19

Scheme 7

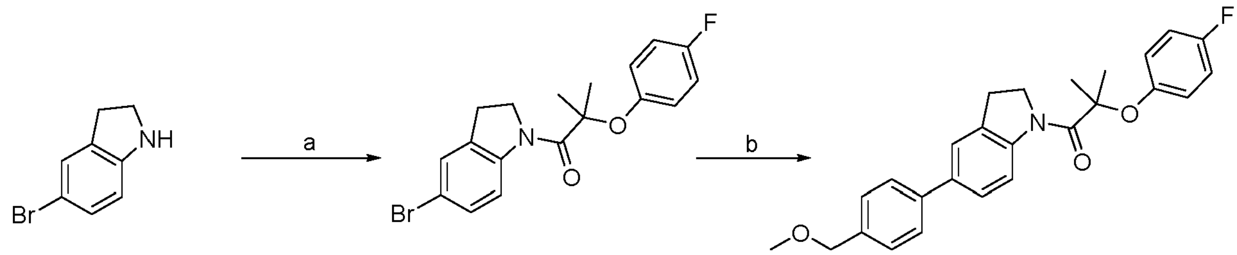


I-20 I-21 I-22

**Reagents:** a) $RCO_2H$, HATU, DIPEA, MeCN (b) $Pd(dppf)Cl_2$, $ArB(OH)_2$, $K_3PO_4$, dioxane-water

**[0233]** Intermediate I-20 underwent amide coupling to give I-21 which was coupled via Suzuki reaction to afford I-22.

Scheme 8

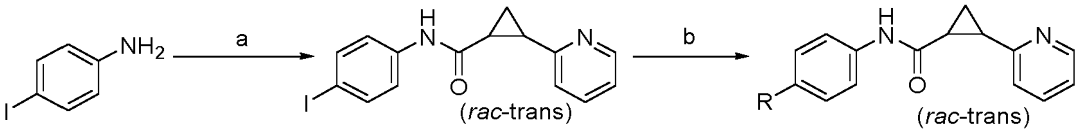


I-1 I-23 I-24

**Reagents:** (a) Carboxylic acid, HATU, DIPEA, MeCN RT (b) $Pd(dppf)Cl_2$, $K_3PO_4$, $ArB(OH)_2$, 1,4-dioxane-water 100 °C.

**[0234]** I-1 was reacted coupled to carboxylic acid to give amide I-23. This was coupled with an arylboronic acid or arylboronic ester under Pd catalysis to afford I-24.

Scheme 9

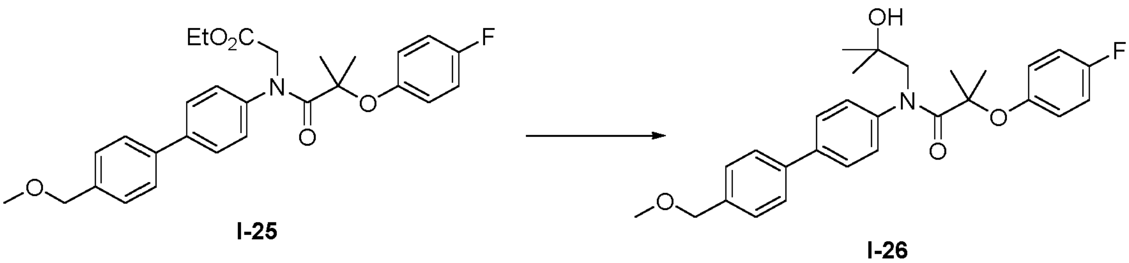

I-25 I-26

**Reagents:** MeMgBr, THF

**[0235]** Methyl magnesium bromide was added to ester I-25 to afford tertiary alcohol I-26.

Scheme 10

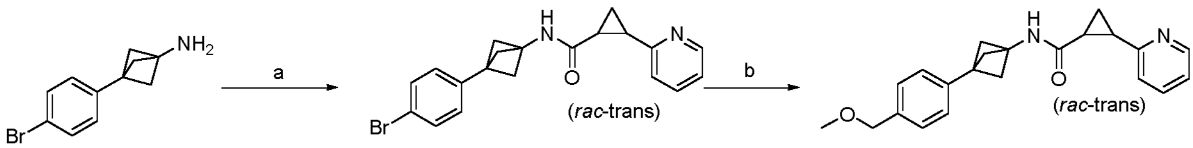


I-27 I-28 I-29

**Reagents:** (a) $RCO_2H$, HATU, DIPEA, MeCN (b) $MeOCH_2BF_3K$, Pd(dppf)$Cl_2$, $K_3PO_4$, 1,4-dioxane-water 100 °C

**[0236]** Amine I-27 underwent amide coupling to afford I-28 which was reacted with methoxymethyl potassium trifluoroboronate under palladium catalysis to give I-29.

General Experimental Conditions

**[0237]** All starting materials and solvents were obtained either from commercial sources or prepared according to the literature citation. Reaction mixtures were magnetically stirred and reactions performed at room temperature (ca. 20 °C) unless otherwise indicated.

**[0238]** Column chromatography was performed on an automated flash chromatography system, such as a CombiFlash Rf system, using pre-packed silica (40 μm) cartridges, unless otherwise indicated.

**[0239]** $^1H$ NMR spectra were recorded using a Bruker Avance III HD spectrometer at 500 MHz, equipped with a Bruker 5 mm SmartProbe™. Chemical shifts are expressed in parts per million using either the central peaks of the residual protic solvent or an internal standard of tetramethylsilane as references. The spectra were recorded at 298 K unless otherwise indicated.

**[0240]** Analytical UPLC-MS experiments to determine retention times and associated mass ions were performed using a Waters ACQUITY UPLC® H-Class system, equipped with ACQUITY PDA Detector and ACQUITY QDa Mass Detector, running one of the analytical methods described below.

**[0241]** Analytical LC-MS experiments to determine retention times and associated mass ions were performed using an Agilent 1200 series HPLC system coupled to an Agilent 1956, 6100 or 6120 series single quadrupole mass spectrometer running one of the analytical methods described below.

**[0242]** Preparative HPLC purifications were performed either using a Waters X-Select CSH C18, 5 μm, 19x50 mm column using a gradient of MeCN and water, both modified with 0.1% v/v formic acid, or on a Waters X-Bridge BEH C18, 5 μm, 19x50 mm column using a gradient of MeCN and 10 mM ammonium bicarbonate(aq). Fractions were collected following detection by UV at a single wavelength measured by a variable wavelength detector.

**[0243]** Nomenclature of structures was generated using 'Structure to Name' conversion from ChemDraw® Professional 19 (PerkinElmer).

## Analytical Methods

### Method 1 - Acidic 3 min method

**[0244]**

| | |
|---|---|
| Column: | Waters ACQUITY UPLC® CSH C18, 1.7 μm, 2.1x30 mm at 40 °C |
| Detection: | UV at 254 nm unless otherwise indicated, MS by electrospray ionisation |
| Solvents: | A: 0.1% v/v Formic acid in water, B: 0.1% v/v Formic acid in MeCN |

Gradient:

**[0245]**

| Time | %A | %B | Flow rate (ml/min) |
|---|---|---|---|
| 0.00 | 95 | 5 | 0.77 |
| 0.11 | 95 | 5 | 0.77 |
| 2.15 | 5 | 95 | 0.77 |
| 2.56 | 5 | 95 | 0.77 |
| 2.83 | 95 | 5 | 0.77 |
| 3.00 | 95 | 5 | 0.77 |

**Method 2 - Basic 3 min method**

**[0246]**

Column: Waters ACQUITY UPLC® BEH C18, 1.7 μm, 2.1x30 mm at 40 °C
Solvents: A: 10 mM ammonium bicarbonate(aq), B: MeCN

(other parameters the same as Method 1)

**Method 3 - Acidic 4 min method**

**[0247]**

Column: Waters X-Select CSH C18, 2.5 μm, 4.6x30 mm at 40 °C
Detection: UV at 254 nm unless otherwise indicated, MS by electrospray ionisation
Solvents: A: 0.1% v/v Formic acid in water, B: 0.1% v/v Formic acid in MeCN

Gradient:

**[0248]**

| Time | %A | %B | Flow rate (ml/min) |
|---|---|---|---|
| 0.0 | 95.0 | 5.0 | 2.5 |
| 3.0 | 5.0 | 95.0 | 2.5 |
| 3.01 | 5.0 | 95.0 | 4.5 |
| 3.6 | 5.0 | 95.0 | 4.5 |
| 3.7 | 95.0 | 5.0 | 2.5 |
| 4.0 | 95.0 | 5.0 | 2.5 |

**Method 4 - Basic 4 min method**

**[0249]**

Column: Waters X-Bridge BEH C18, 2.5 μm, 4.6x30 mm at 40 °C
Solvents: A: 10 mM ammonium bicarbonate(aq), B: MeCN

(other parameters the same as Method 3)

**Chiral SFC Method 1**

**[0250]** Waters $UPC^2$ using anlH 4.6X250, 5um column, flow rate 4 $mL/min^{-1}$ eluting with 30 % MeOH (0.1% Ammonia), 70% $CO_2$ at a wavelength 210 - 400nm and BPR 120 Bar.

**Chiral SFC Method 2**

**[0251]** Waters $UPC^2$. Chiralpak IC 4.6X250, 5um, flow rate 4 $mL/min^{-1}$ eluting with 50 % MeOH (0.1% Ammonia), 50% $CO_2$ at a wavelength 210 - 400nm and BPR 120 Bar.

***Example** 1: **(rac-trans)-N-(4'-(methoxymethyl)-[1,1'-biphenyl]-4-yl)-2-(pyridin-2-yl)cyclo propane-1-carboxamide***

**[0252]**

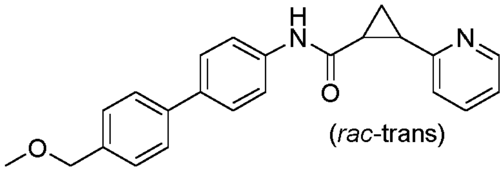

Step 1: 4'-(methoxymethyl)-[1,1'-biphenyl]-4-amine

**[0253]** To a degassed solution of 4-bromoaniline (2.00 g, 11.6 mmol), (4-(methoxymethyl)phenyl)boronic acid (2.10 g, 12.7 mmol) and 1,1'-bis(diphenylphosphino) ferrocenedichloro palladium(II) dichloromethane complex (420 mg, 574 μmol) in 1-4, dioxane (30 mL) was added a solution of $K_3PO_4$ (6.20 g, 29.2 mmol) in water (10 mL). The mixture was degassed then heated to 90 °C for 18 h. The mixture was filtered through celite washing with EtOAc (25 mL) then poured into 10 wt% aq. citric acid (100 mL). The phases were separated and the aqueous phase extracted with EtOAc (2 × 25 mL). The combined organics were washed with water (50 mL), brine (50 mL), dried ($MgSO_4$), filtered and concentrated under reduced pressure. The crude product was purified by chromatography on silica gel (40 g cartridge,0-100% EtOAc/isohexane) to afford title amine (1.32 g, 5.9 mmol, 51%, 95% purity) as a beige solid. UPLC-MS (Method 1) m/z 214.4 $(M+H)^+$ at 0.85 min. $^1$H NMR (500 MHz, DMSO-d6) δ 7.54 - 7.48 (m, 2H), 7.38 - 7.32 (m, 2H), 7.32 - 7.24 (m, 2H), 6.66 - 6.60 (m, 2H), 5.21 (s, 2H), 4.40 (s, 2H), 3.28 (s, 3H).

Step 2: (*rac*-trans)-*N*-(4'-(methoxymethyl)-[1,1'-biphenyl]-4-yl)-2-(pyridin-2-yl)cyclo propane-1-carboxamide

**[0254]** To a solution of 4'-(methoxymethyl)-[1,1'-biphenyl]-4-amine (120 mg, 563 μmol) and (rac-trans)-2-(pyridin-2-yl) cyclopropane-1-carboxylic acid (110 mg, 675 μmol) in MeCN (5 mL) at RT was added N-ethyl-*N*-isopropylpropan-2-amine (0.29 mL, 1.7 mmol) and HATU (321 mg, 844 μmol). The mixture was heated at 50 °C for 18 h. The reaction mixture was cooled to RT, diluted with DCM (5 mL) and sat. $NaHCO_3$ (2 mL), stirred for 10 min then passed through a hydrophobic frit. 10 wt% aqueous citric acid (2 mL) was added to the organics, stirred for 10 min, the mixture was passed through a hydrophobic frit and concentrated under reduced pressure to afford a brown oil. The crude product was purified by chromatography on silica gel (40 g cartridge, 0-100% EtOAc/isohexane) to afford title amide (150 mg, 0.41 mmol, 74%, 99% purity) as a white solid. UPLC-MS (Method 2) m/z 359.4 $(M+H)^+$ at 1.43 min. $^1$H NMR (500 MHz, DMSO-d6) δ 10.38 (s, 1H), 8.47 (d, *J* = 4.8 Hz, 1H), 7.73 - 7.66 (m, 3H), 7.65 - 7.58 (m, 4H), 7.46 (d, *J* = 7.9 Hz, 1H), 7.37 (d, *J* = 8.2 Hz, 2H), 7.20 (ddd, *J* = 7.5, 4.9, 1.1 Hz, 1H), 4.43 (s, 2H), 3.30 (s, 3H), 2.58 (ddd, *J* = 8.7, 6.0, 3.8 Hz, 1H), 2.41 (ddd, *J* = 8.1, 5.4, 3.8 Hz, 1H), 1.53 - 1.46 (m, 2H).

**[0255]** The trans isomers of N-(4'-(methoxymethyl)-[1,1'-biphenyl]-4-yl)-2-(pyridin-2-yl)cyclopropane-1-carboxamide were separated by chiral SFC on a Waters prep 15 with UV detection by DAD at 210 - 400 nm, 40 °C, 120 bar. The column was IH 10×250mm, 5um, flow rate 15mL/ min at 30 % MeOH (0.1% Ammonia), 70 % $CO_2$ to afford:

First eluting isomer (Example 18) Chiral SFC (method 1) 2.18 min, >99% ee.

Second eluting isomer (Example 19) Chiral SFC (method 1) 4.04 min, >99% ee.

**[0256]** The following examples were prepared by methods analogous to Example 1, substituting appropriate starting materials and intermediates where necessary:

| Example | Structure | Name / Analytical Data |
|---|---|---|
| 2 | 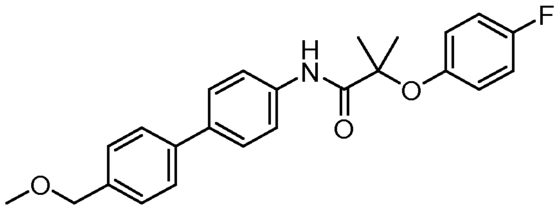 | 2-(4-fluorophenoxy)-*N*-(4'-(methoxymethyl)-[1,1'-biphenyl]-4-yl)-2-methylpropanamide UPLC-MS (Method 2) m/z 394.4 $(M+H)^+$, at 1.77 min. $^1H$ NMR (400 MHz, DMSO-d6) δ 10.11 (s, 1H), 7.88 - 7.74 (m, 2H), 7.72 - 7.56 (m, 4H), 7.39 (d, *J* = 8.1 Hz, 2H), 7.23 - 7.10 (m, 2H), 7.08 - 6.92 (m, 2H), 4.44 (s, 2H), 3.30 (s, 3H), 1.52 (s, 6H). |
| 4 | 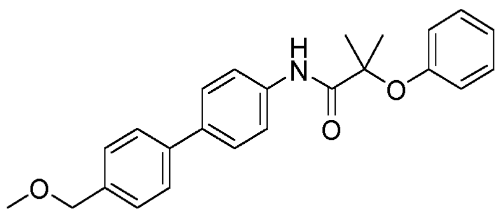 | *N*-(4'-(methoxymethyl)-[1,1'-biphenyl]-4-yl)-2-methyl-2-phenoxypropanamide UPLC-MS (Method 1) m/z 376.2 $(M+H)^+$, at 2.73 min. $^1H$ NMR (500 MHz, Chloroform-d) δ 8.66 (s, 1H), 7.74 - 7.64 (m, 2H), 7.61 - 7.49 (m, 4H), 7.40 (d, *J* = 8.1 Hz, 2H), 7.35 - 7.26 (m, 2H), 7.17 - 7.06 (m, 1H), 7.06 - 6.97 (m, 2H), 4.50 (s, 2H), 3.42 (s, 3H), 1.61 (s, 6H). |

***Example 3: 2-(4-chlorophenoxy)-N-(4'-(methoxymethyl)-[1,1'-biphenyl]-4-yl)-2-methylpropanamide***

**[0257]**

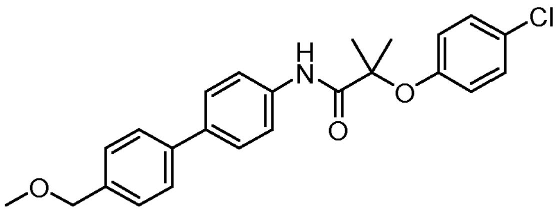

**[0258]** To a solution of 4'-(methoxymethyl)-[1,1'-biphenyl]-4-amine (50 mg, 0.23 mmol) and 2-(4-chlorophenoxy)-2-methylpropanoic acid (55 mg, 0.26 mmol) in MeCN (2.0 mL) were added HATU (98 mg, 0.26 mmol) and DIPEA (0.12 mL, 0.70 mmol) and the reaction was stirred at RT for 3 h. The mixture was diluted with DCM (15 mL) and 10 wt% aq. citric acid (10 mL), stirred for 10 min, passed through a hydrophobic frit and concentrated under reduced pressure. The crude product was purified by chromatography on silica gel (12 g cartridge, 0-100% EtOAc/isohexane) to afford 2-(4-chlorophenoxy)-N-(4'-(methoxymethyl)-[1,1'-biphenyl]-4-yl)-2-methylpropanamide (89 mg, 0.21 mmol, 92%, 99% purity) as a cream solid. UPLC-MS (Method 1) m/z 410.4/412.4 $(M+H)^+$, at 1.91 min. $^1H$ NMR (500 MHz, DMSO-d6) δ 10.10 (s, 1H), 7.81 - 7.74 (m, 2H), 7.62 (dd, *J* = 8.5, 3.1 Hz, 4H), 7.38 (d, *J* = 8.3 Hz, 2H), 7.36 - 7.34 (m, 2H), 6.99 - 6.92 (m, 2H), 4.44 (s, 2H), 3.30 (s, 3H), 1.56 (s, 6H).

**[0259]** The following examples were prepared by methods analogous to Example 3, substituting appropriate starting materials and intermediates where necessary:

| Example | Structure | Name / Analytical Data |
|---|---|---|
| 6 | 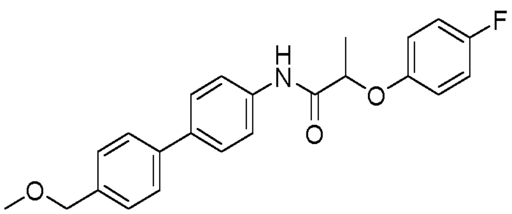 | 2-(4-fluorophenoxy)-*N*-(4'-(methoxymethyl)-[1,1'-biphenyl]-4-yl)propenamide UPLC-MS (Method 1) m/z 380.7 $(M+H)^+$, at 1.65 min. $^1H$ NMR (500 MHz, DMSO-d6) δ 10.20 (s, 1H), 7.75 - 7.69 (m, 2H), 7.66 - 7.59 (m, 4H), 7.38 (d, *J* = 8.1 Hz, 2H), 7.18 - 7.10 (m, 2H), 7.04 - 6.96 (m, 2H), 4.85 (q, *J* = 6.7 Hz, 1H), 4.43 (s, 2H), 3.30 (s, 3H), 1.55 (d, *J* = 6.7 Hz, 3H). |

(continued)

| Example | Structure | Name / Analytical Data |
|---|---|---|
| 7 | 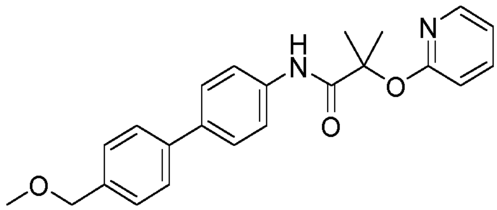 | *N*-(4'-(methoxymethyl)-[1,1'-biphenyl]-4-yl)-2-methyl-2-(pyridin-2-yloxy)propanamide<br>UPLC-MS (Method 1) m/z 377.5 $(M+H)^+$, at 1.65 min. $^1$H NMR (500 MHz, DMSO-d6) δ 9.61 (s, 1H), 8.05 (ddd, *J* = 5.0, 2.0, 0.8 Hz, 1H), 7.72 (ddd, *J* = 8.3, 7.1, 2.0 Hz, 1H), 7.69 - 7.63 (m, 2H), 7.63 - 7.57 (m, 2H), 7.59 - 7.52 (m, 2H), 7.36 (d, *J* = 8.3 Hz, 2H), 6.93 (ddd, *J* = 7.1, 5.0, 1.0 Hz, 1H), 6.90 (d, *J* = 8.3 Hz, 1H), 4.43 (s, 2H), 3.30 (s, 3H), 1.66 (s, 6H). |
| 8 | 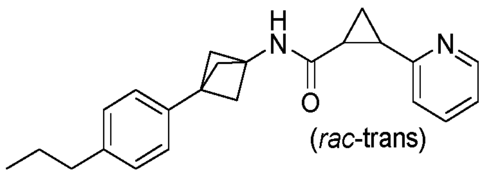<br>(*rac*-trans) | (rac-trans)-N-(3-(4-propylphenyl)bicyclo[1.1.1] pentan-1-yl)-2-(pyridin-2-yl)cyclopropane-1-carboxamide<br>UPLC-MS (Method 1) m/z 369.2 $(M+Na)^+$, at 1.40 min. $^1$H NMR (500 MHz, DMSO-d6) δ 8.77 (s, 1H), 8.42 (ddd, *J* = 4.8, 1.8, 0.9 Hz, 1H), 7.66 (app td, *J* = 7.6, 1.9 Hz, 1H), 7.40 (app dt, *J* = 7.8, 1.1 Hz, 1H), 7.17 (ddd, *J* = 7.5, 4.8, 1.1 Hz, 1H), 7.14 - 7.07 (m, 4H), 2.46 - 2.38 (m, 1H), 2.20 (s, 6H), 2.12 - 2.04 (m, 1H), 1.60 - 1.49 (m, 2H), 1.33 (dd, *J* = 7.8, 6.3 Hz, 2H), 0.87 (t, *J* = 7.3 Hz, 3H). 2 protons not observed (under DMSO solvent peak).<br>The trans isomers were separated by chiral SFC on a Waters prep 15 with UV detection by DAD at 210 - 400 nm, 40 °C, 120 bar. The column was IG 10X250mm, 5um, flow rate 15mL/ min at 30 % MeOH (0.1% Ammonia), 70% $CO_2$ to afford:<br>First eluting isomer (Example 34), Chiral SFC (method 1) 2.19 min, >99% ee<br>Second eluting isomer (Example, 35) Chiral SFC (method 1) 3.04 min, >99% ee |
| 9 | 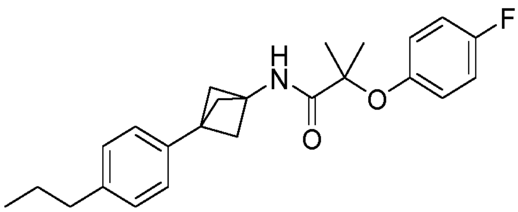 | 2-(4-fluorophenoxy)-2-methyl-N-(3-(4-propylphenyl)bicyclo[1.1.1] pentan-1-yl)propenamide<br>UPLC-MS (Method 1) m/z 404.2 $(M+Na)^+$, at 1.99 min. $^1$H NMR (500 MHz, DMSO-d6) δ 8.70 (s, 1H), 7.17 - 7.08 (m, 6H), 6.98 - 6.90 (m, 2H), 2.23 (s, 6H), 1.60 - 1.49 (m, 2H), 1.37 (s, 6H), 0.87 (t, *J* = 7.3 Hz, 3H). 2 protons not observed (under DMSO solvent peak). |
| 40 | 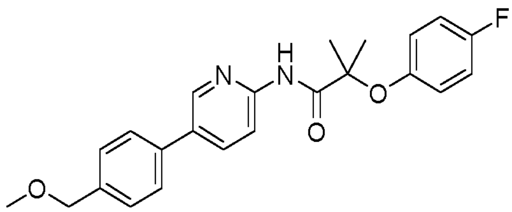 | 2-(4-fluorophenoxy)-*N*-(5-(4-(methoxymethyl) phenyl)pyridin-2-yl)-2-methylpropanamide<br>UPLC-MS (Method 1) m/z 395.4 $(M+H)^+$, at 1.78 min. $^1$H NMR (500 MHz, DMSO-d6) δ 10.02 (s, 1H), 8.67 (app t, J = 1.7 Hz, 1H), 8.16 (d, *J* = 1.7 Hz, 2H), 7.74 - 7.68 (m, 2H), 7.43 (d, *J* = 8.3 Hz, 2H), 7.19 - 7.11 (m, 2H), 7.08 - 7.00 (m, 2H), 4.46 (s, 2H), 3.31 (s, 3H), 1.53 (s, 6H). |

(continued)

| Example | Structure | Name / Analytical Data |
|---|---|---|
| 45 | 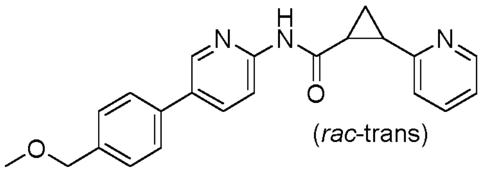 (*rac*-trans) | (*rac*-trans)-*N*-(5-(4-(methoxymethyl)phenyl)pyridin-2-yl)-2-(pyridin-2-yl)cyclopropane-1-carboxamide<br>UPLC-MS (Method 1) 360.3 $(M+H)^+$, at 1.01 min. $^1H$ NMR (500 MHz, Chloroform-d) δ 9.12 (s, 1H), 8.46 (d, *J = 2.3* Hz, 1H), 8.34 (d, *J* = 4.7 Hz, 1H), 8.30 (d, *J* = 8.7 Hz, 1H), 7.91 (dd, *J* = 8.6, 2.4 Hz, 1H), 7.57 - 7.49 (m, 3H), 7.42 (d, *J* = 8.0 Hz, 2H), 7.29 - 7.26 (m, 1H), 7.03 (dd, *J* = 7.2, 5.1 Hz, 1H), 4.51 (s, 2H), 3.42 (s, 3H), 2.74 - 2.66 (m, 1H), 2.39 - 2.32 (m, 1H), 1.78 - 1.70 (m, 1H), 1.70 - 1.61 (m, 1H). |

***Example 5: (rac-trans)-N-((2R,3R)-2-hydroxy-3-methoxybutyl)-N-(4'-(methoxymethyl)-[1,1'-biphenyl]-4-yl)-2-(pyridin-2-yl)cyclopropane-1-carboxamide***

**[0260]**

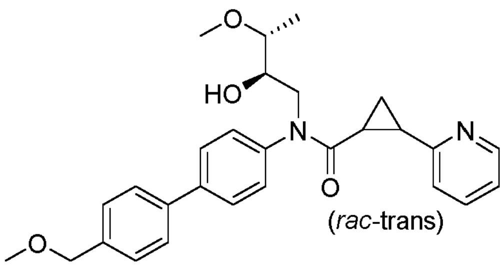

(*rac*-trans)

Step 1: 1-((R)-2,2-dimethyl-1,3-dioxolan-4-yl)ethan-1-ol

**[0261]** To a solution of (R)-2,2-dimethyl-1,3-dioxolane-4-carbaldehyde (2.00 g, 15.4 mmol) in $Et_2O$ (20 mL) cooled to _78 °C was added dropwise methylmagnesium bromide (10.2 mL, 3 molar, 30.7 mmol), the mixture was allowed to warm slowly to RT and stirred for 18 h. The reaction mixture was quenched by drop-wise addition of sat. aq. $NH_4Cl$ (15 mL) and stirred for 15 min. The mixture was extracted with EtOAc (3 × 50 mL), the combined organics were washed with brine (50 mL), dried ($MgSO_4$), filtered and concentrated under reduced pressure to afford a colourless oil. The crude product was purified by chromatography on silica gel (24 g cartridge, 0-50% EtOAc/isohexane) to afford title alcohol (2.06 g, 13 mmol, 87%, 95% purity, 3:1 mixture of diastereoisomers) as a colourless oil. NMR data for major isomer: $^1H$ NMR (500 MHz, DMSO-$d_6$) δ 4.75 (d, *J* = 5.3 Hz, 1H), 3.95 (dd, *J* = 8.0, 6.2 Hz, 1H), 3.76 (dd, *J* = 8.1, 6.0 Hz, 1H), 3.71 (dt, *J* = 7.3, 6.1 Hz, 1H), 3.52 - 3.42 (m, 1H), 1.30 (s, 3H), 1.25 (d, *J* = 0.7 Hz, 3H), 1.08 (d, *J* = 6.2 Hz, 3H).

Step 2: (2R)-3-methoxybutane-1,2-diol

**[0262]** To a solution of 1-((R)-2,2-dimethyl-1,3-dioxolan-4-yl)ethan-1-ol (2.05 g, 14.0 mmol) and MeI (1.32 mL, 21.0 mmol) in THF (20 mL) cooled to 0 °C was added portionwise NaH (617 mg, 60% Wt, 15.4 mmol). After stirring for 30 min, a solution of MeI (1.32 mL, 21.0 mmol) in THF (2.0 mL) was added drop-wise and the mixture was allowed to warm to RT and stirred for 18 h. The reaction mixture was quenched by addition of sat. aq. $NH_4Cl$ (50 mL) and extracted with EtOAc (3 × 25 mL). The combined organics were washed with brine (50 mL), dried ($MgSO_4$), filtered and concentrated under reduced pressure to afford a yellow oil. AcOH/water (4:1, 2.5 mL) was added and the mixture was stirred at RT for 2 h, then concentrated under reduced pressure and azeotroped with toluene (2 × 10 mL). The crude product was purified by chromatography on silica gel (24 g cartridge, 0-100% EtOAc/isohexane) to afford title diol (775 mg, 6.1 mmol, 44 %, 95% Purity, 3:1 mixture of diastereoisomers) as a yellow oil. NMR data for major isomer: $^1H$ NMR (500 MHz, DMSO-$d_6$) δ 3.98 (dd, *J* = 8.3, 6.6 Hz, 1H), 3.87 (q, *J* = 6.3 Hz, 1H), 3.71 (dd, *J* = 8.1, 6.1 Hz, 1H), 3.28 (s, 1H), 3.26 (s, 3H), 1.32 (d, *J* = 2.6 Hz, 3H), 1.26 (d, *J* = 2.9 Hz, 3H), 1.08 (d, *J* = 6.2 Hz, 3H).

Step 3: (2R,3R)-2-hydroxy-3-methoxybutyl 4-methylbenzenesulfonate

**[0263]** To a solution of (2R,3R)-3-methoxybutane-1,2-diol (290 mg, 2.41 mmol) in DCM (20 mL) and pyridine (2 ml) cooled to 0 °C was added drop-wise a solution of 4-methylbenzenesulfonyl chloride (506 mg, 2.66 mmol) in DCM (2.0 mL) and the mixture was stirred for 18 h. The mixture was diluted with 10 wt% aq. citric acid (50 mL) and extracted with DCM (2 × 25 mL). The combined organics were washed with water (20 ml), dried ($MgSO_4$), filtered and concentrated under reduced pressure. The crude product was purified by chromatography on silica gel (12 g cartridge, 0-100% EtOAc/isohexane) to afford (2R,3R)-2-hydroxy-3-methoxybutyl 4-methylbenzenesulfonate (150 mg, 0.54 mmol, 22 %, 98% purity, 3:1 mixture of diastereoisomers) as a colourless oil.

**[0264]** UPLC-MS (Method 1) m/z 274.9 $(M+H)^+$, at 1.15 min. NMR data for major isomer: $^1H$ NMR (500 MHz, DMSO-$d_6$) δ 7.79 (dd, *J* = 8.4, 1.9 Hz, 2H), 7.51 - 7.42 (m, 2H), 5.26 (d, *J* = 5.9 Hz, 1H), 4.02 (dd, *J* = 9.9, 2.9 Hz, 1H), 3.88 (dd, *J* = 9.8, 6.2 Hz, 1H), 3.44 (qd, *J* = 6.2, 2.8 Hz, 1H), 3.09-3.14 (s, 4H), 2.42 (s, 3H), 1.03 (s, 1H). 3H not observed (under DMSO solvent peak)

Step 4: (2R,3R)-3-methoxy-1-((4'-(methoxymethyl)-[1,1'-biphenyl]-4-yl)amino)butan-2-ol

**[0265]** A mixture of (2R,3R)-2-hydroxy-3-methoxybutyl 4-methylbenzenesulfonate (100 mg, 365 μmol), 4'-(methoxymethyl)-[1,1'-biphenyl]-4-amine (77.7 mg, 365 μmol), triethylamine (152 μL, 1.09 mmol) and potassium iodide (60.5 mg, 365 μmol) in acetonitrile (3.0 mL) was heated at 160 °C for 2 h under microwave irradiation. The mixture was then heated at 160 °C for a further 12 h. The mixture was partitioned between DCM (10 mL) and 0.5 M aq. HCl (10 mL), stirred for 15 min, passed through a hydrophobic frit and concentrated under reduced pressure to afford a brown gum. The crude product was purified by chromatography on silica gel (12 g cartridge, 0-50% EtOAc/isohexane) to afford title alcohol (10 mg, 32 μmol, 8.7%, 100% purity, 3:1 mixture of diastereoisomers) as a brown oil. UPLC-MS (Method 1) m/z 330.4 $(M+H)^+$, at 1.28 min. NMR data for major diastereoisomer: $^1H$ NMR (500 MHz, DMSO-d6) δ 9.68 (s, 1H), 7.84 - 7.78 (m, 2H), 7.62 (ddt, *J* = 7.2, 4.8, 2.1 Hz, 4H), 7.40 - 7.30 (m, 2H), 5.74 (dd, *J* = 6.5, 2.2 Hz, 1H), 4.44 (d, *J* = 2.2 Hz, 2H), 3.95 (dd, *J* = 6.5, 3.3 Hz, 1H), 3.76 - 3.68 (m, 3H), 3.35 (s, 1H), 3.22 (d, *J* = 1.7 Hz, 3H), 1.15 (dd, *J* = 6.5, 2.1 Hz, 3H).

Step 5: (rac-trans)-N-((2R,3R)-2-hydroxy-3-methoxybutyl)-N-(4'-(methoxymethyl)-[1,1'-biphenyl]-4-yl)-2-(pyridin-2-yl) cyclopropane-1-carboxamide

**[0266]** To a solution of (rac-trans)-2-(pyridin-2-yl)cyclopropane-1-carboxylic acid (9.3 mg, 57 μmol) and (2R,3R)-3-methoxy-1-((4'-(methoxymethyl)-[1,1'-biphenyl]-4-yl)amino)butan-2-ol (18 mg, 57 μmol) in acetonitrile (2.0 mL) was added HATU (22 mg, 57 μmol) followed by DIPEA (30 μL, 0.17 mmol) and the mixture was stirred at RT for 18 h. The mixture was diluted with sat. aq. $NH_4Cl$ (10 mL) and DCM (10 mL), stirred for 10 min, passed through a hydrophobic frit and concentrated under reduced pressure. The crude product was purified by chromatography on silica gel (4 g cartridge, 0-100% EtOAc/isohexane, product eluted at 100% EtOAc) to afford title amide (17.9 mg, 38.9 μmol, 68%, 100% purity, mixture of 4 diastereoisomers 3:3:1:1) as a colourless solid. UPLC-MS (Method 1) m/z 461.7 $(M+H)^+$, at 1.22 min.

***Example 10: 2-(4-fluorophenoxy)-2-methyl-N-(4'-(trifluoromethyl)-[1,1'-biphenyl]-4- yl)propenamide***

**[0267]**

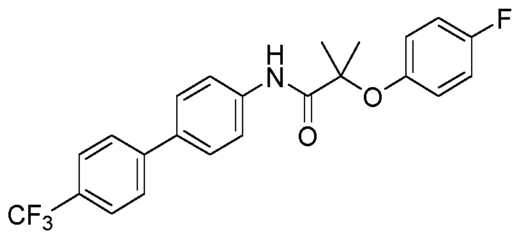

Step 1: 2-(4-fluorophenoxy)-N-(4-iodophenyl)-2-methylpropanamide

**[0268]** To a stirred solution of 2-(4-fluorophenoxy)-2-methylpropanoic acid (268 mg, 1.35 mmol) and 4-iodoaniline (326 mg, 1.49 mmol) in MeCN (5.0 mL) under a nitrogen atmosphere was added N-ethyl-N-isopropylpropan-2-amine (0.71 mL, 4.1 mmol) followed by HATU (617 mg, 1.62 mmol). The reaction mixture was stirred for 5 hours at 50 °C. The reaction mixture was cooled to RT, diluted with DCM (5 mL) and sat. aq. $NaHCO_3$ (2 mL), stirred for 10 min then passed through a hydrophobic frit. 10 wt% aqueous citric acid (2 mL) was added to the organics, stirred for 10 min, the mixture was passed

through a hydrophobic frit and concentrated under reduced pressure to afford a brown oil. The crude product was purified by chromatography on silica gel (40 g cartridge, 0-50% EtOAc/isohexane) to afford 2-(4-fluorophenoxy)-N-(4-iodophenyl)-2-methyl propanamide (490 mg, 1.2 mmol, 90%, 99% purity) as a light brown solid. LC-MS (Method 3) m/z 400.1 $(M+H)^+$, at 2.73 min. $^1H$ NMR (500 MHz, Chloroform-d) δ 8.62 (s, 1H), 7.80 - 7.58 (m, 2H), 7.52 - 7.30 (m, 2H), 7.17 - 6.83 (m, 4H), 1.53 (s, 6H).

Step 2: 2-(4-fluorophenoxy)-2-methyl-N-(4'-(trifluoromethyl)-[1,1'-biphenyl]-4-yl) propenamide

**[0269]** 2-(4-fluorophenoxy)-*N*-(4-iodophenyl)-2-methylpropanamide (51 mg, 0.13 mmol), (4-(trifluoromethyl)phenyl) boronic acid (36 mg, 0.19 mmol), potassium phosphate (67 mg, 0.32 mmol), and $Pd(dppf)Cl_2$ (9.3 mg, 13 μmol) were dissolved in 1,4-dioxane (5.0 mL) and water (0.5 mL). The reaction mixture was purged with nitrogen and heated to 95 °C for 3 h. The reaction mixture was filtered through celite and the cake was washed with EtOAc (20 mL). The filtrate was concentrated under reduced pressure and crude product was purified by chromatography on silica gel (24 g cartridge, 0-50% EtOAc/isohexane) to afford title amide (48 mg, 0.11 mmol, 90%, 99% purity) as a pale brown solid. LC-MS (Method 3) m/z 418.2 $(M+H)^+$, at 3.00 min. $^1H$ NMR (500 MHz, Chloroform-d) δ 8.71 (s, 1H), 7.73 (d, *J* = 1.8 Hz, 1H), 7.70 (s, 5H), 7.65 - 7.58 (m, 2H), 7.07 - 6.95 (m, 4H), 1.57 (s, 6H).

**[0270]** The following examples were prepared by methods analogous to Example 10, substituting appropriate starting materials and intermediates where necessary:

| Example | Structure | Name / Analytical Data |
|---|---|---|
| 11 | 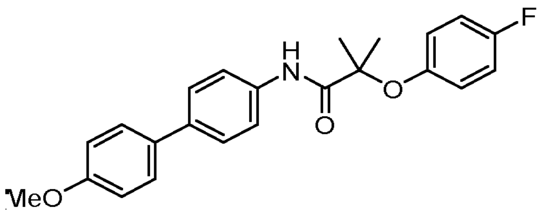 | 2-(4-fluorophenoxy)-N-(4'-methoxy-[1,1'-biphenyl]-4-yl)-2-methylpropanamide<br>LC-MS (Method 3) m/z 380.2 $(M+H)^+$, at 2.76 min. $^1H$ NMR (500 MHz, Chloroform-d) δ 8.63 (s, 1H), 7.71 - 7.61 (m, 2H), 7.58 - 7.49 (m, 4H), 7.04 - 6.92 (m, 6H), 3.85 (s, 3H), 1.56 (s, 6H). |
| 12 | 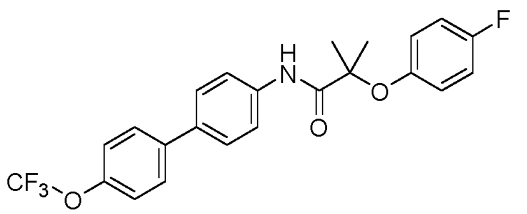 | 2-(4-fluorophenoxy)-2-methyl-N-(4'-(trifluoromethoxy)-[1,1'-biphenyl]-4-yl)propanamide<br>LC-MS (Method 3) m/z 434.2 $(M+H)^+$, at 3.02 min. $^1H$ NMR (500 MHz, Chloroform-d) δ 8.68 (s, 1H), 7.76 - 7.64 (m, 2H), 7.63 - 7.47 (m, 4H), 7.31 - 7.26 (m, 2H), 7.10 - 6.88 (m, 4H), 1.57 (s, 6H). |
| 16 | 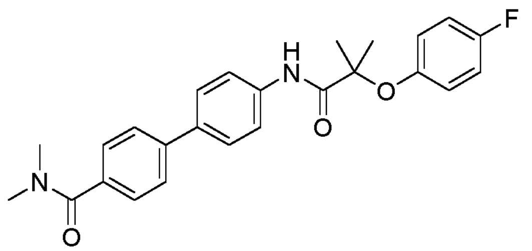 | 4'-(2-(4-fluorophenoxy)-2-methylpropanamido)-*N,N*-dimethyl-[1,1'-biphenyl]-4-carboxamide<br>LC-MS (Method 3) m/z 421.3 $(M+H)^+$, at 2.36 min. $^1H$ NMR (500 MHz, Methanol-d4) δ 7.78 - 7.65 (m, 6H), 7.58 - 7.50 (m, 2H), 7.06 (d, *J* = 6.4 Hz, 4H), 3.15 - 3.08 (m, 6H), 1.61 (s, 6H). One exchangeable proton not observed |
| 17 | 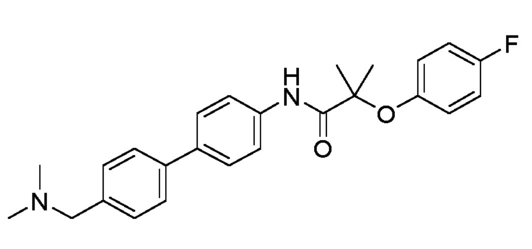 | *N*-(4'-((dimethylamino)methyl)-[1,1'-biphenyl]-4-yl)-2-(4-fluorophenoxy)-2-methylpropanamide<br>LC-MS (Method 3) m/z 407.3 $(M+H)^+$, at 1.62 min. $^1H$ NMR (500 MHz, Chloroform-d) δ 8.68 - 8.51 (m, 1H), 7.70 (d, *J* = 8.5 Hz, 2H), 7.65 - 7.55 (m, 4H), 7.50 (d, *J* = 8.0 Hz, 2H), 7.06 - 6.93 (m, 4H), 3.92 (s, 2H), 2.57 (s, 6H), 1.57 (s, 6H). |

(continued)

| Example | Structure | Name / Analytical Data |
|---|---|---|
| 24 | 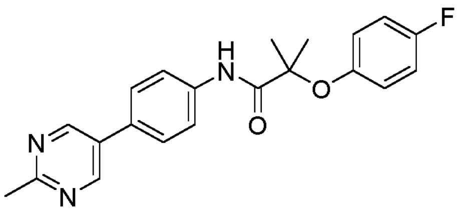 | 2-(4-fluorophenoxy)-2-methyl-N-(4-(2-methylpyrimidin-5-yl) phenyl) propenamide<br>LC-MS (Method 3) m/z 366.2 $(M+H)^+$, at 2.17 min. $^1H$ NMR (500 MHz, Chloroform-d) δ 8.82 (s, 2H), 8.75 (s, 1H), 7.75 (d, *J* = 8.6 Hz, 2H), 7.54 (d, *J* = 8.6 Hz, 2H), 7.06 - 6.91 (m, 4H), 2.77 (s, 3H), 1.56 (s, 6H). |
| 25 | 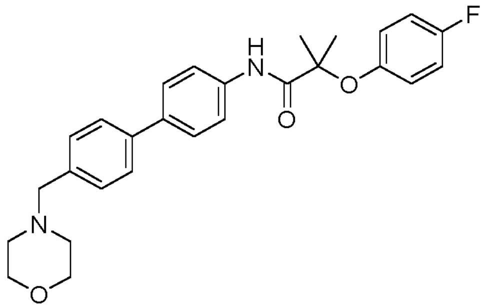 | 2-(4-fluorophenoxy)-2-methyl-N-(4'-(morpholinomethyl)-[1,1'-biphenyl]-4-yl)propenamide<br>LC-MS (Method 3) m/z 449.3 $(M+H)^+$, at 1.65 min. $^1H$ NMR (500 MHz, Methanol-d4) δ 7.74 - 7.66 (m, 2H), 7.68 - 7.54 (m, 4H), 7.44 - 7.40 (m, 2H), 7.06 (dd, *J* = 6.4, 1.3 Hz, 4H), 3.72 (app t, *J* = 4.6 Hz, 4H), 3.57 (s, 2H), 2.50 (br s, 4H), 1.60 (s, 6H). One exchangeable proton not observed |
| 26 | 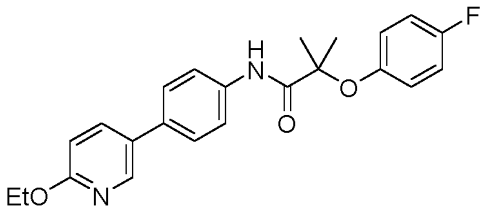 | *N*-(4-(6-ethoxypyridin-3-yl)phenyl)-2-(4-fluorophenoxy)-2-methylpropanamide<br>LC-MS (Method 3) m/z 395.2 $(M+H)^+$, at 2.74 min. $^1H$ NMR (500 MHz, Chloroform-d) δ 8.66 (s, 1H), 8.35 (d, *J* = 2.6 Hz, 1H), 7.77 (dd, *J* = 8.6, 2.6 Hz, 1H), 7.73 - 7.64 (m, 2H), 7.57 - 7.47 (m, 2H), 7.08 - 6.95 (m, 4H), 6.79 (d, *J* = 8.6 Hz, 1H), 4.40 (q, *J* = 7.0 Hz, 2H), 1.54 (s, 6H), 1.42 (t, *J* = 7.0 Hz, 3H). |
| 27 | 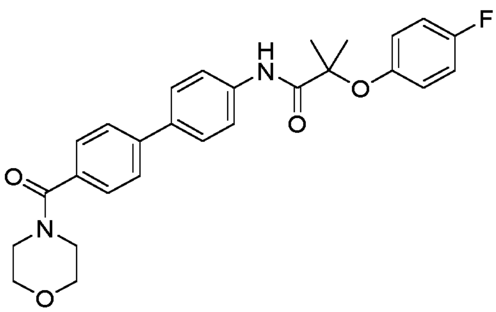 | 2-(4-fluorophenoxy)-2-methyl-*N*-(4'-(morpholine-4-carbonyl)-[1,1'-biphenyl]-4-yl)propenamide<br>UPLC-MS (Method 2) m/z 463.3 $(M+H)^+$, at 2.33 min. $^1H$ NMR (500 MHz, Chloroform-d) δ 8.69 (s, 1H), 7.77 - 7.65 (m, 2H), 7.65 - 7.51 (m, 2H), 7.60 - 7.56 (m, 2H), 7.51 - 7.44 (m, 2H), 7.07 - 6.89 (m, 4H), 3.73 - 3.55 (m, 8H), 1.57 (s, 6H). |
| 30 | 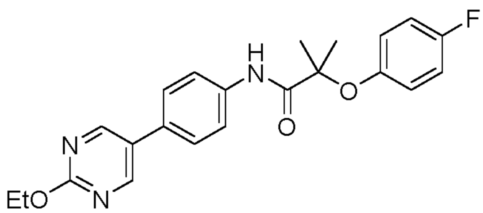 | *N*-(4-(2-ethoxypyrimidin-5-yl)phenyl)-2-(4-fluorophenoxy)-2-methylpropanamide<br>LC-MS (Method 3) m/z 396.3 $(M+H)^+$, at 2.49 min. $^1H$ NMR (500 MHz, Chloroform-d) δ 8.70 (br s, 3H), 7.81 - 7.65 (m, 2H), 7.57 - 7.41 (m, 2H), 7.09 - 6.93 (m, 4H), 4.46 (q, *J* = 7.1 Hz, 2H), 1.56 (s, 6H), 1.45 (t, *J* = 7.1 Hz, 3H). |

(continued)

| Example | Structure | Name / Analytical Data |
|---|---|---|
| 36 | 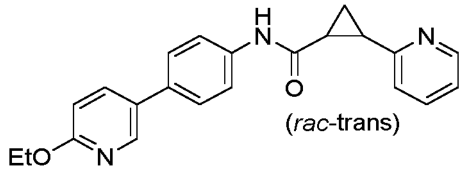 (*rac*-trans) | (*rac*-trans)-*N*-(4-(6-ethoxypyridin-3-yl)phenyl)-2-(pyridin-2-yl)cyclopropane-1-carboxamide<br>LC-MS (Method 3) m/z 360.2 $(M+H)^+$, at 1.66 min. $^1H$ NMR (500 MHz, Chloroform-d) δ 8.47 (d , *J* = 5.1 Hz, 1H), 8.33 (d, *J* = 2.3 Hz, 1H), 7.74 (dd, *J* = *8.6,* 2.6 Hz, 2H), 7.65 - 7.54 (m, 3H), 7.45 (d, *J* = 8.7 Hz, 2H), 7.30 (d, *J* = 7.8 Hz, 1H), 7.11 (dd, *J* = 7.4, 5.1 HZ, 1H), 6.77 (d, *J* = 8.5 Hz, 1H), 4.38 (q, *J* = 7.1 Hz, 2H), 2.77 - 2.66 (m, 1H), 2.35 - 2.22 (m, 1H), 1.74 (m, 1H), 1.61 (m, 1H), 1.41 (t, *J* = 7.1 Hz, 3H). |
| 37 | 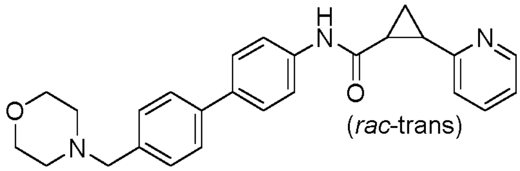 (*rac*-trans) | (*rac*-trans)-*N*-(4'-(morpholinomethyl)-[1,1'-biphenyl]-4-yl)-2-(pyridin-2-yl)cyclopropane-1-carboxamide<br>LC-MS (Method 3) m/z 414.3 $(M+H)^+$, at 0.93 min. $^1H$ NMR (500 MHz, Chloroform-d) δ 8.45 (d, *J* = 4.9 Hz, 1H), 7.81 - 7.46 (m, 7H), 7.40 (d, *J* = 7.8 Hz, 2H), 7.29 (d, *J* = 7.8 Hz, 1H), 7.10 (dd, *J* = 7.5, 4.8 Hz, 1H), 3.74 (app t, *J* = 4.5 Hz, 4H), 3.58 (s, 2H), 2.68 (m, 1H), 2.49 (br s, 4H), 2.25 (m, 1H), 1.73 (ddd, *J* = 9.0. 5.3, 3.6 Hz, 1H), 1.62 (ddd, *J* = 8.3, 6.0, 3.7 Hz, 1H). One exchangeable proton not observed |
| 38 | 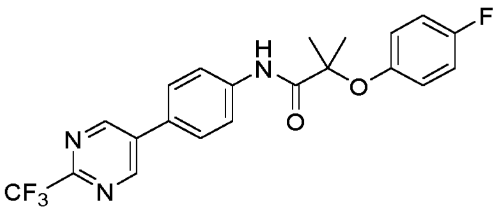 | 2-(4-fluorophenoxy)-2-methyl-*N*-(4-(2-(trifluoromethyl)pyrimidin-5-yl) phenyl) propenamide<br>LC-MS (Method 3) m/z 420.2 $(M+H)^+$, at 2.66 min. $^1H$ NMR (500 MHz, Methanol-d4) δ 9.27 (s, 2H), 7.96 - 7.88 (m, 2H), 7.86 - 7.79 (m, 2H), 7.11 - 7.01 (m, 4H), 1.61 (s, 6H). One exchangeable proton not observed |
| 41 | 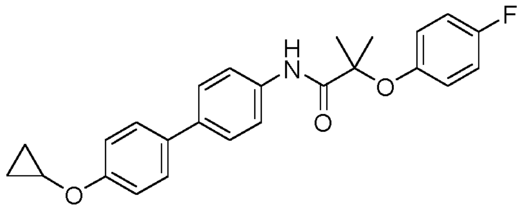 | *N*-(4'-cyclopropoxy-[1,1'-biphenyl]-4-yl)-2-(4-fluorophenoxy)-2-methylpropanamide<br>LC-MS (Method 3) m/z 406.3 $(M+H)^+$, at 2.93 min. $^1H$ NMR (500 MHz, Chloroform-d) δ 8.65 (s, 1H), 7.66 (d, *J* = 8.6 Hz, 2H), 7.57 - 7.53 (m, 2H), 7.53 - 7.49 (m, 2H), 7.16 - 7.06 (m, 2H), 7.07 - 6.93 (m, 4H), 3.82 - 3.72 (m, 1H), 1.57 (s, 6H), 0.83 - 0.77 m, 4H). |
| 42 | 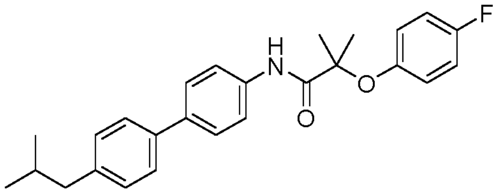 | 2-(4-fluorophenoxy)-*N*-(4'-isobutyl-[1,1'-biphenyl]-4-yl)-2-methylpropan amide<br>LC-MS (Method 3) m/z 406.3 $(M+H)^+$, at 3.20 min. $^1H$ NMR (500 MHz, Chloroform-d) δ 8.65 (s, 1H), 7.67 (d, *J* = 8.7 Hz, 2H), 7.59 (d, *J* = 8.7 Hz, 2H), 7.50 (d, *J* = 8.2 Hz, 2H), 7.21 (d, *J* = 8.2 Hz, 2H), 7.06 - 6.96 (m, 4H), 2.52 (d, *J* = 7.2 Hz, 2H), 1.95 - 1.86 (m, 1H), 1.57 (s, 6H), 0.94 (d, *J* = 6.6 Hz, 6H). |

(continued)

| Example | Structure | Name / Analytical Data |
|---|---|---|
| 46 | 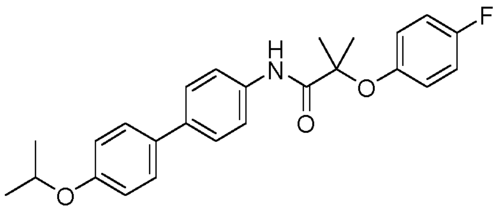 | 2-(4-fluorophenoxy)-*N*-(4'-isopropoxy-[1,1'-biphenyl]-4-yl)-2-methylpropanamide LC-MS (Method 3) m/z 408.3 $(M+H)^+$, at 2.98 min. $^1H$ NMR (500 MHz, Chloroform-d) δ 8.64 (s, 1H), 7.71 - 7.61 (m, 2H), 7.58 - 7.52 (m, 2H), 7.51 - 7.43 (m, 2H), 7.08 - 6.87 (m, 6H), 4.61 - 4.56 (m, 1H), 1.57 (s, 6H), 1.37 (d, *J* = 6.0 Hz, 6H). |
| 47 | 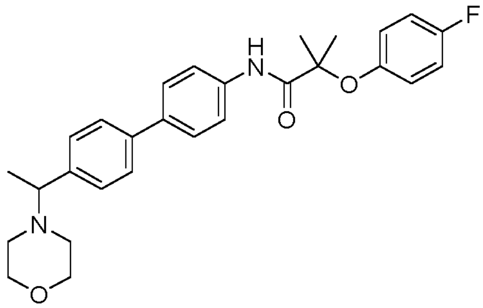 | 2-(4-fluorophenoxy)-2-methyl-*N*-(4'-(1-morpholinoethyl)-[1,1'-biphenyl]-4-yl)propanamide LC-MS (Method 3) m/z 463.3 $(M+H)^+$, at 1.69 min. $^1H$ NMR (500 MHz, Chloroform-d) δ 8.67 (s, 1H), 7.68 (d, *J* = 8.6 Hz, 2H), 7.58 (d, *J* = 8.6 HZ, 2H), 7.54 (d, *J* = 8.3 Hz, 2H), 7.39 (d, *J* = 7.8 Hz, 2H), 7.11 - 6.90 (m, 4H), 3.73 (br s, 4H), 3.39 (br s, 1H), 2.55 - 2.44 (m, 4H), 1.56 (s, 6H), 1.42 (br s, 3H). |
| 48 | 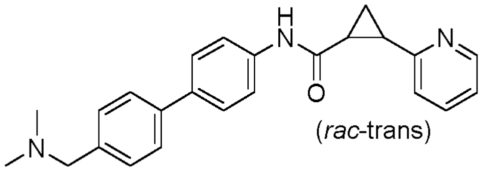 (*rac*-trans) | (*rac*-trans)-*N*-(4'-((dimethylamino)methyl)-[1,1'-biphenyl]-4-yl)-2-(pyridin-2-yl)cyclopropane-1-carboxamide LC-MS (Method 3) m/z 372.2 $(M+H)^+$, at 0.88 min. $^1H$ NMR (500 MHz, Methanol-d4) δ 8.52 - 8.39 (m, 1H), 7.80 - 7.69 (m, 5H), 7.64 (d, *J* = 8.9 Hz, 2H), 7.53 (d, *J* = 8.0 Hz, 2H), 7.40 (dt, J= 7.9, 1.1 Hz, 1H), 7.25 (ddd, *J* = 7.6, 5.0, 1.1 Hz, 1H), 4.08 (s, 2H), 2.69 (br s, 6H), 2.68 - 2.63 (m, 1H) 2.39 (ddd, *J* = 8.4, 5.5, 3.9 Hz, 1H), 1.70 - 1.65 (m, 1H), 1.65 - 1.58 (m, 1H). One exchangeable proton not observed |
| 49 | 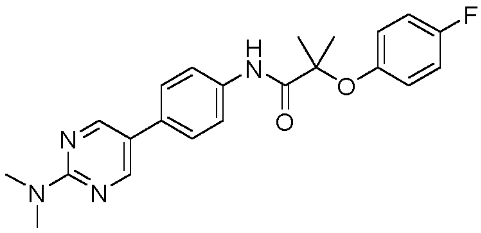 | *N*-(4-(2-(dimethylamino)pyrimidin-5-yl)phenyl)-2-(4-fluorophenoxy)-2-methylpropanamide UPLC-MS (Method 2) m/z 395.3 $(M+H)^+$, at 2.44 min. $^1H$ NMR (500 MHz, Chloroform-d) δ 8.64 (s, 1H), 8.54 (s, 2H), 7.67 (d, *J* = 8.9 Hz, 2H), 7.46 (d, *J* = 8.9 Hz, 2H), 7.06 - 6.93 (m, 4H), 3.24 (s, 6H), 1.56 (s, 6H). |
| 72 | 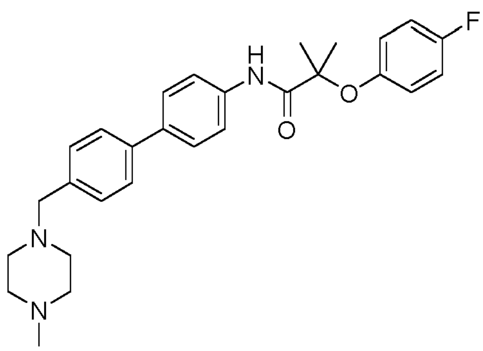 | 2-(4-fluorophenoxy)-2-methyl-*N*-(4'-((4-methylpiperazin-1-yl)methyl)-[1,1'-biphenyl]-4-yl)propanamide LC-MS (Method 3) m/z 462.3 $(M+H)^+$, at 1.65 min. $^1H$ NMR (500 MHz, DMSO-d6) δ 10.10 (s, 1H), 8.16 (s, 1H), 7.85 - 7.75 (m, 2H), 7.65 - 7.54 (m, 4H), 7.41 - 7.30 (m, 2H), 7.20 - 7.09 (m, 2H), 7.04 - 6.93 (m, 2H), 3.47 (s, 3H), 2.16 (s, 9H), 1.52 (s, 6H). |

***Example 13: 2-(4-fluorophenoxy)-N-(4'-(methoxymethyl)-[1,1'-biphenyl]-4-yl)-N,2-dimethylpropanamide***

**[0271]**

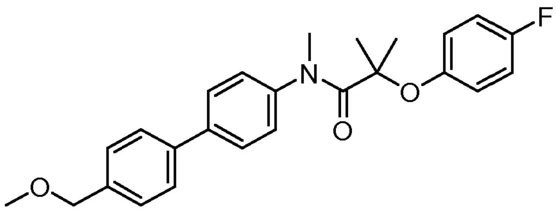

**[0272]** To a solution of 2-(4-fluorophenoxy)-*N*-(4'-(methoxymethyl)-[1,1'-biphenyl]-4-yl)-2-methylpropanamide (Example 2) (30 mg, 76 μmol) in THF (2.0 mL) at 0 °C was added NaH (3.7 mg, 60 wt%, 91 μmol). The reaction mixture was warmed to RT and after 20 min iodomethane (6.2 μL, 99 μmol) was added. After stirring for 90 min the mixture was heated at 50 °C and stirred for a further 16 h. After cooling to RT, MeOH (1.0 mL) was added and the mixture was concentrated under reduced pressure. The crude product was purified by chromatography on silica gel (24 g cartridge, 0-50% EtOAc/isohexane) to afford 2-(4-fluorophenoxy)-*N*-(4'-(methoxymethyl)-[1,1'-biphenyl]-4-yl)-N,2-dimethyl propenamide (14 mg, 33 μmol, 43%, 95% purity) as a pale yellow solid.

**[0273]** LC-MS (Method 3) m/z 408.2 $(M+H)^+$, at 2.75 min. $^1H$ NMR (500 MHz, Chloroform-d) δ 7.55 (d, *J* = 8.3 Hz, 2H), 7.57 - 7.45 (m, 2H), 7.41 (d, *J* = 8.3 Hz, 2H), 7.20 - 7.00 (m, 2H), 7.00 - 6.87 (m, 2H), 6.86 - 6.39 (m, 2H), 4.50 (s, 2H), 3.42 (s, 3H), 3.38 (br s, 3H), 1.59 (br s, 6H).

**[0274]** The following examples were prepared by methods analogous to Example 13, substituting appropriate starting materials and intermediates where necessary:

| Example | Structure | Name / Analytical Data |
|---|---|---|
| 14 | 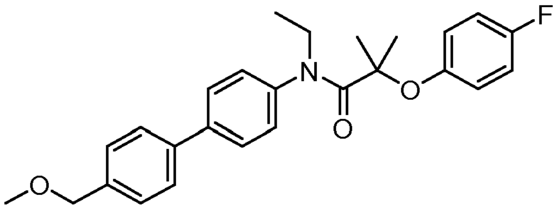 | *N*-ethyl-2-(4-fluorophenoxy)-*N*-(4'-(methoxymethyl)-[1,1'-biphenyl]-4-yl)-2-methylpropanamide<br>UPLC-MS (Method 3) m/z 422.3 $(M+H)^+$, at 2.87 min. $^1H$ NMR (500 MHz, Chloroform-d) δ 7.68 - 7.39 (m, 2H), 7.55 (d, *J* = 8.2 Hz, 2H), 7.40 (d, *J* = 8.2 Hz, 2H), 7.15 - 6.96 (m, 2H), 6.96 - 6.85 (m, 2H), 6.76 - 6.43 (m, 2H), 4.50 (s, 2H), 3.75 (br s, 2H), 3.42 (s, 3H), 1.82 - 1.43 (m, 6H), 1.15 (br s, 3H). |
| 15 | 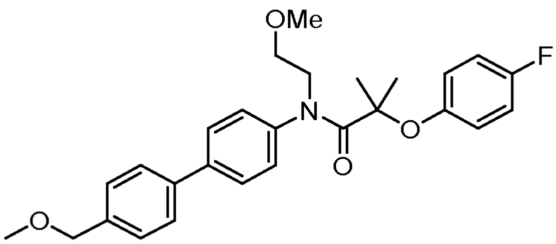 | 2-(4-fluorophenoxy)-*N*-(2-methoxyethyl)-N-(4'-(methoxymethyl)-[1,1'-biphenyl]-4-yl)-2-methylpropanamide<br>UPLC-MS (Method 3) m/z 452.3 $(M+H)^+$, at 2.76 min. $^1H$ NMR (500 MHz, Chloroform-d) δ 7.55 (d, *J* = 8.1 Hz, 2H), 7.50 - 7.42 (m, 2H), 7.41 (d, *J* = 8.1 Hz, 2H), 7.12 - 7.03 (m, 2H), 6.97 - 6.86 (m, 2H), 6.77 - 6.52 (m, 2H), 4.50 (s, 2H), 4.01 - 3.85 (m, 2H), 3.63 - 3.48 (m, 2H), 3.42 (s, 3H), 3.36 - 3.25 (m, 3H), 1.55 - 1.45 (m, 6H). |
| 22 | 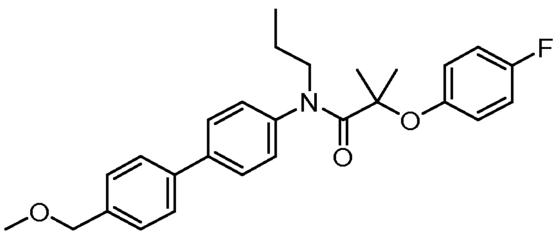 | 2-(4-fluorophenoxy)-N-(4'-(methoxymethyl)-[1,1'-biphenyl]-4-yl)-2-methyl-N-propylpropanamide<br>LC-MS (Method 3) m/z 436.3 $(M+H)^+$, at 3.03 min. $^1H$ NMR (500 MHz, Chloroform-d) δ 7.55 (d, *J* = 8.4 Hz, 2H), 7.45 (br s , 2H), 7.44 (d, *J* = 8.4 Hz, 2H), 7.15 - 6.95 (m, 2H), 6.96 - 6.86 (m, 2H), 6.75 - 6.43 (m, 2H), 4.50 (s, 2H), 4.06 - 3.54 (m, 2H), 3.42 (s, 3H), 1.85 - 1.41 (m, 8H), 0.96 - 0.69 (m, 3H). |

(continued)

| Example | Structure | Name / Analytical Data |
|---|---|---|
| 23 | 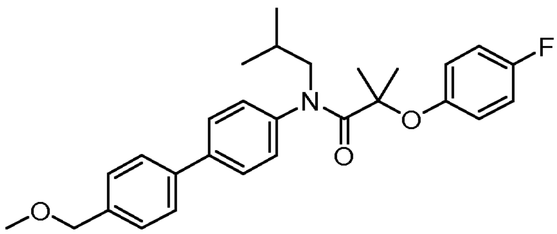 | 2-(4-fluorophenoxy)-*N*-isobutyl-*N*-(4'-(methoxymethyl)-[1,1'-biphenyl]-4-yl)-2-methylpropanamide<br>LC-MS (Method 3) m/z 450.3 $(M+H)^+$, at 3.13 min. $^1H$ NMR (500 MHz, Chloroform-d) δ 7.55 ( d, *J* = 8.2 Hz, 2H), 7.44 (br s, 2H), 7.41 (d, *J* = 8.2 Hz, 2H), 7.12 - 6.96 (m, 2H), 6.96 - 6.84 (m, 2H), 6.66 - 6.45 (m, 2H), 4.50 (s, 2H), 3.73 - 3.50 (m, 2H), 3.42 (s, 3H), 1.92 - 1.71 (m, 1H), 1.69 - 1.44 (m, 6H), 1.00 - 0.75 (m, 6H). |
| 28 | 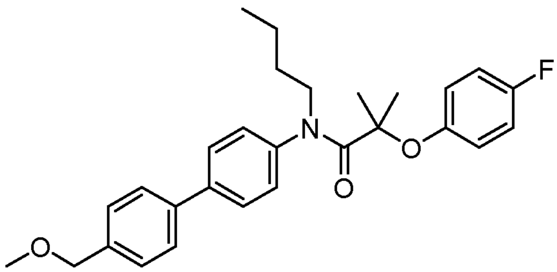 | *N*-butyl-2-(4-fluorophenoxy)-*N*-(4'-(methoxymethyl)-[1,1'-biphenyl]-4-yl)-2-methylpropanamide<br>LC-MS (Method 3) m/z 450.4 $(M+H)^+$, at 2.04 min. $^1H$ NMR (500 MHz, DMSO-d6) δ 7.65 (d, *J* = 8.1 Hz, 2H), 7.60 (br s , 2H), 7.40 (d, *J* = 8.1 Hz, 2H), 7.19 - 7.02 (m, 4H), 6.87 - 6.48 (m, 2H), 4.45 (s, 2H), 4.01 - 3.49 (m, 2H), 1.70 - 1.24 (m, 8H), 1.28 - 1.10 (m, 2H), 0.94 - 0.62 (m, 3H). 3 protons not observed, obscured by water signal. |
| 29 | 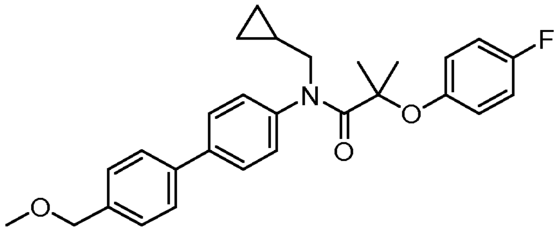 | *N*-(cyclopropylmethyl)-2-(4-fluorophenoxy)-*N*-(4'-(methoxymethyl)-[1,1'-biphenyl]-4-yl)-2-methylpropanamide<br>LC-MS (Method 3) m/z 448.3 $(M+H)^+$, at 1.97 min. $^1H$ NMR (500 MHz, DMSO-d6) δ 7.66 (d, *J* = 8.0 Hz, 2H), 7.60 (br s, 2H), 7.40 (d, *J* = 8.0 Hz, 2H), 7.22 - 7.06 (m, 4H), 6.90 - 6.54 (m, 2H), 4.45 (s, 2H), 3.93 - 3.41 (m, 2H), 3.31 (s, 3H), 1.76 - 1.28 (m, 6H), 1.06 - 0.75 (m, 1H), 0.60 - 0.17 (m, 2H), 0.22 - 0.01 (m, 2H). |
| 73 & 74 | 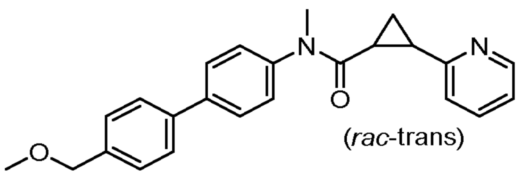<br>(*rac*-trans) | (*rac*-trans)-*N*-(4'-(methoxymethyl)-[1,1'-biphenyl]-4-yl)-*N*-methyl-2-(pyridin-2-yl)cyclopropane-1-carboxamide<br>UPLC-MS (Method 2) 373.2 $(M+H)^+$, at 1.41 min. $^1H$ NMR (500 MHz, DMSO-d6) δ 8.25 (s, 1H), 7.67 - 7.60 (m, 5H), 7.42 - 7.34 (m, 5H), 7.13 - 7.07 (m, 1H), 4.45 (s, 2H), 3.31 (s, 3H), 3.25 (s, 3H), 2.58 (ddd, *J* = 8.5, 5.8, 3.9 Hz, 1H), 1.94 (br s, 1H), 1.47 (ddd, *J* = 8.5, 5.4, 3.1 Hz, 1H), 1.33 (br s, 1H).<br>The trans isomers were separated by preparative chiral SFC to afford:<br>First eluting isomer (Example 73), Chiral SFC (method 2) 3.16 min, >99% ee<br>Second eluting isomer (Example, 74) Chiral SFC (method 2) 3.83 min, >99% ee |

***Example 20: 2-(4-fluorophenoxy)-2-methyl-N-(4'-methyl-[1,1'-biphenyl]-4-yl)-N-propylpropanamide***

**[0275]**

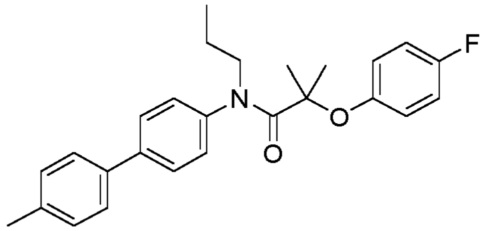

Step 1: 2-(4-fluorophenoxy)-*N*-(4'-(methoxymethyl)-[1,1'-biphenyl]-4-yl)-2-methyl-*N*-(2-methylallyl)propenamide

**[0276]** The title compound was prepared by methods analogous to Example 13, substituting appropriate starting materials and intermediates where necessary to afford 2-(4-fluorophenoxy)-*N*-(4'-(methoxymethyl)-[1,1'-biphenyl]-4-yl)-2-methyl-*N*-(2-methylallyl) propanamide (63 mg, 0.13 mmol, 88%, 95% purity) as a pale yellow oil. LC-MS (Method 3) m/z 434.3 $(M+H)^+$, at 2.90 min. $^1$H NMR (500 MHz, Chloroform-d) δ 7.61 - 7.52 (m, 2H), 7.53 - 7.36 (m, 4H), 7.13 - 6.96 (m, 2H), 6.96 - 6.83 (m, 2H), 6.76 - 6.46 (m, 2H), 6.07 - 5.74 (m, 1H), 5.31 - 4.96 (m, 2H), 4.50 (s, 2H), 4.43 - 4.06 (m, 2H), 3.42 (s, 3H), 1.84 - 1.40 (m, 6H).

Step 2: *N*-propyl-2-(4-fluorophenoxy)-*N*-(4'-(methoxymethyl)-[1,1'-biphenyl]-4-yl)-2-methylpropanamide

**[0277]** To a vial containing palladium (20 mg, 5wt%, 9.2 μmol) was added EtOH (ca 0.5 mL) followed by careful addition of a solution of *N*-allyl-2-(4-fluorophenoxy)-*N*-(4'-(methoxymethyl)-[1,1'-biphenyl]-4-yl)-2-methylpropanamide (40 mg, 92 μmol) in EtOH (3.0 mL), then stirred under an atmosphere of hydrogen 1.0 bar at RT. After 2 h the reaction mixture was filtered through a pad of celite and filtrate was concentrated to afford 2-(4-fluorophenoxy)-2-methyl-*N*-(4'-methyl-[1,1'-biphenyl]-4-yl)-*N*-propylpropanamide (37 mg, 94%, 95% purity) as a beige solid. LC-MS (Method 3) m/z 406.3 $(M+H)^+$, at 3.16 min. $^1$H NMR (500 MHz, Chloroform-d) δ 7.51 - 7.38 (m, 4H), 7.26 - 7.22 (m, 2H), 7.06 - 6.94 (m, 2H), 6.94 - 6.83 (m, 2H), 6.72 - 6.45 (m, 2H), 3.74 - 3.54 (m, 2H), 2.40 (s, 3H), 0.95 - 0.76 (m, 3H). 8 aliphatic protons obscured by $H_2O$ signal.

***Example 21: 2-(4-fluorophenoxy)-N-isobutyl-2-methyl-N-(4'-methyl-[1,1'-biphenyl]-4-yl)-2-methylpropanamide***

**[0278]**

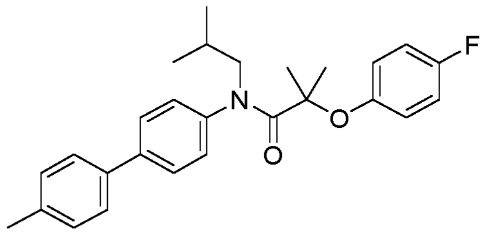

**[0279]** The title compound was prepared by methods analogous to Example 20, substituting appropriate starting materials and intermediates where necessary to afford:

Step 1: 2-(4-fluorophenoxy)-*N*-(4'-(methoxymethyl)-[1,1'-biphenyl]-4-yl)-2-methyl-*N*-(2-methylallyl)propanamide as a pale yellow oil. LC-MS (Method 3) m/z 448.3 $(M+H)^+$, at 2.99 min. $^1$H NMR (500 MHz, Chloroform-d) δ 7.57 - 7.51 (m, 2H), 7.46 - 7.38 (m, 4H), 7.07 - 6.97 (m, 2H), 6.94 - 6.87 (m, 2H), 6.68 - 6.49 (m, 2H), 4.84 - 4.80 (m, 1H), 4.69 (s, 1H), 4.50 (s, 2H), 4.43 - 4.21 (m, 2H), 3.42 (s, 3H), 1.84 - 1.67 (m, 3H), 1.67 - 1.51 (m, 6H).

Step 2: 2-(4-fluorophenoxy)-N-isobutyl-2-methyl-N-(4'-methyl-[1,1'-biphenyl]-4-yl)-2-methylpropanamide as a beige solid. LC-MS (Method 3) m/z 420.3 $(M+H)^+$, at 3.21 min. $^1$H NMR (500 MHz, Chloroform-d) δ 7.50 - 7.36 (m, 4H), 7.25 (d, *J* = 7.9 Hz, 2H), 7.07 - 6.95 (m, 2H), 6.95 - 6.84 (m, 2H), 6.63 - 6.46 (m, 2H), 3.68 - 3.47 (m, 2H), 2.40 (s, 3H), 1.89 - 1.72 (m, 1H), 0.98 - 0.82 (m, 6H). 6 aliphatic protons obscured by $H_2O$ signal.

***Example 31: 2-(4-fluorophenoxy)-1-(6-(4-(methoxymethyl)phenyl)-3,4-dihydroquinolin-1(2H)-yl)-2-methylpropan-1-one***

**[0280]**

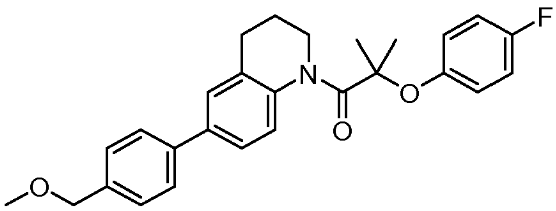

Step 1: 6-(4-(methoxymethyl)phenyl)-1,2,3,4-tetrahydroquinoline

**[0281]** To a solution of 6-bromo-1,2,3,4-tetrahydroquinoline (200 mg, 943 μmol), (4-(methoxymethyl)phenyl)boronic acid (235 mg, 1.41 mmol) and 1,1'-bis(diphenylphosphino)ferrocene-palladium(II) dichloride (69.0 mg, 94.3 μmol) in 1,4-dioxane (10 mL) was added a solution of $K_3PO_4$ (500 mg, 2.36 mmol) in water (2.4 mL). The mixture was degassed then heated to 100 °C for 18 h. The mixture was filtered through celite washing with EtOAc (25 mL) and treated with sat aq $NH_4Cl$ (30 mL). The phases were separated and the aqueous phase extracted with EtOAc (2 × 25 mL), combined organics were washed with water (50 mL), brine (50 mL), dried ($MgSO_4$), filtered and concentrated under reduced pressure. The crude product was purified by chromatography on silica gel (40 g cartridge, 0-100% EtOAc/isohexane) to afford 6-(4-(methoxymethyl)phenyl)-1,2,3,4-tetrahydroquinoline (105 mg, 43%, 98% purity) as a beige solid. UPLC-MS (method 1) m/z 254.6 $(M+H)^+$, at 1.18 min. $^1H$ NMR (500 MHz, DMSO-d6) δ 7.52 - 7.46 (m, 2H), 7.31 - 7.26 (m, 2H), 7.21 - 7.15 (m, 2H), 6.49 (d, *J* = 8.1 Hz, 1H), 5.83 - 5.79 (m, 1H), 4.39 (s, 2H), 3.28 (s, 3H), 3.23 - 3.17 (m, 2H), 2.72 (t, *J* = 6.3 Hz, 2H), 1.85 - 1.77 (m, 2H).

Step 2: 2-(4-fluorophenoxy)-1-(6-(4-(methoxymethyl)phenyl)-3,4-dihydroquinolin-1(2*H*)-yl)-2-methylpropan-1-one

**[0282]** To a solution of 2-(4-fluorophenoxy)-2-methylpropanoic acid (40 mg, 0.20 mmol) and *N,N*-dimethylformamide (3 μL , 17 μmol) in DCM (2 mL) at 0 °C was added oxalyl dichloride (22 μL, 0.25 mmol) and the reaction was warmed to rt. To the solution of acid chloride was added a solution of 6-(4-(methoxymethyl)phenyl)-1,2,3,4-tetrahydroquinoline (43 mg, 0.17 mmol) in DCM (5.0 mL) and DIPEA (0.12 mL, 0.67 mmol) and the reaction mixture was stirred at rt. The mixture was concentrated under reduced pressure and the crude product was purified by chromatography on silica gel (24 g cartridge, 0-50% EtOAc/isohexane) then further purified by preparative HPLC to afford 2-(4-fluorophenoxy)-1-(6-(4-(methoxymethyl)phenyl)-3,4-dihydroquinolin-1(2*H*)-yl)-2-methylpropan-1-one (26 mg, 36%, 100% Purity) as a clear colourless oil. UPLC-MS (Method 1) m/z 434.5 $(M+H)^+$, at 1.98 min. $^1H$ NMR (500 MHz, Chloroform-d) δ 7.78 (d, *J* = 8.5 Hz, 1H), 7.54 (d, *J* = 8.0 Hz, 2H), 7.43 - 7.36 (m, 3H), 7.32 (br s, 1H), 6.97 - 6.85 (m, 4H), 4.49 (s, 2H), 4.12 - 4.06 (m, 2H), 3.41 (s, 3H), 2.81 (t, *J* = 7.1 Hz, 2H), 1.94 - 1.85 (m, 2H), 1.72 (s, 6H).

***Example 32: 2-(4-fluorophenoxy)-1-(5-(4-(methoxymethyl)phenyl)indolin-1-yl)-2-methylpropan-1-one***

**[0283]**

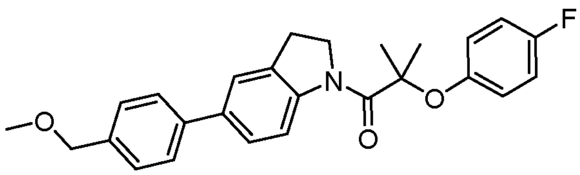

Step 1: 1-(5-bromoindolin-1-yl)-2-(4-fluorophenoxy)-2-methylpropan-1-one

**[0284]** To a solution of 5-bromoindoline (150 mg, 757 μmol) and 2-(4-fluorophenoxy)-2-methylpropanoic acid (150 mg, 757 μmol) in MeCN (2.0 mL) were added DIPEA (396 μL, 2.27 mmol) and HATU (302 mg, 795 μmol) and the reaction was stirred at RT for 3 days. The mixture was diluted with DCM (15 mL) and 10 wt% aq. citric acid (10 mL), stirred for 10 min, passed through a hydrophobic frit and concentrated under reduced pressure. The crude product was purified by chromatography on silica gel (40 g cartridge, 0-100% EtOAc/isohexane) to afford 1-(5-bromoindolin-1-yl)-2-(4-fluorophenoxy)-2-methylpropan-1-one (210 mg, 0.55 mmol, 73%, 99% purity) as a cream solid. LC-MS (Method 3) m/z 378.1/380.1 $(M+H)^+$, at 1.95 min. $^1H$ NMR (500 MHz, DMSO-d6) δ 8.11 (d, *J* = 8.6 Hz, 1H), 7.46 - 7.42 (m, 1H), 7.40 - 7.34 (m, 1H), 7.16 - 7.07 (m, 2H), 6.88 - 6.80 (m, 2H), 4.26 (t, *J* = 8.3 Hz, 2H), 3.05 (t, *J* = 8.2 Hz, 2H), 1.59 (s, 6H).

Step 2: 2-(4-fluorophenoxy)-1-(5-(4-(methoxymethyl)phenyl)indolin-1-yl)-2-methylpropan-1-one

**[0285]** To a solution of 1-(5-bromoindolin-1-yl)-2-(4-fluorophenoxy)-2-methylpropan-1-one (70 mg, 0.19 mmol), (4-(methoxymethyl)phenyl)boronic acid (46 mg, 0.28 mmol) and 1,1'-bis(diphenylphosphino)ferrocene-palladium(II) dichloride (14 mg, 19 μmol) in 1,4-dioxane (3.0 mL) in microwave vial was added a solution of $K_3PO_4$ (98 mg, 0.46 mmol) in water (0.50 mL). The mixture was degassed, sealed, and heated under microwave at 130 °C for 2 h. The mixture was filtered through celite washing with EtOAc (25 mL) and treated with sa.t aq. $NH_4Cl$ (30 mL). The phases were separated and the aqueous extracted with EtOAc (2 × 25 mL), combined organics were washed with water (50 mL), brine (50 mL), dried ($MgSO_4$), filtered and concentrated under reduced pressure. The crude product was purified by chromatography on silica gel (24 g cartridge, 0-50% EtOAc/isohexane) to afford 2-(4-fluorophenoxy)-1-(5-(4-(methoxymethyl)phenyl)indolin-1-yl)-2-methylpropan-1-one (52 mg, 0.12 mmol, 65%, 97% purity) as an orange oil. UPLC-MS (Method 1) m/z 420.5 $(M+H)^+$, at 1.96 min. $^1H$ NMR (500 MHz, DMSO-d6) δ 8.24 (d, *J* = 8.5 Hz, 1H), 7.64 - 7.59 (m, 2H), 7.57 - 7.48 (m, 2H), 7.40 - 7.35 (m, 2H), 7.16 - 7.09 (m, 2H), 6.90 - 6.83 (m, 2H), 4.44 (s, 2H), 4.30 (t, *J* = 8.2 Hz, 2H), 3.10 (t, *J* = 8.2 Hz, 2H), 1.62 (s, 6H). 3H signal obscured by water signal.

***Example* 33: *2-(4-fluorophenoxy)-N-(2-hydroxyethyl)-N-(4'-(methoxymethyl)-[1,1'-biphenyl]-4-yl)-2-methylpropanamide***

**[0286]**

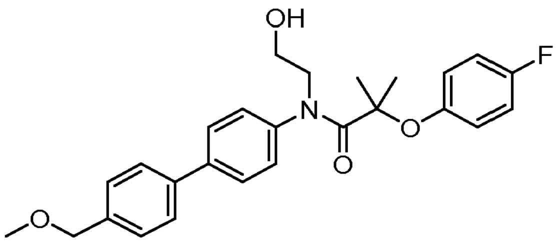

Step 1: *N*-(2-((tert-butyldimethylsilyl)oxy)ethyl)-2-(4-fluorophenoxy)-N-(4'-(methoxymethyl)-[1,1'-biphenyl]-4-yl)-2-methylpropanamide

**[0287]** The title compound was prepared by methods analogous to Example 20, substituting appropriate starting materials and intermediates where necessary to afford *N*-(2-((tert-butyldimethylsilyl)oxy)ethyl)-2-(4-fluorophenoxy)-*N*-(4'-(methoxymethyl)-[1,1'-biphenyl]-4-yl)-2-methylpropanamide (20 mg, 31 μmol, 30%, 85% purity) as a pale yellow solid. UPLC-MS (Method 1) m/z 552.6 $(M+H)^+$, at 2.35 min. $^1H$ NMR (500 MHz, Chloroform-d) δ 7.59 - 7.52 (m, 2H), 7.50 - 7.37 (m, 4H), 7.08 (s, 2H), 6.90 (s, 2H), 6.67 - 6.45 (m, 2H), 4.50 (s, 2H), 4.25 - 3.61 (m, 4H), 3.42 (s, 3H), 1.91 - 1.41 (m, 6H), 0.86 (s, 9H), 0.07 - -0.03 (m, 6H).

Step 2: 2-(4-fluorophenoxy)-*N*-(2-hydroxyethyl)-*N*-(4'-(methoxymethyl)-[1,1'-biphenyl]-4-yl)-2-methylpropanamide

**[0288]** To a solution of *N*-(2-((tert-butyldimethylsilyl)oxy)ethyl)-2-(4-fluorophenoxy)-N-(4'-(methoxymethyl)-[1,1'-biphenyl]-4-yl)-2-methylpropanamide (20 mg, 36 μmol) in THF (5.0 mL) at rt was added TBAF (1.0M in THF) (54 μL, 54 μmol) drop-wise. After 16 h the reaction mixture was treated with MeOH (~1 mL) and concentrated under reduced pressure. The crude product was purified by chromatography on silica gel (24 g cartridge, 0-50% EtOAc/isohexane) to afford 2-(4-fluorophenoxy)-*N*-(2-hydroxyethyl)-N-(4'-(methoxymethyl)-[1,1'-biphenyl]-4-yl)-2-methylpropanamide (13 mg, 28 μmol, 78%, 95% purity) as a pale yellow solid. LC-MS (Method 3) m/z 438.4 $(M+H)^+$, at 1.88 min. $^1H$ NMR (500 MHz, Chloroform-d) δ 7.52 (d, *J* = 7.9 Hz, 2H), 7.44 (d, *J* = 8.3 Hz, 2H), 7.37 (d, *J* = 7.9 Hz, 2H), 6.93 - 6.86 (m, 2H), 6.86 - 6.79 (m, 2H), 6.62 (d, *J* = 8.1 Hz, 2H), 4.48 (s, 2H), 4.40 (t, *J* = 5.4 Hz, 2H), 3.45 (t, *J* = 5.5 Hz, 2H), 3.41 (s, 3H), 1.58 (s, 6H). One exchangeable proton not observed.

***Example* 39: *2-(4-fluorophenoxy)-N-(2-hydroxy-2-methylpropyl)-N-(4'-(methoxymethyl)-[1,1'-biphenyl]-4-yl)-2-methylpropanamide***

**[0289]**

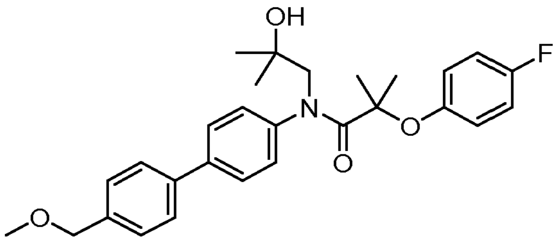

Step 1: ethyl-*N*-(2-(4-fluorophenoxy)-2-methylpropanoyl)-*N*-(4'-(methoxymethyl)-[1,1'-biphenyl]-4-yl)glycinate

**[0290]** To a solution of 2-(4-fluorophenoxy)-*N*-(4'-(methoxymethyl)-[1,1'-biphenyl]-4-yl)-2-methylpropanamide (200 mg, 508 μmol) in THF (5.0 mL) at 0 °C was added NaH (40.7 mg, 60% Wt, 1.02 mmol). The mixture was warmed to RTand after 20 min ethyl 2-bromoacetate (169 μL, 1.52 mmol) was added and reaction was heated at 50 °C. After 16 h, the reaction was cooled to RT, treated with MeOH (~1 mL) and concentrated under reduced pressure. The crude product was purified by chromatography on silica gel (40 g cartridge, 0-50% EtOAc/isohexane) to afford ethyl-N-(2-(4-fluorophenoxy)-2-methylpropanoyl)-*N*-(4'-(methoxymethyl)-[1,1'-biphenyl]-4-yl)glycinate (75 mg, 0.13 mmol, 25%, 80% purity) as a pale yellow solid. LC-MS (Method 3) m/z 480.3 $(M+H)^+$, at 1.88 min. $^1H$ NMR (500 MHz, Chloroform-d) δ 7.53 (d, J = 7.9 Hz, 2H), 7.46 - 7.37 (m, 4H), 7.26 - 7.20 (m, 2H), 6.94 - 6.87 (m, 2H), 6.68 - 6.61 (m, 2H), 4.49 (s, 2H), 4.31 (s, 2H), 4.27 - 4.20 (m, 2H), 3.42 (s, 3H), 1.56 (s, 6H), 1.35 - 1.22 (m, 3H).

Step 2: 2-(4-fluorophenoxy)-*N*-(2-hydroxy-2-methylpropyl)-*N*-(4'-(methoxymethyl)-[1,1'-biphenyl]-4-yl)-2-methylpropanamide

**[0291]** To a solution of ethyl-*N*-(2-(4-fluorophenoxy)-2-methylpropanoyl)-*N*-(4'-(methoxymethyl)-[1,1'-biphenyl]-4-yl) glycinate (43 mg, 90 μmol) in THF (2.0 mL) at 0 °C was added drop-wsie a solution of methylmagnesium bromide (3M in $Et_2O$) (0.12 mL, 0.36 mmol). The reaction was allowed to warm to RT and stirred for 90 min. The reaction mixture was treated with sat. aq. $NH_4Cl$ (5 mL), extracted with EtOAc (3 × 10 mL), washed with brine (20 mL), passed through a phase separator, and concentrated. The crude product was purified by chromatography on silica gel (24 g cartridge, 0-50% EtOAc/isohexane) then further purified by preparative HPLC to afford 2-(4-fluorophenoxy)-*N*-(2-hydroxy-2-methylpropyl)-N-(4'-(methoxymethyl)-[1,1'-biphenyl]-4-yl)-2-methylpropanamide (0.9 mg, 2 μmol, 2%, 96% purity) as a clear colourless oil. UPLC-MS (Method 1) m/z 466.6 $(M+H)^+$, at 1.79 min. $^1H$ NMR (500 MHz, Chloroform-d) δ 7.53 (d, *J* = 8.1 Hz, 2H), 7.42 (dd, *J* = 10.4, 8.2 Hz, 4H), 7.08 (d, *J* = 8.4 Hz, 2H), 6.94 - 6.87 (m, 2H), 6.59 - 6.53 (m, 2H), 4.50 (s, 2H), 3.81 (s, 2H), 3.43 (s, 3H), 1.58 - 1.51 (m, 3H), 1.26 (s, 3H), 1.23 (s, 6H). One exchangeable proton not observed.

***Example 43: (6-(4-(methoxymethyl)phenyl)-3,4-dihydroquinolin-1(2H)-yl)((rac-trans)-2-(pyridin-2-yl)cyclopropyl)methanone***

**[0292]**

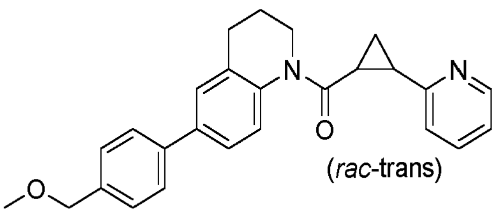

(*rac*-trans)

**[0293]** To a solution of 6-(4-(methoxymethyl)phenyl)-1,2,3,4-tetrahydroquinoline (70 mg, 0.28 mmol) and (rac-trans)-2-(pyridin-2-yl)cyclopropane-1-carboxylic acid (45 mg, 0.28 mmol) in acetonitrile (4.0 mL) at RT was added HATU (0.12 g, 0.30 mmol) and DIPEA (0.14 mL, 0.83 mmol). After 3 days the reaction was diluted with water (10 mL) and EtOAc (30 mL), washed with sat. aq. $NaHCO_3$ (10 mL) and brine (10 mL), passed through a hydrophobic frit and concentrated under reduced pressure. The crude was purified by chromatography on silica gel (24 g cartridge, 0-100% EtOAc/isohexane) to afford (6-(4-(methoxymethyl)phenyl)-3,4-dihydroquinolin-1(2*H*-yl)((rac-trans)-2-(pyridin-2-yl)cyclopropyl)methanone (85 mg, 0.20 mmol, 73%, 95% purity) as a white solid. UPLC-MS (Method 1) m/z 399.4 $(M+H)^+$, at 1.38 min. $^1H$ NMR (500 MHz, Chloroform-d) δ 8.39 (d, *J* = 4.6 Hz, 1H), 7.59 - 7.53 (m, 1H), 7.52 (d, *J* = 8.1 Hz, 2H), 7.40 - 7.33 (m, 3H), 7.33 - 7.17 (m, 3H), 7.08 - 7.02 (m, 1H), 4.48 (s, 2H), 3.94 - 3.87 (m, 1H), 3.87 - 3.78 (m, 1H), 3.41 (s, 3H), 2.84 - 2.73 (m, 3H), 2.68 - 2.61 (m, 1H), 2.06 - 1.92 (m, 2H), 1.82 - 1.75 (m, 1H), 1.66 - 1.58 (m, 1H).

***Example 44: (rac-trans)-N-(3-(4-(methoxymethyl)phenyl)bicyclo[1.1.1]pentan-1-yl)-2-(pyridin-2-yl)cyclopropane-1-carboxamide***

**[0294]**

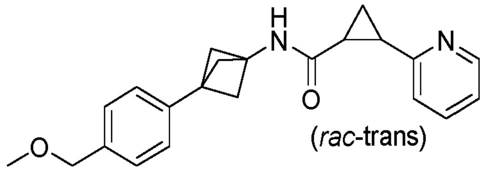

(*rac*-trans)

Step 1: (rac-trans)-N-(3-(4-bromophenyl)bicyclo[1.1.1]pentan-1-yl)-2-(pyridin-2-yl)

**[0295]** To a solution of 3-(4-bromophenyl)bicyclo[1.1.1]pentan-1-amine, HCl (100 mg, 364 μmol) and (rac-trans)-2-(pyridin-2-yl)cyclopropane-1-carboxylic acid (59.4 mg, 1 Eq, 364 μmol) in acetonitrile (2.0 mL) at RT was added HATU (152 mg, 401 μmol) and DIPEA (190 μL, 1.09 mmol). After 3 days the mixture was diluted with water (10 mL) and EtOAc (30 mL), washed with sat. aq. $NaHCO_3$ (10 mL) and brine (10 mL), passed through a hydrophobic frit and concentrated under reduced pressure. The crude was purified by chromatography on silica gel (24 g cartridge, 0-100% EtOAc/isohexane) to afford (rac-trans)-N-(3-(4-bromophenyl)bicyclo[1.1.1]pentan-1-yl)-2-(pyridin-2-yl)cyclopropane-1-carboxamide (118 mg, 0.29 mmol, 80%, 95% purity) as a white solid. UPLC-MS (Method 1) m/z 383.4/385.4 $(M+H)^+$, at 1.25 min. $^1H$ NMR (500 MHz, DMSO-d6) δ 8.80 (s, 1H), 8.44 - 8.39 (m, 1H), 7.70 - 7.63 (m, 1H), 7.52 - 7.45 (m, 2H), 7.43 - 7.38 (m, 1H), 7.21 - 7.14 (m, 3H), 2.46 - 2.38 (m, 1H), 2.23 (s, 6H), 2.11 - 2.04 (m, 1H), 1.37 - 1.30 (m, 2H).

Step 2: (*rac*-trans)-*N*-(3-(4-(methoxymethyl)phenyl)bicyclo[1.1.1]pentan-1-yl)-2-(pyridin-2-yl)cyclopropane-1-carboxamide

**[0296]** To a degassed solution of (rac-trans)-N-(3-(4-bromophenyl)bicyclo[1.1.1]pentan-1-yl)-2-(pyridin-2-yl)cyclopropane-1-carboxamide (95.0 mg, 248 μmol), trifluoro(methoxymethyl)-4-borane, potassium salt (75.3 mg, 496 μmol) and 1,1'-bis(diphenylphosphino)ferrocene-palladium(II) dichloride (18.1 mg, 24.8 μmol) in 1,4-dioxane (5.0 mL) was added a solution of $Cs_2CO_3$ (162 mg, 496 μmol) in water (0.50 mL). The mixture was de-gassed with nitrogen for 15 min, then sealed and heated at 90 °C. After 16 h the reaction mixture was filtered through a pad of celite, the cake was washed with EtOAc (30 mL) and the filtrate was poured into sat. aq. $NaHCO_3$ (30 mL). The mixture was extracted with EtOAc (3 × 30 mL), the combined organic phases were dried (phase separator), and concentrated. The crude product was purified by chromatography on silica gel (40 g cartridge, 0-100% EtOAc/isohexane) to afford title carboxamide (18 mg, 49 μmol, 20%, 95% purity) as a brown crystalline solid. UPLC-MS (Method 1) m/z 349.4 $(M+H)^+$, at 0.99 min. $^1H$ NMR (500 MHz, Chloroform-d) δ 8.47 - 8.39 (m, 1H), 7.60 - 7.53 (m, 1H), 7.37 - 7.12 (m, 5H), 7.11 - 7.05 (m, 1H), 6.28 - 6.15 (m, 1H), 4.54 - 4.37 (m, 2H), 3.43 - 3.32 (m, 3H), 2.61 - 2.54 (m, 1H), 2.49 - 2.29 (m, 6H), 2.09 - 1.98 (m, 1H), 1.92 - 1.82 (m, 1H), 1.66 - 1.58 (m, 1H).

***Example 50: (5-(4-(methoxymethyl)phenyl)indolin-1-yl)((rac-trans)-2-(pyridin-2-yl) cyclopropyl)methanone***

**[0297]**

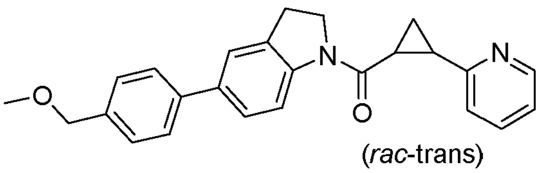

(*rac*-trans)

Step 1: (5-bromoindolin-1-yl)((*rac*-trans)-2-(pyridin-2-yl)cyclopropyl)methanone

**[0298]** To a solution of 5-bromoindoline (150 mg, 757 μmol) and (rac-trans)-2-(pyridin-2-yl)cyclopropane-1-carboxylic acid (124 mg, 757 μmol) in MeCN (2.0 mL) were added DIPEA (396 μL, 2.27 mmol) and HATU (302 mg, 795 μmol) and the reaction was stirred at RT for 16 h. The mixture was diluted with DCM (15 mL) and 10 wt% aq. citric acid (10 mL), stirred for 10 min, passed through a hydrophobic frit and concentrated under reduced pressure. The crude product was purified by chromatography on silica gel (40 g cartridge, 0-100% EtOAc/isohexane) to afford (5-bromoindolin-1-yl)((*rac*-trans)-2-(pyridin-2-yl)cyclopropyl)methanone (338 mg, 0.54 mmol, 72%, 55% purity) as a cream solid. LC-MS (Method 3) m/z 343.1/345.2 $(M+H)^+$, at 1.16min. $^1H$ NMR (500 MHz, Chloroform-d) δ 8.48 (d, *J* = 5.1 Hz, 1H), 8.09 - 8.03 (m, 1H), 7.78 - 7.71 (m, 1H), 7.40 (d, *J* = 7.9 Hz, 1H), 7.31 - 7.27 (m, 2H), 7.26 - 7.21 (m, 1H), 4.37 - 4.21 (m, 2H), 3.24 - 3.13 (m, 2H), 2.80 - 2.75 (m, 1H), 2.58 - 2.51 (m, 1H), 1.82 - 1.75 (m, 1H), 1.74 - 1.67 (m, 1H).

Step 2: (5-(4-(methoxymethyl)phenyl)indolin-1-yl)((rac-trans)-2-(pyridin-2-yl) cyclopropyl)methanone

**[0299]** To a solution of (5-bromoindolin-1-yl)((*rac*-trans)-2-(pyridin-2-yl)cyclopropyl)methanone (100 mg, 55 wt%, 160 μmol), (4-(methoxymethyl)phenyl)boronic acid (39.9 mg, 240 μmol) and 1,1'-bis(diphenylphosphino)ferrocene-palladium(II) dichloride (11.7 mg, 16.0 μmol) in 1,4-dioxane (3.0 mL) in microwave vial was added a solution of $K_3PO_4$ (85.0 mg, 401 μmol) in water (0.50 mL). The mixture was degassed, sealed, and heated at 100 °C for 16 h. The mixture was filtered through celite washing with EtOAc (25 mL) and treated with sat. aq. $NH_4Cl$ (30 mL). The phases were separated and the aqueous extracted with EtOAc (2 × 25 mL), the combined organics were washed with water (50 mL), brine (50 mL), dried ($MgSO_4$), filtered and concentrated under reduced pressure. The crude product was purified by chromatography on silica gel (24 g cartridge, 0-50% EtOAc/isohexane) to afford (5-(4-(methoxymethyl)phenyl)indolin-1-yl)((rac-trans)-2-(pyridin-2-yl)cyclopropyl)methanone (54 mg, 0.13 mmol, 83%, 95% purity) as a pale yellow oil. UPLC-MS (Method 1) m/z 385.3$(M+H)^+$, at 1.23 min. $^1H$ NMR (500 MHz, Chloroform-d) δ 8.47 (d, *J* = 4.9 Hz, 1H), 8.24 (d, *J* = 8.4 Hz, 1H), 7.64 - 7.57 (m, 1H), 7.55 (d, *J* = 7.9 Hz, 2H), 7.43 (d, *J* = 8.4 Hz, 1H), 7.42 - 7.35 (m, 3H), 7.32 (d, *J* = 7.8 Hz, 1H), 7.16 - 7.09 (m, 1H), 4.49 (s, 2H), 4.40 - 4.31 (m, 1H), 4.31 - 4.22 (m, 1H), 3.41 (s, 3H), 3.32 - 3.18 (m, 2H), 2.78 - 2.71 (m, 1H), 2.54 - 2.47 (m, 1H), 1.80 - 1.73 (m, 1H), 1.71 - 1.64 (m, 1H).

***Example 51: N-(4'-ethoxy-[1,1'-biphenyl]-4-yl)-2-(4-fluorophenoxy)-2-methylpropanamide***

**[0300]**

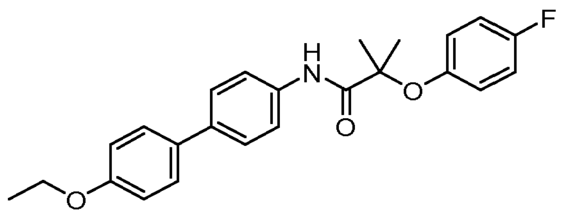

**[0301]** To a mixture of 2-(4-fluorophenoxy)-N-(4-iodophenyl)-2-methylpropanamide (50 mg, 0.13 mmol), (4-ethoxyphenyl)boronic acid (31 mg, 0.19 mmol) and Pd(dppf)$Cl_2$ (9.2 mg, 13 μmol) in dioxane (2.0 mL) was added a solution of potassium phosphate tribasic (80 mg, 0.38 mmol) in water (0.2 mL). The mixture was degassed then heated at 120 °C for 60 min under microwave irradiation. The reaction mixture was poured into 10 wt% aq. citric acid (20 mL) then DCM (10 mL) was added. The mixture was stirred for 15 min, passed through a hydrophobic frit and concentrated under reduced pressure. The crude product was purified by chromatography on silica gel (4 g cartridge, 0-50% EtOAc/isohexane) to afford N-(4'-ethoxy-[1,1'-biphenyl]-4-yl)-2-(4-fluorophenoxy)-2-methylpropanamide (35 mg, 89 μmol, 71%, 97% purity) as a colourless solid. UPLC-MS (Method 1) m/z 394.8 $(M+H)^+$, at 1.73 min. $^1H$ NMR (500 MHz, DMSO-d6) δ 10.08 (s, 1H), 7.83 - 7.69 (m, 2H), 7.61 - 7.53 (m, 4H), 7.21 - 7.13 (m, 2H), 7.03 - 6.97 (m, 4H), 4.08 (q, *J* = 7.0 Hz, 2H), 1.54 (s, 6H), 1.36 (t, *J* = 7.0 Hz, 3H).

***Example 52: 2-(4-fluorophenoxy)-2-methyl-N-(4'-(methylsulfonyl)-[1,1'-biphenyl]-4-yl)propenamide***

**[0302]**

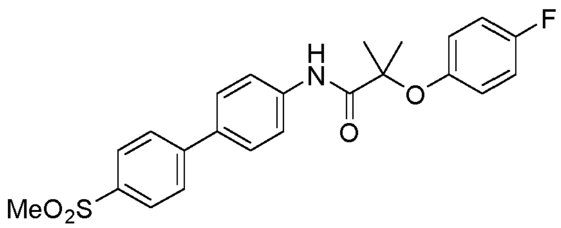

**[0303]** The following example was prepared by methods analogous to Example 51, substituting appropriate starting materials and intermediates where necessary to afford 2-(4-fluorophenoxy)-2-methyl-*N*-(4'-(methylsulfonyl)-[1,1'-biphenyl]-4-yl)propanamide (36 mg, 84 μmol, 67%, 99% purity) as a colourless solid. UPLC-MS (Method 1) no ionisation at 1.42 min. $^1$H NMR (500 MHz, DMSO-d6) δ 10.20 (s, 1H), 7.97 (d, *J = 8.7 Hz,* 2H), 7.93 (d, *J = 8.7 H,* 2H), 7.88 (d, *J = 8.7* Hz, 2H), 7.74 (d, *J* = 8.7 Hz, 2H), 7.19 - 7.10 (m, 2H), 7.03 - 6.93 (m, 2H), 3.24 (s, 3H), 1.53 (s, 6H).

***Example 53: 1-(4-fluorophenoxy)-N-(4'-(methoxymethyl)-[1,1'-biphenyl]-4-yl)cyclopropane-1-carboxamide***

**[0304]**

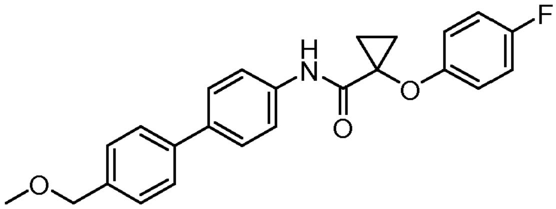

**[0305]** To a solution of 1-(4-fluorophenoxy)cyclopropane-1-carboxylic acid (0.17 mmol) in DMF (0.5 mL) in a 96 well plate were added sequentially a solution of 4'-(methoxymethyl)-[1,1'-biphenyl]-4-amine (32 mg, , 0.15 mmol) in DMF (0.5 mL), followed by a solution of HATU (68 mg, 0.18 mmol) in DMF (0.5 mL) and DIPEA (78 μL, 0.45 mmol). The reactions were shaken for 20 h then filtered. The crude reaction mixture was purified by preparative HPLC using a Waters X-Bridge BEH C18, 5 μm, 19x50 mm column using a gradient of MeCN and 10 mM ammonium bicarbonate(aq) then dried in a Genevac to afford the title compound (38.4 mg, 6.04 mmol, 65% yield, 99% purity) as a colourless solid. UPLC-MS (Method 2) m/z 392.2 (M+H)$^+$, at 1.69 min. $^1$H NMR (500 MHz, DMSO-d6) δ 10.04 (s, 1H), 7.75 - 7.66 (m, 2H), 7.64 - 7.56 (m, 4H), 7.44 - 7.31 (m, 2H), 7.21 - 7.11 (m, 2H), 7.04 - 6.94 (m, 2H), 4.43 (s, 2H), 3.30 (s, 3H), 1.60 - 1.51 (m, 2H), 1.25 - 1.16 (m, 2H).

**[0306]** The following examples were prepared by methods analogous to Example 53, substituting appropriate starting materials and intermediates where necessary:

| Example | Structure | Name / Analytical Data |
|---|---|---|
| 54 | 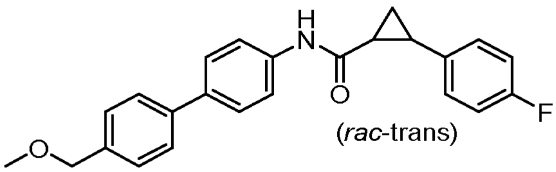 (*rac*-trans) | (*rac*-trans)-2-(4-fluorophenyl)-*N*-(4'-(methoxymethyl)-[1,1'-biphenyl]-4-yl)cyclopropane-1-carboxamide UPLC-MS (Method 2) m/z 376.2 (M+H)$^+$, at 1.61 min. $^1$H NMR (500 MHz, DMSO-d6) δ 10.34 (s, 1H), 7.69 (d, *J* = 8.7 Hz, 2H), 7.65 - 7.58 (m, 4H), 7.37 (d, *J* = 8.0 Hz, 2H), 7.30 - 7.22 (m, 2H), 7.18 - 7.08 (m, 2H), 4.43 (s, 2H), 3.30 (s, 3H), 2.41 (m, 1H), 2.11 - 1.99 (m, 1H), 1.50 (m, 1H), 1.37 (m, 1H). |
| 55 | 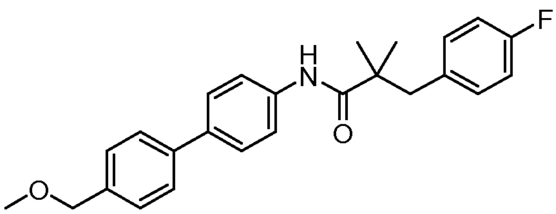 | 3-(4-fluorophenyl)-*N*-(4'-(methoxymethyl)-[1,1'-biphenyl]-4-yl)-2,2-dimethylpropanamide UPLC-MS (Method 2) m/z 392.2 (M+H)$^+$, at 1.70 min. $^1$H NMR (500 MHz, DMSO-d6) δ 9.37 (s, 1H), 7.77 - 7.67 (m, 2H), 7.67 - 7.58 (m, 4H), 7.38 (d, *J* = 8.0 Hz, 2H), 7.22 - 7.14 (m, 2H), 7.13 - 7.04 (m, 2H), 4.44 (s, 2H), 3.31 (s, 3H), 2.94 (s, 2H), 1.19 (s, 6H). |

(continued)

| Example | Structure | Name / Analytical Data |
|---|---|---|
| 56 | 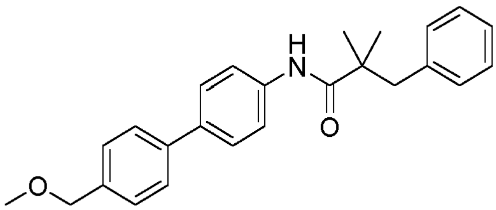 | *N*-(4'-(methoxymethyl)-[1,1'-biphenyl]-4-yl)-2,2-dimethyl-3-phenylpropanamide UPLC-MS (Method 2) m/z 374.5 $(M+H)^+$, at 1.69 min. $^1H$ NMR (500 MHz, DMSO-d6) δ 9.38 (s, 1H), 7.78 - 7.69 (m, 2H), 7.68 - 7.59 (m, 4H), 7.38 (d, *J* = 8.1 Hz, 2H), 7.24 (m, 2H), 7.22 - 7.16 (m, 1H), 7.16 - 7.09 (m, 2H), 4.44 (s, 2H), 3.31 (s, 3H), 2.95 (s, 2H), 1.20 (s, 6H). |
| 57 | 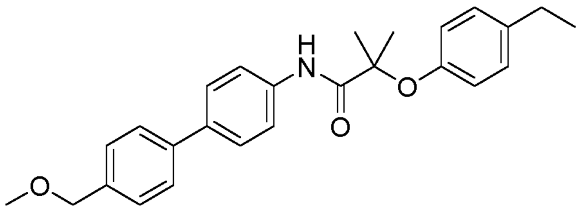 | 2-(4-ethylphenoxy)-*N*-(4'-(methoxymethyl)-[1,1'-biphenyl]-4-yl)-2-methylpropanamide UPLC-MS (Method 2) m/z 404.2 $(M+H)^+$, at 1.89 min. $^1H$ NMR (500 MHz, DMSO-d6) δ 10.09 (s, 1H), 7.80 (d, *J* = 8.8 Hz, 2H), 7.70 - 7.55 (m, 4H), 7.38 (d, *J* = 8.1 Hz, 2H), 7.12 (d, *J* = 8.3 Hz, 2H), 6.85 (d, *J* = 8.3 Hz, 2H), 4.44 (s, 2H), 3.30 (s, 3H), 2.54 (d, *J* = 7.6 Hz, 2H), 1.52 (s, 6H), 1.13 (t, *J* = 7.6 Hz, 3H). |
| 58 | 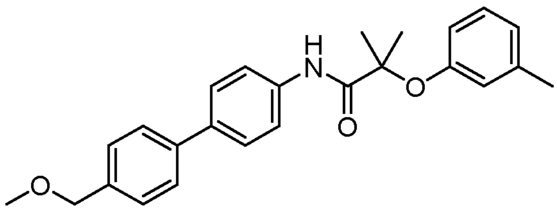 | *N*-(4'-(methoxymethyl)-[1,1'-biphenyl]-4-yl)-2-methyl-2-(m-tolyloxy)propenamide UPLC-MS (Method 2) m/z 390.1 $(M+H)^+$, at 1.81 min. $^1H$ NMR (500 MHz, DMSO-$d_6$) δ 10.07 (s, 1H), 7.78 (d, *J* = 8.8 Hz, 2H), 7.66 - 7.56 (m, 4H), 7.38 (d, *J* = 8.2 Hz, 2H), 7.16 (app t, *J* = 7.9 Hz, 1H), 6.83 (m, 1H), 6.78 (br s, 1H), 6.71 (dd, *J* = 8.2, 2.5 Hz, 1H), 4.44 (s, 2H), 3.30 (s, 3H), 2.25 (s, 3H), 1.54 (s, 6H). |
| 59 | 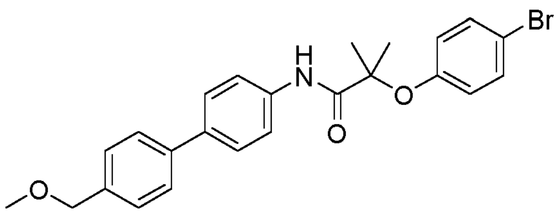 | 2-(4-bromophenoxy)-*N*-(4'-(methoxymethyl)-[1,1'-biphenyl]-4-yl)-2-methylpropanamide UPLC-MS (Method 2) m/z 454.0/456.0 $(M+H)^+$, at 1.84 min. $^1H$ NMR (500 MHz, DMSO-d6) δ 10.10 (s, 1H), 7.77 (d, *J* = 8.7 Hz, 2H), 7.66 - 7.58 (m, 4H), 7.53 - 7.44 (m, 2H), 7.38 (d, *J* = 8.2 Hz, 2H), 6.96 - 6.79 (m, 2H), 4.44 (s, 2H), 3.30 (s, 3H), 1.56 (s, 6H). |
| 60 | 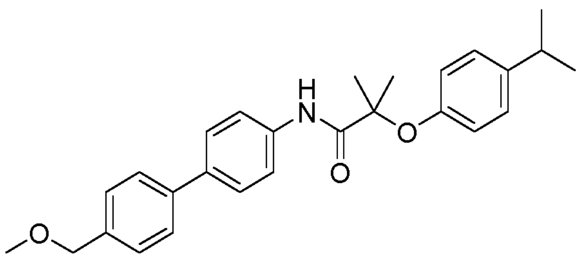 | 2-(4-isopropylphenoxy)-*N*-(4'-(methoxymethyl)-[1,1'-biphenyl]-4-yl)-2-methylpropanamide UPLC-MS (Method 2) m/z 418.2 $(M+H)^+$, at 1.96 min. $^1H$ NMR (500 MHz, DMSO-d6) δ 10.08 (s, 1H), 7.86 - 7.75 (m, 2H), 7.69 - 7.56 (m, 4H), 7.38 (d, *J* = 8.3 Hz, 2H), 7.19 - 7.09 (m, 2H), 6.90 - 6.80 (m, 2H), 4.44 (s, 2H), 3.30 (s, 3H), 2.82 (hept, *J* = 6.9 Hz, 1H), 1.53 (s, 6H), 1.15 (d, *J* = 6.9 Hz, 6H). |

(continued)

| Example | Structure | Name / Analytical Data |
|---|---|---|
| 61 | 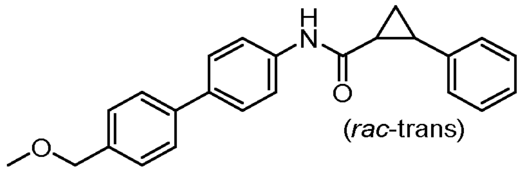 | (*rac*-trans)-*N*-(4'-(methoxymethyl)-[1,1'-biphenyl]-4-yl)-2-phenylcyclopropane-1-carboxamide<br>UPLC-MS (Method 2) m/z 358.2 $(M+H)^+$, at 1.61 min. $^1H$ NMR (500 MHz, DMSO-d6) δ 10.33 (s, 1H), 7.69 (d, *J* = 8.6 Hz, 2H), 7.66 - 7.55 (m, 4H), 7.37 (d, *J* = 8.1 Hz, 2H), 7.32 - 7.27 (m, 2H), 7.24 - 7.13 (m, 3H), 4.43 (s, 2H), 3.30 (s, 3H), 2.39 (m, 1H), 2.09 (m, 1H), 1.51 (m, 1H), 1.38 (m, 1H). |
| 62 | 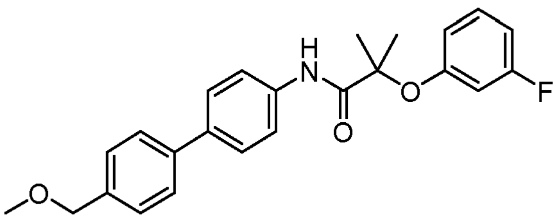 | 2-(3-fluorophenoxy)-*N*-(4'-(methoxymethyl)-[1,1'-biphenyl]-4-yl)-2-methylpropanamide<br>UPLC-MS (Method 2) m/z 394.2 $(M+H)^+$, at 1.73 min. $^1H$ NMR (500 MHz, DMSO-d6) δ 10.11 (s, 1H), 7.82 - 7.71 (m, 2H), 7.67 - 7.55 (m, 4H), 7.42 - 7.27 (m, 3H), 6.86 (m, 1H), 6.82 - 6.71 (m, 2H), 4.44 (s, 2H), 3.30 (s, 3H), 1.58 (s, 6H). |
| 63 | 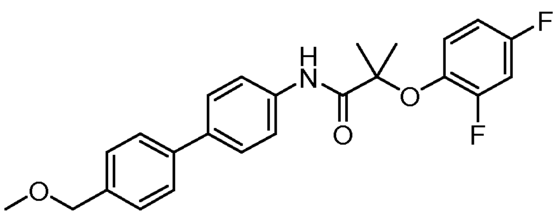 | 2-(2,4-difluorophenoxy)-*N*-(4'-(methoxymethyl)-[1,1'-biphenyl]-4-yl)-2-methylpropanamide<br>UPLC-MS (Method 2) m/z 412.1 $(M+H)^+$, at 1.76 min. $^1H$ NMR (500 MHz, DMSO-$d_6$) δ 10.14 (s, 1H), 7.83 (d, *J* = 8.7 Hz, 2H), 7.64 (d, *J* = 8.3 Hz, 4H), 7.42 - 7.30 (m, 3H), 7.12 (app td, *J* = 9.2, 5.6 Hz, 1H), 7.07 - 7.00 (m, 1H), 4.44 (s, 2H), 3.31 (s, 3H), 1.52 (s, 6H). |
| 64 | 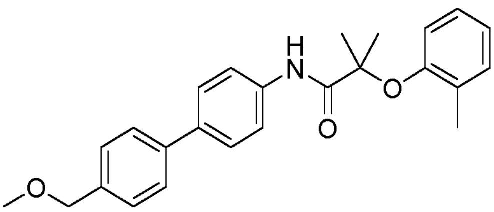 | *N*-(4'-(methoxymethyl)-[1,1'-biphenyl]-4-yl)-2-methyl-2-(o-tolylox)propenamide<br>UPLC-MS (Method 2) m/z 390.2 $(M+H)^+$, at 1.82 min. $^1H$ NMR (500 MHz, DMSO-$d_6$) δ 10.07 (s, 1H), 7.81 (d, *J* = 8.6 Hz, 2H), 7.69 - 7.57 (m, 4H), 7.38 (d, *J* = 8.1 Hz, 2H), 7.21 (d, *J* = 7.6 Hz, 1H), 7.08 (app t, *J* = 7.8 Hz, 1H), 6.91 (app t, *J* = 7.4 Hz, 1H), 6.76 (d, *J* = 8.2 Hz, 1H), 4.44 (s, 2H), 3.30 (s, 3H), 2.27 (s, 3H), 1.54 (s, 6H). |
| 65 | 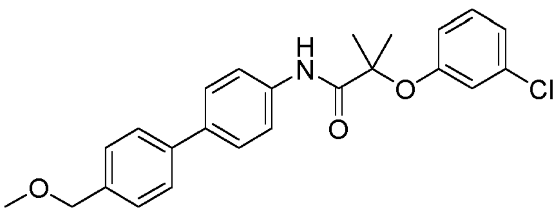 | 2-(3-chlorophenoxy)-*N*-(4'-(methoxymethyl)-[1,1'-biphenyl]-4-yl)-2-methylpropanamide<br>UPLC-MS (Method 2) m/z 410.1 $(M+H)^+$, at 1.82 min. $^1H$ NMR (500 MHz, DMSO-$d_6$) δ 10.11 (s, 1H), 7.76 (d, *J* = 8.8 Hz, 2H), 7.63 (br d, J 8.3 Hz, 4H), 7.38 (d, *J* = 8.0 Hz, 2H), 7.33 (app t, *J* = 8.2 Hz, 1H), 7.09 (dd, J = 8.0, 2.0 Hz, 1H), 7.00 (app t, *J* = 2.2 Hz, 1H), 6.90 (dd, *J* = 8.4, 2.4 Hz, 1H), 4.44 (s, 2H), 3.30 (s, 3H), 1.57 (s, 6H). |

(continued)

| Example | Structure | Name / Analytical Data |
|---|---|---|
| 66 | 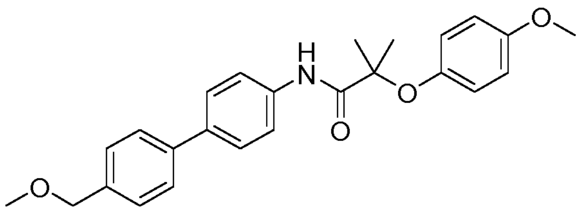 | *N*-(4'-(methoxymethyl)-[1,1'-biphenyl]-4-yl)-2-(4-methoxyphenoxy)-2-methylpropanamide UPLC-MS (Method 2) m/z 406.2 $(M+H)^+$, at 1.70 min. $^1H$ NMR (500 MHz, DMSO-$d_6$) δ 10.10 (s, 1H), 7.83 (d, *J* = 8.6 Hz, 2H), 7.73 - 7.58 (m, 4H), 7.38 (d, *J* = 8.2 Hz, 2H), 6.95 - 6.89 (m, 2H), 6.89 - 6.81 (m, 2H), 4.44 (s, 2H), 3.69 (s, 3H), 3.31 (s, 3H), 1.48 (s, 6H). |
| 67 | 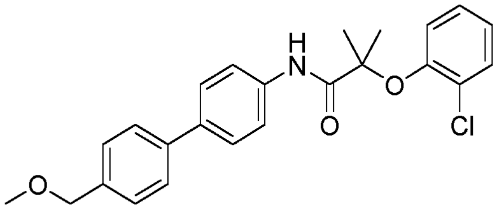 | 2-(2-chlorophenoxy)-*N*-(4'-(methoxymethyl)-[1,1'-biphenyl]-4-yl)-2-methylpropanamide UPLC-MS (Method 2) m/z 410.1 $(M+H)^+$, at 1.84 min. $^1H$ NMR (500 MHz, DMSO-$d_6$) δ 9.61 (s, 1H), 8.05 (ddd, *J* = 5.0, 2.0, 0.8 Hz, 1H), 7.72 (ddd, *J* = 8.3, 7.1, 2.0 Hz, 1H), 7.69 - 7.63 (m, 2H), 7.63 - 7.57 (m, 2H), 7.59 - 7.52 (m, 2H), 7.36 (d, *J* = 8.3 Hz, 2H), 6.93 (ddd, *J* = 7.1, 5.0, 0.9 Hz, 1H), 6.90 (dd, J = 8.3, 0.9 Hz, 1H), 4.43 (s, 2H), 3.30 (s, 3H), 1.66 (s, 6H). |
| 68 | 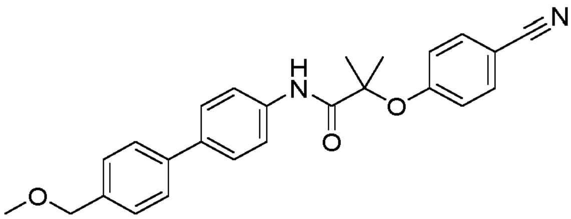 | 2-(4-cyanophenoxy)-*N*-(4'-(methoxymethyl)-[1,1'-biphenyl]-4-yl)-2-methylpropanamide UPLC-MS (Method 2) m/z 401.2 $(M+H)^+$, at 1.89 min. $^1H$ NMR (500 MHz, DMSO-$d_6$) δ 10.11 (s, 1H), 7.81 - 7.75 (m, 2H), 7.75 - 7.69 (m, 2H), 7.66 - 7.58 (m, 4H), 7.37 (d, *J* = 8.2 Hz, 2H), 7.07 - 7.00 (m, 2H), 4.43 (s, 2H), 3.30 (s, 3H), 1.64 (s, 6H). |
| 69 | 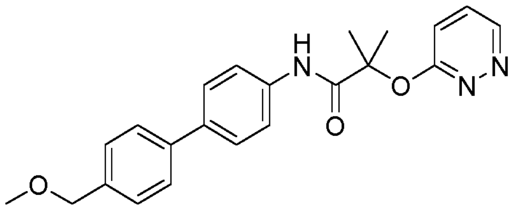 | *N*-(4'-(methoxymethyl)-[1,1'-biphenyl]-4-yl)-2-methyl-2-(pyridazin-3-yloxy)propenamide UPLC-MS (Method 2) m/z 378.2 $(M+H)^+$, at 1.22 min. $^1H$ NMR (500 MHz, DMSO-d6) δ 9.39 (s, 1H), 8.05 (dd, *J* = 3.8, 1.7 Hz, 1H), 7.67 - 7.57 (m, 7H), 7.47 (dd, *J* = 9.4, 3.8 Hz, 1H), 7.40 - 7.36 (m, 2H), 6.90 (dd, *J* = 9.4, 1.7 Hz, 1H), 4.43 (s, 2H), 3.30 (s, 4H), 1.66 (s, 6H). |
| 70 | 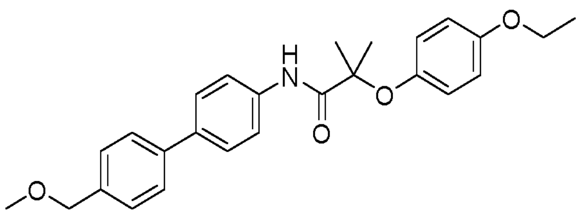 | 2-(4-ethoxyphenoxy)-*N*-(4'-(methoxymethyl)-[1,1'-biphenyl]-4-yl)-2-methylpropanamide UPLC-MS (Method 2) m/z 420.2 $(M+H)^+$, at 1.79 min. $^1H$ NMR (500 MHz, DMSO-$d_6$) δ 10.09 (s, 1H), 7.92 - 7.78 (m, 2H), 7.68 - 7.58 (m, 4H), 7.38 (d, *J* = 8.4 Hz, 2H), 6.93 - 6.88 (m, 2H), 6.88 - 6.81 (m, 2H), 4.44 (s, 2H), 3.94 (q, *J* = 7.0 Hz, 2H), 3.31 (s, 3H), 1.48 (s, 6H), 1.28 (t, *J* = 7.0 Hz, 3H). |

(continued)

| Example | Structure | Name / Analytical Data |
|---|---|---|
| 71 | 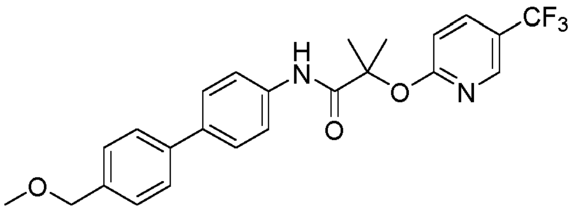 | *N*-(4'-(methoxymethyl)-[1,1'-biphenyl]-4-yl)-2-methyl-2-((5-(trifluoromethyl)pyridin-2-yl)oxy)propenamide UPLC-MS (Method 2) m/z 445.2 $(M+H)^+$, at 1.71 min. $^1H$ NMR (500 MHz, DMSO-$d_6$) δ 9.68 (s, 1H), 8.49 (m, 1H), 8.10 (dd, *J* = 8.8, 2.6 Hz, 1H), 7.70 - 7.63 (m, 2H), 7.63 - 7.54 (m, 4H), 7.36 (d, *J* = 8.2 Hz, 2H), 7.10 (d, *J* = 8.8 Hz, 1H), 4.43 (s, 2H), 3.30 (s, 3H), 1.72 (s, 6H). |

## ADME Properties Testing procedures

### (i) Plasma Stability (Human, mouse and/or Rat)

**[0307]** To quantify the degradation of the test compound in plasma over a 1 hour period. The percent of parent compound present at 0, 30 and 60 mins after initiating incubations in plasma is determined. Compounds were taken from 10 mM DMSO stock solutions and added to plasma, which had previously been incubated at 37°C, to give a final concentration of 25 μM and re-incubated. Aliquots were removed at the appropriate timepoints and quenched with an equal volume of cold acetonitrile. After mixing vigorously, the precipitated protein matter was removed by filtration (Multiscreen Solvinert filter plates, Millipore, Bedford, MA, USA) and the filtrate analysed by reverse phase HPLC with mass spectrometric detection, using single ion monitoring of the $[M+H]^+$ species. Metabolic turnover was determined by comparison of peak areas from the ion chromatograms of the parent before and after incubation and expressed as percent remaining at each timepoint.

### (ii) Microsomal Metabolic Stability (Human, mouse or Rat)

**[0308]** Test compound (3μM) is incubated with pooled liver microsomes. Test compound is incubated at 5 time points over the course of a 45 min experiment and the test compound is analysed by LC-MS/MS. An intrinsic clearance value ($CL_{int}$) with standard error and $t_{½}$ value are calculated.

**[0309]** Microsomes (final protein concentration 0.5mg/mL), 0.1M phosphate buffer pH7.4 and test compound (final substrate concentration 3μM; final DMSO concentration 0.25%) are pre-incubated at 37 C prior to the addition of NADPH (final concentration 1mM) to initiate the reaction. The final incubation volume is 50μL. A minus cofactor control incubation is included for each compound tested where 0.1M phosphate buffer pH7.4 is added instead of NADPH (minus NADPH). Two control compounds are included with each species. All incubations are performed singularly for each test compound. Each compound is incubated for 0, 5, 15, 30 and 45min. The control (minus NADPH) is incubated for 45min only. The reactions are stopped by transferring 20μL of incubate to 60μL methanol at the appropriate time points. The termination plates are centrifuged at 2,500rpm for 20min at 4 C to precipitate the protein. Following protein precipitation, the sample supernatants are combined in cassettes of up to 4 compounds and analysed using generic LC-MS/MS conditions. From a plot of In peak area ratio (compound peak area/internal standard peak area) against time, the gradient of the line is determined. Subsequently, half-life and intrinsic clearance are calculated using the equations below:

$$\text{Elimination rate constant (k)} = (\text{- gradient})$$

$$\text{Half-life } (t_{½}) \text{ (min)} = \frac{0.693}{k}$$

$$\text{Intrinsic clearance } (CL_{int}) \text{ (μL/min/mg protein)} = \frac{V \times 0.693}{t_{1/2}}$$

where V = Incubation volume (μL)/Microsomal protein (mg)

**[0310]** Relevant control compounds are assessed, ensuring intrinsic clearance values fall within the specified limits.

**(iii) Hepatocyte Stability (Human, mouse, rat or doq)**

**[0311]** Test compound (3μM) is incubated with cryopreserved hepatocytes in suspension. Samples are removed at 6 time points over the course of a 60 min experiment and test compound is analysed by LC-MS/MS. An intrinsic clearance value ($CL_{int}$) with standard error and half-life ($t_{½}$) are calculated. Cryopreserved pooled hepatocytes are stored in liquid nitrogen prior to use. Williams E media supplemented with 2mM L-glutamine and 25mM HEPES and test compound (final substrate concentration 3μM; final DMSO concentration 0.25 %) are pre-incubated at 37 C prior to the addition of a suspension of cryopreserved hepatocytes (final cell density $0.5\times10^6$ viable cells/mL in Williams E media supplemented with 2mM L-glutamine and 25mM HEPES) to initiate the reaction. The final incubation volume is 500μL. A control incubation is included for each compound tested where lysed cells are added instead of viable cells. Two control compounds are included with each species.

**[0312]** The reactions are stopped by transferring 50μL of incubate to 100μL methanol containing internal standard at the appropriate time points. The control (lysed cells) is incubated for 60min only. The termination plates are centrifuged at 2500rpm at 4°C for 30min to precipitate the protein. Following protein precipitation, the sample supernatants are combined in cassettes of up to 4 compounds and analysed using generic LC-MS/MS conditions. From a plot of In peak area ratio (compound peak area/internal standard peak area) against time, the gradient of the line is determined. Subsequently, half-life ($t_{½}$) and intrinsic clearance ($CL_{int}$) are calculated using the equations below:

$$\text{Elimination rate constant (k)} = (\text{- gradient})$$

$$\text{Half-life } (t_{½}) \text{ (min)} = \frac{0.693}{k}$$

$$\text{Intrinsic clearance } (CL_{int}) \text{ (μL/min/million cells)} = \frac{V\times0.693}{t_{1/2}}$$

where V = Incubation volume (μL)/Number of cells

**[0313]** Two control compounds for each species are included in the assay and if the values for these compounds are not within the specified limits the results are rejected and the experiment repeated.

**[0314]** Compounds of the invention are compared to the stability of literature comparison 1; RTI-13951-33 (e.g. see Jin, C et al, J. Med. Chem. 61, 6748-6758, 2018 and references cited therein), Table 1.

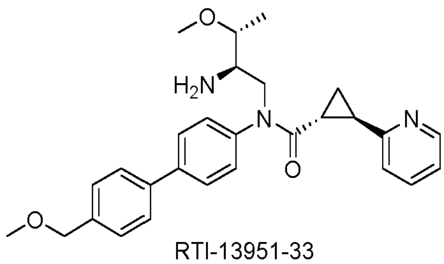

RTI-13951-33

**Table 1.** Mouse hepatocyte stability data

| Example | Mouse heps Clint (μL/min/$10^6$ cells) |
|---|---|
| Literature comparison 1 | 176 |
| (1) | 65 |
| (2) | 54 |
| (3) | 30 |
| (4) | 51 |
| (7) | 46 |
| (8) | 55 |

(continued)

| Example | Mouse heps Clint ($\mu$L/min/$10^6$ cells) |
|---|---|
| (9) | 39 |
| (13) | 90 |
| (17) | 37 |
| (18) | 70 |
| (25) | 41 |
| (26) | 70 |
| (31) | 76 |
| (32) | 65 |
| (35) | 49 |
| (36) | 80 |
| (53) | 71 |
| (68) | 37 |
| (70) | 74 |

**(v) LogD Determinations:**

**[0315]** $LogD_{(PBS)}$ determinations were performed in 96 well microtitre plates using a miniaturised "shake-flask" method. In brief, compounds were taken from 10 mM DMSO stock solutions and added to wells containing equal volumes of phosphate buffered saline (10 mM; pH 7.4) (PBS) and 1-octanol (Sigma-Aldrich, Poole, Dorset, UK) to give a final concentration of 50 $\mu$M. The plates were then capped and mixed vigorously for 1 hour on a microtitre plate shaker, after which they were left to stand, allowing the PBS and octanol phases to separate. The PBS layer was analysed by reverse phase HPLC with mass spectrometric detection, using single ion monitoring of the $[M+H]^+$ species. $LogD_{(PBS)}$ was determined by comparison of the peak area from the ion chromatogram of the compound in the PBS phase with that of a 50$\mu$M standard of the same compound dissolved in acetonitrile/water (50:50) and calculated using the following formula:

$$LogD = Log\left[\frac{AUCstd - AUCpbs}{AUCpbs}\right]$$

**[0316]** Where *AUCstd* and *AUCpbs* are the peak areas from the standard and test ion chromatograms respectively. $LogD_{(PBS)}$ determinations were also made using PBS at pH6.9 and 5.5 by adjusting the pH of the buffer prior to the start of the assay, with 0.1 M HCl

**Biological Investigations**

**[0317]** The following assays can be used to illustrate the commercial utilities of the compounds according to the present invention.

**Biological Assay 1: hGPR88-HEK cAMP accumulation assay**

**[0318]** To evaluate the agonist activity of compounds at the hGPR88 receptor, test compounds are dispensed into 384-well white shallow well ProxiPlate assay plates (Perkin Elmer 6008280) using ECHO acoustic dispensing with DMSO backfill. Forskolin, prepared in KRH assay buffer (5 mM KCl, 1.25 mM $MgSO_4$, 124 mM NaCl, 25 mM HEPES, 13.3 mM Glucose, 1.25 mM $KH_2PO_4$, 1.45 mM $CaCl_2$ freshly supplemented with 0.05% (w/v) BSA and 0.5 mM IBMX), is dispensed into wells containing test compounds using Thermo Scientific™ Multidrop™ Combi Reagent Dispenser in 5 $\mu$l volume to provide a final assay concentration of 200 nM ($EC_{90}$). Cryopreserved vials of HEK-293 cells expressing human recombinant GPR88 receptor are re-suspended in KRH assay buffer and 5 $\mu$l of cell solution is suspended in test wells at a seeding density of 2500 $\pm$ 500 cells per well using the multidrop to provide a final reaction volume of 10 $\mu$l containing 0.5% DMSO. Assay plate is incubated for 30 min at room temperature and the reaction is terminated by addition of 5 $\mu$l of

each of the cAMP detection reagents of the cAMP $G_i$ kit (Cisbio Bioassays, 62AM9PEJ), diluted in cell lysis buffer, to each well using the multidrop in the following order: first the cAMP-d2 conjugate, then the anti-cAMP cryptate conjugate. The plate is further incubated for 1 hour at room temperature before reading the fluorescence emission ratio (665nm/620nm) on PHERAstar® FSX (BMG Labtech). Raw counts were converted to cAMP concentrations via a standard curve before $EC_{50}$ and $E_{max}$ determination. Data is expressed as % decrease in forskolin stimulated cAMP compared to cells treated with vehicle alone in the same buffer and on the same plate.

**Supplementary Information**

**Cloning of the GPR88 receptor gene:**

**[0319]** The coding region encoding the GPR88 receptor was cloned in pEFIN3, a proprietary bicistronic expression vector developed at EPICS, in which the transcription of both the receptor and the gene of selection (neomycin) are under the control of a strong promoter of transcription through an IRES (internal ribosome entry site) sequence (Ghattas et al., 1991, Mol. Cell. Biol. 11, 5848-5859).

**Cell line development**

**[0320]** EPICS's proprietary bicistronic expression plasmids containing the coding sequence of the human GPR88 receptor was transfected, using Lipofectamine 2000, in HEK293 cells. After selection with antibiotics, the mix of antibiotic-resistant cells has been frozen and further used in a cAMP assay using 2-PCCA as reference agonist.

**Preparation of cryovials**

**[0321]** GPR88-HEK cells were grown in standard TC conditions with the supplier's recommended media (EMEM, 10% FBS, 100 IU/ml penicillin, 100 μg/ml streptomycin, 100 μg/ml Geneticin - Gibco ref 10131-027). Cells were harvested between 50-80% confluency by washing flasks once with PBS, then detaching cells with a 10-15 min incubation with Versene (5 mL per 225 cm2 flask). Detached cells were harvested using 5 mL media (without G418) per flask and dissociated by pipetting aggressively against the wall of the flask 10-15 times. Cells were visually inspected under a microscope to ensure adequate dissociation. Cells were counted using AOPI stain, centrifuged at 300 x g for 5 min, then resuspended in freezing media (90% media without G418, 10% DMSO) for a final concentration of $2.5 \times 10^6$ live cells/mL. Cells were frozen in 0.5 and 1 mL aliquots using a cell freezing container in a -80°C freezer overnight. Cells were then stored at -80°C until required.

**[0322]** The results for selected compounds according to the invention are shown in Table 2. The skilled person will realise that the assays described herein exhibit some variability. The variability arises due to the fact that the assay is a cell-based assay (involving batches of cells being thawed for each assay run). The inter assay variability might range by an amount of +/- 30%. For that reason, the activity of the compounds is quoted in High/Medium/Low 'bands' rather than as precise results.

| | | | | | | | |
|---|---|---|---|---|---|---|---|
| **1** | High | **29** | Medium | **57** | High | | |
| **2** | High | **30** | Medium | **58** | High | | |
| **3** | High | **31** | High | **59** | High | **85** | High |
| **4** | High | **32** | Medium | **60** | High | **86** | High |
| **5** | Low | **33** | Medium | **61** | Low | **87** | High |
| **6** | Low | **34** | Medium | **62** | High | **88** | Medium |
| **7** | Medium | **35** | High | **63** | High | **89** | High |
| **8** | High | **36** | Medium | **64** | High | **90** | Medium |
| **9** | Medium | **37** | Medium | **65** | High | **91** | Medium |
| **10** | Low | **38** | Low | **66** | High | **92** | High |
| **11** | Low | **39** | Medium | **67** | High | **93** | Low |
| **12** | Low | **40** | Medium | **68** | High | | |
| **13** | Medium | **41** | Low | **69** | Low | | |
| **14** | Medium | **42** | Low | **70** | High | | |

| | | | | | | | |
|---|---|---|---|---|---|---|---|
| **15** | Medium | **43** | Low | **71** | Low | | |
| **16** | Low | **44** | Medium | **72** | Medium | | |
| **17** | Medium | **45** | Medium | **73** | Low | | |
| **18** | High | **46** | High | **74** | Low | | |
| **19** | Low | **47** | Medium | **75** | Medium | | |
| **20** | Low | **48** | Low | **76** | Medium | | |
| **21** | Low | **49** | High | **77** | Medium | | |
| **22** | Medium | **50** | Medium | **78** | Medium | | |
| **23** | Low | **51** | Low | **79** | High | | |
| **24** | Low | **52** | Low | **80** | Medium | | |
| **25** | High | **53** | High | **81** | High | | |
| **26** | High | **54** | Low | **82** | Low | | |
| **27** | Medium | **55** | High | **83** | Medium | | |
| **28** | High | **56** | Medium | **84** | Low | | |

**Table 2**. GRP88 agonist activity ($EC_{50}$) wherein High (<1000nM), Medium (1000nM to 5000nM); Low (>5000nM). In this assay, literature comparison 1 (RTI-13951-33) exhibited a value of 420nM (lit value e.g. see Jin, C et al, J. Med. Chem. 61, 6748-6758, 2018 and references cited therein is 25nM).

**[0323]** **Table 2.** GRP88 agonist activity ($EC_{50}$) wherein High (<1000nM), Medium (1000nM to 5000nM); Low (>5000nM). In this assay, literature comparison 1 (RTI-13951-33) exhibited a value of 420nM (lit value e.g. see Jin, C et al, J. Med. Chem. 61, 6748-6758, 2018 and references cited therein is 25nM).

**Biological Assay 2: Dopamine Transporter Uptake Assay**

**[0324]** Evaluation of the inhibition of dopamine uptake transporter with 10μM compound is determined in rat striatum synaptosomes following [$^3$H]dopamine scintillation counting (see Janowsky, A. et al. J. Neurochem., 46, 1272-1276, 1986). Compounds of the invention are compared to the literature comparison 1; RTI-13951-33 and literature comparison 2; 2-PCCA (e.g. see Jin, C et al, J. Med. Chem. 61, 6748-6758, 2018 and references cited therein), Table 3,

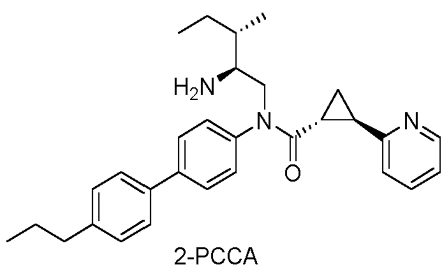

2-PCCA

**Table 3.** Off-target selectivity screening vs rat DAT

| Example | Inhibition of dopamine uptake transporter (% inhibition at 10μM compound) |
|---|---|
| Literature comparative 1 | 70 |
| Literature comparative 2 | 90 |
| (1) | 11 |
| (2) | 3 |
| (18) | 0 |
| (26) | 7 |
| (31) | 52 |
| (45) | 28 |
| (49) | 0 |

## Claims

**1.** A compound of Formula (I) or a pharmaceutically acceptable salt thereof:

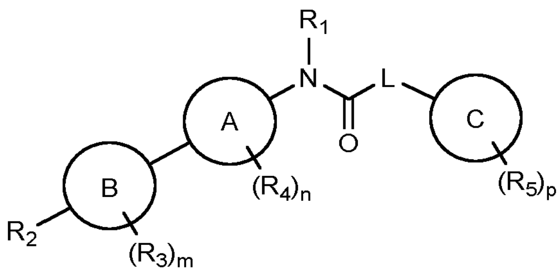

(I)

wherein:

i) -L- is selected from the group consisting of

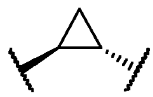, 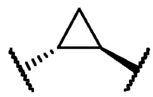,

and

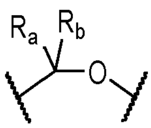;

Ring A is selected from the group consisting of: a 6-membered cycloalkyl, a 5-membered cycloalkyl ring system, a 8-membered cycloalkyl ring system, a 6-membered cycloalkenyl, a 6-membered aromatic ring system, and a 6-membered heteroaromatic ring system;
Ring B is selected from the group consisting of: a 6-membered cycloalkyl, a 6-membered aromatic and a 6-membered heteroaromatic ring;
Ring C is selected from the group consisting of: a 5-6 membered aromatic and a 5-6 membered heteroaromatic ring;
m is 0, 1 or 2;
n is 0, 1 or 2;
p is 0, 1 or 2;

$R_1$ is selected from the group consisting of: hydrogen, substituted or unsubstituted $C_1$-$C_4$ alkyl, substituted or unsubstituted $C_3$-$C_4$ cycloalkyl and substituted or unsubstituted $C_3$-$C_4$ cycloalkyl $C_1$-$C_2$ alkyl; wherein each substituent is independently selected from the group consisting of -$OR_c$, nitrile or halo; wherein each $R_c$ is independently selected from the group consisting of H, Me, Et and cyclopropyl;
$R_2$ is selected from the group consisting of: substituted or unsubstituted $C_1$-$C_6$ alkyl, substituted or unsubstituted $C_2$-$C_6$ alkenyl, substituted or unsubstituted $C_2$-$C_6$ alkynyl; substituted or unsubstituted $C_3$-$C_6$ cycloalkyl; substituted or unsubstituted $C_1$-$C_6$ alkoxy, substituted or unsubstituted $C_3$-$C_6$ cycloalkoxy; substituted or unsubstituted $C_1$-$C_6$ alkoxy-$C_1$-$C_6$ alkyl; substituted or unsubstituted $C_3$-$C_6$ cycloalkoxy-$C_1$-$C_6$ alkyl; substituted or unsubstituted $C_1$-$C_6$ haloalkyl, substituted or unsubstituted $C_2$-$C_6$ haloalkenyl, substituted or unsubstituted $C_2$-$C_6$ haloalkynyl; substituted or unsubstituted $C_3$-$C_6$ cyclohaloalkyl; substituted or unsubstituted $C_1$-$C_6$ haloalkoxy, substituted or unsubstituted $C_3$-$C_6$ cyclohaloalkoxy; substituted or unsubstituted $C_1$-$C_6$ haloalkoxy-$C_1$-$C_6$ alkyl, substituted or unsubstituted $C_3$-$C_6$ cyclohaloalkoxy-$C_1$-$C_6$ alkyl, substituted or unsubstituted $C_1$-$C_6$ alkoxy-$C_1$-$C_6$ haloalkyl, substituted or unsubstituted $C_3$-$C_6$ cycloalkoxy-$C_1$-$C_6$ haloalkyl, substituted or unsubstituted $C_1$-$C_6$ haloalkoxy-$C_1$-$C_6$ haloalkyl, substituted or unsubstituted $C_3$-$C_6$ cyclohaloalkoxy-$C_1$-$C_6$ haloalkyl, -$SO_2R_d$, and -$NR_eR_e$;

wherein $R_d$ is $C_{1-4}$ alkyl;
wherein each $R_e$ is independently selected from the group consisting of H and $C_{1-4}$ alkyl;
wherein each substituent is independently selected from the group consisting of =O, -$NR_eR_e$, and a substituted or unsubstituted monocyclic, bicyclic or bridged 3-8 membered cycloalkyl or heterocycloalkyl ring, wherein each $R_e$ is independently selected from the group consisting of H and $C_{1-4}$ alkyl;
wherein each substituent of the monocyclic, bicyclic or bridged 3-8 membered cycloalkyl or heterocycloalkyl ring is independently selected from the group consisting of -OH, nitrile, halo, methyl or methoxy;

each $R_3$ is independently selected from the group consisting of: halogen, $C_1$-$C_3$ alkyl, $C_1$-$C_3$ haloalkyl, $C_1$-$C_3$ alkoxy, $C_1$-$C_3$ haloalkoxy or -CN;
each $R_4$ is independently selected from the group consisting of: halogen, $C_1$-$C_3$ alkyl, $C_1$-$C_3$ haloalkyl, $C_1$-$C_3$ alkoxy, $C_1$-$C_3$ haloalkoxy or -CN;
each $R_5$ is independently selected from the group consisting of: halogen, $C_1$-$C_3$ alkyl, $C_1$-$C_3$ haloalkyl, $C_1$-$C_3$ alkoxy, $C_1$-$C_3$ haloalkoxy, -CN, or -C(O)$NR_eR_e$;
$R_a$ and $R_b$ are each independently selected from the group consisting of: hydrogen, halogen, $C_1$-$C_6$ alkyl, and $C_1$-$C_6$ alkoxy; or wherein $R_a$ and $R_b$, together with the atom to which they are bonded, form a 3-membered to 6-membered cycloalkyl or heterocycloalkyl ring comprising -O- or -$NR_f$-, wherein $R_f$ is H or methyl;
provided that the compound is not:

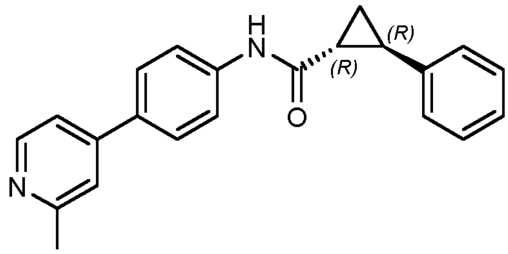

or

ii) -L-, Ring A, Ring B, Ring C, m, p, $R_2$, $R_3$, $R_5$, $R_a$, $R_b$, $R_c$, $R_d$, $R_e$ and $R_f$ are as defined above under i),

$R_1$ is a $C_1$ or $C_2$ alkylenyl residue,
n is 1 or 2, and
one $R_4$ moiety is a $C_1$ alkylenyl residue and, when present, the other $R_4$ moiety is as defined above under i), wherein the $C_1$ alkylenyl $R_4$ moeity is bonded to a carbon atom in Ring A that is ortho to the carbon atom of Ring A having the amide residue, and, together with the atoms to which they are bonded, the $R_1$ and $R_4$ moieties form a 5- or 6-membered ring;

and/or
iii) -L-, Ring A, Ring B, Ring C, n, p, $R_1$, $R_4$, $R_5$, $R_a$, $R_b$, $R_c$ and $R_f$ are as defined above under i),

m is 1 or 2;
$R_2$ is selected from the group consisting of: substituted or unsubstituted -O-$C_{1-2}$ alkylenyl, wherein the oxygen atom is connected to Ring B;
wherein each substituent is independently selected from the group consisting of -OH, nitrile, halo, methyl or methoxy;
one $R_3$ moiety is an -O- residue and, when present, the other $R_3$ moiety is as defined above under i);
wherein, together with the atoms to which they are bonded, the $R_2$ moiety and $R_3$ moiety, which is an -O- residue, form a 5- or 6-membered ring.

2. The compound of claim 1, wherein $R_1$ is selected from the group consisting of: hydrogen, substituted or unsubstituted $C_1$-$C_4$ alkyl, substituted or unsubstituted $C_3$-$C_4$ cycloalkyl and substituted or unsubstituted $C_3$-$C_4$ cycloalkyl $C_1$-$C_2$ alkyl; wherein each substituent is independently selected from -$OR_c$ and nitrile; and wherein each $R_c$ is independently selected from the group consisting of H, Me, Et and cyclopropyl.

3. The compound of claim 2, wherein

- $R_1$ is selected from the group consisting of: hydrogen, substituted or unsubstituted $C_1$-$C_4$ alkyl and substituted or unsubstituted $C_3$-$C_4$ cycloalkyl $C_1$-$C_2$ alkyl; wherein each substituent is independently selected from -$OR_c$ and nitrile; and wherein each $R_c$ is independently selected from the group consisting of H, Me, Et and cyclopropyl; or
- $R_1$ is selected from the group consisting of: hydrogen and substituted or unsubstituted $C_1$-$C_4$ alkyl and substituted or unsubstituted $C_3$ cycloalkyl $C_1$ alkyl; wherein each substituent is independently selected from -$OR_c$ and nitrile; and wherein each $R_c$ is independently selected from the group consisting of H and Me, or
- $R_1$ is selected from the group consisting of:

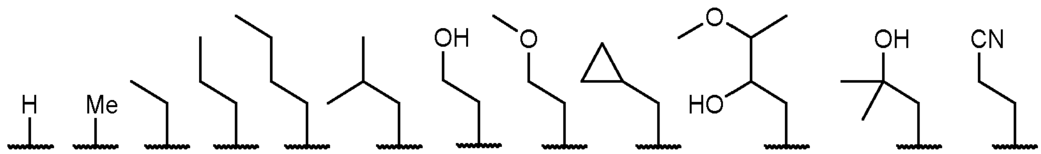.

4. The compound of any preceding claim, wherein

- n is 1; and/or
- each $R_4$ is independently selected from the group consisting of: halogen, $C_1$-$C_2$ alkyl, and $C_1$-$C_2$ alkoxy, wherein optionally $R_4$ is methyl.

5. The compound of any of claims 1 to 3, wherein n is 0.

6. The compound of claim 1, wherein $R_1$ is a $C_1$ or $C_2$ alkylenyl residue and one $R_4$ moiety is a $C_1$ alkylenyl residue wherein the $C_1$ alkylenyl $R_4$ moeity is bonded to a carbon atom in Ring A that is ortho to the carbon atom of Ring A having the amide residue, and, together with the atoms to which they are bonded, the $R_1$ and $R_4$ moieties form a 5- or 6-membered ring.

7. The compound of any preceding claim, wherein $R_2$ is selected from the group consisting of: substituted or unsubstituted $C_1$-$C_4$ alkyl; substituted or unsubstituted $C_3$-$C_4$ cycloalkyl; substituted or unsubstituted $C_1$-$C_4$ alkoxy, substituted or unsubstituted $C_3$-$C_4$ cycloalkoxy; substituted or unsubstituted $C_1$-$C_4$ alkoxy-$C_1$-$C_4$ alkyl; substituted or unsubstituted $C_1$-$C_4$ haloalkyl; substituted or unsubstituted $C_1$-$C_4$ haloalkoxy; $SO_2R_4$; and -$NR_eR_e$.;
wherein optionally:

- $R_2$ is selected from the group consisting of: substituted or unsubstituted $C_1$-$C_4$ alkyl; substituted or unsubstituted $C_1$-$C_4$ alkoxy, substituted or unsubstituted $C_3$-$C_4$ cycloalkoxy; substituted or unsubstituted $C_1$-$C_4$ alkoxy-$C_1$-$C_4$ alkyl; substituted or unsubstituted $C_1$-$C_4$ haloalkyl; substituted or unsubstituted $C_1$-$C_4$ haloalkoxy; $SO_2R_d$; and -$NR_eR_e$; or
- $R_2$ is selected from the group consisting of:

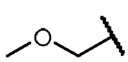 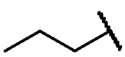 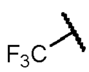 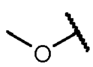 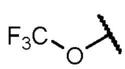

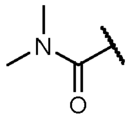 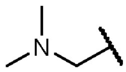 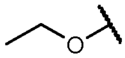 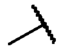 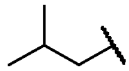

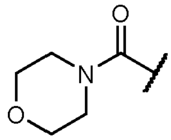 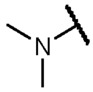 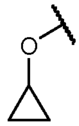 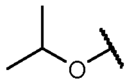 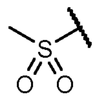

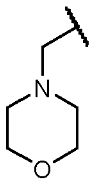 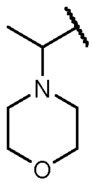 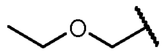 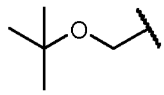 .

**8.** The compound of any preceding claim, wherein

- m is 1; and/or
- $R_3$ is independently selected from the group consisting of: halogen, $C_1$-$C_2$ alkyl, and $C_1$-$C_2$ alkoxy.

**9.** The compound of any of claims 1 to 7, wherein m is 0.

**10.** The compound of any of claims 1 to 6, wherein $R_2$ is a substituted or unsubstituted - O-$C_1$ alkylenyl residue, wherein the oxygen atom is connected to Ring B; and one $R_3$ moiety is an -O- residue, wherein together with the atoms to which they are bonded, the -O-$C_1$ alkylenyl and -O- residues form a 5-membered ring.

**11.** The compound of any preceding claim, wherein

- p is 0; or
- p is 1; and/or
- each $R_5$ is independently selected from the group consisting of: halogen, $C_1$-$C_3$ alkyl, $C_1$-$C_3$ haloalkyl, $C_1$-$C_3$ alkoxy, -CN or -C(O)$NR_eR_e$.

**12.** The compound of any preceding claim, wherein

- Ring A is selected from the group consisting of: a 6 membered aromatic and heteroaromatic ring, optionally wherein Ring A is phenyl; or
- Ring A is a 5 or 8 membered cycloalkyl ring system, optionally wherein Ring A is bicyclo-[1.1.1]-pentyl.

**13.** The compound of any preceding claim, wherein

- Ring B is selected from the group consisting of: phenyl, pyridinyl or pyrimidinyl, and/or
- Ring C is selected from the group consisting of: a 6 membered aromatic and a 6 membered heteroaromatic ring, optionally phenyl or pyridinyl; and/or
- $R_a$ and $R_b$ are each independently selected from the group consisting of: hydrogen, halogen, $C_1$-$C_4$ alkyl, and $C_1$-$C_4$ alkoxy; or wherein $R_a$ and $R_b$, together with the atom to which they are bonded, form a 3-membered to 4-membered cycloalkyl or heterocycloalkyl ring comprising -O- or -$NR_f$-, wherein $R_f$ is H or methyl, wherein optionally $R_a$ and $R_b$ are each independently selected from the group consisting of: hydrogen and methyl.

**14.** The compound of claim 1, wherein the compound is selected from:

| | | | |
|---|---|---|---|
| (1) | 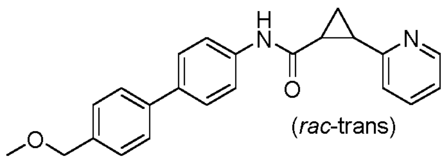 (*rac*-trans) | (2) | 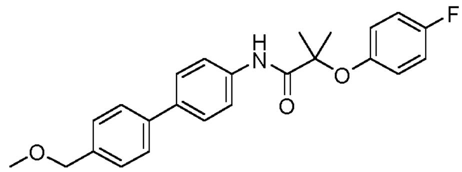 |
| (3) | 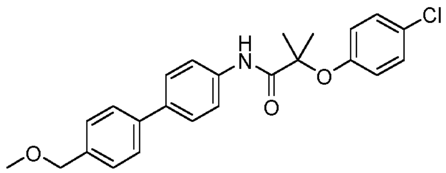 | (4) | 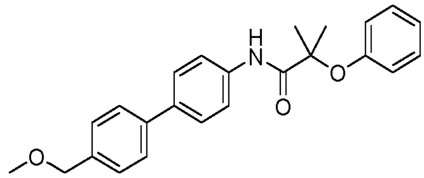 |
| (5) | 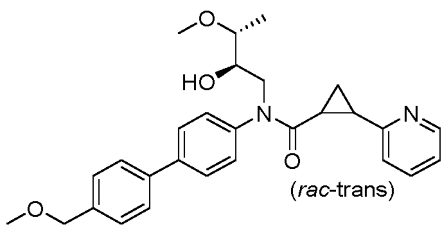 (*rac*-trans) | (6) | 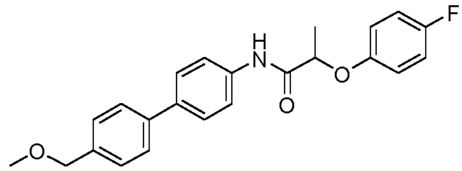 |
| (7) | 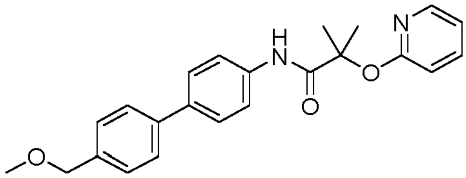 | (8) | 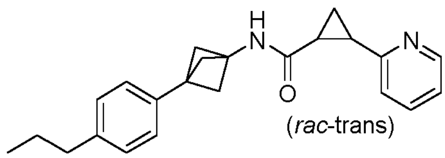 (*rac*-trans) |
| (9) | 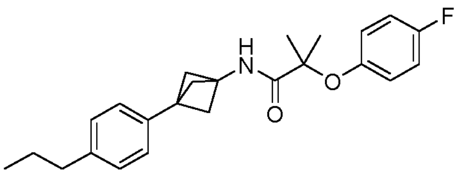 | (10) | 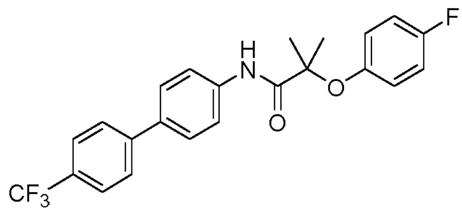 |
| (11) | 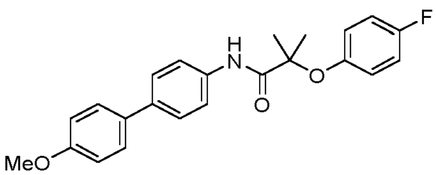 | (12) | 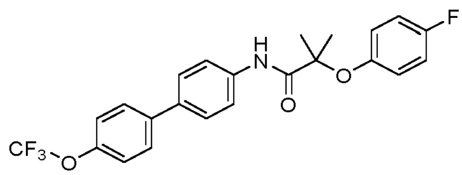 |
| (13) | 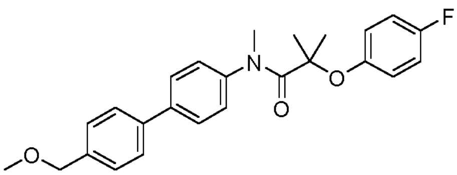 | (14) | 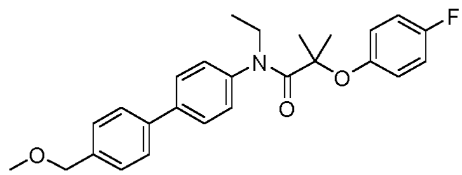 |
| (15) | 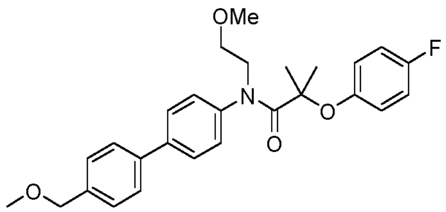 | (16) | 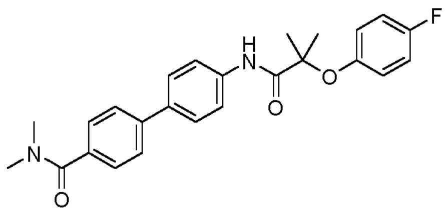 |

(continued)

| | | | |
|---|---|---|---|
| (17) | 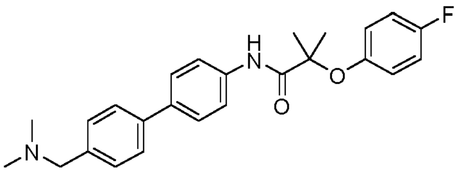 | (18) D1 | 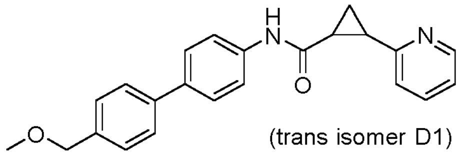 (trans isomer D1) |
| (19) D2 | 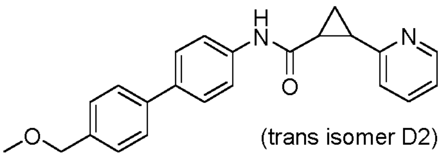 (trans isomer D2) | (20) | 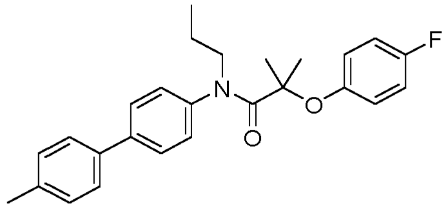 |
| (21) | 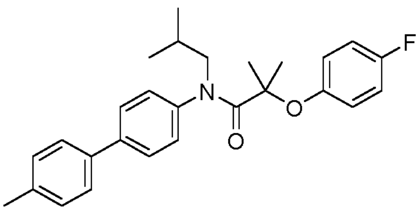 | (22) | 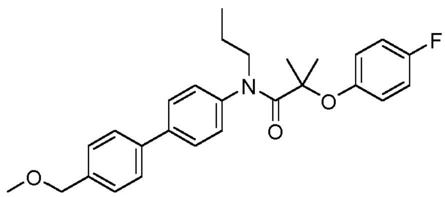 |
| (23) | 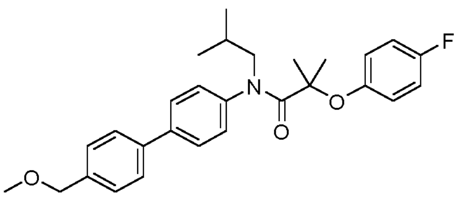 | (24) | 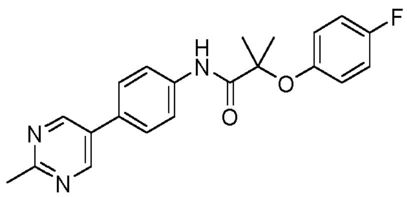 |
| (25) | 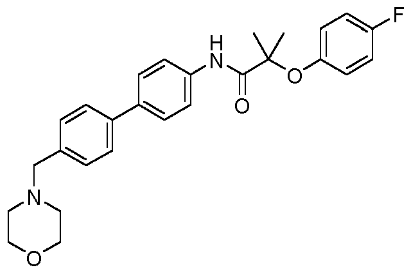 | (26) | 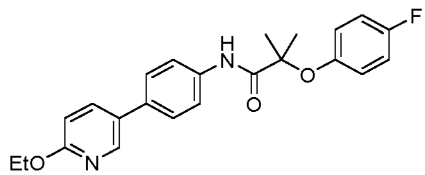 |
| (27) | 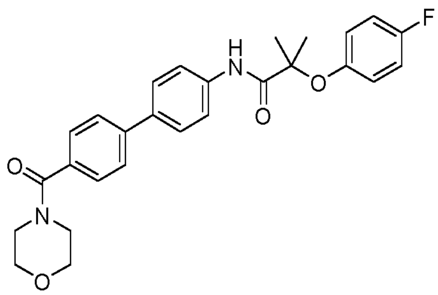 | (28) | 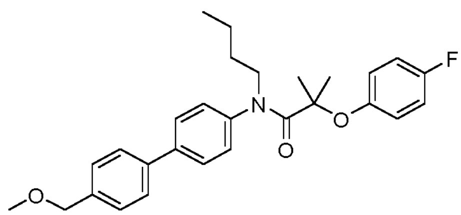 |
| (29) | 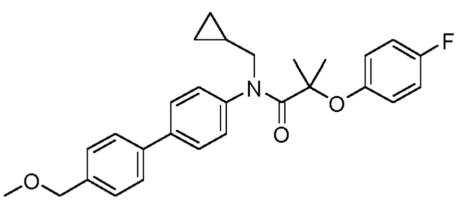 | (30) | 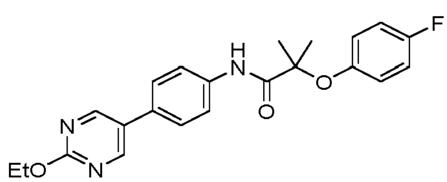 |

(continued)

| | | | |
|---|---|---|---|
| (31) | 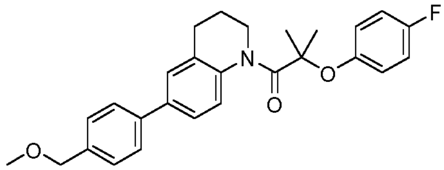 | (32) | 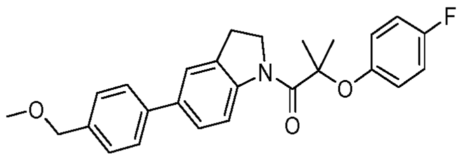 |
| (33) | 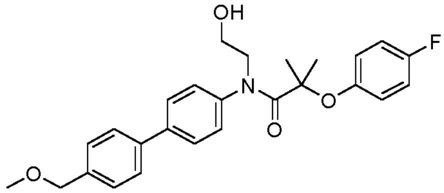 | (34) D1 | 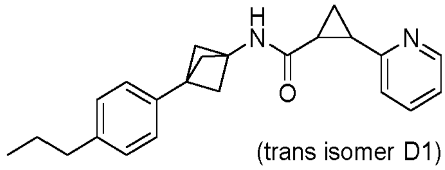 (trans isomer D1) |
| (35) D2 | 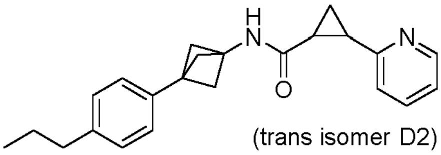 (trans isomer D2) | (36) | 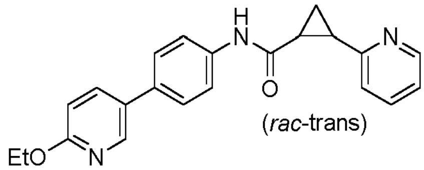 (*rac*-trans) |
| (37) | 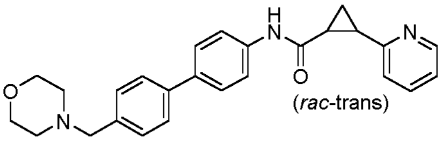 (*rac*-trans) | (38) | 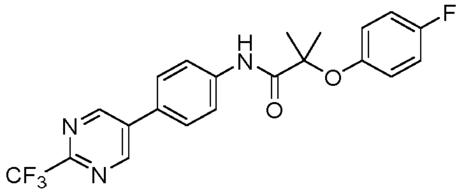 |
| (39) | 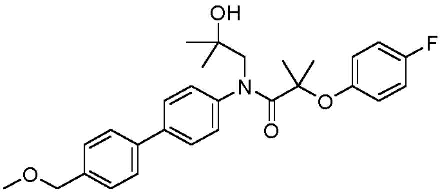 | (40) | 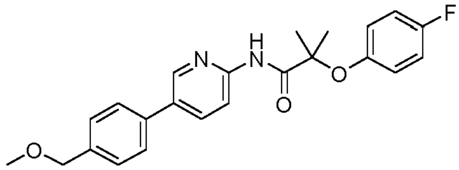 |
| (41) | 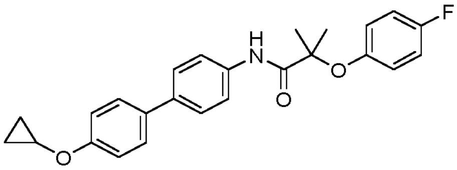 | (42) | 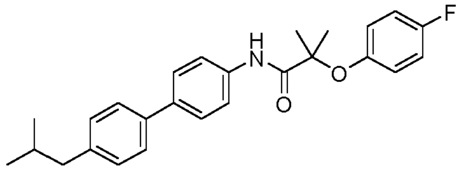 |
| (43) | 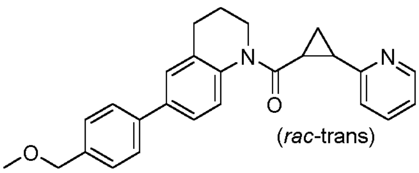 (*rac*-trans) | (44) | 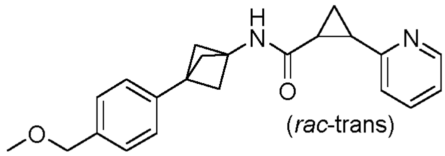 (*rac*-trans) |
| (45) | 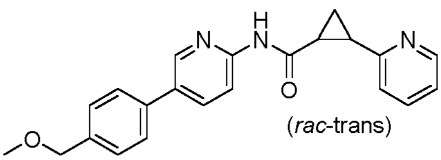 (*rac*-trans) | (46) | 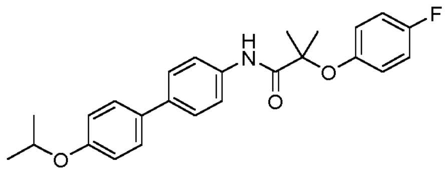 |

(continued)

| | | | |
|---|---|---|---|
| (47) | 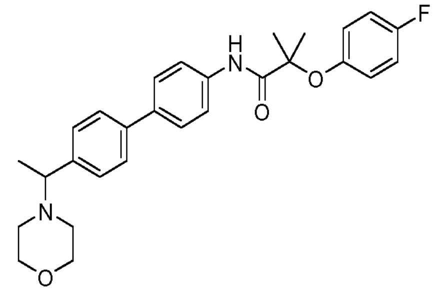 | (48) | 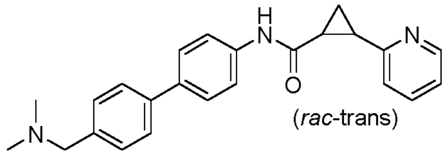 (*rac*-trans) |
| (49) | 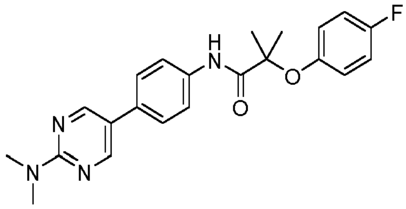 | (50) | 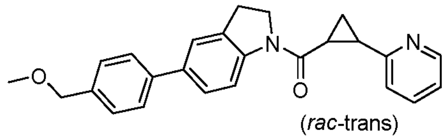 (*rac*-trans) |
| (51) | 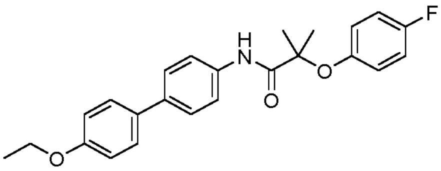 | (52) | 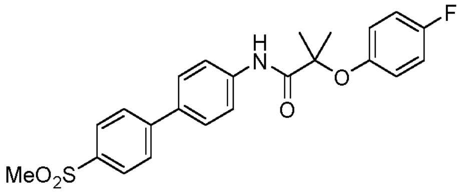 |
| (53) | 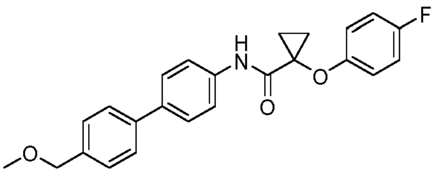 | (54) | 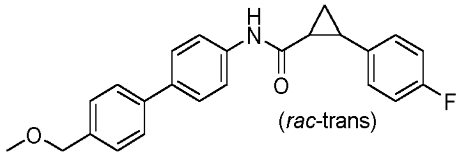 (*rac*-trans) |
| (55)* | 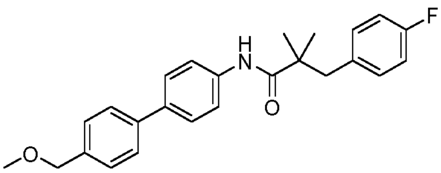 | (56)* | 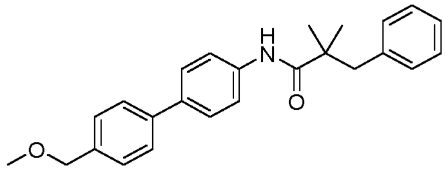 |
| (57) | 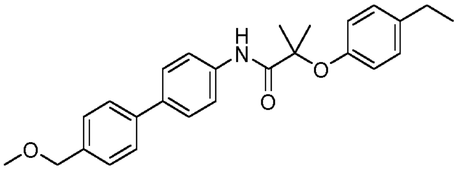 | (58) | 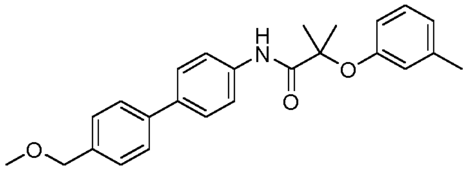 |
| (59) | 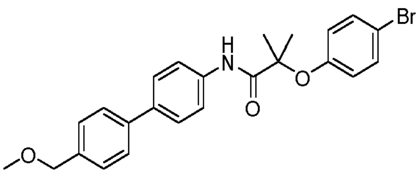 | (60) | 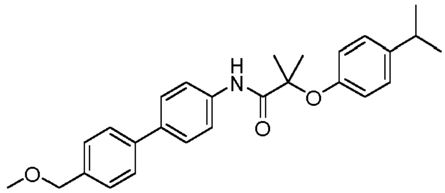 |
| (61) | 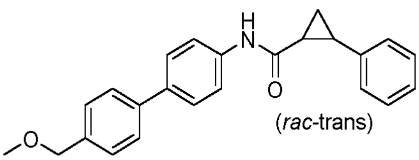 (*rac*-trans) | (62) | 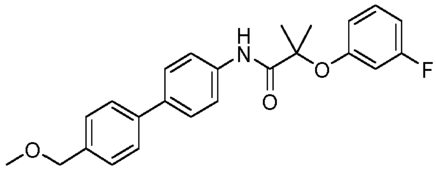 |

(continued)

| | | | |
|---|---|---|---|
| (63) | 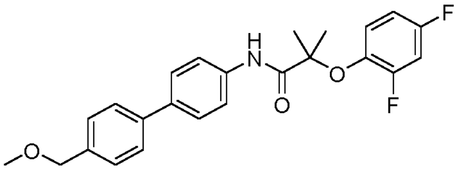 | (64) | 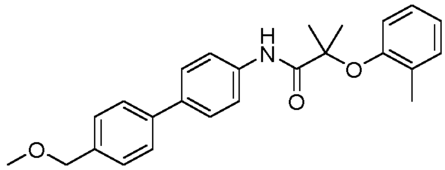 |
| (65) | 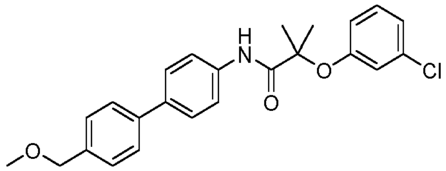 | (66) | 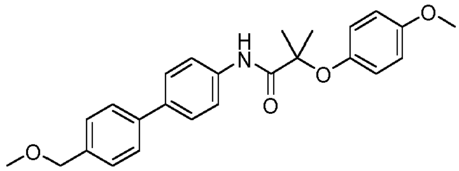 |
| (67) | 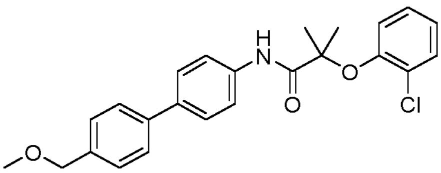 | (68) | 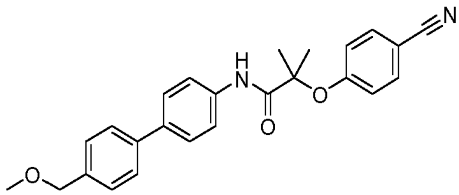 |
| (69) | 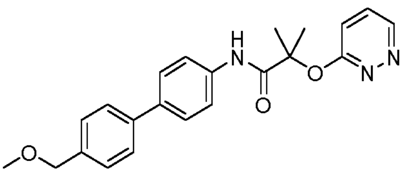 | (70) | 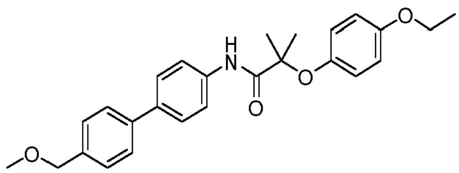 |
| (71) | 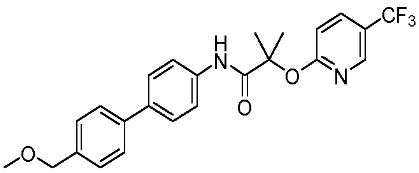 | (72) | 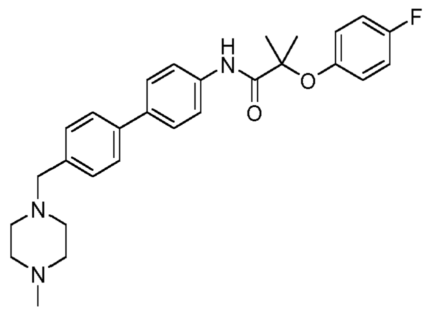 |
| (73) D1 | 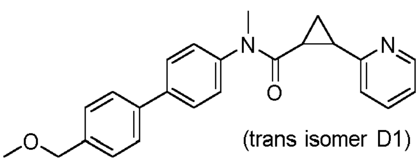 (trans isomer D1) | (74) D2 | 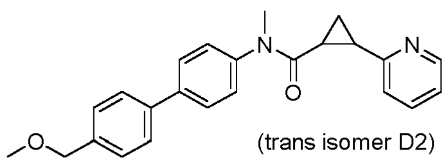 (trans isomer D2) |
| (75) D1 | 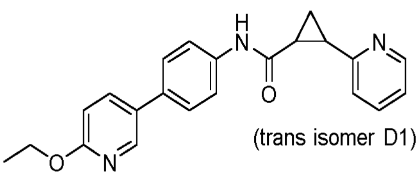 (trans isomer D1) | (76) D2 | 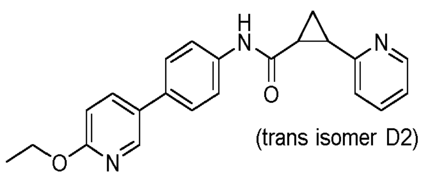 (trans isomer D2) |
| (77) D1 | 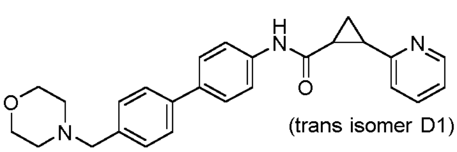 (trans isomer D1) | (78) D2 | 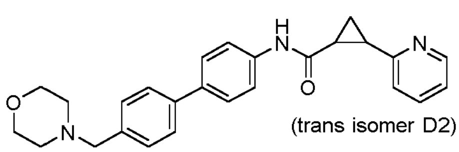 (trans isomer D2) |

(continued)

| | | | |
|---|---|---|---|
| (79) | 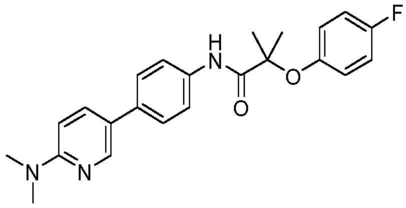 | (80) | 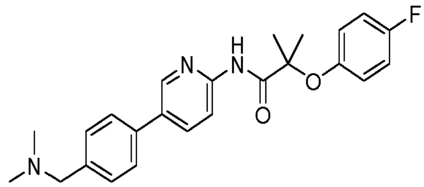 |
| (81) | 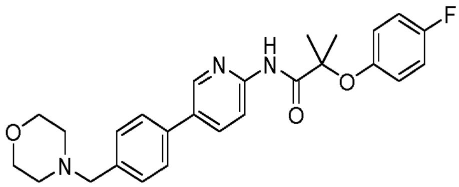 | (82) | 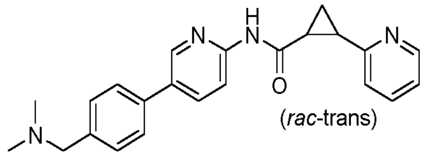 (*rac*-trans) |
| (83) | 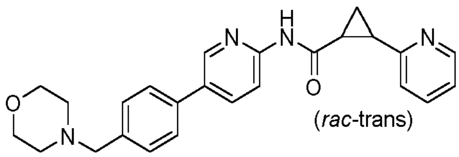 (*rac*-trans) | (84) | 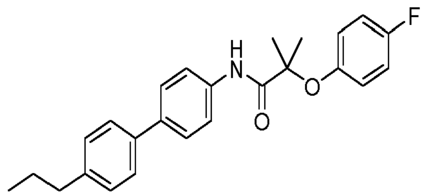 |
| (85) | 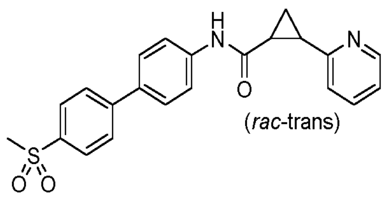 (*rac*-trans) | (86) | 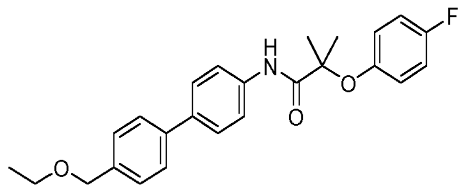 |
| (87) | 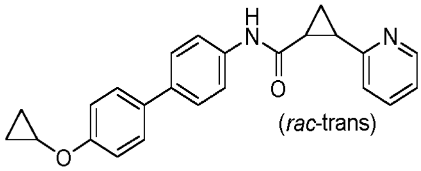 (*rac*-trans) | (88) | 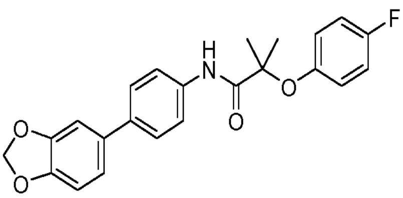 |
| (89) | 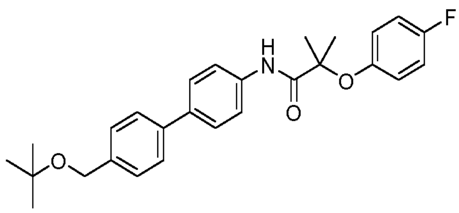 | (90) | 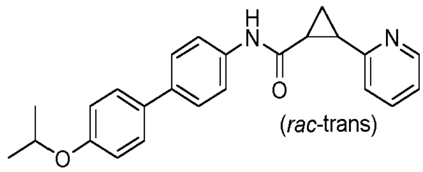 (*rac*-trans) |
| (91) | 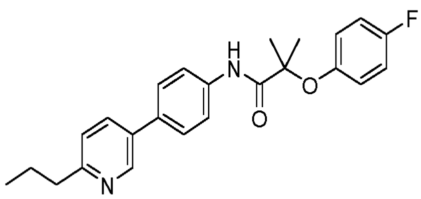 | (92) | 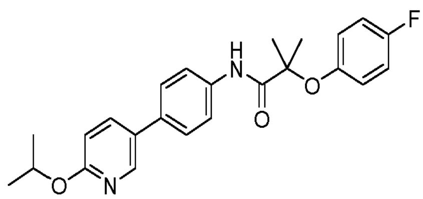 |
| (93) | 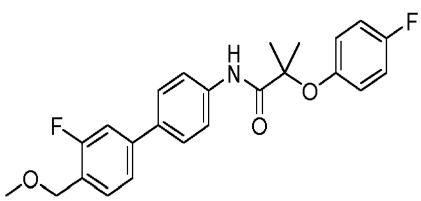 | (94) | 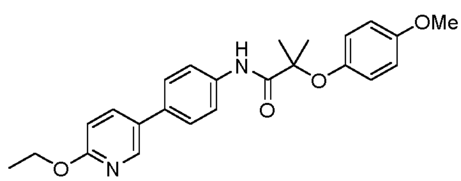 |

(continued)

| | | | |
|---|---|---|---|
| (95) | 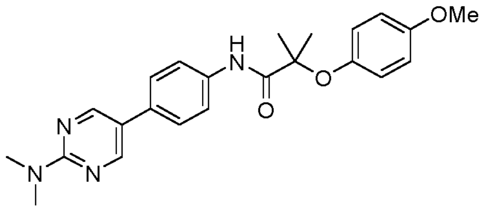 | (96) | 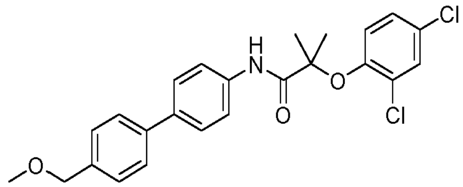 |
| (97) | 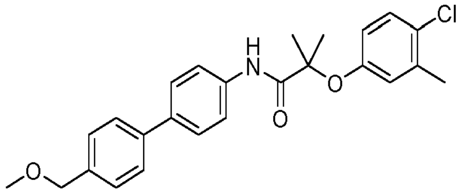 | (98) | 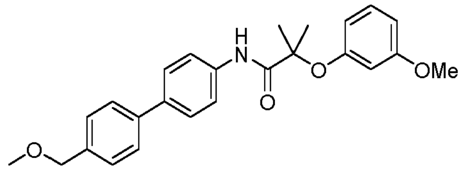 |
| (99) | 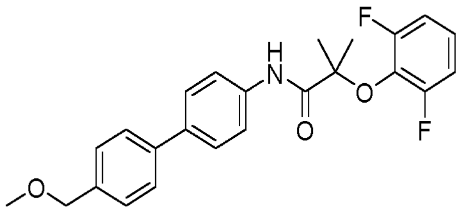 | (100) | 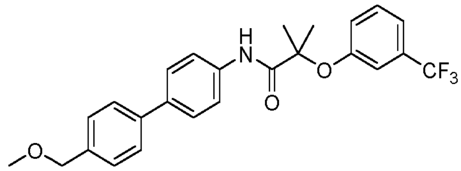 |
| (101) | 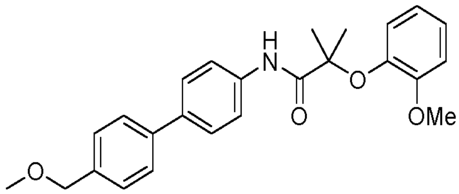 | (102) | 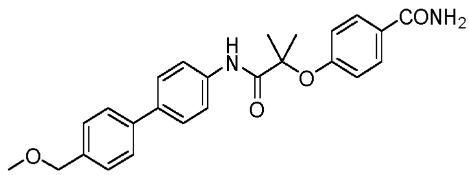 |
| (103) | 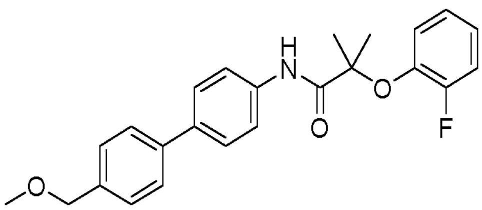 | (104) | 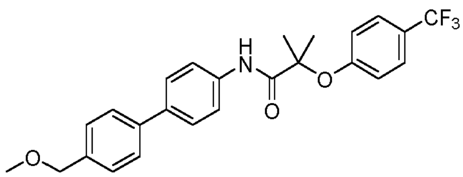 |
| (105) | 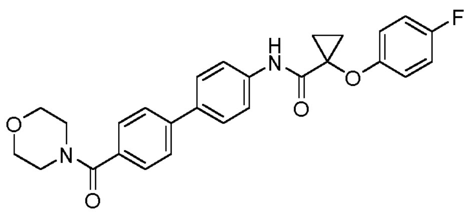 | (106) | 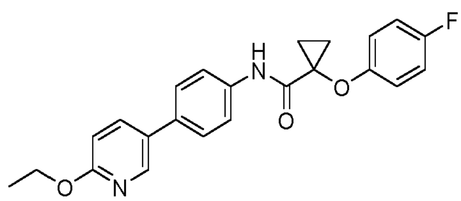 |
| (107) | 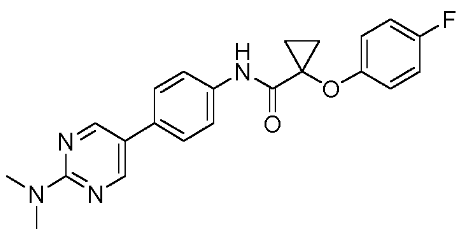 | (108) | 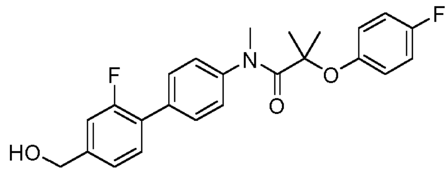 |

(continued)

| | | | |
|---|---|---|---|
| (109) | 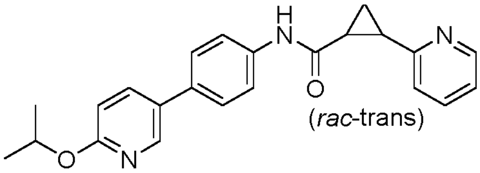 (*rac*-trans) | (110) D1 | 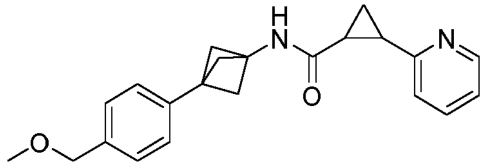 |
| (111) D2 | 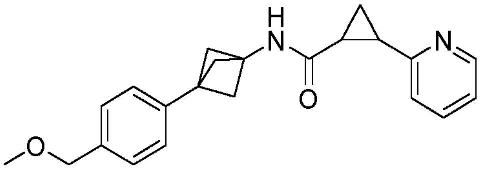 | (112) D3 | 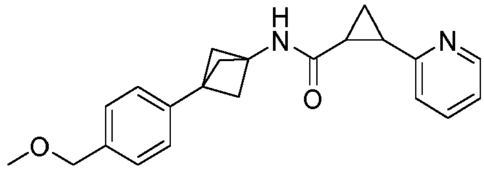 |
| (113) | 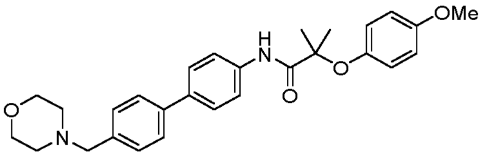 | (114) | 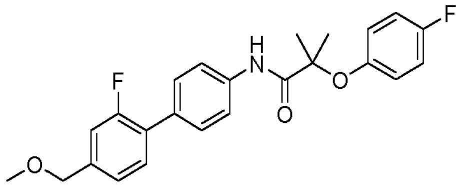 |
| (115) | 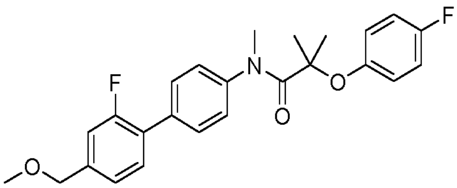 | (116) | 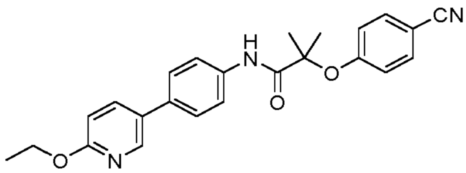 |
| (117) | 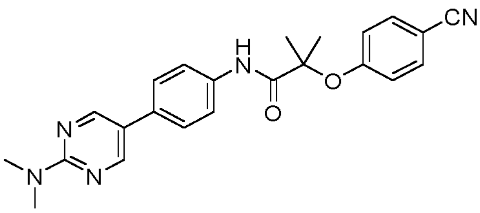 | (118) | 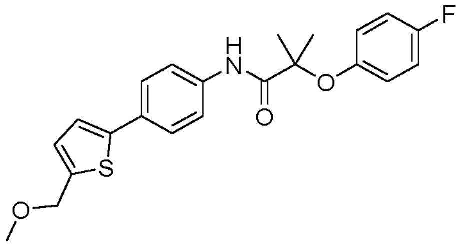 |
| (119) | 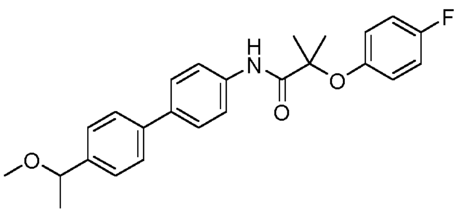 | (120) D1 | 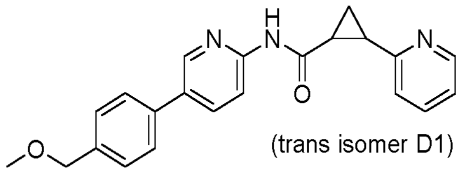 (trans isomer D1) |
| (121) D2 | 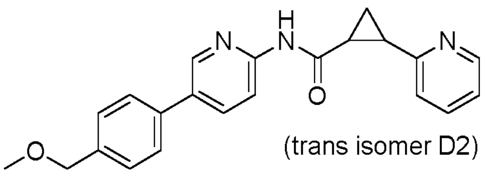 (trans isomer D2) | (122) E1 | 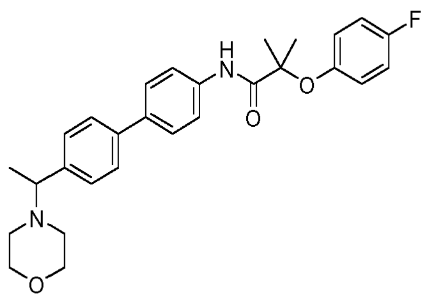 |

(continued)

| | | | |
|---|---|---|---|
| (123)<br>E2 | 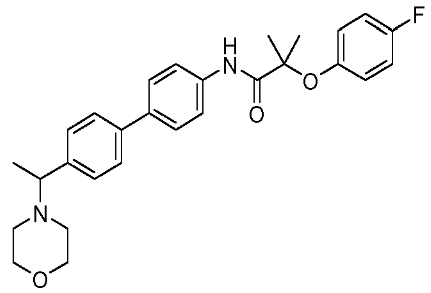 | (124) | 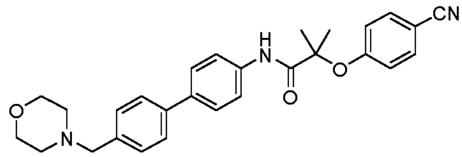 |
| (125) | 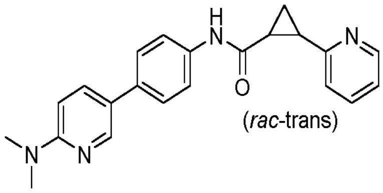<br>(*rac*-trans) | (126) | 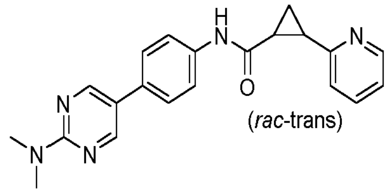<br>(*rac*-trans) |
| (127) | 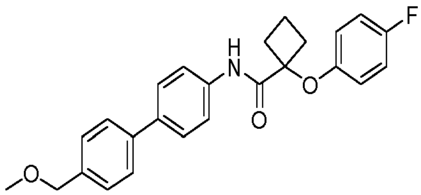 | (128) | 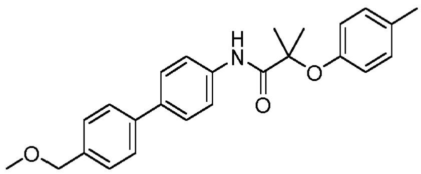 |
| (129) | 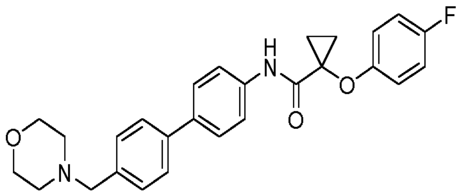 | (130)<br>D2 | 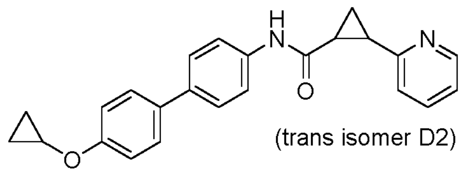<br>(trans isomer D2) |
| (131) | 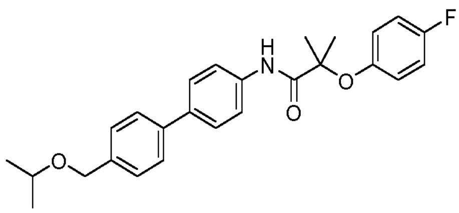 | (132) | 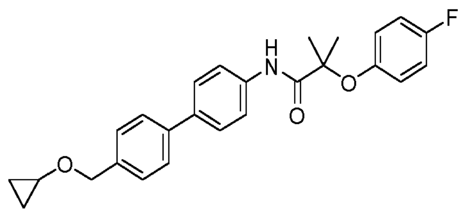 |
| (133)<br>D1 | 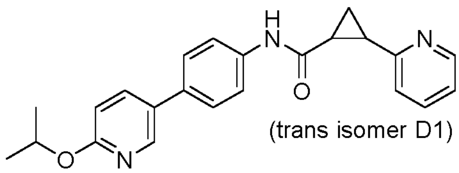<br>(trans isomer D1) | (134)<br>D2 | 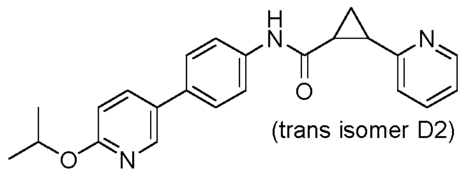<br>(trans isomer D2) |
| (135) | 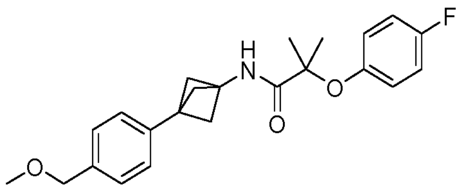 | (136) | 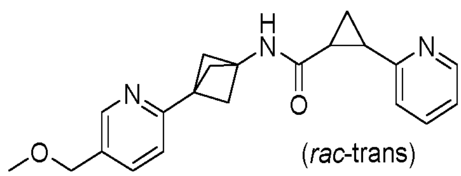<br>(*rac*-trans) |
| (137) | 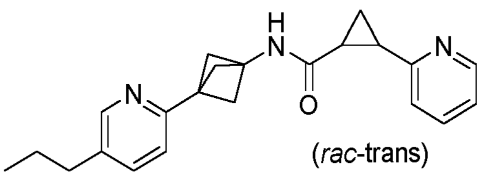<br>(*rac*-trans) | (138) | 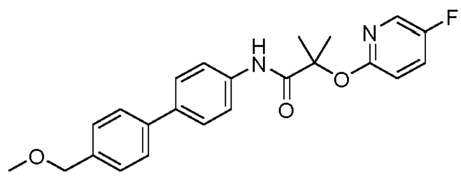 |

(continued)

| | | | |
|---|---|---|---|
| (139) | 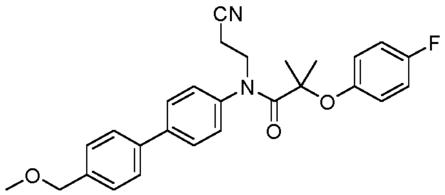 | (140) | 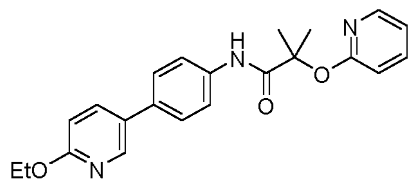 |
| (141)<br>D1 | 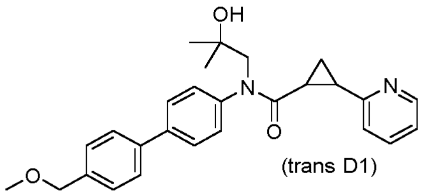<br>(trans D1) | (142)<br>D2 | 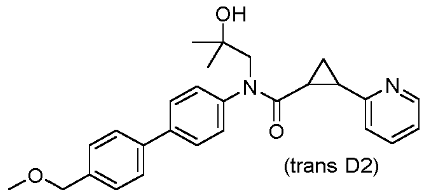<br>(trans D2) |

or a pharmaceutically acceptable salt thereof.

**15.** The compound of claim 1 or 14, wherein wherein the compound is selected from:

| | | | |
|---|---|---|---|
| (1) | 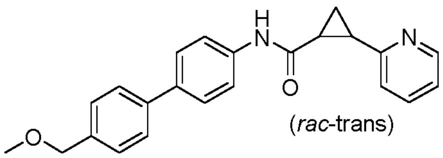<br>(*rac*-trans) | (2) | 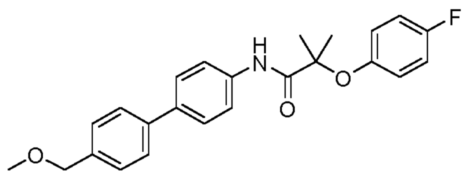 |
| (3) | 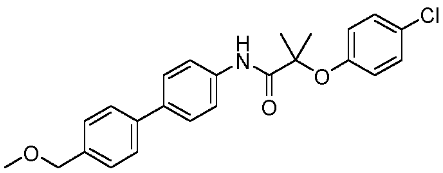 | (4) | 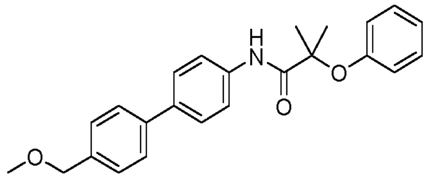 |
| (7) | 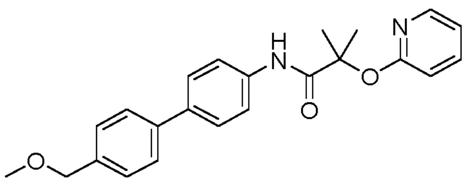 | (8) | 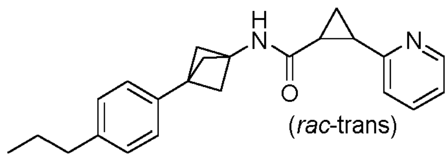<br>(*rac*-trans) |
| (9) | 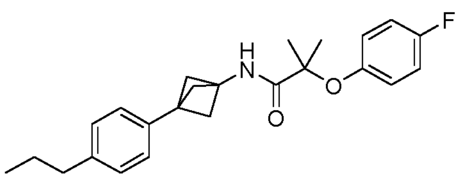 | (10) | 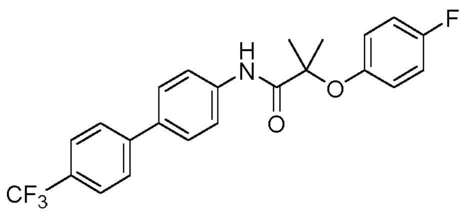 |
| (11) | 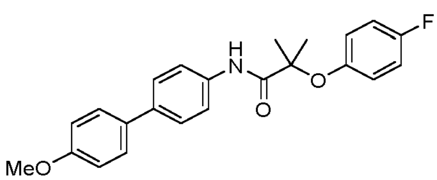 | (12) | 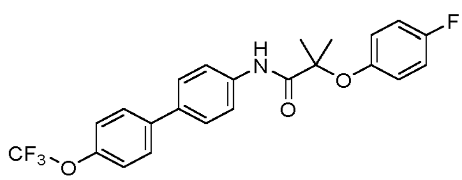 |

(continued)

| | | | |
|---|---|---|---|
| (13) | 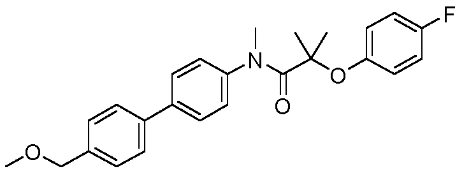 | (14) | 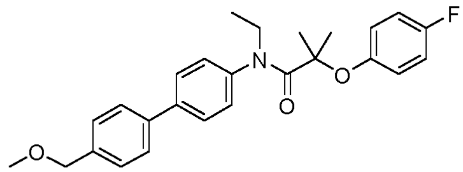 |
| (15) | 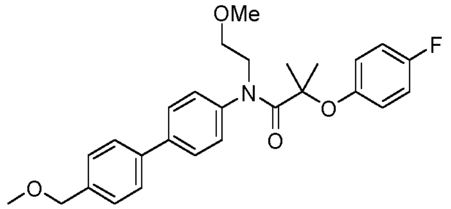 | (17) | 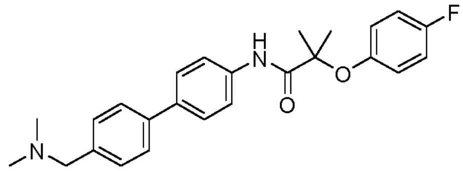 |
| (18)<br>D1 | 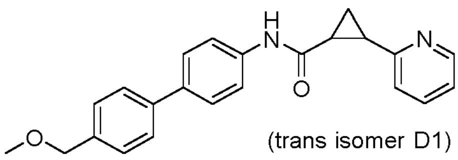 (trans isomer D1) | (22) | 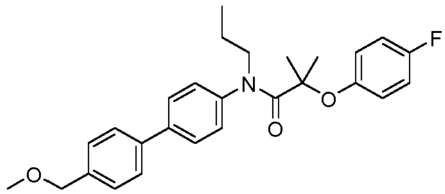 |
| (25) | 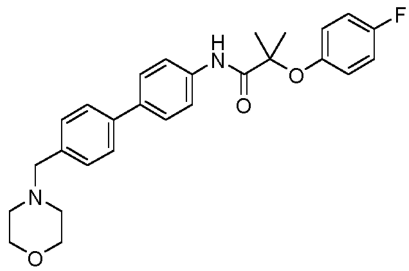 | (26) | 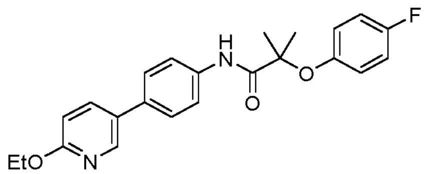 |
| (27) | 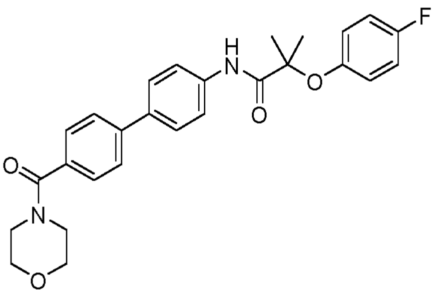 | (28) | 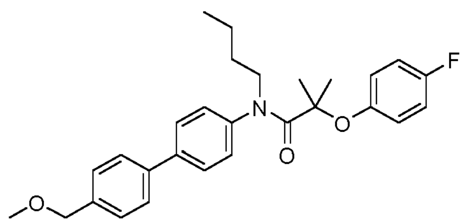 |
| (29) | 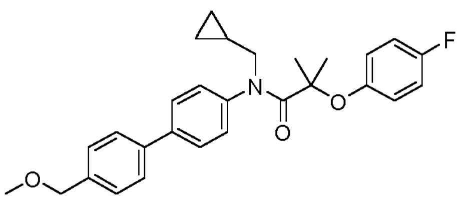 | (30) | 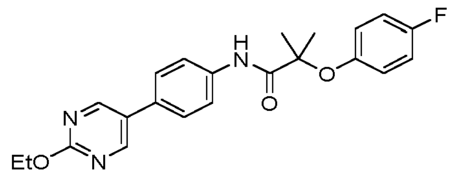 |
| (31) | 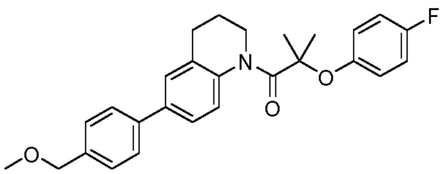 | (32) | 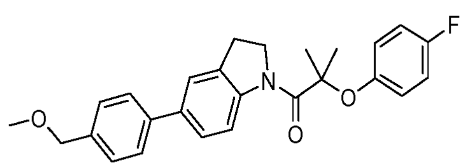 |

(continued)

| | | | |
|---|---|---|---|
| (33) | 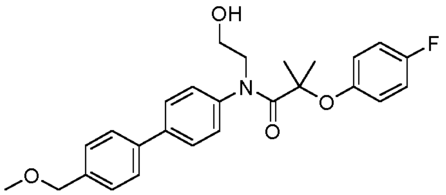 | (34)<br>D1 | 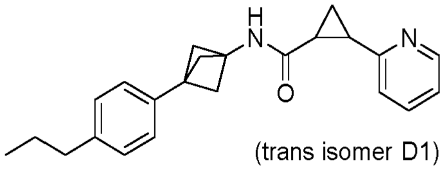 (trans isomer D1) |
| (35)<br>D2 | 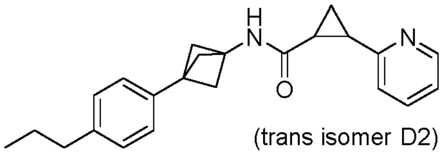 (trans isomer D2) | (36) | 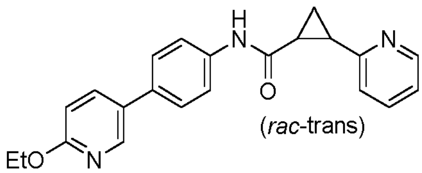 (*rac*-trans) |
| (37) | 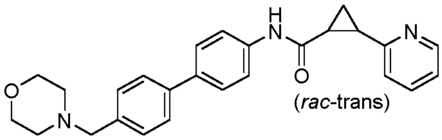 (*rac*-trans) | (39) | 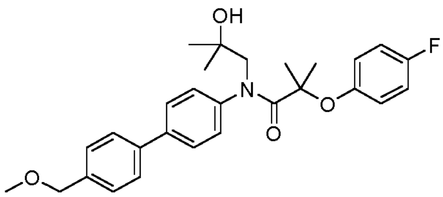 |
| (40) | 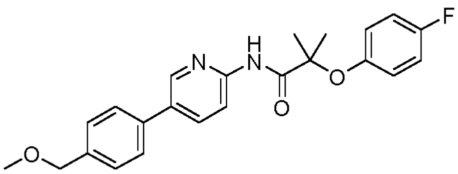 | (44) | 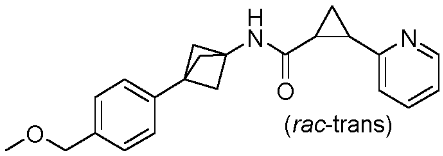 (*rac*-trans) |
| (45) | 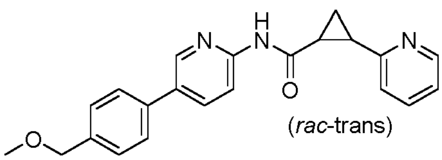 (*rac*-trans) | (46) | 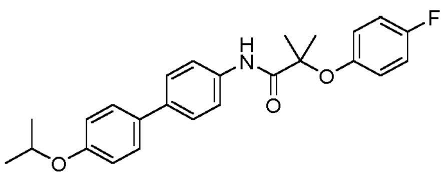 |
| (47) | 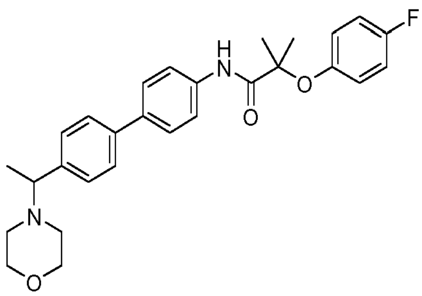 | (49) | 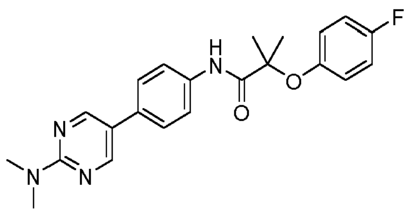 |
| (50) | 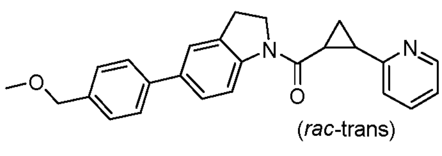 (*rac*-trans) | (53) | 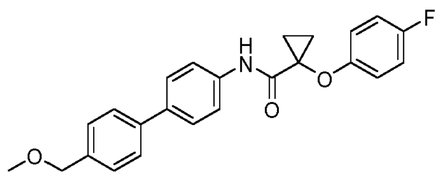 |
| (55)* | 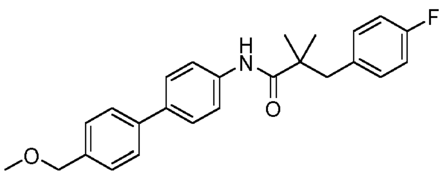 | (56)* | 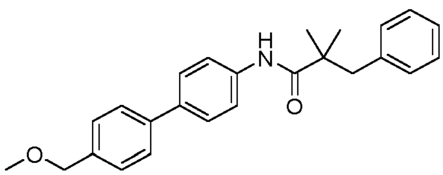 |

(continued)

| | | | |
|---|---|---|---|
| (57) | 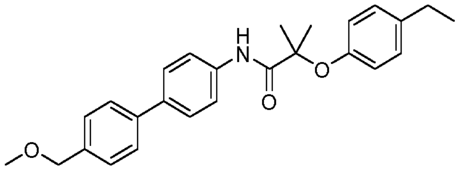 | (58) | 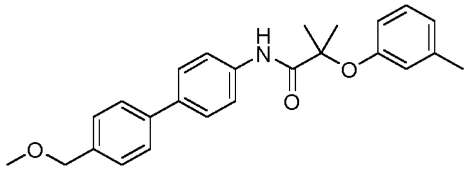 |
| (59) | 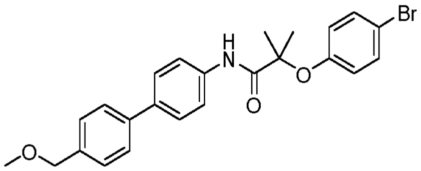 | (60) | 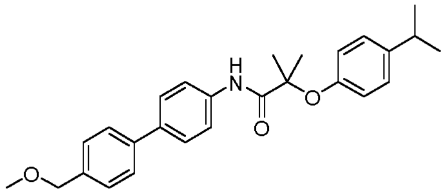 |
| (62) | 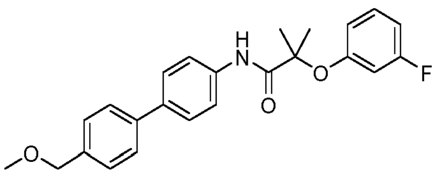 | (63) | 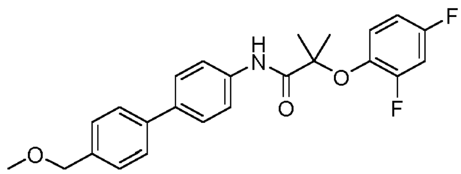 |
| (64) | 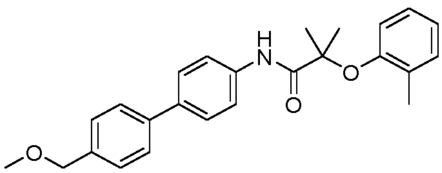 | | |
| (65) | 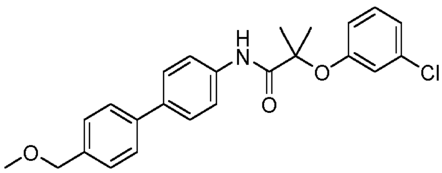 | (66) | 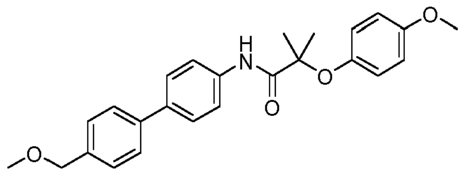 |
| (67) | 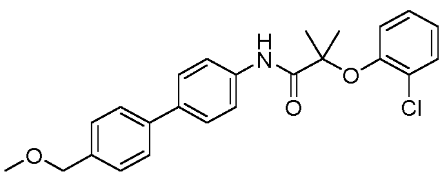 | (68) | 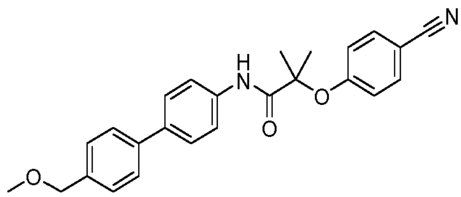 |
| (70) | 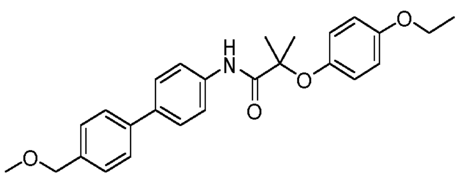 | (72) | 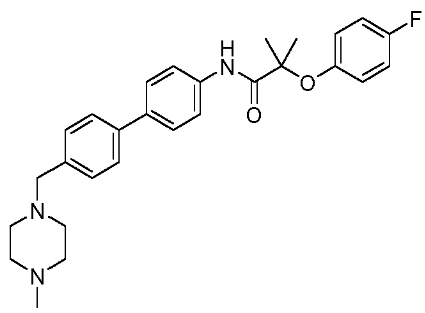 |
| (75) D1 | 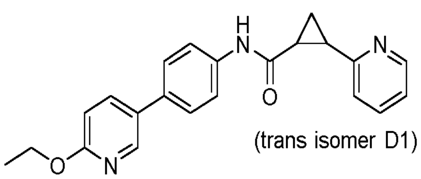 (trans isomer D1) | (76) D2 | 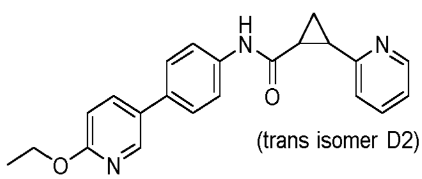 (trans isomer D2) |

(continued)

| | | | |
|---|---|---|---|
| (77)<br>D1 | 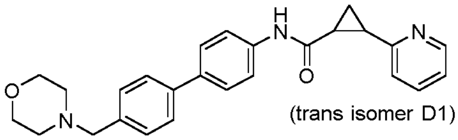<br>(trans isomer D1) | (78)<br>D2 | 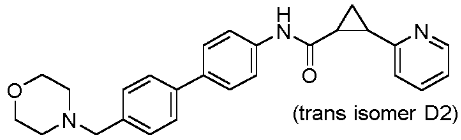<br>(trans isomer D2) |
| (79) | 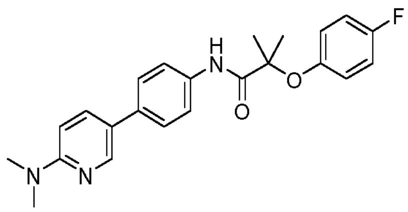 | (80) | 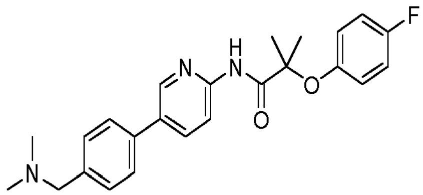 |
| (81) | 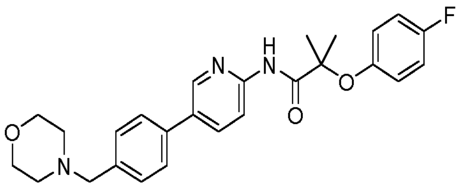 | (83) | 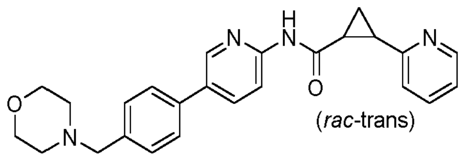<br>(*rac*-trans) |
| (85) | 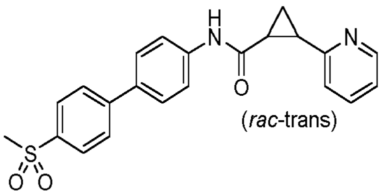<br>(*rac*-trans) | (86) | 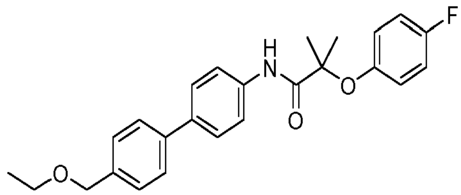 |
| (87) | 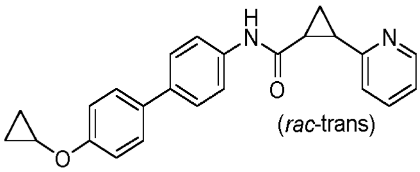<br>(*rac*-trans) | (88) | 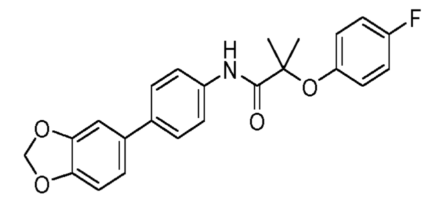 |
| (89) | 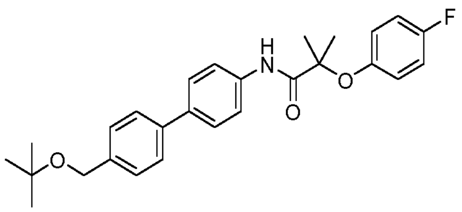 | (90) | 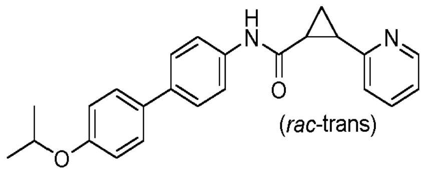<br>(*rac*-trans) |
| (91) | 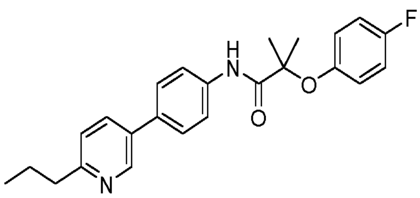 | (92) | 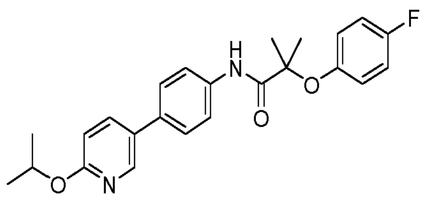 |
| (94) | 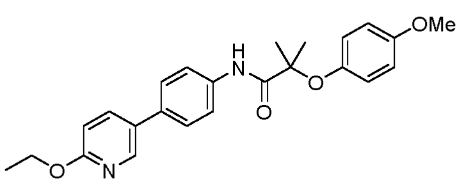 | (95) | 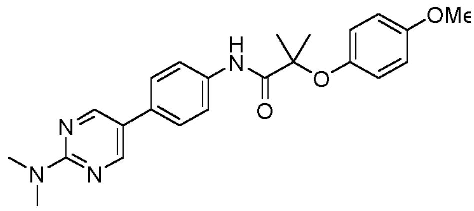 |

(continued)

| | | | |
|---|---|---|---|
| (97) | 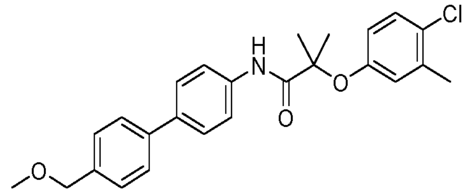 | (98) | 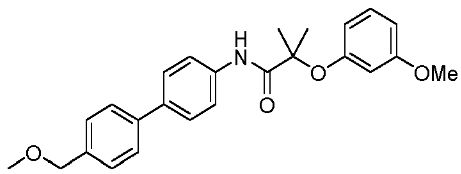 |
| (99) | 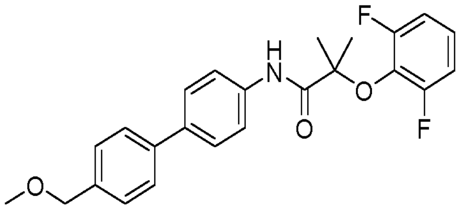 | (100) | 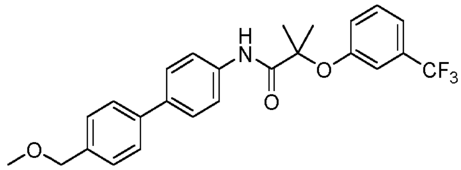 |
| (101) | 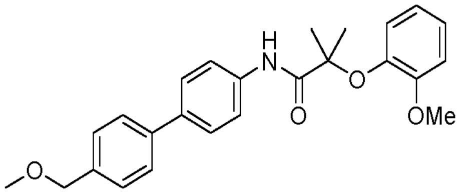 | (102) | 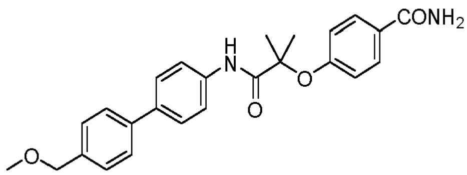 |
| (103) | 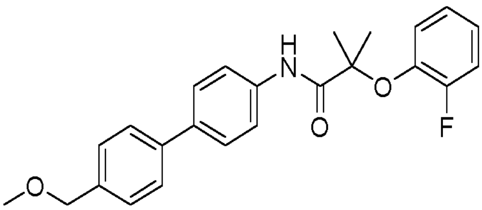 | (104) | 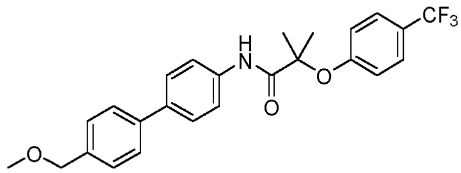 |
| (105) | 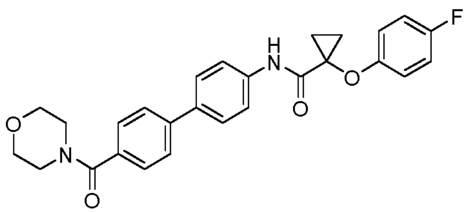 | (106) | 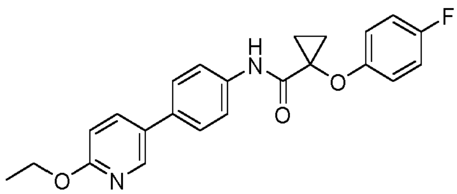 |
| (107) | 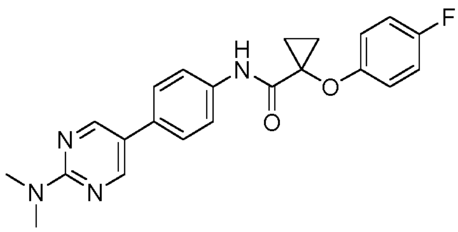 | (108) | 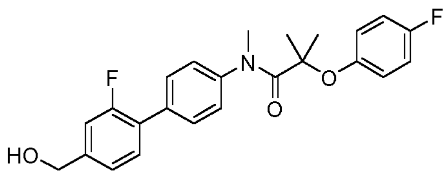 |
| (109) | 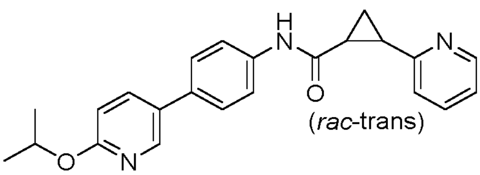 (*rac*-trans) | (110)<br>D1 | 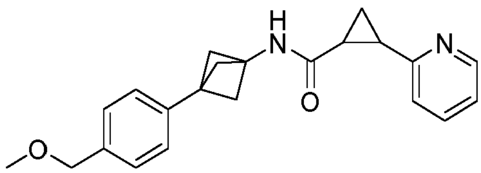 |

(continued)

| | | | |
|---|---|---|---|
| (111) D2 | 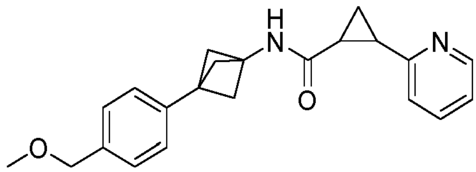 | | |
| (113) | 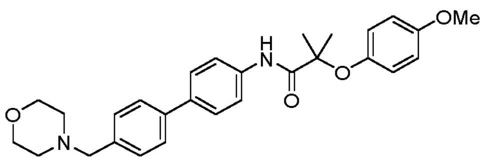 | (114) | 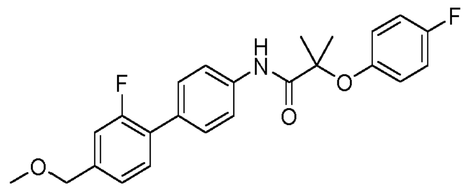 |
| (115) | 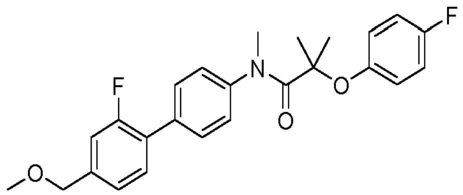 | (116) | 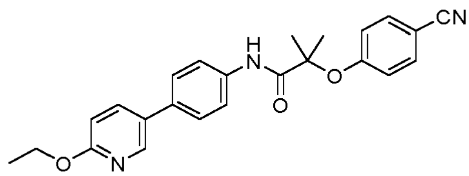 |
| (117) | 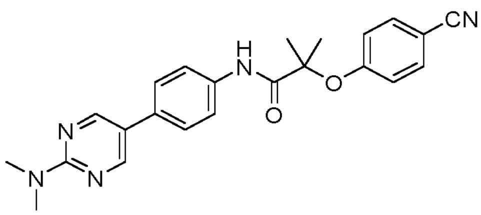 | | |
| (119) | 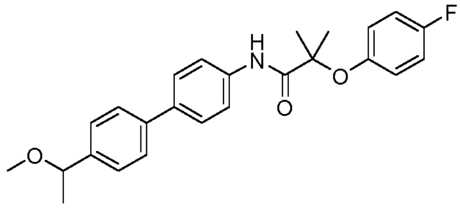 | (120) D1 | 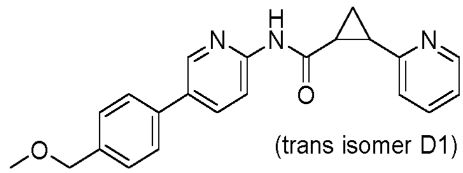 (trans isomer D1) |
| (121) D2 | 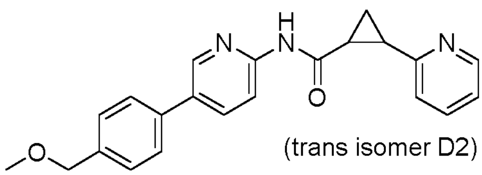 (trans isomer D2) | (122) E1 | 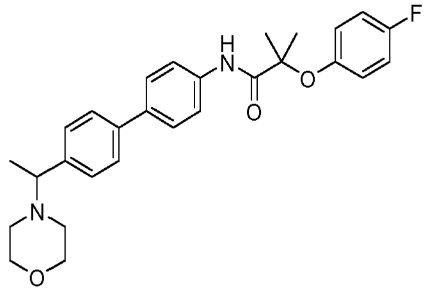 |
| (123) E2 | 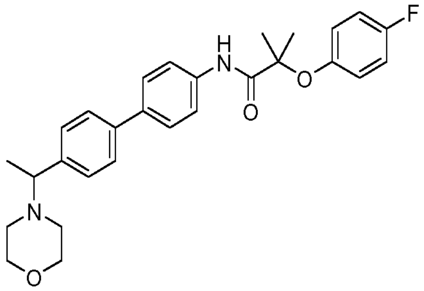 | (124) | 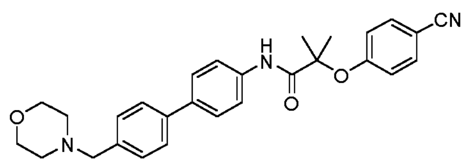 |

(continued)

| | | | |
|---|---|---|---|
| (127) | 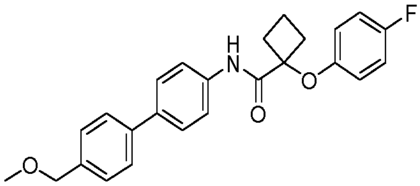 | (128) | 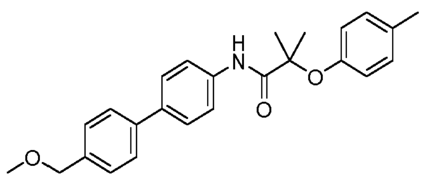 |
| (129) | 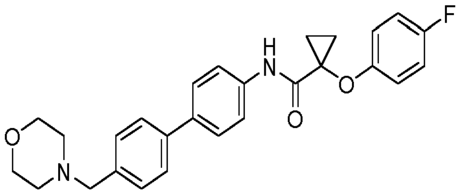 | (132) | 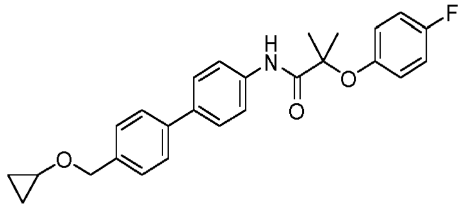 |
| (133) D1 | 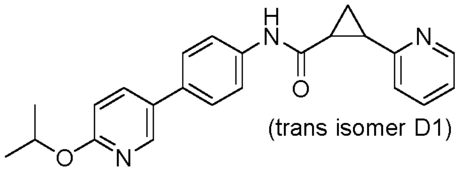 (trans isomer D1) | | |
| (135) | 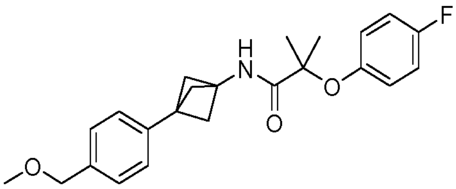 | (136) | 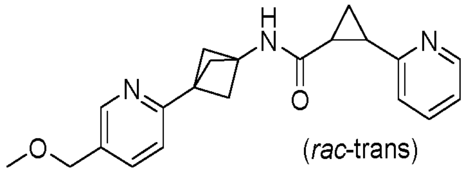 (*rac*-trans) |
| (137) | 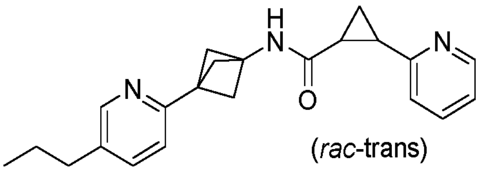 (*rac*-trans) | (138) | 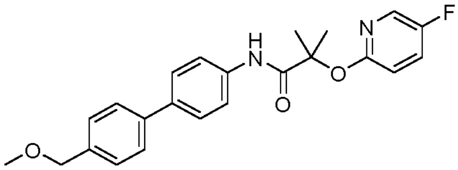 |
| (140) | 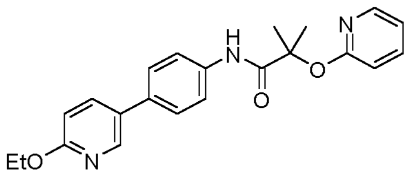 | | |

or a pharmaceutically acceptable salt thereof.

**16.** A pharmaceutical formulation comprising a compound of any of claims 1 to 15, or a pharmaceutically acceptable salt thereof, and a pharmaceutically acceptable excipient.

**17.** A compound of any of claims 1 to 15, or a pharmaceutically acceptable salt thereof, for use as a medicament; or for use in the treatment of a disease mediated by GPR88; or for use in the treatment of Tourette's Syndrome, Huntington's Disease (HD), Addiction, Parkinson's Disease (PD), Schizophrenia, and Attention Deficit Hyperactivity Disorder (ADHD), choreiform movements, speech delay, learning disabilities, depression, hyperkinetic movement disorders **characterised by** chorea and/or dystonia, psychosis, cognitive deficits in schizophrenia, affective disorders, bipolar disorder, Alzheimer's disease and basal ganglia disorders.

## Patentansprüche

**1.** Eine Verbindung der Formel (I) oder einem pharmazeutisch-verträglichen Salz davon:

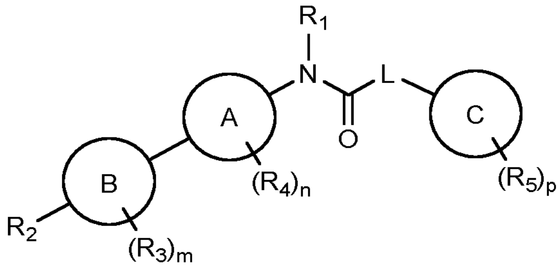

(I)

wobei:

i) -L- ausgewählt ist aus der Gruppe bestehend aus

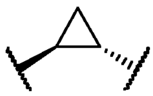,
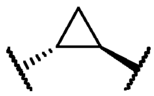,

und

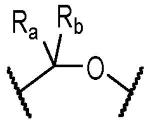;

Ring A ausgewählt ist aus der Gruppe bestehend aus: einem 6-gliedrigen Cycloalkyl, einem 5-gliedrigen Cycloalkylringsystem, einem 8-gliedrigen Cycloalkylringsystem, einem 6-gliedrigen Cycloalkenyl, einem 6-gliedrigen aromatischen Ringsystem, und einem 6-gliedrigen heteroaromatischen Ringsystem;
Ring B ausgewählt ist aus der Gruppe bestehend aus: einem 6-gliedrigen Cycloalkyl, einem 6-gliedrigen aromatischen und einem 6-gliedrigen heteroaromatischen Ring;
Ring C ausgewählt ist aus der Gruppe bestehend aus: einem 5-6 gliedrigen aromatischen und einem 5-6 gliedrigen heteroaromatischen Ring;
m 0, 1 oder 2 ist;
n 0, 1 oder 2 ist;
p 0, 1 oder 2 ist;
$R_1$ ausgewählt ist aus der Gruppe bestehend aus: Wasserstoff, substituiertem oder unsubstituiertem $C_1$-$C_4$-Alkyl, substituiertem oder unsubstituiertem $C_3$-$C_4$ Cycloalkyl und substituiertem oder unsubstituiertem $C_3$-$C_4$ Cycloalkyl $C_1$-$C_2$ Alkyl; wobei jeder Substituent unabhängig ausgewählt ist aus der Gruppe bestehend aus -$OR_c$, Nitril oder Halo; wobei jedes $R_c$ unabhängig ausgewählt ist aus der Gruppe bestehend aus H, Me, Et und Cyclopropyl;
$R_2$ ausgewählt ist aus der Gruppe bestehend aus: substituiertem oder unsubstituiertem $C_1$-$C_6$ Alkyl, substituiertem oder unsubstituiertem $C_2$-$C_6$ Alkenyl, substituiertem oder unsubstituiertem $C_2$-$C_6$ Alkinyl; substituiertem oder unsubstituiertem $C_3$-$C_6$ Cycloalkyl, substituiertem oder unsubstituiertem $C_1$-$C_6$ Alkoxy, substituiertem oder unsubstituiertem $C_3$-$C_6$ Cycloalkoxy; substituiertem oder unsubstituiertem $C_1$-$C_6$ Alkoxy-$C_1$-$C_6$ Alkyl; substituiertem oder unsubstituiertem $C_3$-$C_6$ Cycloalkoxy-$C_1$-$C_6$ Alkyl; substituiertem oder unsubstituiertem $C_1$-$C_6$ Haloalkyl, substituiertem oder unsubstituiertem $C_2$-$C_6$ Haloalkenyl, substituiertem oder unsubstituiertem $C_2$-$C_6$ Haloalkinyl; substituiertem oder unsubstituiertem $C_3$-$C_6$ Cyclohaloalkyl; substituiertem oder unsubstituiertem $C_1$-$C_6$ Haloalkoxy, substituiertem oder unsubstituiertem $C_3$-$C_6$ Cyclohaloalkoxy; substituiertem oder unsubstituiertem $C_1$-$C_6$ Haloalkoxy-$C_1$-$C_6$ Alkyl, substituiertem oder unsubstituiertem $C_3$-$C_6$ Cyclohaloalkoxy-$C_1$-$C_6$ Alkyl, substituiertem oder unsubstituiertem $C_1$-$C_6$ Alkoxy-$C_1$-$C_6$ Haloalkyl, substituiertem oder unsubstituiertem $C_3$-$C_6$ Cycloalkoxy-$C_1$-$C_6$ Haloalkyl, substituiertem oder unsubstituiertem $C_1$-$C_6$ Haloalkoxy-$C_1$-$C_6$ Haloalkyl, substituiertem oder unsubstituiertem $C_3$-$C_6$ Cycloha-

loalkoxy-$C_1$-$C_6$ Haloalkyl, -$SO_2R_d$, und -$NR_eR_e$;
wobei $R_d$ $C_{1-4}$ Alkyl ist;
wobei jedes $R_e$ unabhängig ausgewählt ist aus der Gruppe bestehend aus H und $C_{1-4}$ Alkyl;
wobei jeder Substituent unabhängig ausgewählt ist aus der Gruppe bestehend aus =O, -$NR_eR_e$, und einem substituierten oder unsubstituierten monozyklischen, bizyklischen oder verbrückten 3-8-gliedrigen Cycloalkyl- oder Heterocycloalkylring, wobei jedes $R_e$ ausgewählt ist aus der Gruppe bestehend aus H und $C_{1-4}$ Alkyl;
wobei jeder Substituent des monozyklischen, bizyklischen oder verbrückten 3-8-gliedrigen Cycloalkyl- oder Heterocycloalkylrings unabhängig ausgewählt ist aus der Gruppe bestehend aus -OH, Nitril, Halo, Methyl oder Methoxy;
jedes $R_3$ unabhängig ausgewählt ist aus der Gruppe bestehend aus: Halogen, $C_1$-$C_3$ Alkyl, $C_1$-$C_3$ Haloalkyl, $C_1$-$C_3$ Alkoxy, $C_1$-$C_3$ Haloalkoxy oder -CN;
jedes $R_4$ unabhängig ausgewählt ist aus der Gruppe bestehend aus: Halogen, $C_1$-$C_3$ Alkyl, $C_1$-$C_3$ Haloalkyl, $C_1$-$C_3$ Alkoxy, $C_1$-$C_3$ Haloalkoxy oder -CN;
jedes $R_5$ unabhängig ausgewählt ist aus der Gruppe bestehend aus: Halogen, $C_1$-$C_3$ Alkyl, $C_1$-$C_3$ Haloalkyl, $C_1$-$C_3$ Alkoxy, $C_1$-$C_3$ Haloalkoxy, -CN, oder -C(O)$NR_eR_e$;
$R_a$ und $R_b$ jeweils unabhängig ausgewählt sind aus der Gruppe bestehend aus: Wasserstoff, Halogen, $C_1$-$C_6$ Alkyl, und $C_1$-$C_6$ Alkoxy; oder wobei $R_a$ und $R_b$, zusammen mit dem Atom, an das sie gebunden sind, einen 3-gliedrigen bis 6-gliedrigen Cycloalkyl- oder Heterocycloalkylring bilden, umfassend -O- oder -$NR_f$-, wobei $R_f$ H oder Methyl ist;
vorausgesetzt, dass die Verbindung nicht:

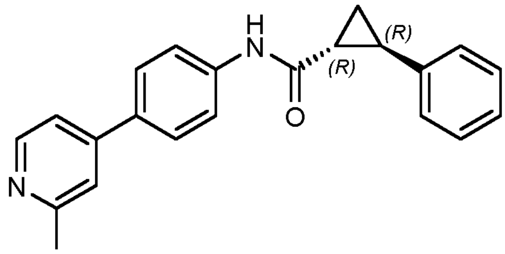

ist
oder

ii) -L-, Ring A, Ring B, Ring C, m, p, $R_2$, $R_3$, $R_5$, $R_a$, $R_b$, $R_c$, $R_d$, $R_e$ und $R_f$ wie oben unter i) definiert sind,

$R_1$ ein $C_1$ oder $C_2$ Alkylenylrest ist,
n 1 oder 2 ist, und
eine $R_4$ Einheit ein $C_1$ Alkylenylrest ist und, wenn vorliegend, die andere $R_4$ Einheit wie oben unter i) definiert ist,
wobei die $C_1$ Alkylenyl $R_4$ Einheit an ein Kohlenstoffatom in Ring A gebunden ist, das ortho zu dem Kohlenstoffatom von Ring A liegt, das einen Amidrest aufweist, und, wobei die $R_1$ und $R_4$ Einheiten zusammen mit den Atomen, an die sie gebunden sind, einen 5- oder 6-gliedrigen Ring bilden;

und/oder
iii) -L-, Ring A, Ring B, Ring C, n, p, $R_1$, $R_4$, $R_5$, $R_a$, $R_b$, $R_c$ und $R_f$ wie oben unter i) definiert sind,

m 1 oder 2 ist;
$R_2$ ausgewählt ist aus der Gruppe bestehend aus: substituiertem oder unsubstituiertem -O-$C_{1-2}$ Alkylenyl, wobei das Sauerstoffatom mit Ring B verbunden ist;
wobei jeder Substituent unabhängig ausgewählt ist aus der Gruppe bestehend aus -OH, Nitril, Halo, Methyl oder Methoxy;
eine $R_3$ Einheit ein -O- Rest ist und, wenn vorliegend, die andere $R_3$ Einheit wie oben unter i) definiert ist;
wobei die $R_2$ Einheit und die $R_3$ Einheit, die ein -O- Rest ist, zusammen mit den Atomen, an die sie gebunden sind, einen 5- oder 6-gliedrigen Ring bilden.

**2.** Die Verbindung gemäß Anspruch 1, wobei $R_1$ ausgewählt ist aus der Gruppe bestehend aus: Wasserstoff, substituiertem oder unsubstituiertem $C_1$-$C_4$ Alkyl, substituiertem oder unsubstituiertem $C_3$-$C_4$ Cycloalkyl und sub-

stituiertem oder unsubstituiertem $C_3$-$C_4$ Cycloalkyl $C_1$-$C_2$ Alkyl; wobei jeder Substituent unabhängig ausgewählt ist aus -$OR_c$ und Nitril; und wobei jedes $R_c$ unabhängig ausgewählt ist aus der Gruppe bestehend aus H, Me, Et und Cyclopropyl.

**3.** Die Verbindung gemäß Anspruch 2, wobei

- $R_1$ ausgewählt ist aus der Gruppe bestehend aus: Wasserstoff, substituiertem oder unsubstituiertem $C_1$-$C_4$ Alkyl und substituiertem oder unsubstituiertem $C_3$-$C_4$ Cycloalkyl $C_1$-$C_2$ Alkyl; wobei jeder Substituent unabhängig ausgewählt ist aus -$OR_c$ und Nitril; und wobei jedes $R_c$ unabhängig ausgewählt ist aus der Gruppe bestehend aus H, Me, Et und Cyclopropyl; oder
- $R_1$ ausgewählt ist aus der Gruppe bestehend aus: Wasserstoff und substituiertem oder unsubstituiertem $C_1$-$C_4$ Alkyl und substituiertem oder unsubstituiertem $C_3$ Cycloalkyl $C_1$ Alkyl; wobei jeder Substituent ausgewählt ist aus -$OR_c$ und Nitril; und wobei jedes $R_c$ unabhängig ausgewählt ist aus der Gruppe bestehend aus H und Me, oder
- $R_1$ ausgewählt ist aus der Gruppe bestehend aus:

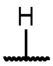 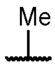 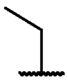 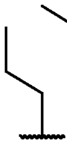 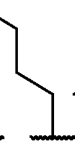 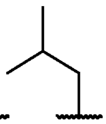 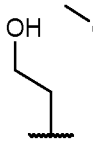 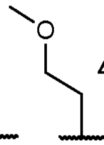 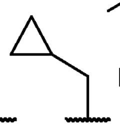 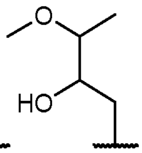 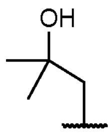 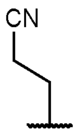 .

**4.** Die Verbindung gemäß einem der vorangegangenen Ansprüche, wobei

- n 1 ist; und/oder
- jedes $R_4$ unabhängig ausgewählt ist aus der Gruppe bestehend aus: Wasserstoff, $C_1$-$C_2$ Alkyl, und $C_1$-$C_2$ Alkoxy, wobei gegebenenfalls $R_4$ Methyl ist.

**5.** Die Verbindung gemäß einem der Ansprüche 1 bis 3, wobei n 0 ist.

**6.** Die Verbindung gemäß Anspruch 1, wobei $R_1$ ein $C_1$ oder $C_2$ Alkylenylrest ist und eine $R_4$ Einheit ein $C_1$ Alkylenylrest ist, wobei die $C_1$ Alkylenyl $R_4$ Einheit an ein Kohlenstoffatom in Ring A gebunden ist, das ortho zu dem Kohlenstoffatom von Ring A, das einen Amidrest aufweist, steht, und wobei die $R_1$ und $R_4$ Einheiten, zusammen mit den Atomen, an die sie gebunden sind, einen 5- oder 6-gliedrigen Ring bilden.

**7.** Die Verbindung gemäß einem der vorangegangenen Ansprüche, wobei $R_2$ ausgewählt ist aus der Gruppe bestehend aus: substituiertem oder unsubstituiertem $C_1$-$C_4$ Alkyl; substituiertem oder unsubstituiertem $C_3$-$C_4$ Cycloalkyl, substituiertem oder unsubstituiertem $C_1$-$C_4$ Alkoxy, substituiertem oder unsubstituiertem $C_3$-$C_4$ Cycloalkoxy; substituiertem oder unsubstituiertem $C_1$-$C_4$ Alkoxy-$C_1$-$C_4$ Alkyl; substituiertem oder unsubstituiertem $C_1$-$C_4$ Haloalkyl; substituiertem oder unsubstituiertem $C_1$-$C_4$ Haloalkoxy; $SO_2R_d$; und -$NR_eR_e$;
wobei gegebenenfalls:

- $R_2$ ausgewählt ist aus der Gruppe bestehend aus: substituiertem oder unsubstituiertem $C_1$-$C_4$ Alkyl; substituiertem oder unsubstituiertem $C_1$-$C_4$ Alkoxy, substituiertem oder unsubstituiertem $C_3$-$C_4$ Cycloalkoxy; substituiertem oder unsubstituiertem $C_1$-$C_4$ Alkoxy-$C_1$-$C_4$ Alkyl; substituiertem oder unsubstituiertem $C_1$-$C_4$ Haloalkyl; substituiertem oder unsubstituiertem $C_1$-$C_4$ Haloalkoxy; $SO_2R_d$; und -$NR_eR_e$; oder
- $R_2$ ausgewählt ist aus der Gruppe bestehend aus:

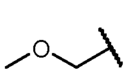 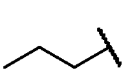 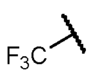 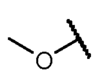 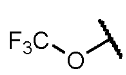

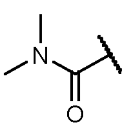 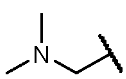 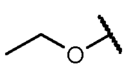 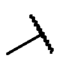 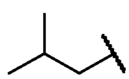

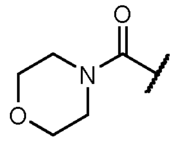 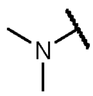 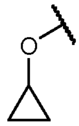 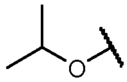 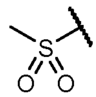

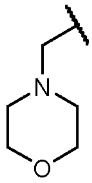 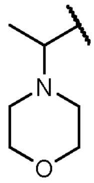 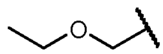 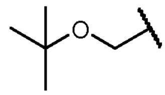 .

**8.** Die Verbindung gemäß einem der vorangegangenen Ansprüche, wobei

- m 1 ist; und/oder
- $R_3$ unabhängig ausgewählt ist aus der Gruppe bestehend aus: Halogen, $C_1$-$C_2$ Alkyl, und $C_1$-$C_2$ Alkoxy.

**9.** Die Verbindung gemäß einem der Ansprüche 1 bis 7, wobei m 0 ist.

**10.** Die Verbindung gemäß einem der Ansprüche 1 bis 6, wobei $R_2$ ein substituierter oder unsubstituierter -O-$C_1$ Alkylenylrest ist, wobei das Sauerstoffatom mit Ring B verbunden ist; und wobei eine $R_3$ Einheit ein -O- Rest ist, wobei die -O-$C_1$-Alkylenyl- und -O- Reste zusammen mit den Atomen, an die sie gebunden sind, einen 5-gliedrigen Ring bilden.

**11.** Die Verbindung gemäß einem der vorangegangenen Ansprüche, wobei

- p 0 ist; oder
- p 1 ist; und/oder
- jedes $R_5$ unabhängig ausgewählt ist aus der Gruppe bestehend aus: Halogen, $C_1$-$C_3$ Alkyl, $C_1$-$C_3$ Haloalkyl, $C_1$-$C_3$ Alkoxy, -CN oder -C(O)$NR_eR_e$.

**12.** Die Verbindung gemäß einem der vorangegangenen Ansprüche, wobei

- Ring A ausgewählt ist aus der Gruppe bestehend aus: einem 6-gliedrigen aromatischen und heteroaromatischen Ring, gegebenenfalls wobei Ring A Phenyl ist; oder
- Ring A ein 5- oder 8-gliedriges Cycloalkylringsystem ist, gegebenenfalls wobei Ring A Bicyclo-[1.1.1]-Pentyl ist.

**13.** Die Verbindung gemäß einem der vorangegangenen Ansprüche, wobei

- Ring B ausgewählt ist aus der Gruppe bestehend aus: Phenyl, Pyridinyl oder Pyrimidinyl, und/oder
- Ring C ausgewählt ist aus der Gruppe bestehend aus: einem 6-gliedrigen aromatischen und einem 6-gliedrigen heteroaromatischen Ring, gegebenenfalls Phenyl oder Pyridinyl; und/oder
- $R_a$ und $R_b$ jeweils unabhängig ausgewählt sind aus der Gruppe bestehend aus: Wasserstoff, Halogen, $C_1$-$C_4$ Alkyl, und $C_1$-$C_4$ Alkoxy; oder wobei $R_a$ und $R_b$, zusammen mit dem Atom, an das sie gebunden sind, einen 3-gliedrigen oder 4-gliedrigen Cycloalkyl- oder Heterocycloalkylring, umfassend -O- oder -$NR_f$-, wobei $R_f$ H oder Methyl ist, bilden, wobei gegebenenfalls $R_a$ und $R_b$ jeweils unabhängig ausgewählt sind aus der Gruppe bestehend aus Wasserstoff und Methyl.

**14.** Die Verbindung gemäß Anspruch 1, wobei die Verbindung ausgewählt ist aus:

| | | | |
|---|---|---|---|
| (1) | 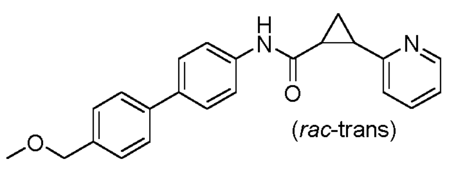 (*rac*-trans) | (2) | 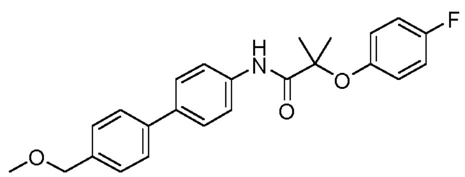 |

(continued)

| | | | |
|---|---|---|---|
| (3) | 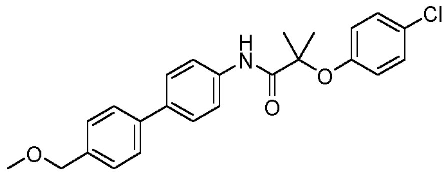 | (4) | 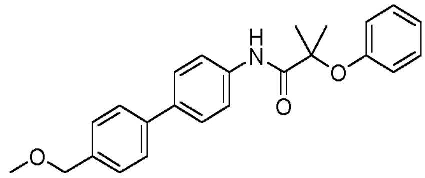 |
| (5) | 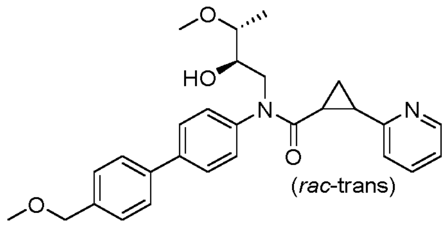 (*rac*-trans) | (6) | 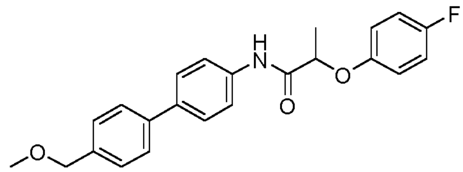 |
| (7) | 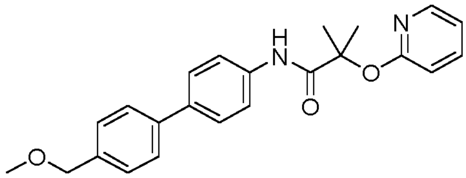 | (8) | 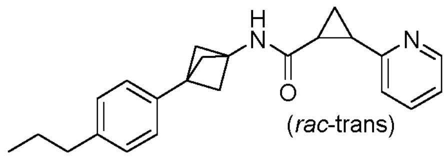 (*rac*-trans) |
| (9) | 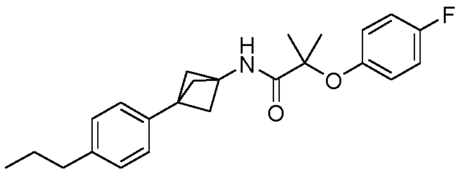 | (10) | 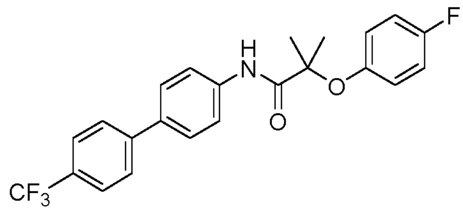 |
| (11) | 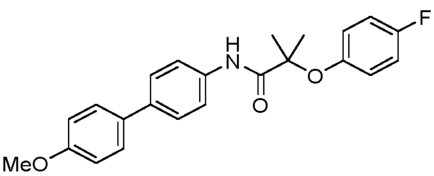 | (12) | 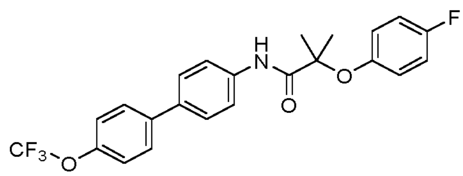 |
| (13) | 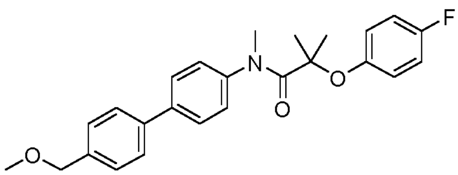 | (14) | 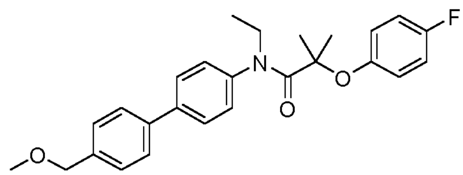 |
| (15) | 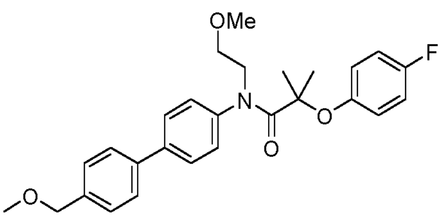 | (16) | 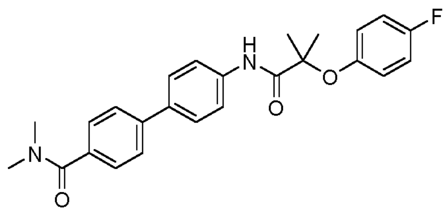 |
| (17) | 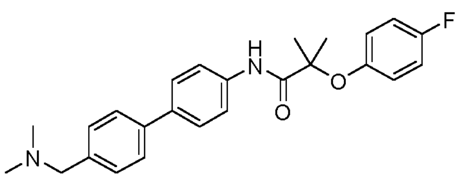 | (18) D1 | 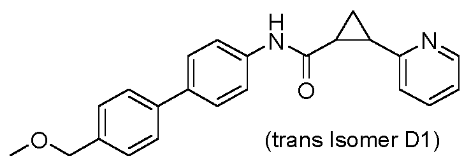 (trans Isomer D1) |

(continued)

| | | | |
|---|---|---|---|
| (19) D2 | 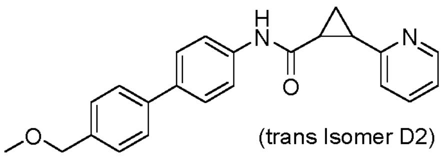 (trans Isomer D2) | (20) | 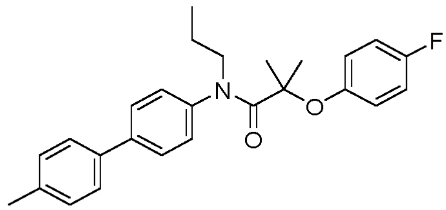 |
| (21) | 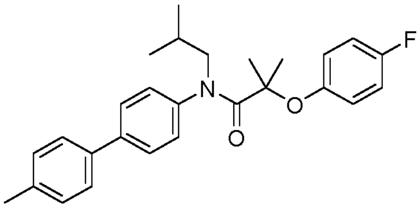 | (22) | 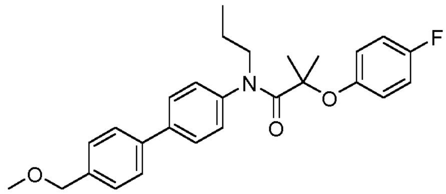 |
| (23) | 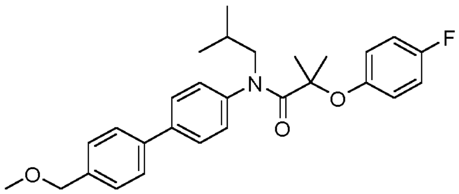 | (24) | 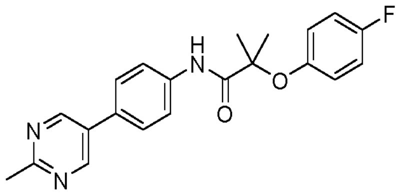 |
| (25) | 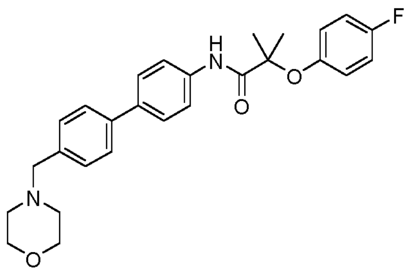 | (26) | 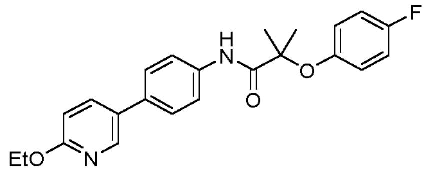 |
| (27) | 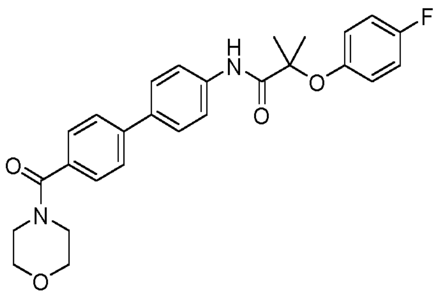 | (28) | 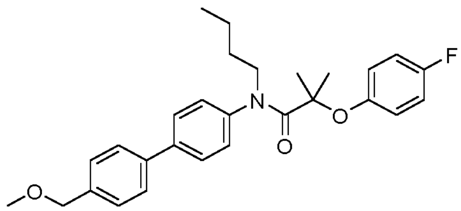 |
| (29) | 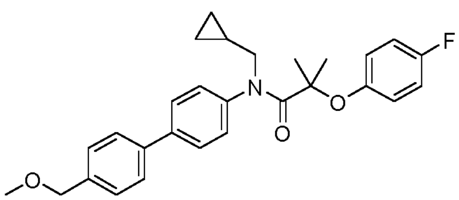 | (30) | 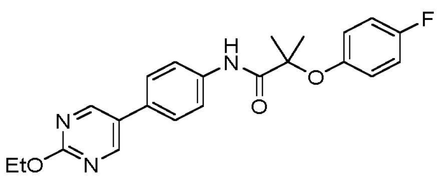 |
| (31) | 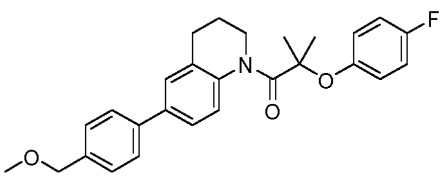 | (32) | 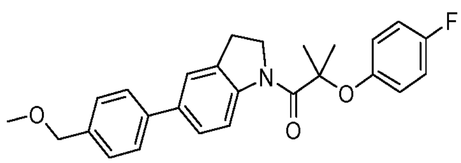 |

(continued)

| | | | |
|---|---|---|---|
| (33) | 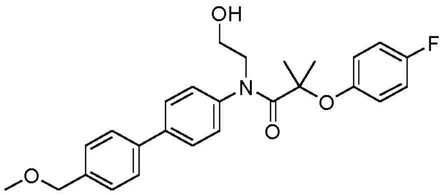 | (34) D1 | 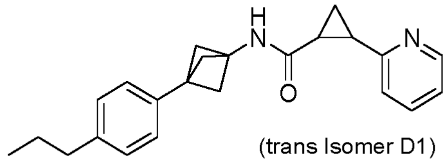 (trans Isomer D1) |
| (35) D2 | 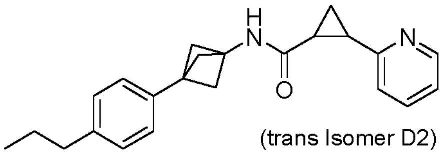 (trans Isomer D2) | (36) | 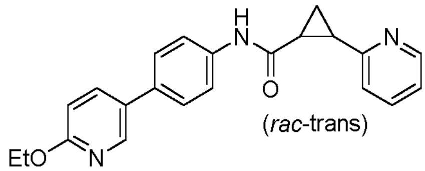 (*rac*-trans) |
| (37) | 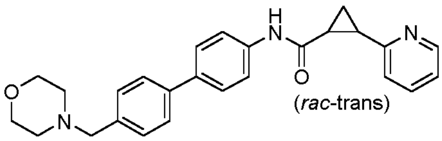 (*rac*-trans) | (38) | 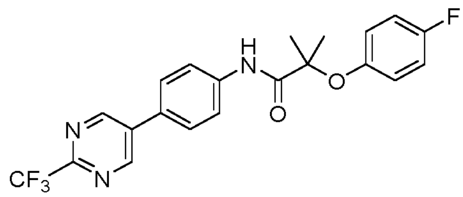 |
| (39) | 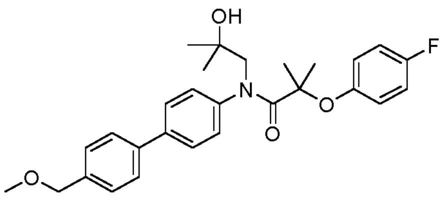 | (40) | 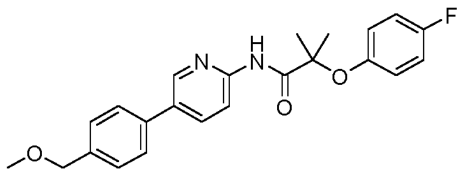 |
| (41) | 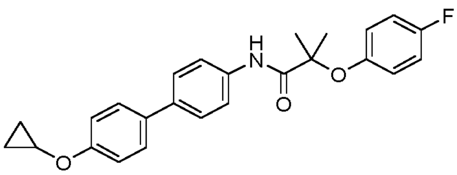 | (42) | 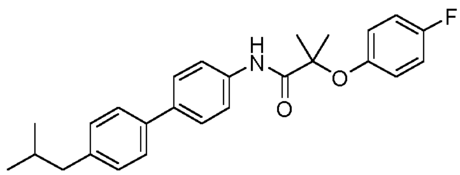 |
| (43) | 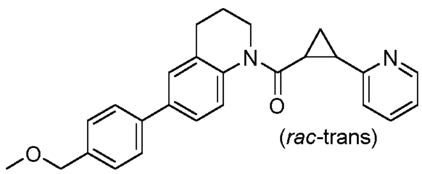 (*rac*-trans) | (44) | 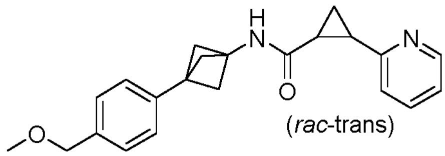 (*rac*-trans) |
| (45) | 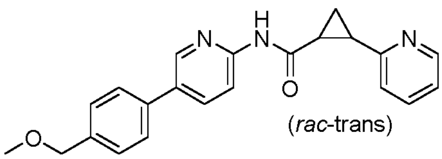 (*rac*-trans) | (46) | 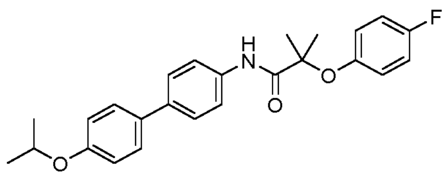 |
| (47) | 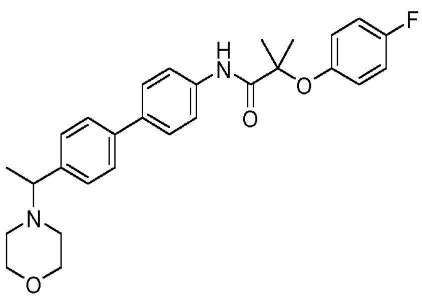 | (48) | 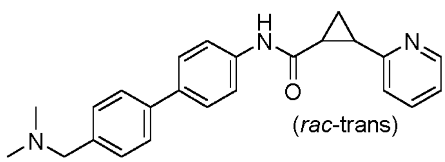 (*rac*-trans) |

(continued)

| | | | |
|---|---|---|---|
| (49) | 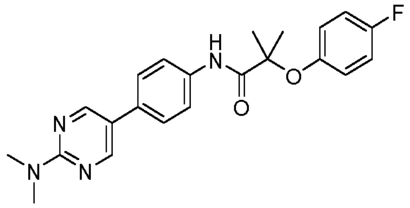 | (50) | 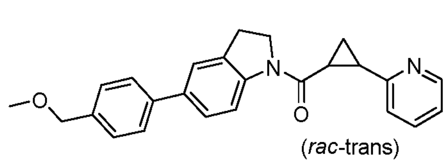<br>(*rac*-trans) |
| (51) | 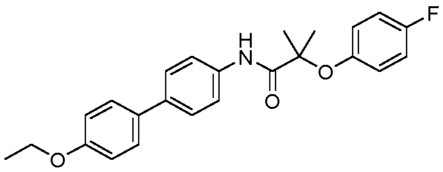 | (52) | 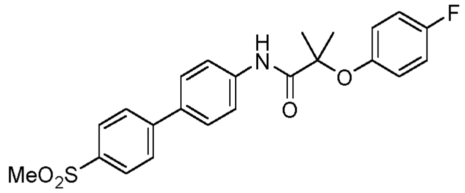 |
| (53) | 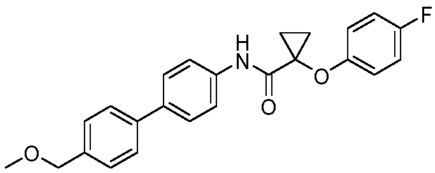 | (54) | 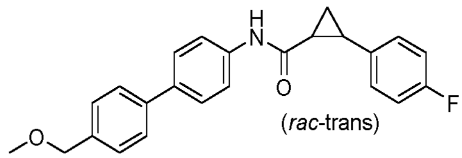<br>(*rac*-trans) |
| (55)* | 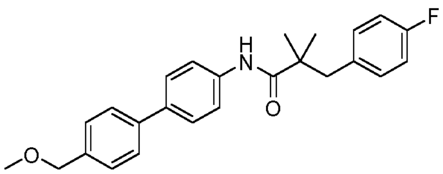 | (56)* | 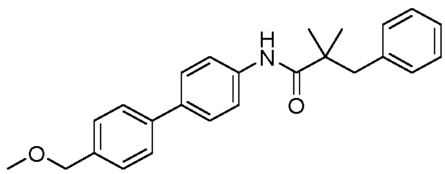 |
| (57) | 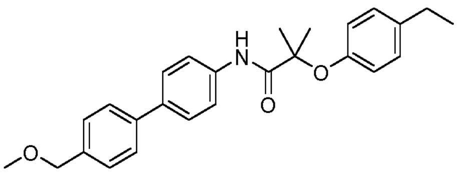 | (58) | 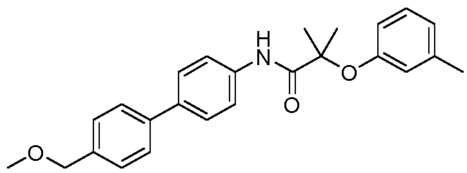 |
| (59) | 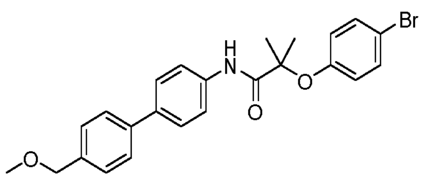 | (60) | 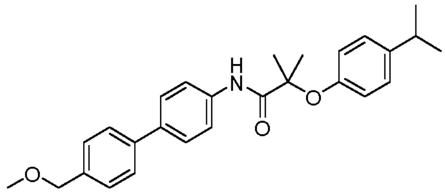 |
| (61) | 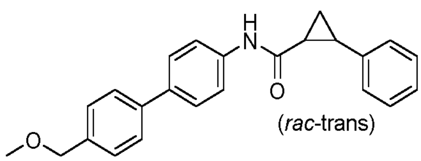<br>(*rac*-trans) | (62) | 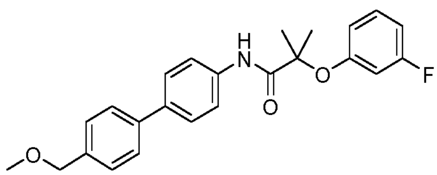 |
| (63) | 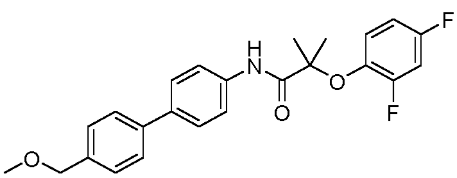 | (64) | 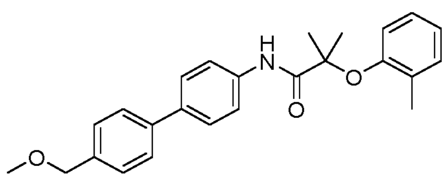 |

(continued)

| | | | |
|---|---|---|---|
| (65) | 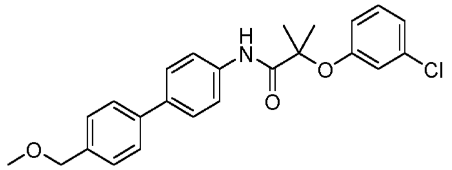 | (66) | 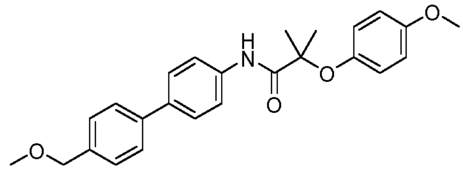 |
| (67) | 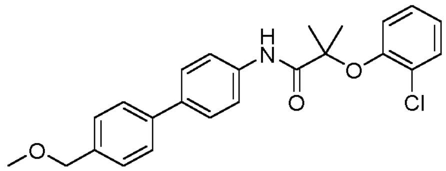 | (68) | 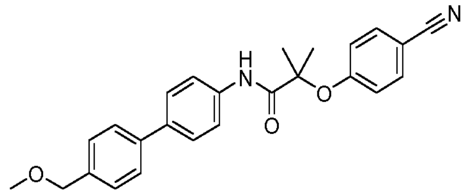 |
| (69) | 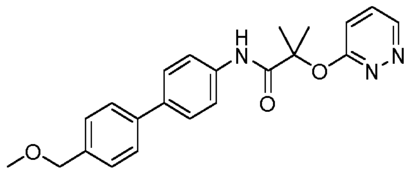 | (70) | 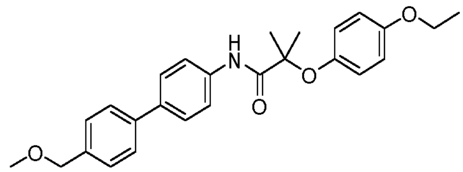 |
| (71) | 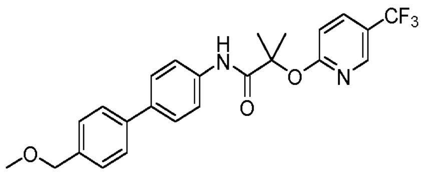 | (72) | 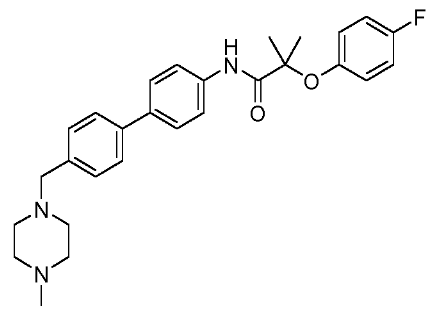 |
| (73) D1 | 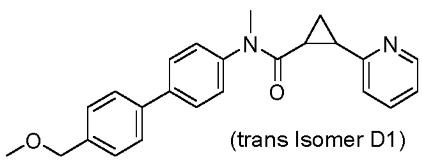 (trans Isomer D1) | (74) D2 | 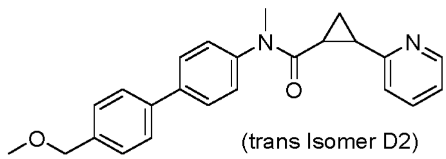 (trans Isomer D2) |
| (75) D1 | 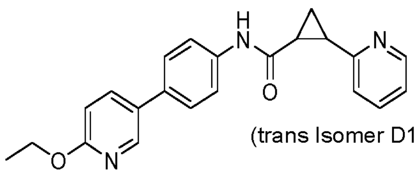 (trans Isomer D1) | (76) D2 | 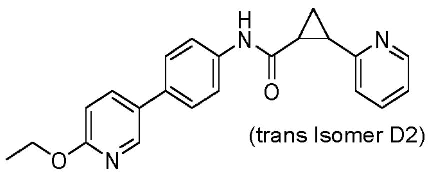 (trans Isomer D2) |
| (77) D1 | 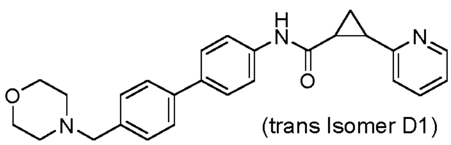 (trans Isomer D1) | (78) D2 | 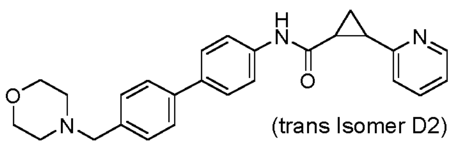 (trans Isomer D2) |
| (79) | 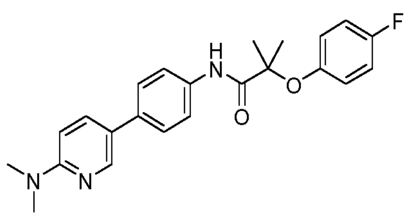 | (80) | 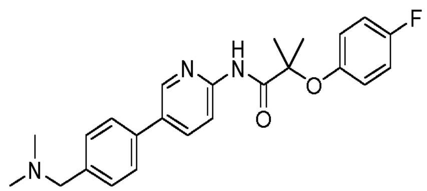 |

(continued)

| | | | |
|---|---|---|---|
| (81) | 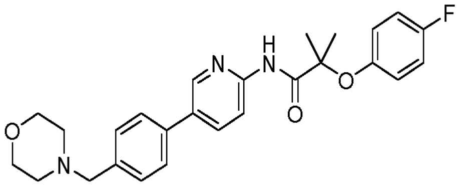 | (82) | 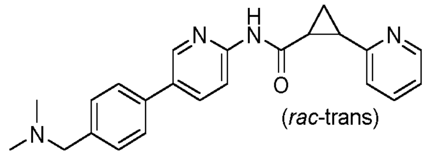 (*rac*-trans) |
| (83) | 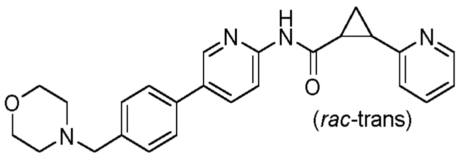 (*rac*-trans) | (84) | 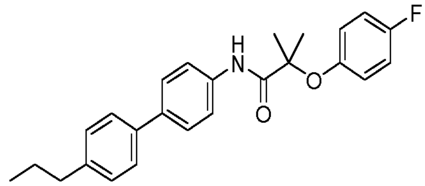 |
| (85) | 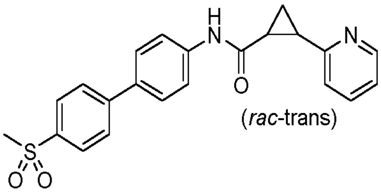 (*rac*-trans) | (86) | 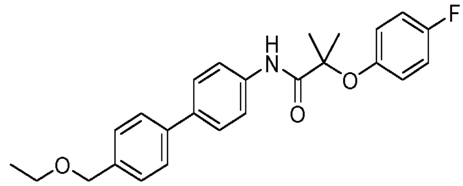 |
| (87) | 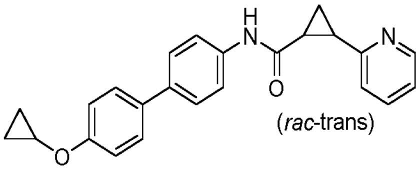 (*rac*-trans) | (88) | 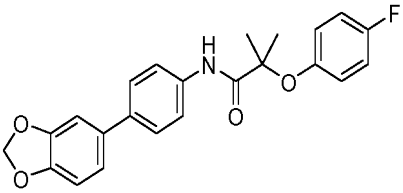 |
| (89) | 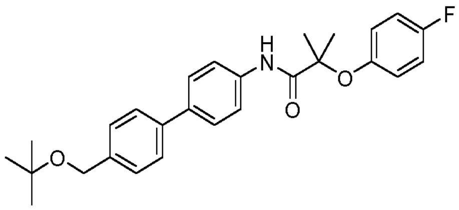 | (90) | 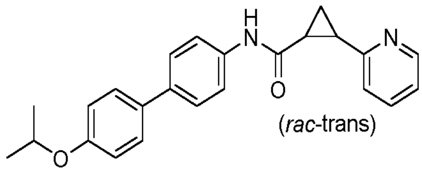 (*rac*-trans) |
| (91) | 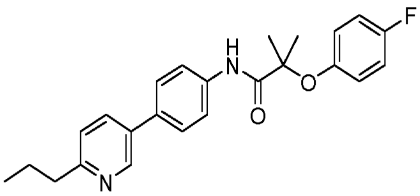 | (92) | 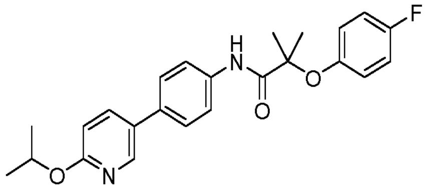 |
| (93) | 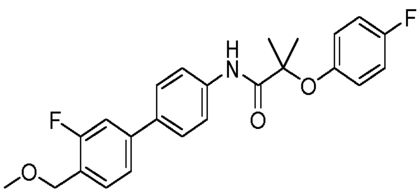 | (94) | 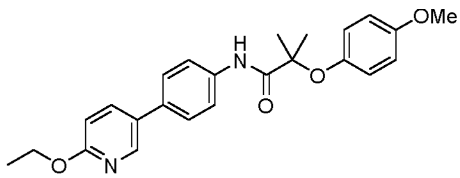 |

(continued)

| | | | |
|---|---|---|---|
| (95) | 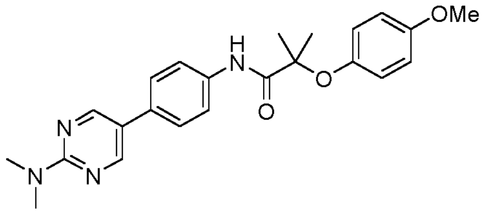 | (96) | 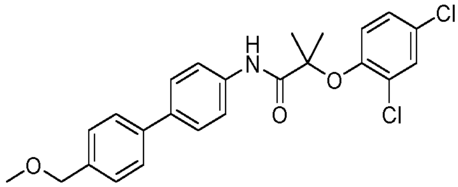 |
| (97) | 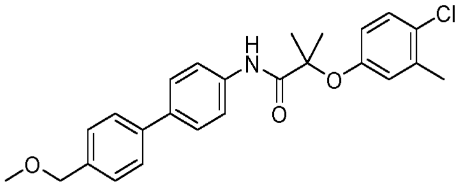 | (98) | 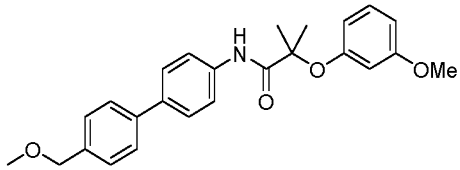 |
| (99) | 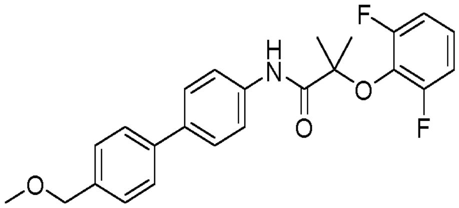 | (100) | 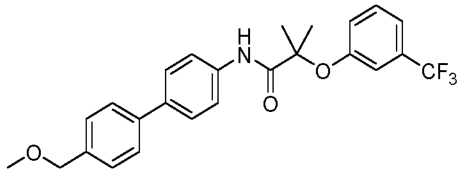 |
| (101) | 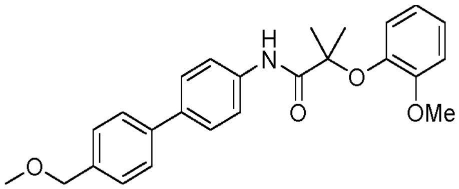 | (102) | 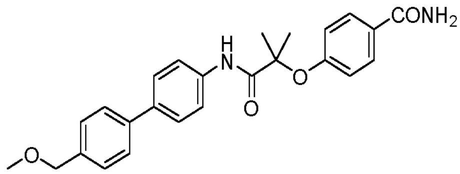 |
| (103) | 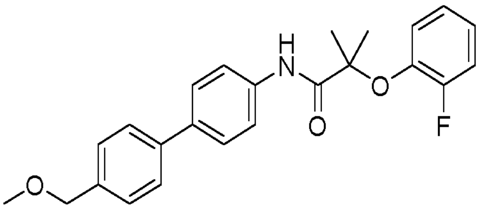 | (104) | 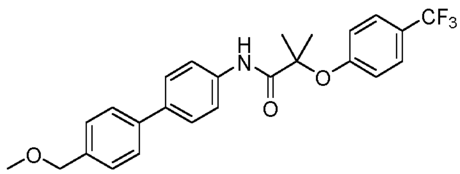 |
| (105) | 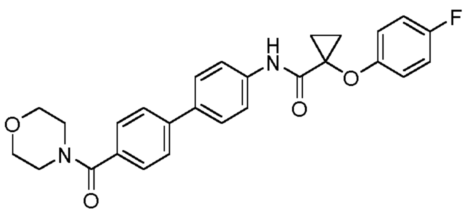 | (106) | 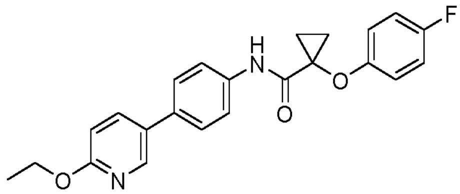 |
| (107) | 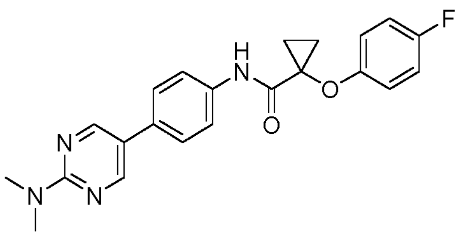 | (108) | 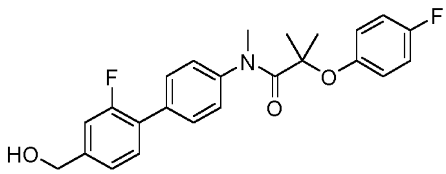 |

(continued)

| | | | |
|---|---|---|---|
| (109) | 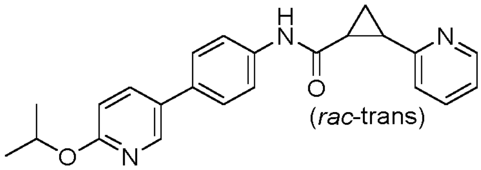 (*rac*-trans) | (110) D1 | 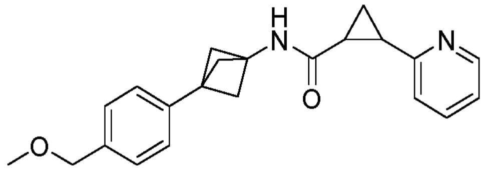 |
| (111) D2 | 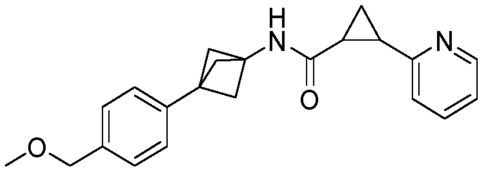 | (112) D3 | 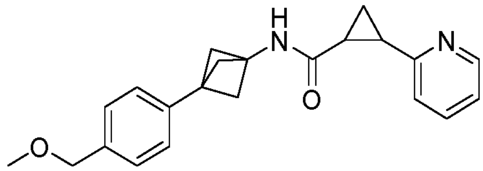 |
| (113) | 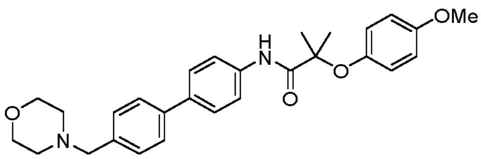 | (114) | 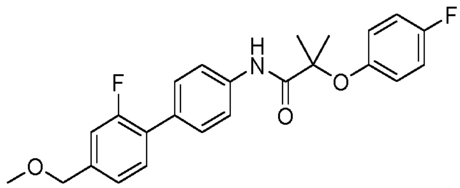 |
| (115) | 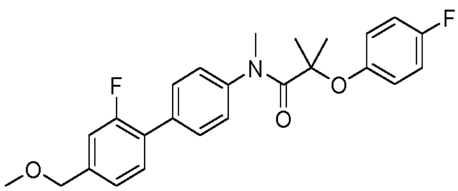 | (116) | 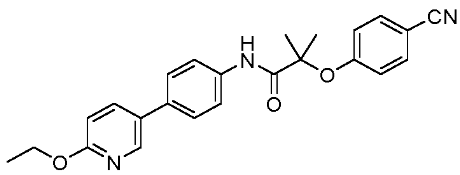 |
| (117) | 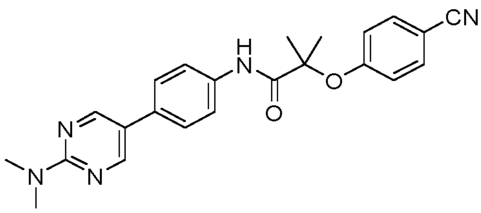 | (118) | 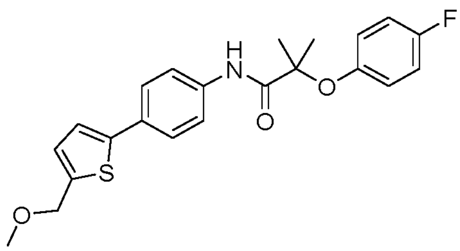 |
| (119) | 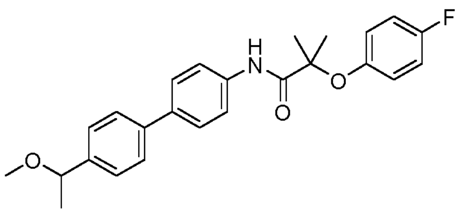 | (120) D1 | 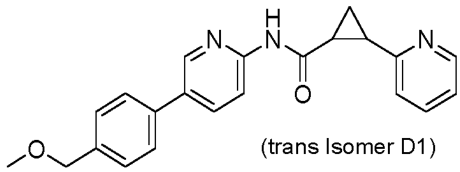 (trans Isomer D1) |
| (121) D2 | 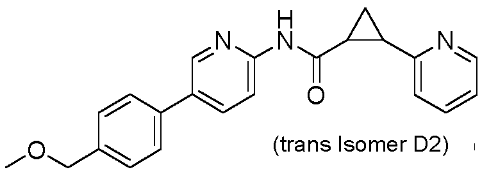 (trans Isomer D2) | (122) E1 | 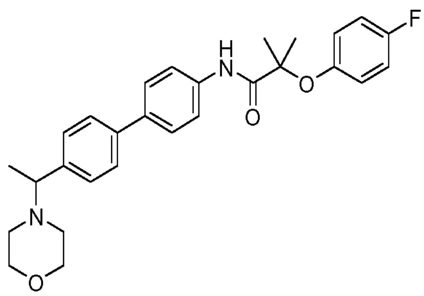 |

(continued)

| | | | |
|---|---|---|---|
| (123) E2 | 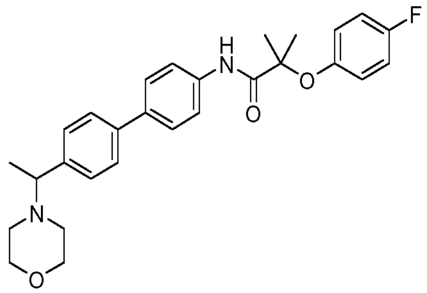 | (124) | 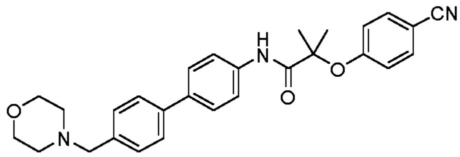 |
| (125) | 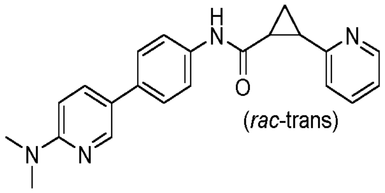 (*rac*-trans) | (126) | 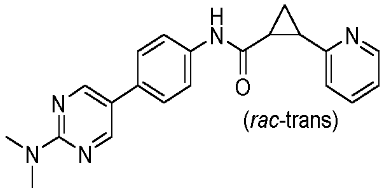 (*rac*-trans) |
| (127) | 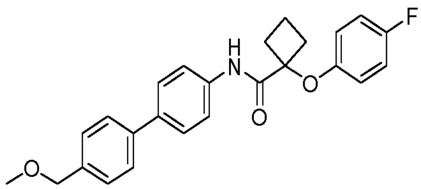 | (128) | 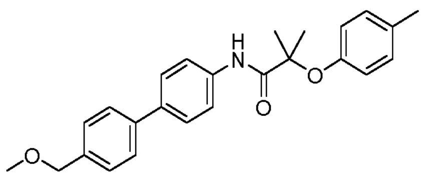 |
| (129) | 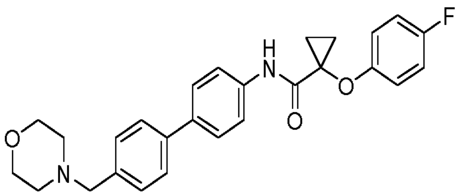 | (130) D2 | 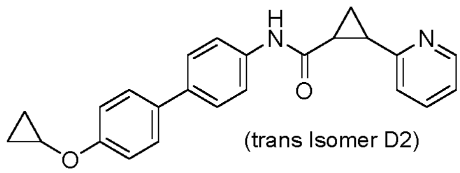 (trans Isomer D2) |
| (131) | 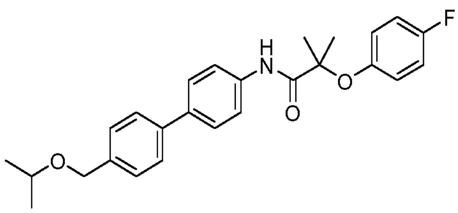 | (132) | 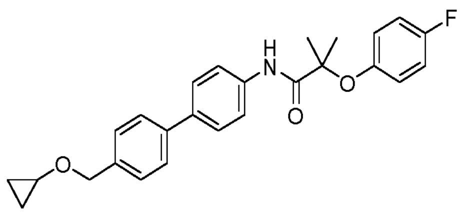 |
| (133) D1 | 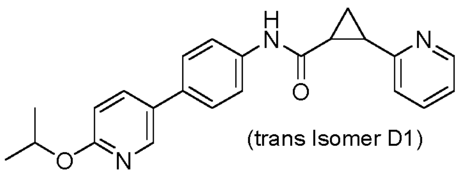 (trans Isomer D1) | (134) D2 | 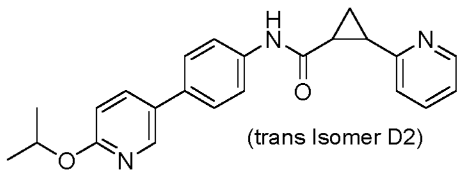 (trans Isomer D2) |
| (135) | 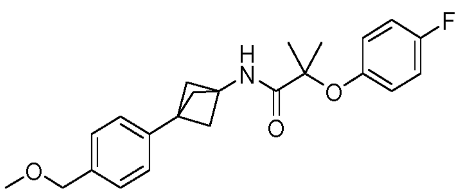 | (136) | 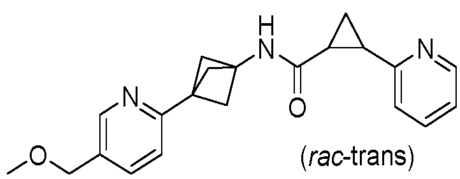 (*rac*-trans) |
| (137) | 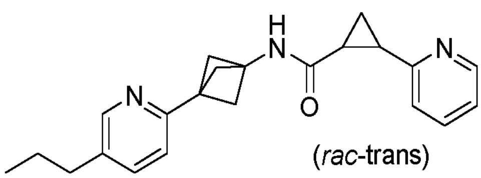 (*rac*-trans) | (138) | 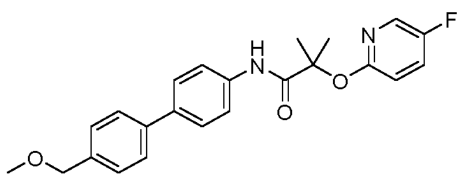 |

(continued)

| | | | |
|---|---|---|---|
| (139) | 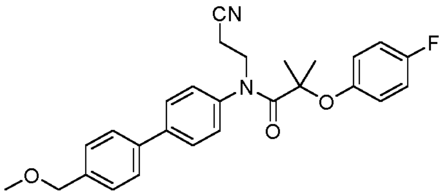 | (140) | 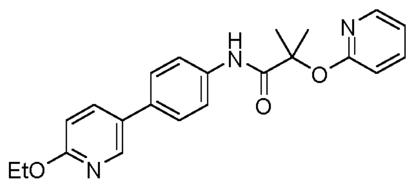 |
| (141) D1 | 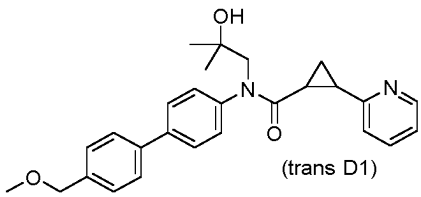 (trans D1) | (142) D2 | 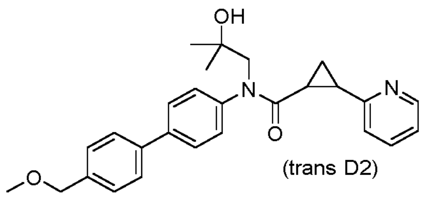 (trans D2) |

oder einem pharmazeutisch-verträgliches Salz davon.

**15.** Die Verbindung gemäß einem der Ansprüche 1 oder 14, wobei die Verbindung ausgewählt ist aus:

| | | | |
|---|---|---|---|
| (1) | 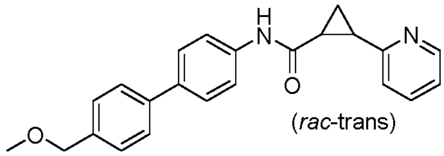 (*rac*-trans) | (2) | 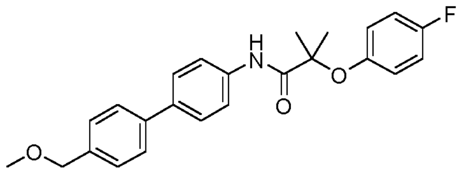 |
| (3) | 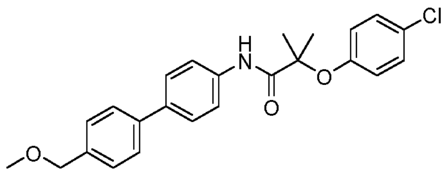 | (4) | 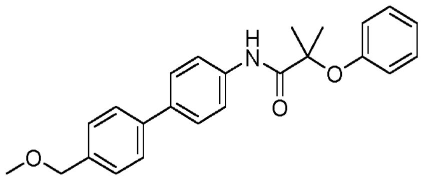 |
| (7) | 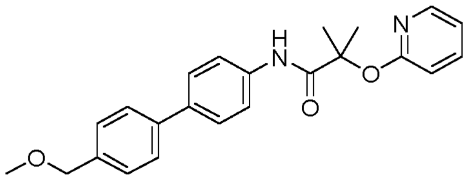 | (8) | 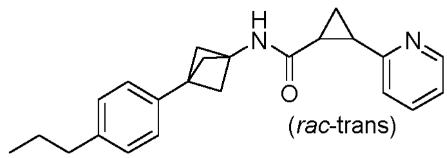 (*rac*-trans) |
| (9) | 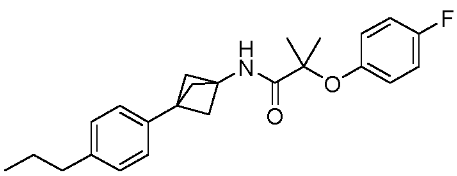 | (10) | 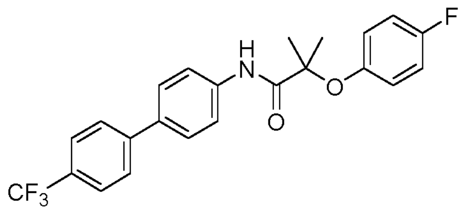 |
| (11) | 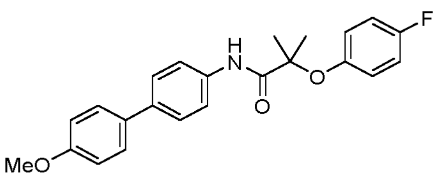 | (12) | 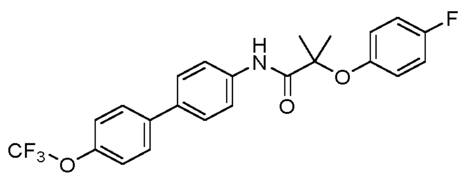 |

(continued)

| | | | |
|---|---|---|---|
| (13) | 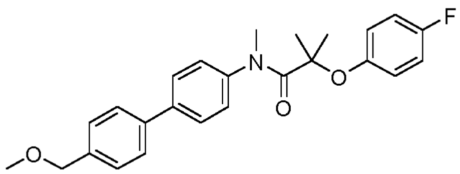 | (14) | 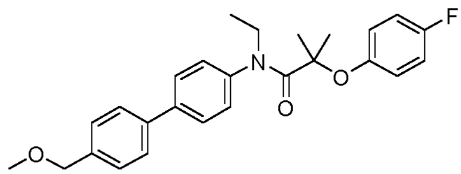 |
| (15) | 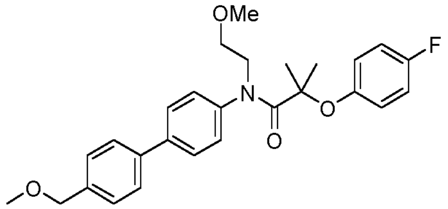 | (17) | 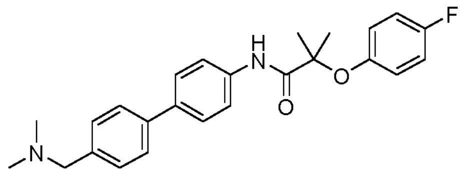 |
| (18)<br>D1 | 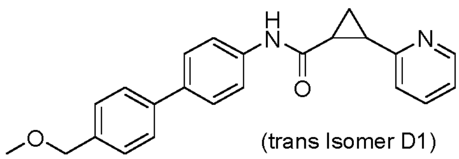<br>(trans Isomer D1) | (22) | 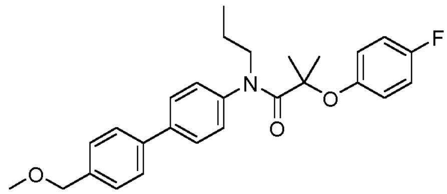 |
| (25) | 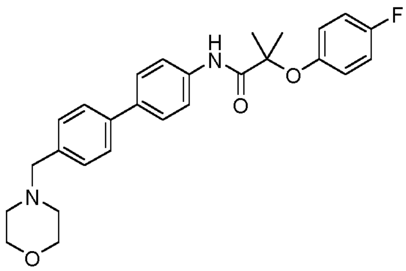 | (26) | 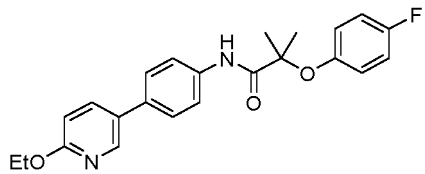 |
| (27) | 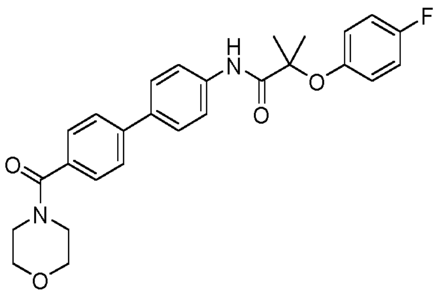 | (28) | 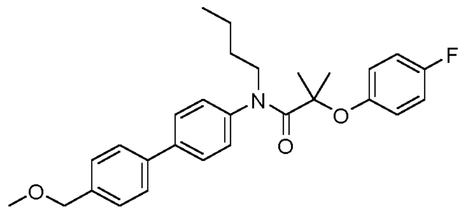 |
| (29) | 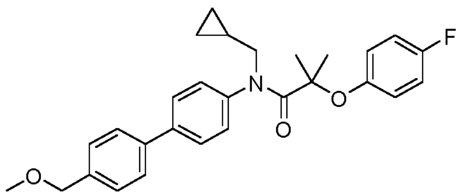 | (30) | 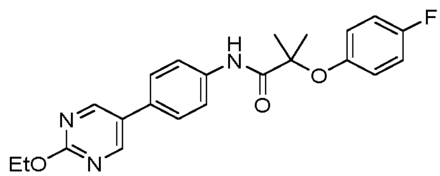 |
| (31) | 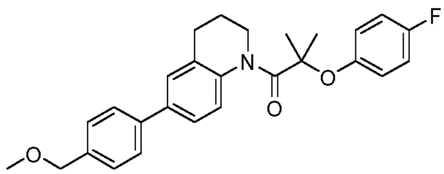 | (32) | 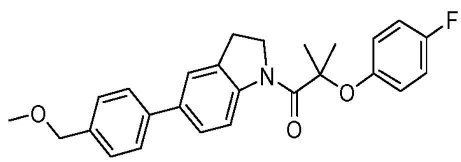 |

(continued)

| | | | |
|---|---|---|---|
| (33) | 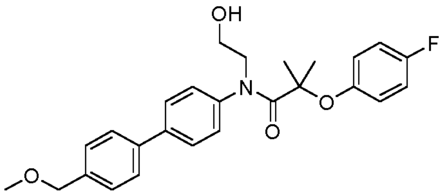 | (34)<br>D1 | 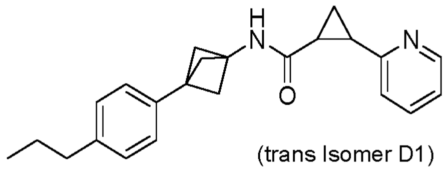<br>(trans Isomer D1) |
| (35)<br>D2 | 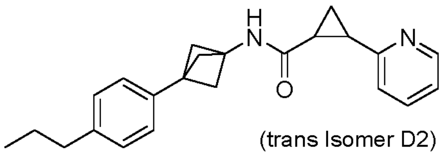<br>(trans Isomer D2) | (36) | 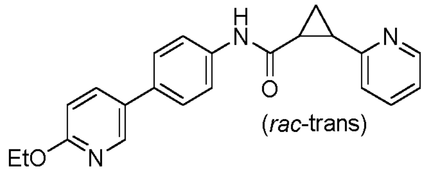<br>(*rac*-trans) |
| (37) | 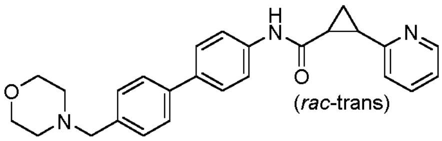<br>(*rac*-trans) | (39) | 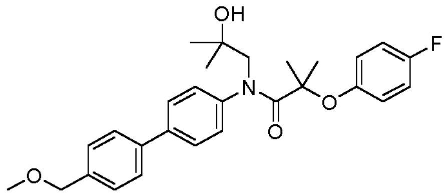 |
| (40) | 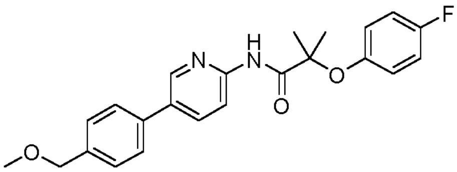 | (44) | 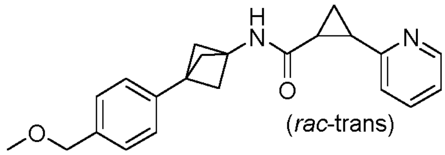<br>(*rac*-trans) |
| (45) | 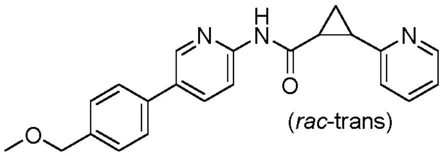<br>(*rac*-trans) | (46) | 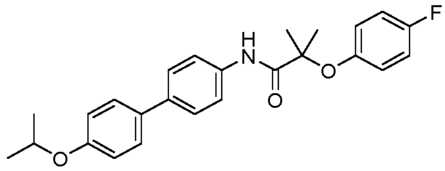 |
| (47) | 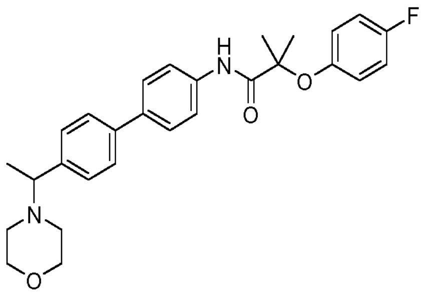 | (49) | 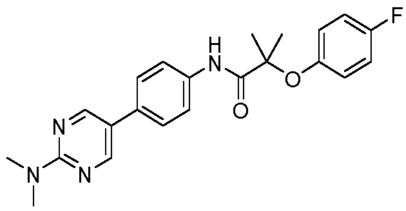 |
| (50) | 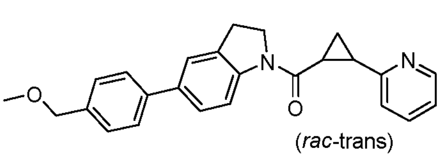<br>(*rac*-trans) | (53) | 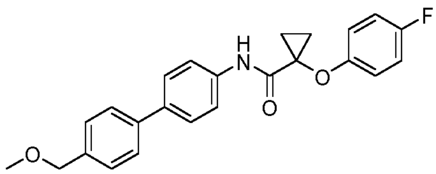 |
| (55)* | 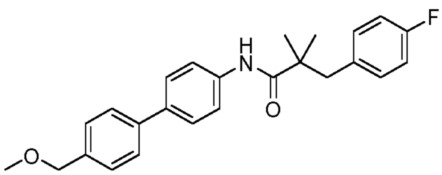 | (56)* | 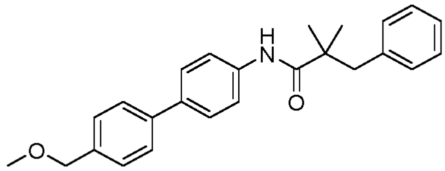 |

(continued)

| | | | |
|---|---|---|---|
| (57) | 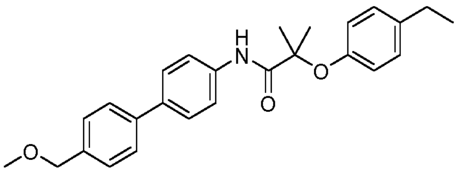 | (58) | 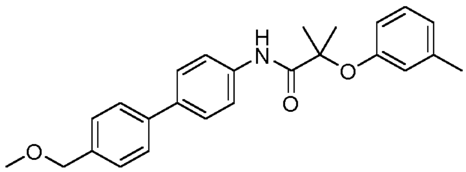 |
| (59) | 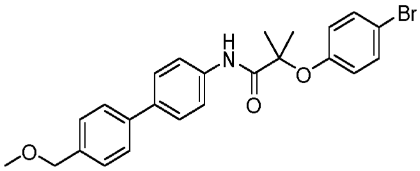 | (60) | 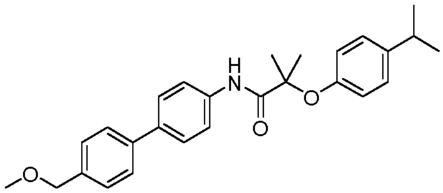 |
| (62) | 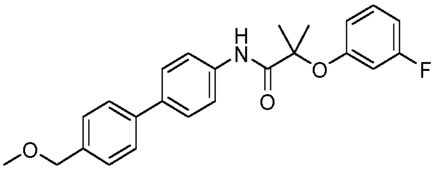 | (63) | 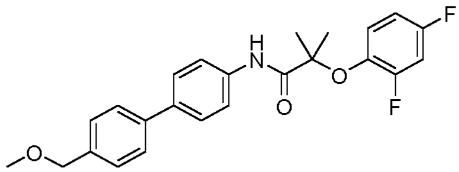 |
| (64) | 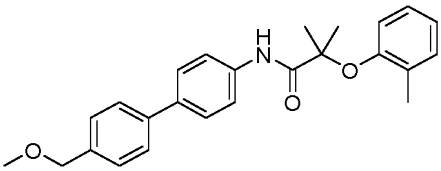 | | |
| (65) | 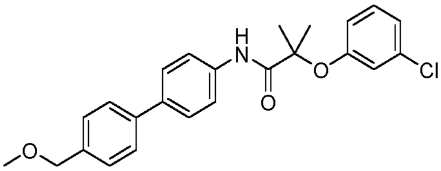 | (66) | 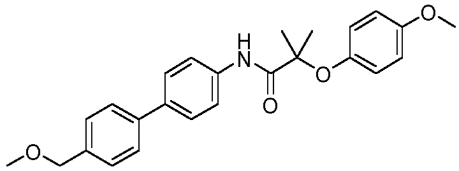 |
| (67) | 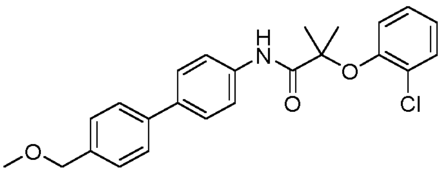 | (68) | 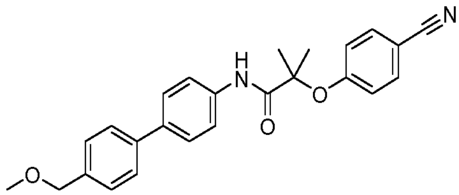 |
| (70) | 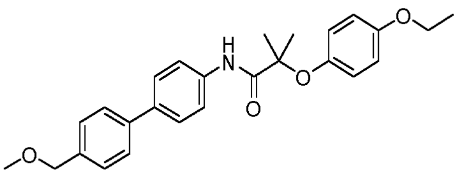 | (72) | 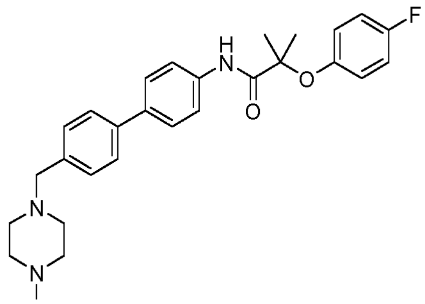 |
| (75) D1 | 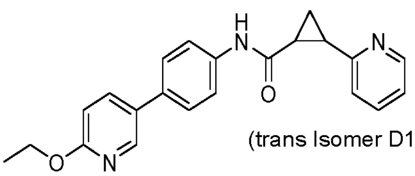 (trans Isomer D1) | (76) D2 | 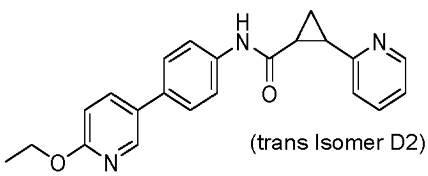 (trans Isomer D2) |

(continued)

| | | | |
|---|---|---|---|
| (77) D1 | 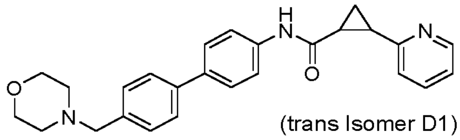 (trans Isomer D1) | (78) D2 | 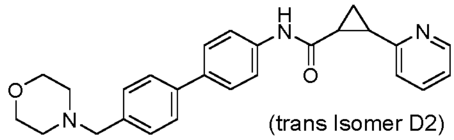 (trans Isomer D2) |
| (79) | 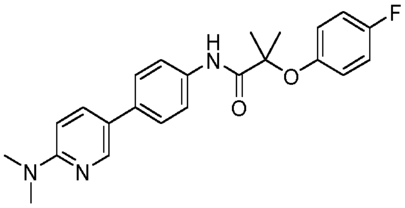 | (80) | 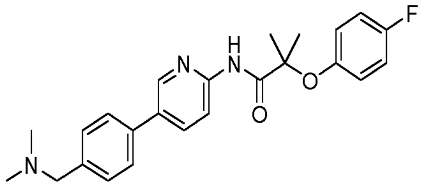 |
| (81) | 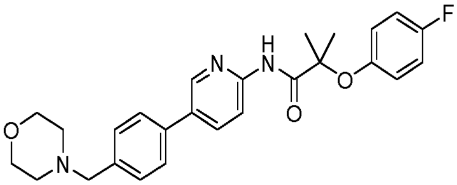 | (83) | 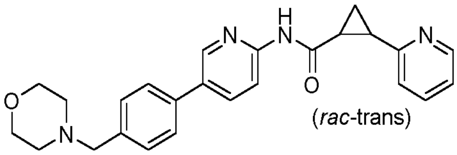 (*rac*-trans) |
| (85) | 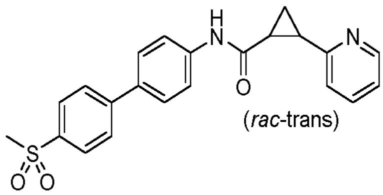 (*rac*-trans) | (86) | 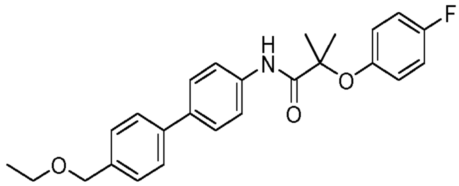 |
| (87) | 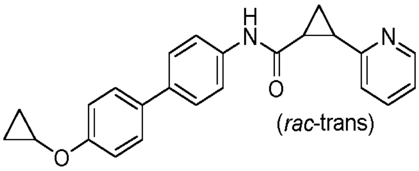 (*rac*-trans) | (88) | 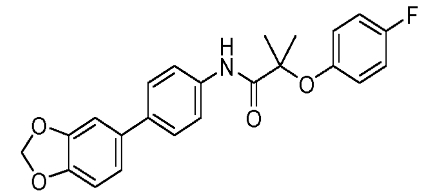 |
| (89) | 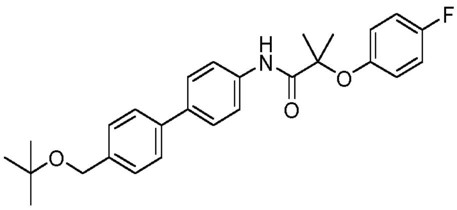 | (90) | 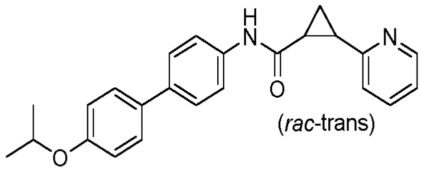 (*rac*-trans) |
| (91) | 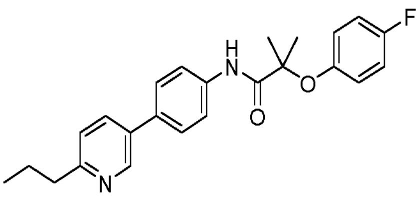 | (92) | 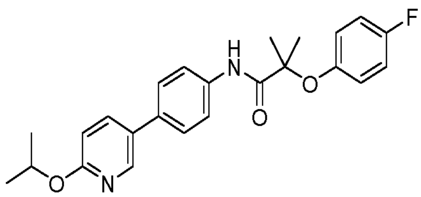 |
| (94) | 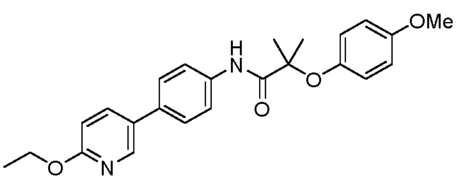 | (95) | 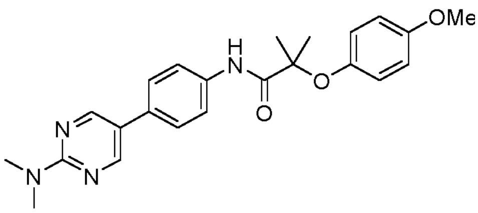 |

(continued)

| | | | |
|---|---|---|---|
| (97) | 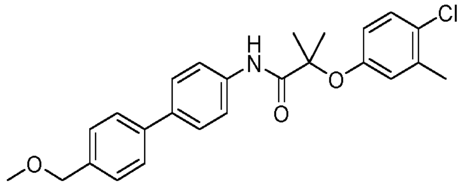 | (98) | 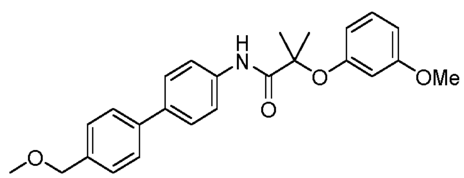 |
| (99) | 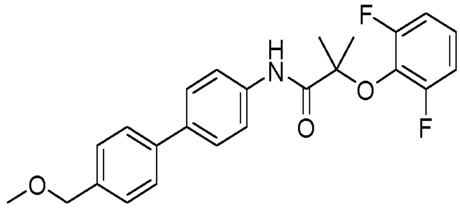 | (100) | 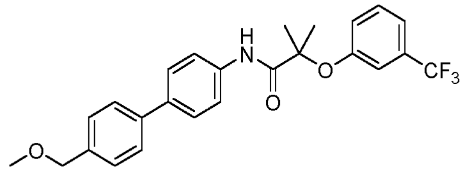 |
| (101) | 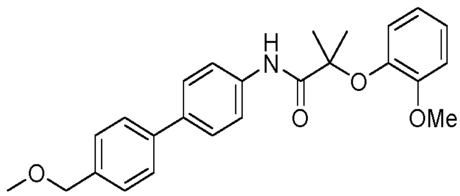 | (102) | 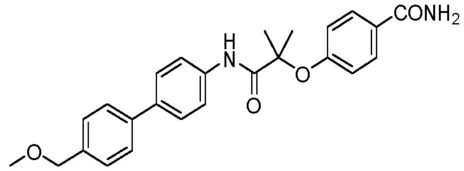 |
| (103) | 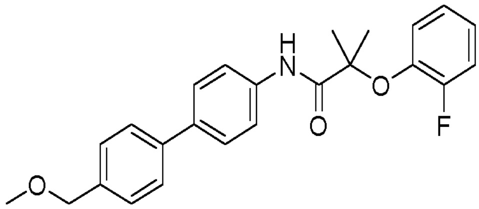 | (104) | 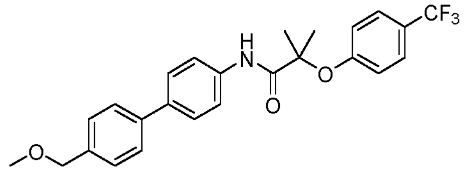 |
| (105) | 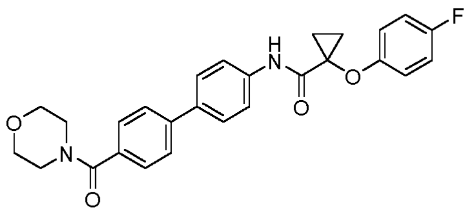 | (106) | 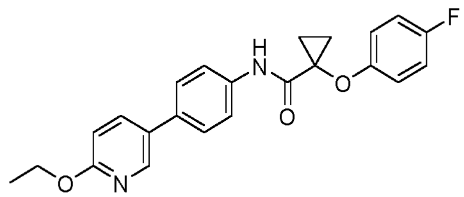 |
| (107) | 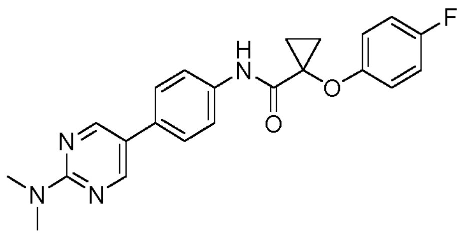 | (108) | 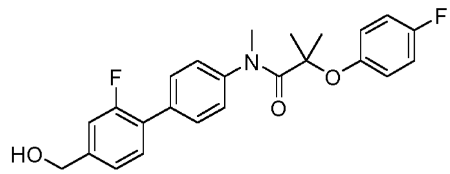 |
| (109) | 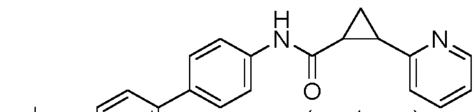 (*rac*-trans) | (110) D1 | 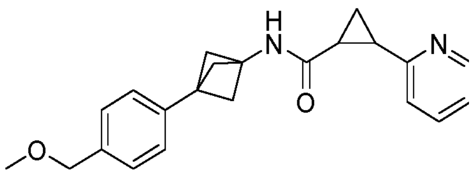 |
| (111) D2 | 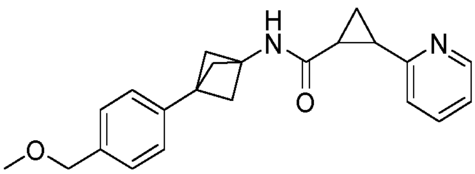 | | |

(continued)

| | | | |
|---|---|---|---|
| (113) | 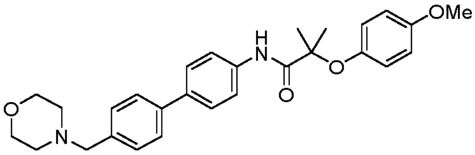 | (114) | 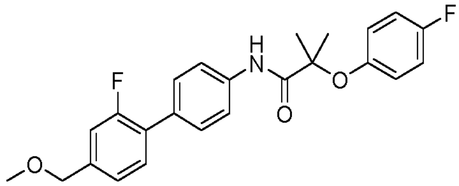 |
| (115) | 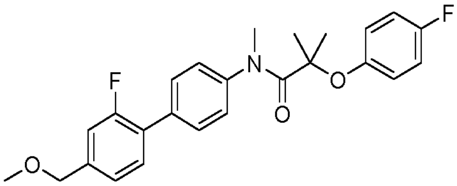 | (116) | 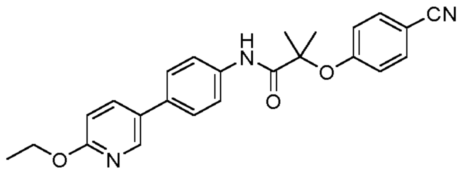 |
| (117) | 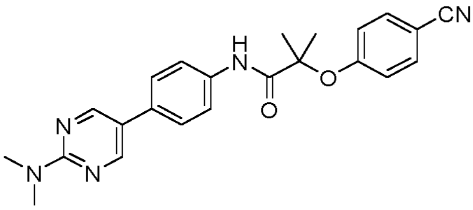 | | |
| (119) | 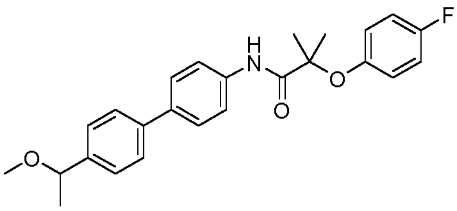 | (120)<br>D1 | 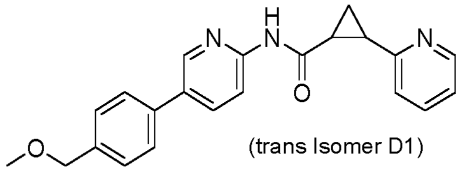 (trans Isomer D1) |
| (121)<br>D2 | 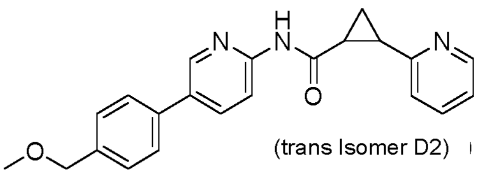 (trans Isomer D2) | (122)<br>E1 | 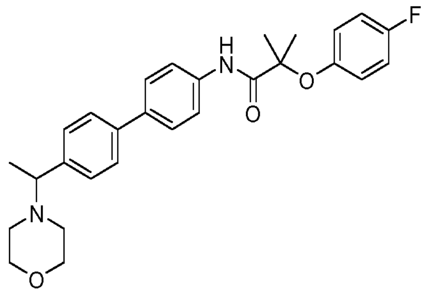 |
| (123)<br>E2 | 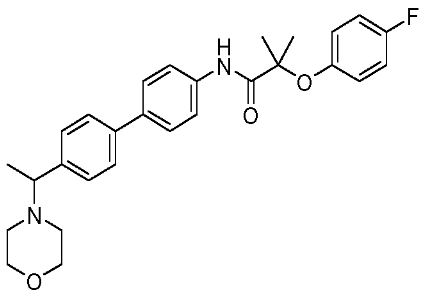 | (124) | 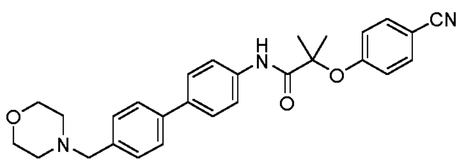 |
| (127) | 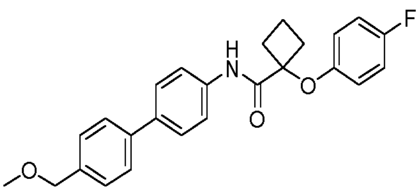 | (128) | 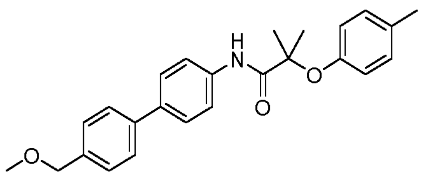 |

(continued)

| | | | |
|---|---|---|---|
| (129) | 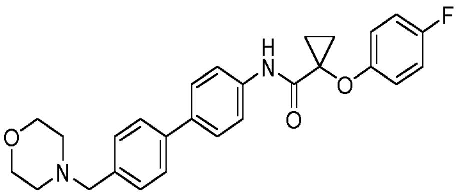 | (132) | 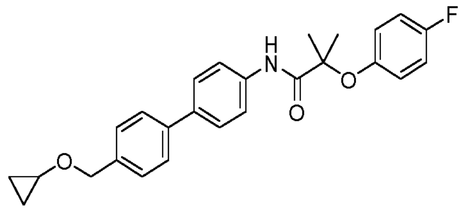 |
| (133) D1 | 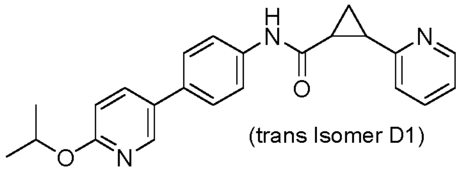 (trans Isomer D1) | | |
| (135) | 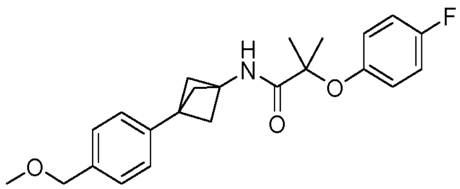 | (136) | 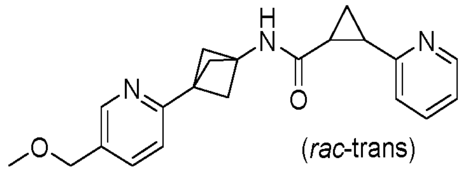 (*rac*-trans) |
| (137) | 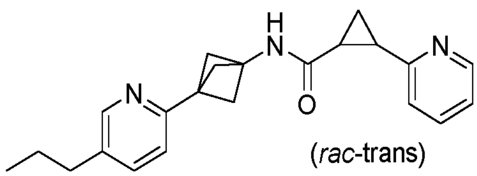 (*rac*-trans) | (138) | 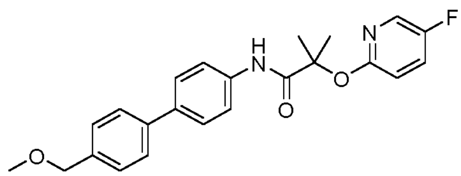 |
| (140) | 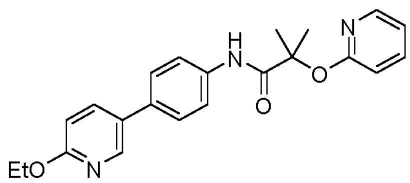 | | |

oder einem pharmazeutisch-verträglichen Salz davon.

**16.** Eine pharmazeutische Formulierung umfassend eine Verbindung gemäß einem der Ansprüche 1 bis 15, oder einem pharmazeutisch-verträglichen Salz davon, und einem pharmazeutisch-verträglichen Hilfsstoff.

**17.** Eine Verbindung gemäß einem der Ansprüche 1 bis 15, oder einem pharmazeutisch-verträglichen Salz davon, zur Verwendung als ein Medikament; oder zur Verwendung bei der Behandlung einer Krankheit, die von GPR88 vermittelt wird; oder zur Verwendung bei der Behandlung von Tourette-Syndrom, der Huntington-Krankheit (HD), Sucht, der Parkinson-Krankheit (PD), Schizophrenie, und Aufmerksamkeitsdefizit-/Hyperaktivitätsstörung (ADHD), choreiformen Bewegungen, Sprachverzögerung, Lernbehinderungen, Depression, hyperkinetischen Bewegungsstörungen **gekennzeichnet durch** Chorea und/oder Dystonie, Psychosen, kognitiven Defiziten bei Schizophrenie, affektiven Störungen, bipolarer Störung, der Alzheimer-Krankheit und Basalganglienstörungen.

## Revendications

**1.** Composé de Formule (I) ou sel pharmaceutiquement acceptable de celui-ci :

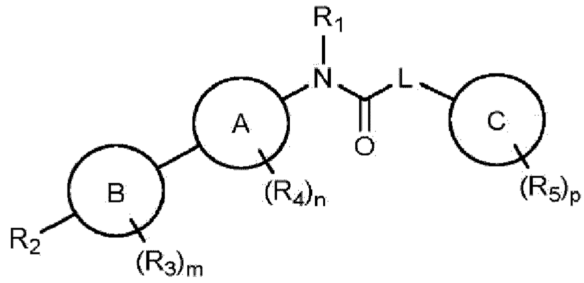

(I)

sachant que :

i) -L- est sélectionné dans le groupe constitué par

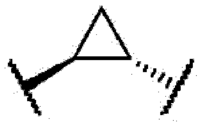 , 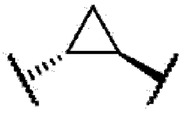 , et 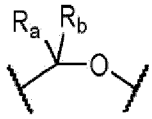 ;

l'anneau A est sélectionné dans le groupe constitué par : un cycloalkyle à 6 chaînons, un système d'anneau de cycloalkyle à 5 chaînons, un système d'anneau de cycloalkyle à 8 chaînons, un cycloalkényle à 6 chaînons, un système d'anneau aromatique à 6 chaînons, et un système d'anneau hétéroaromatique à 6 chaînons ;
l'anneau B est sélectionné dans le groupe constitué par : un cycloalkyle à 6 chaînons, un anneau aromatique à 6 chaînons et un anneau hétéroaromatique à 6 chaînons ;
l'anneau C est sélectionné dans le groupe constitué par : un anneau aromatique à 5-6 chaînons et un anneau hétéroaromatique à 5-6 chaînons ;
m est 0, 1 ou 2 ;
n est 0, 1 ou 2 ;
p est 0, 1 ou 2 ;
$R_1$ est sélectionné dans le groupe constitué par : de l'hydrogène, un alkyle en $C_1$-$C_4$ substitué ou non substitué, un cycloalkyle en $C_3$-$C_4$ substitué ou non substitué et un cycloalkyle en $C_3$-$C_4$ alkyle en $C_1$-$C_2$ substitué ou non substitué ; sachant que chaque substituant est sélectionné indépendamment dans le groupe constitué par -$OR_c$, du nitrile ou de l'halo ; sachant que chaque $R_c$ est sélectionné indépendamment dans le groupe constitué par H, Me, Et et du cyclopropyle ;
$R_2$ est sélectionné dans le groupe constitué par : un alkyle en $C_1$-$C_6$ substitué ou non substitué, un alkényle en $C_2$-$C_6$ substitué ou non substitué, un alkynyle en $C_2$-$C_6$ substitué ou non substitué ; un cycloalkyle en $C_3$-$C_6$ substitué ou non substitué ; un alkoxy en $C_1$-$C_6$ substitué ou non substitué, un cycloalkoxy en $C_3$-$C_6$ substitué ou non substitué ; un alkoxy en $C_1$-$C_6$ alkyle en $C_1$-$C_6$ substitué ou non substitué ; un cycloalkoxy en $C_3$-$C_6$ alkyle en $C_1$-$C_6$ substitué ou non substitué ; un haloalkyle en $C_1$-$C_6$ substitué ou non substitué, un haloalkényle en $C_2$-$C_6$ substitué ou non substitué, un haloalkynyle en $C_2$-$C_6$ substitué ou non substitué ; un cyclohaloalkyle en $C_3$-$C_6$ substitué ou non substitué ; un haloalkoxy en $C_1$-$C_6$ substitué ou non substitué, un cyclohaloakoxy en $C_3$-$C_6$ substitué ou non substitué ; un haloalkoxy en $C_1$-$C_6$ alkyle en $C_1$-$C_6$ substitué ou non substitué, un cyclohaloalkoxy en $C_3$-$C_6$ alkyle en $C_1$-$C_6$ substitué ou non substitué, un alkoxy en $C_1$-$C_6$ haloalkyle en $C_1$-$C_6$ substitué ou non substitué, un cycloalkoxy en $C_3$-$C_6$ haloalkyle en $C_1$-$C_6$ substitué ou non substitué, un haloalkoxy en $C_1$-$C_6$ haloalkyle en $C_1$-$C_6$ substitué ou non substitué, un cyclohaloalkoxy en $C_3$-$C_6$ haloalkyle en $C_1$-$C_6$ substitué ou non substitué, -$SO_2R_d$, et - $NR_eR_e$ ;
sachant que $R_d$ est un alkyle en $C_{1-4}$ ;
sachant que chaque $R_e$ est sélectionné indépendamment dans le groupe constitué par H et un alkyle en $C_{1-4}$ ;
sachant que chaque substituant est sélectionné indépendamment dans le groupe constitué par =O, -$NR_eR_e$, et un anneau cycloalkyle ou hétérocycloalkyle monocyclique, bicyclique ou ponté à 3-8 chaînons substitué ou non substitué, sachant que chaque $R_e$ est sélectionné indépendamment dans le groupe constitué par H et un alkyle en $C_{1-4}$ ;
sachant que chaque substituant de l'anneau cycloalkyle ou hétérocycloalkyle monocyclique, bicyclique ou ponté à 3-8 chaînons est sélectionné indépendamment dans le groupe constitué par -OH, du nitrile, de l'halo, du méthyle ou du méthoxy ;

chaque $R_3$ est sélectionné indépendamment dans le groupe constitué par : de l'halogène, un alkyle en $C_1$-$C_3$, un haloalkyle en $C_1$-$C_3$, un alkoxy en $C_1$-$C_3$, un haloalkoxy en $C_1$-$C_3$ ou -CN ;
chaque $R_4$ est sélectionné indépendamment dans le groupe constitué par : de l'halogène, un alkyle en $C_1$-$C_3$, un haloalkyle en $C_1$-$C_3$, un alkoxy en $C_1$-$C_3$, un haloalkoxy en $C_1$-$C_3$ ou -CN ;
chaque $R_5$ est sélectionné indépendamment dans le groupe constitué par : de l'halogène, un alkyle en $C_1$-$C_3$, un haloalkyle en $C_1$-$C_3$, un alkoxy en $C_1$-$C_3$, un haloalkoxy en $C_1$-$C_3$, -CN, ou -C(O)$NR_eR_e$ ;
$R_a$ et $R_b$ sont sélectionnés chacun indépendamment dans le groupe constitué par : de l'hydrogène, de l'halogène, un alkyle en $C_1$-$C_6$, et un alkoxy en $C_1$-$C_6$ ; ou sachant que $R_a$ et $R_b$, conjointement avec l'atome auquel ils sont liés, forment un anneau cycloalkyle ou hétérocycloalkyle à 3 à 6 chaînons comprenant -O- ou -$NR_f$-, sachant que $R_f$ est H ou du méthyle ;
à condition que le composé ne soit pas :

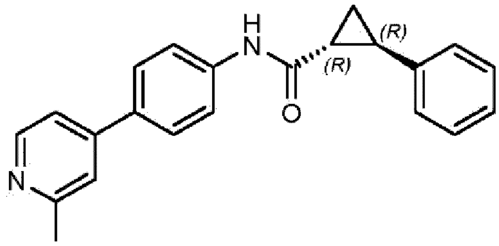

ou

ii) -L-, l'anneau A, l'anneau B, l'anneau C, m, p, $R_2$, $R_3$, $R_5$, $R_a$, $R_b$, $R_c$, $R_d$, $R_e$ et $R_f$ sont tels que définis ci-dessous en i),

$R_1$ est un résidu d'alkylényle en $C_1$ ou $C_2$,
n est 1 ou 2, et
un fragment $R_4$ est un résidu d'alkylényle en $C_1$ et, lorsqu'il est présent, l'autre fragment $R_4$ est tel que défini ci-dessus en i),
sachant que le fragment $R_4$ d'alkylényle en $C_1$ est lié à un atome de carbone dans l'anneau A qui est ortho par rapport à l'atome de carbone de l'anneau A comportant le résidu d'amide, et, conjointement avec les atomes auxquels ils sont liés, les fragments $R_1$ et $R_4$ forment un anneau à 5 ou 6 chaînons ;

et/ou
iii) -L-, l'anneau A, l'anneau B, l'anneau C, n, p, $R_1$, $R_4$, $R_5$, $R_a$, $R_b$, $R_c$ et $R_f$ sont tels que définis ci-dessus en i),

m est 1 ou 2 ;
$R_2$ est sélectionné dans le groupe constitué par : de l'alkylényle -O- en $C_{1-2}$ substitué ou non substitué, sachant que l'atome d'oxygène est connecté à l'anneau B ;
sachant que chaque substituant est sélectionné indépendamment dans le groupe constitué par -OH, du nitrile, de l'halo, du méthyle ou du méthoxy ;
un fragment $R_3$ est un résidu -O- et, lorsqu'il est présent, l'autre fragment $R_3$ est tel que défini en i) ;
sachant que, conjointement aux atomes auxquels ils sont liés, le fragment $R_2$ et le fragment $R_3$, qui est un résidu -O-, forment un anneau à 5 ou 6 chaînons.

**2.** Le composé de la revendication 1, sachant que $R_1$ est sélectionné dans le groupe constitué par : de l'hydrogène, un alkyle en $C_1$-$C_4$ substitué ou non substitué, un cycloalkyle en $C_3$-$C_4$ substitué ou non substitué et un cycloalkyle en $C_3$-$C_4$ alkyle en $C_1$-$C_2$ substitué ou non substitué ; sachant que chaque substituant est sélectionné indépendamment parmi -$OR_c$ et du nitrile ; et sachant que chaque $R_c$ est sélectionné indépendamment dans le groupe constitué par H, Me, Et et du cyclopropyle.

**3.** Le composé de la revendication 2, sachant que

- $R_1$ est sélectionné dans le groupe constitué par : de l'hydrogène, un alkyle en $C_1$-$C_4$ substitué ou non substitué et un cycloalkyle en $C_3$-$C_4$ alkyle en $C_1$-$C_2$ substitué ou non substitué ; sachant que chaque substituant est sélectionné indépendamment parmi -$OR_c$ et du nitrile ; et sachant que chaque $R_c$ est sélectionné indépendamment dans le groupe constitué par H, Me, Et et du cyclopropyle ; ou

- $R_1$ est sélectionné dans le groupe constitué par : de l'hydrogène et un alkyle en $C_1$-$C_4$ substitué ou non substitué et un cycloalkyle en $C_3$ alkyle en $C_1$ substitué ou non substitué ; sachant que chaque substituant est sélectionné indépendamment parmi -$OR_c$ et du nitrile ; et sachant que chaque $R_c$ est sélectionné indépendamment dans le groupe constitué par H et Me, ou
- $R_1$ est sélectionné dans le groupe constitué par :

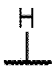 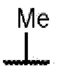 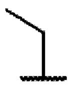 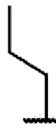 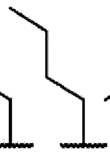 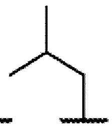 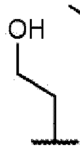 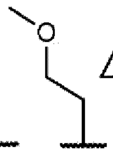 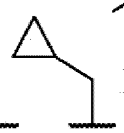 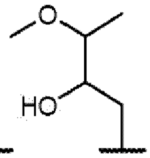 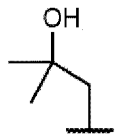 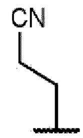.

**4.** Le composé d'une quelconque revendication précédente, sachant que

- n est 1 ; et/ou
- chaque $R_4$ est sélectionné indépendamment dans le groupe constitué par : de l'halogène, un alkyle en $C_1$-$C_2$, et un alkoxy en $C_1$-$C_2$, sachant que facultativement $R_4$ est du méthyle.

**5.** Le composé de l'une quelconque des revendications 1 à 3, sachant que n est 0.

**6.** Le composé de la revendication 1, sachant que $R_1$ est un résidu d'alkylényle en $C_1$ ou $C_2$ et un fragment $R_4$ est un résidu d'alkylényle en $C_1$, sachant que le fragment $R_4$ d'alkylényle en $C_1$ est lié à un atome de carbone dans l'anneau A qui est ortho par rapport à l'atome de carbone de l'anneau A comportant le résidu d'amide, et, conjointement avec les atomes auxquels ils sont liés, les fragments $R_1$ et $R_4$ forment un anneau à 5 ou 6 chaînons.

**7.** Le composé d'une quelconque revendication précédente, sachant que $R_2$ est sélectionné dans le groupe constitué par : un alkyle en $C_1$-$C_4$ substitué ou non substitué ; un cycloalkyle en $C_3$-$C_4$ substitué ou non substitué ; un alkoxy en $C_1$-$C_4$ substitué ou non substitué, un cycloalkoxy en $C_3$-$C_4$ substitué ou non substitué ; un alkoxy en $C_1$-$C_4$ alkyle en $C_1$-$C_4$ substitué ou non substitué ; un haloalkyle en $C_1$-$C_4$ substitué ou non substitué ; un haloalkoxy en $C_1$-$C_4$ substitué ou non substitué ; $SO_2R_d$ ; et -$NR_eR_e$ ;
sachant que facultativement :

- $R_2$ est sélectionné dans le groupe constitué par : un alkyle en $C_1$-$C_4$ substitué ou non substitué ; un alkoxy en $C_1$-$C_4$ substitué ou non substitué, un cycloalkoxy en $C_3$-$C_4$ substitué ou non substitué ; un alkoxy en $C_1$-$C_4$ alkyle en $C_1$-$C_4$ substitué ou non substitué ; un haloalkyle en $C_1$-$C_4$ substitué ou non substitué ; un haloalkoxy en $C_1$-$C_4$ substitué ou non substitué ; $SO_2R_d$ ; et -$NR_eR_e$ ; ou
- $R_2$ est sélectionné dans le groupe constitué par :

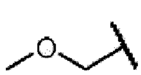 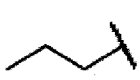 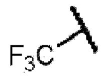 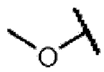 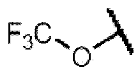

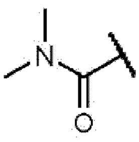 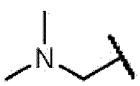 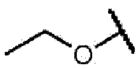 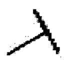 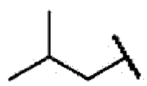

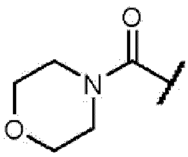 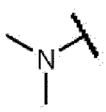 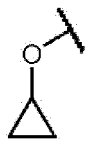 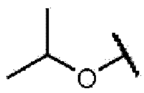 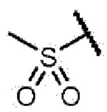

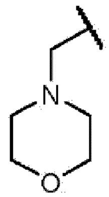 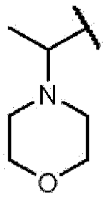 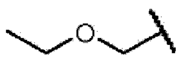 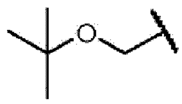

**8.** Le composé d'une quelconque revendication précédente, sachant que

- m est 1 ; et/ou
- $R_3$ est sélectionné indépendamment dans le groupe constitué par : de l'halogène, un alkyle en $C_1$-$C_2$, et un alkoxy en $C_1$-$C_2$.

**9.** Le composé de l'une quelconque des revendications 1 à 7, sachant que m est 0.

**10.** Le composé de l'une quelconque des revendications 1 à 6, sachant que $R_2$ est un résidu d'alkylényle -O- en $C_1$ substitué ou non substitué, sachant que l'atome d'oxygène est lié à l'anneau B ; et un fragment $R_3$ est un résidu -O-, sachant que conjointement avec les atomes auxquels ils sont liés, les résidus d'alkylényle -O- en $C_1$ et -O- forment un anneau à 5 chaînons.

**11.** Le composé d'une quelconque revendication précédente, sachant que

- p est 0 ; ou
- p est 1 ; et/ou
- chaque $R_5$ est sélectionné indépendamment dans le groupe constitué par : de l'halogène, un alkyle en $C_1$-$C_3$, un haloalkyle en $C_1$-$C_3$, un alkoxy en $C_1$-$C_3$, -CN ou -C(O)$NR_eR_e$.

**12.** Le composé d'une quelconque revendication précédente, sachant que

- l'anneau A est sélectionné dans le groupe constitué par : un anneau aromatique et un anneau hétéroaromatique à 6 chaînons, facultativement sachant que l'anneau A est du phényle ; ou
- l'anneau A est un système d'anneau de cycloalkyle à 5 ou 8 chaînons, facultativement sachant que l'anneau A est du bicyclo-[1.1.1]-pentyle.

**13.** Le composé d'une quelconque revendication précédente, sachant que

- l'anneau B est sélectionné dans le groupe constitué par : du phényle, du pyridinyle ou du pyrimidinyle, et/ou
- l'anneau C est sélectionné dans le groupe constitué par : un anneau aromatique à 6 chaînons et un anneau hétéroaromatique à 6 chaînons, facultativement du phényle ou du pyridinyle ; et/ou
- $R_a$ et $R_b$ sont sélectionnés chacun indépendamment dans le groupe constitué par : de l'hydrogène, de l'halogène, un alkyle en $C_1$-$C_4$, et un alkoxy en $C_1$-$C_4$ ; ou sachant que $R_a$ et $R_b$, conjointement avec l'atome auquel ils sont liés, forment un anneau cycloalkyle ou hétérocycloalkyle à 3 à 4 chaînons comprenant -O- ou -$NR_f$-, sachant que $R_f$ est H ou du méthyle, sachant que facultativement $R_a$ et $R_b$ sont sélectionnés chacun indépendamment dans le groupe constitué par : de l'hydrogène et du méthyle.

**14.** Le composé de la revendication 1, sachant que le composé est sélectionné parmi :

| | | | |
|---|---|---|---|
| (1) | 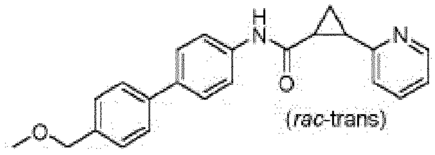 (rac-trans) | (2) | 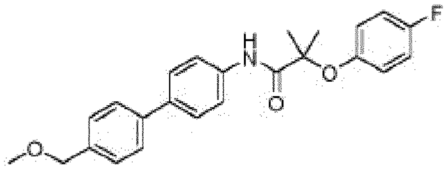 |

(continued)

| | | | |
|---|---|---|---|
| (3) | 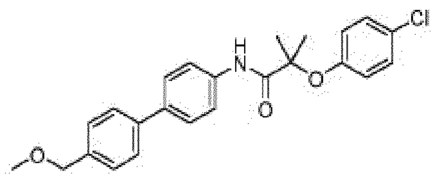 | (4) | 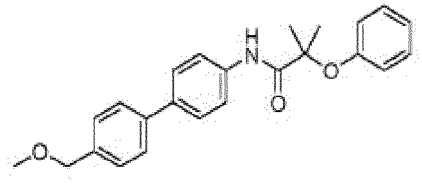 |
| (5) | 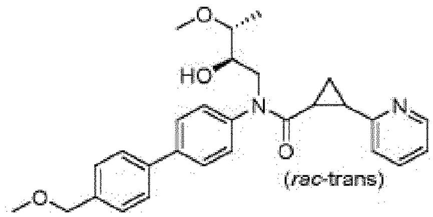 (*rac*-trans) | (6) | 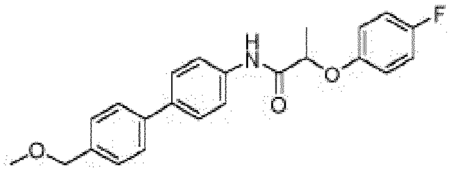 |
| (7) | 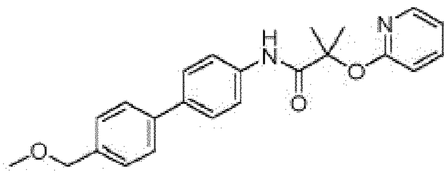 | (8) | 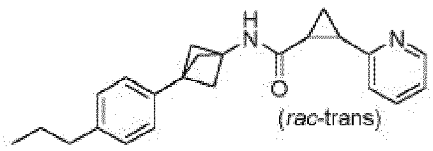 (*rac*-trans) |
| (9) | 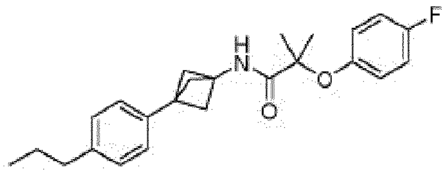 | (10) | 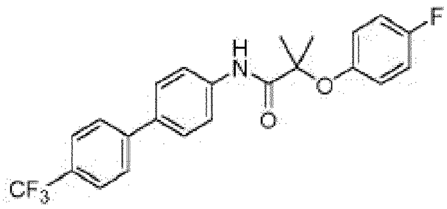 |
| (11) | 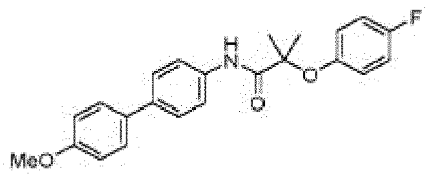 | (12) | 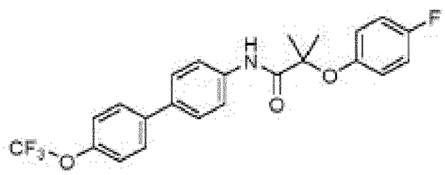 |
| (13) | 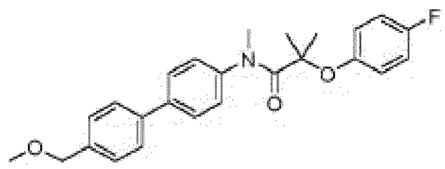 | (14) | 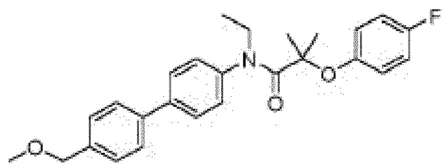 |
| (15) | 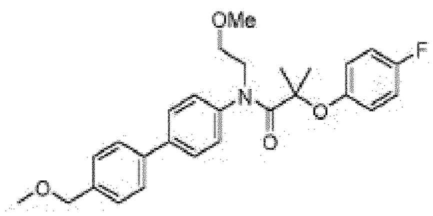 | (16) | 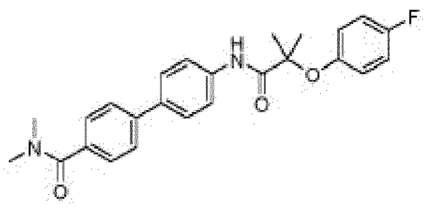 |
| (17) | 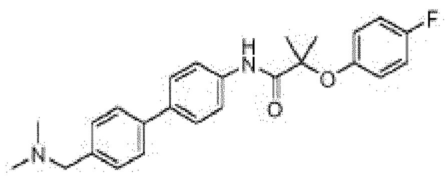 | (18) D1 | 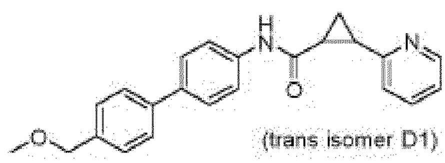 (trans isomer D1) |

(continued)

| | | | |
|---|---|---|---|
| (19) D2 | 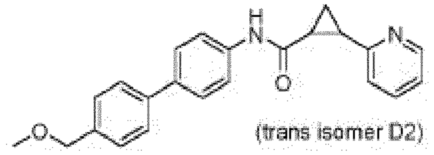 (trans isomer D2) | (20) | 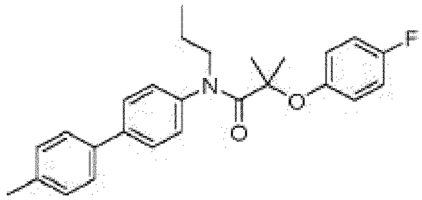 |
| (21) | 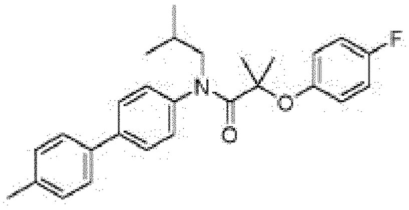 | (22) | 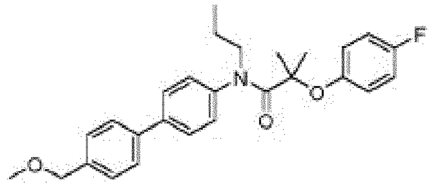 |
| (23) | 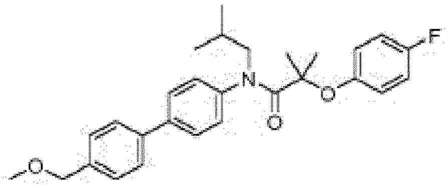 | (24) | 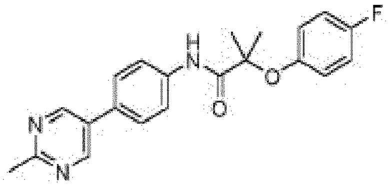 |
| (25) | 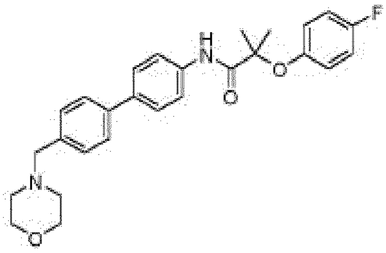 | (26) | 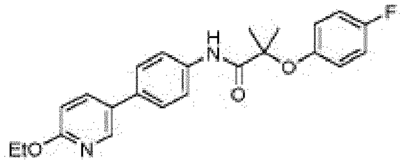 |
| (27) | 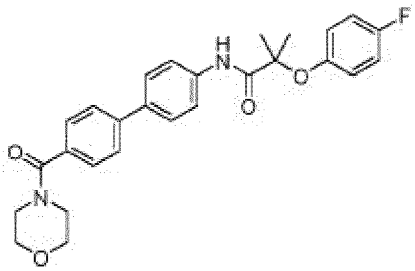 | (28) | 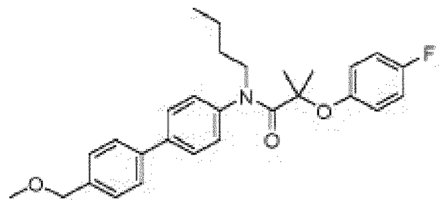 |
| (29) | 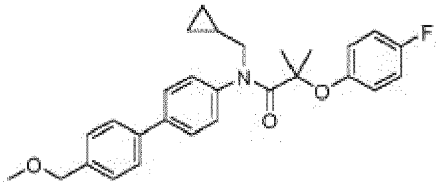 | (30) | 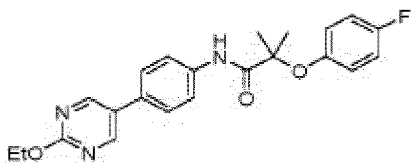 |
| (31) | 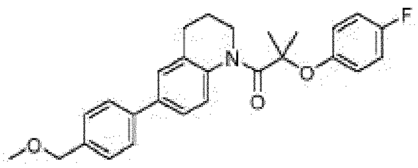 | (32) | 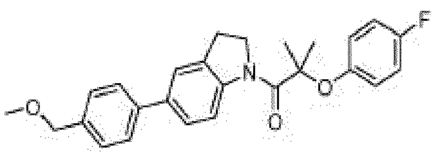 |

(continued)

| | | | |
|---|---|---|---|
| (33) | 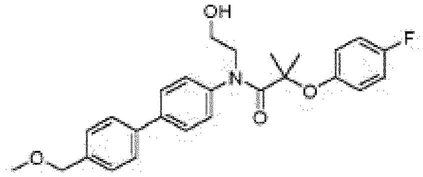 | (34) D1 | 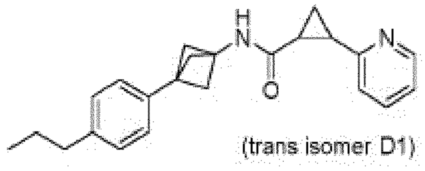 (trans isomer D1) |
| (35) D2 | 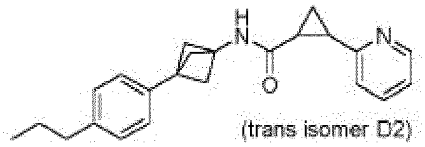 (trans isomer D2) | (36) | 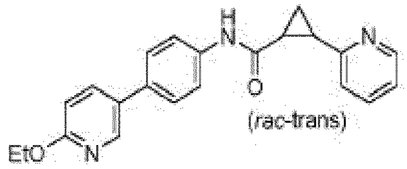 (*rac*-trans) |
| (37) | 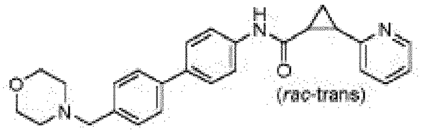 (*rac*-trans) | (38) | 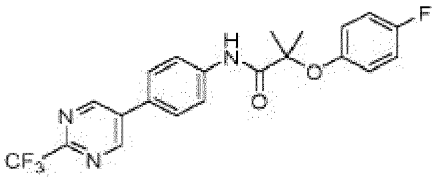 |
| (39) | 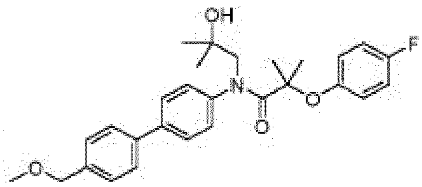 | (40) | 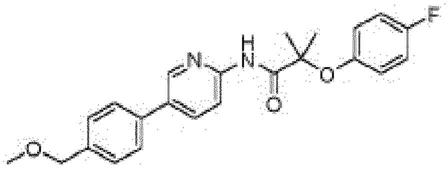 |
| (41) | 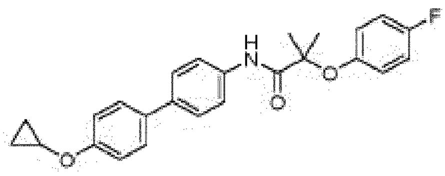 | (42) | 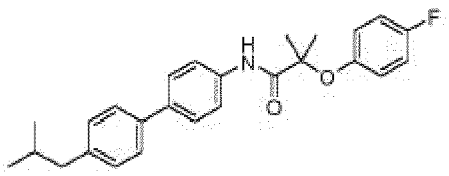 |
| (43) | 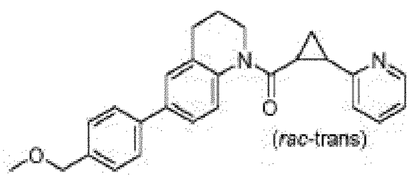 (*rac*-trans) | (44) | 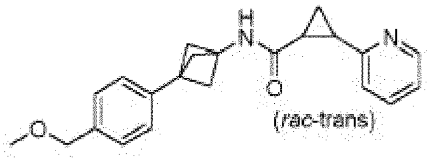 (*rac*-trans) |
| (45) | 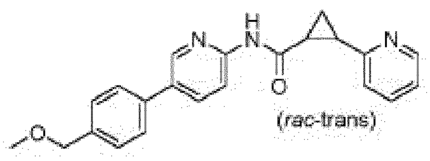 (*rac*-trans) | (46) | 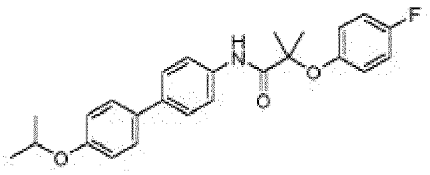 |
| (47) | 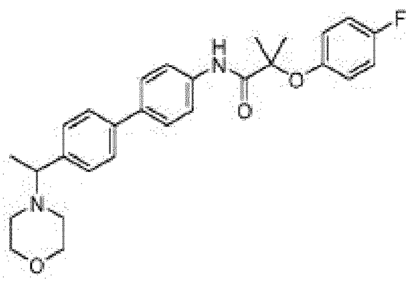 | (48) | 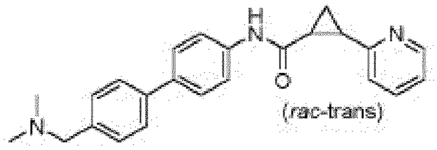 (*rac*-trans) |

(continued)

| | | | |
|---|---|---|---|
| (49) | 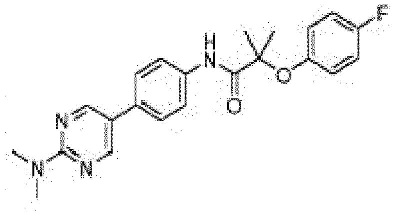 | (50) | 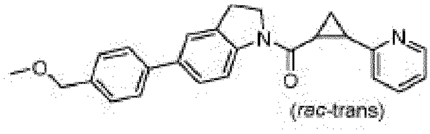 (*rac*-trans) |
| (51) | 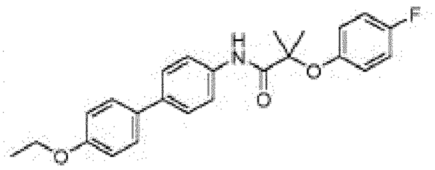 | (52) | 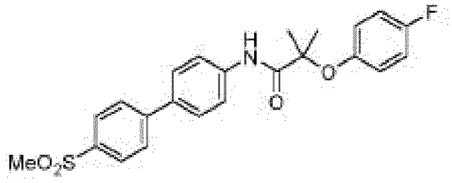 |
| (53) | 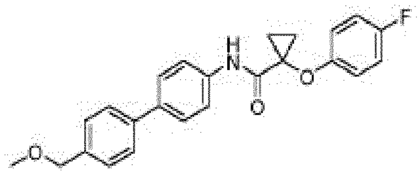 | (54) | 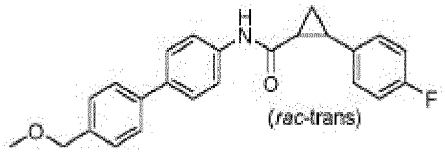 (*rac*-trans) |
| (55)* | 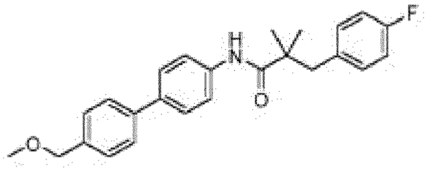 | (56)* | 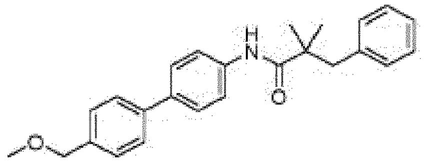 |
| (57) | 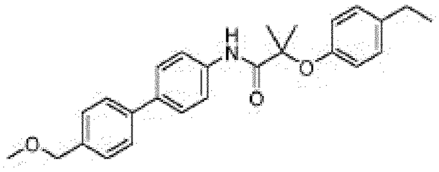 | (58) | 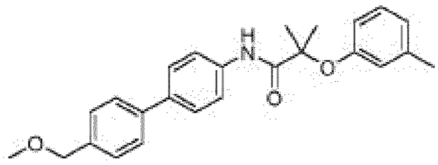 |
| (59) | 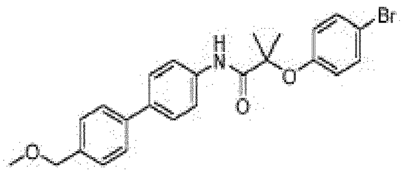 | (60) | 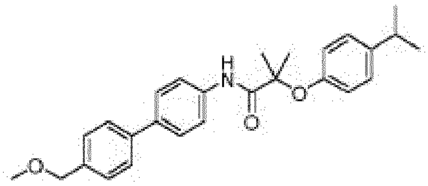 |
| (61) | 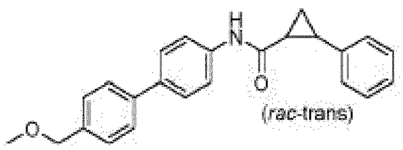 (*rac*-trans) | (62) | 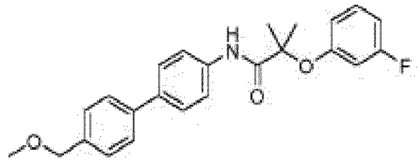 |
| (63) | 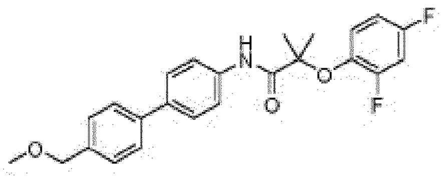 | (64) | 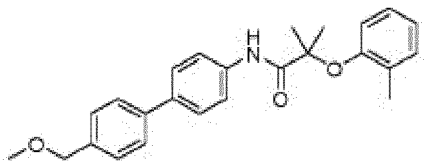 |

(continued)

| | | | |
|---|---|---|---|
| (65) | 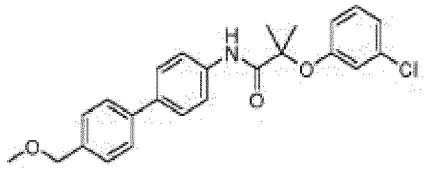 | (66) | 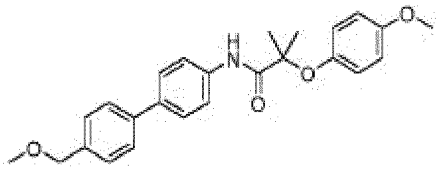 |
| (67) | 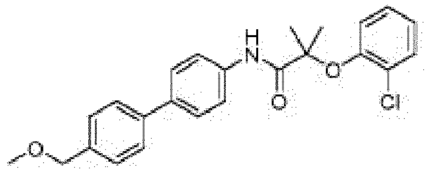 | (68) | 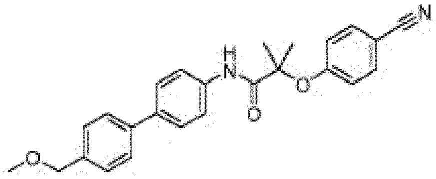 |
| (69) | 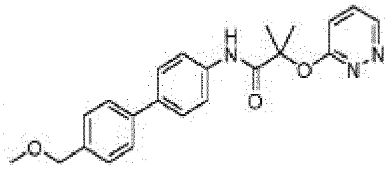 | (70) | 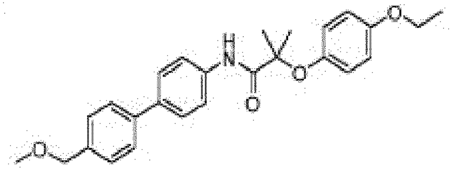 |
| (71) | 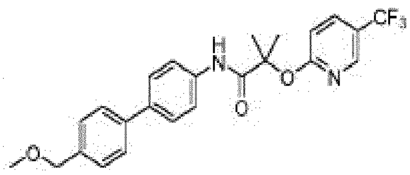 | (72) | 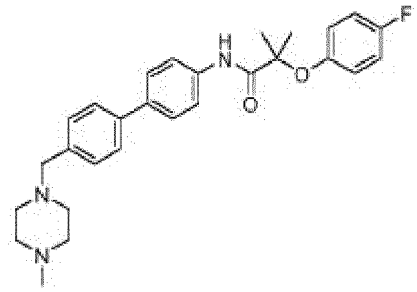 |
| (73) D1 | 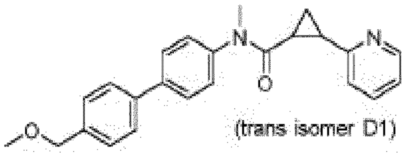 (trans isomer D1) | (74) D2 | 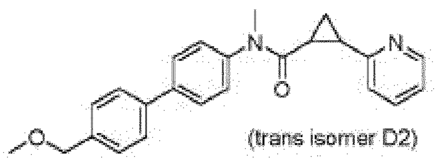 (trans isomer D2) |
| (75) D1 | 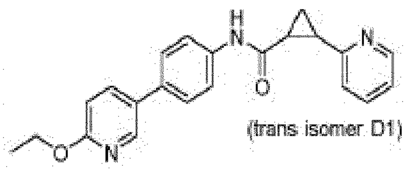 (trans isomer D1) | (76) D2 | 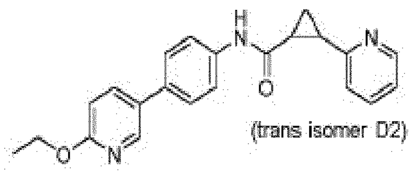 (trans isomer D2) |
| (77) D1 | 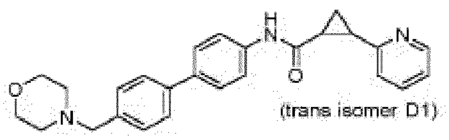 (trans isomer D1) | (78) D2 | 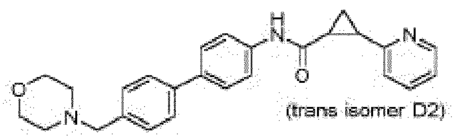 (trans isomer D2) |
| (79) | 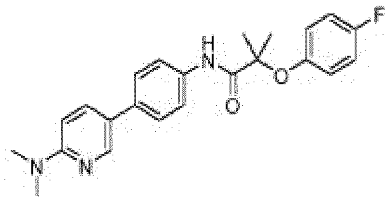 | (80) | 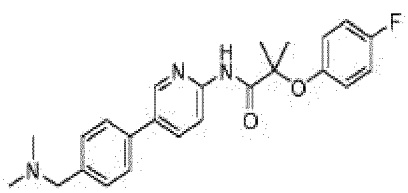 |

(continued)

| | | | |
|---|---|---|---|
| (81) | 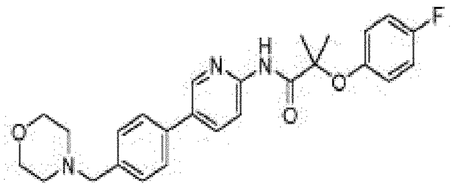 | (82) | 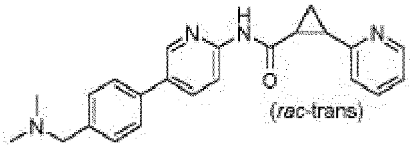 (rac-trans) |
| (83) | 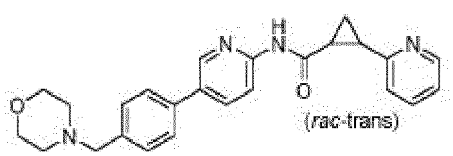 (rac-trans) | (84) | 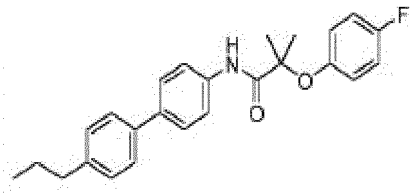 |
| (85) | 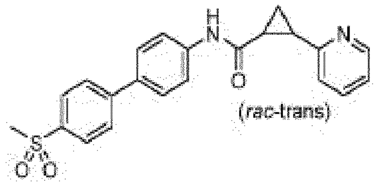 (rac-trans) | (86) | 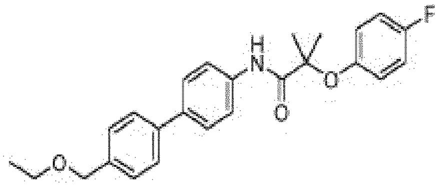 |
| (87) | 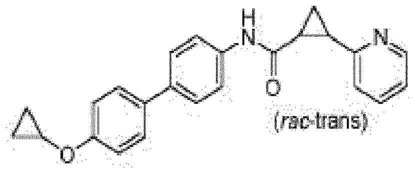 (rac-trans) | (88) | 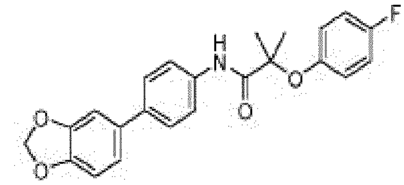 |
| (89) | 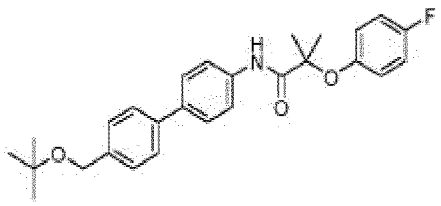 | (90) | 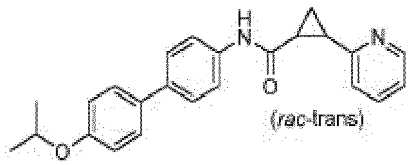 (rac-trans) |
| (91) | 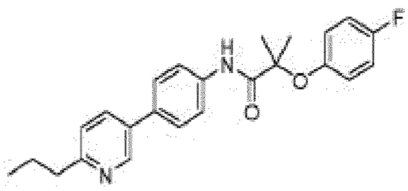 | (92) | 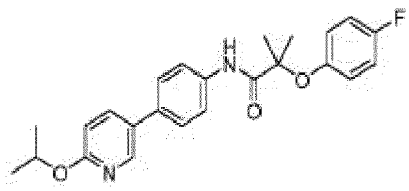 |
| (93) | 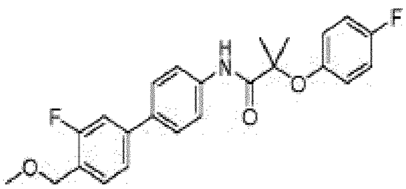 | (94) | 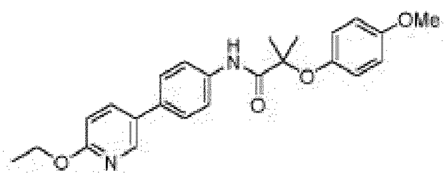 |
| (95) | 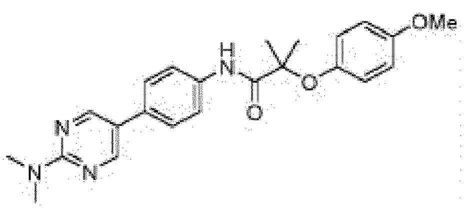 | (96) | 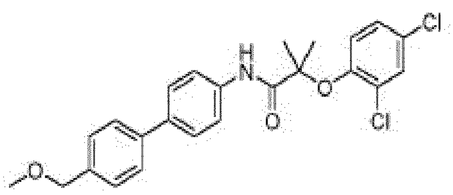 |

(continued)

| | | | |
|---|---|---|---|
| (97) | 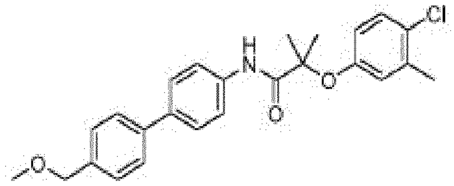 | (98) | 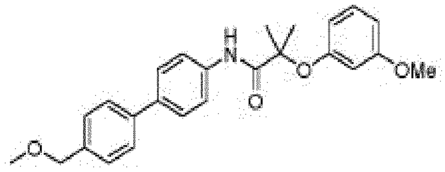 |
| (99) | 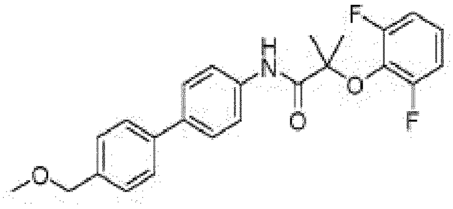 | (100) | 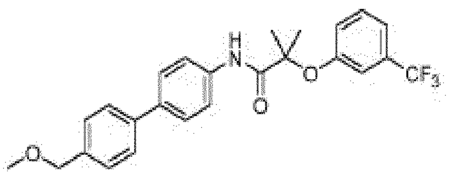 |
| (101) | 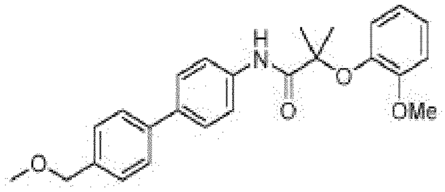 | (102) | 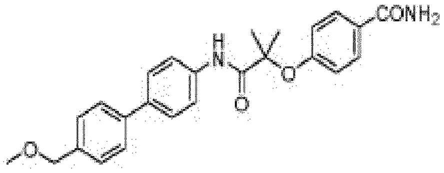 |
| (103) | 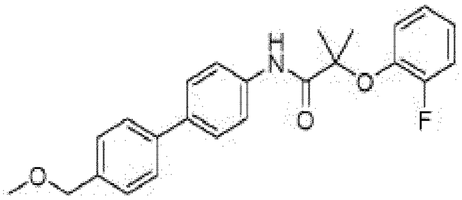 | (104) | 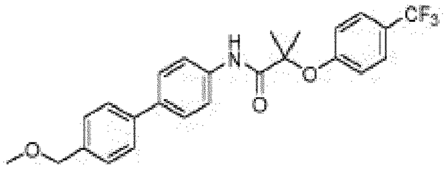 |
| (105) | 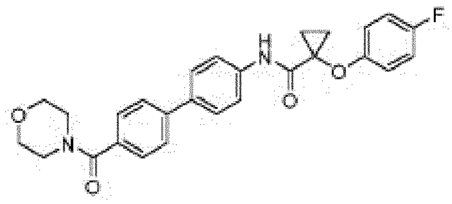 | (106) | 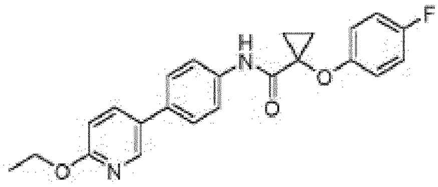 |
| (107) | 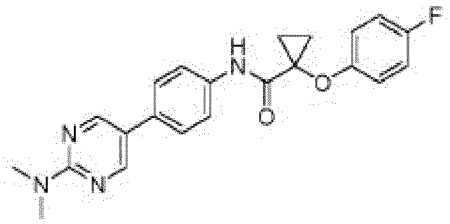 | (108) | 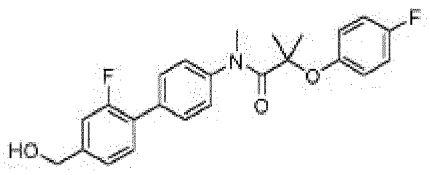 |
| (109) | 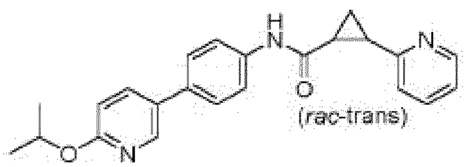 (rac-trans) | (110) D1 | 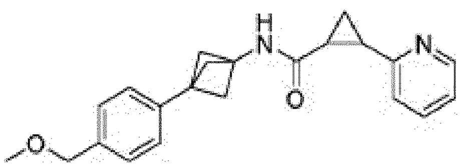 |
| (111) D2 | 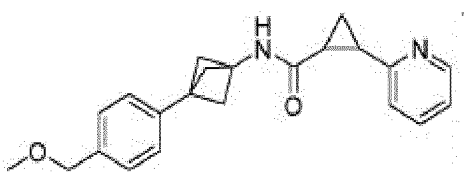 | (112) D3 | 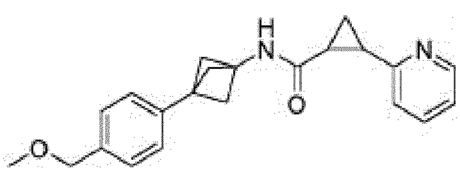 |

(continued)

| | | | |
|---|---|---|---|
| (113) | 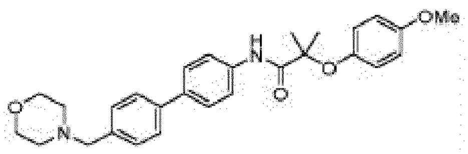 | (114) | 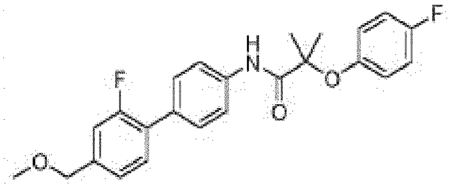 |
| (115) | 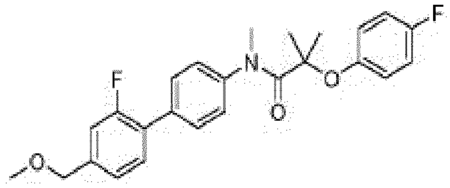 | (116) | 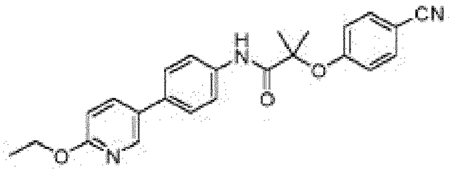 |
| (117) | 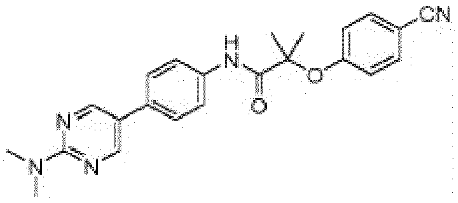 | (118) | 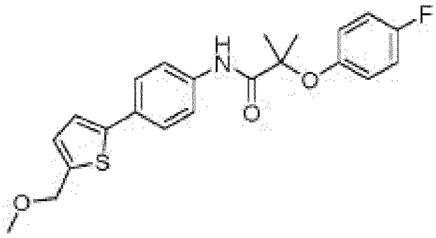 |
| (119) | 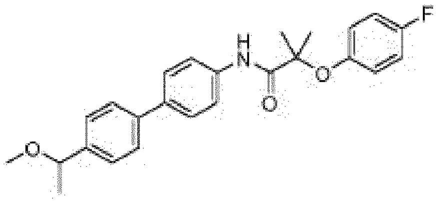 | (120) D1 | 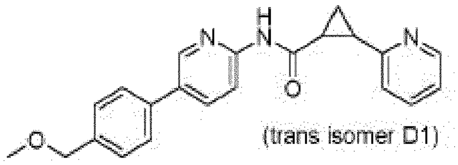 (trans isomer D1) |
| (121) D2 | 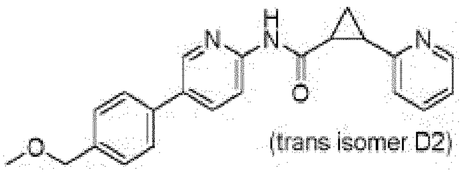 (trans isomer D2) | (122) E1 | 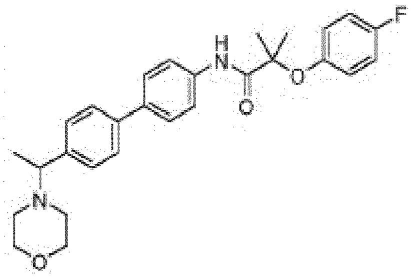 |
| (123) E2 | 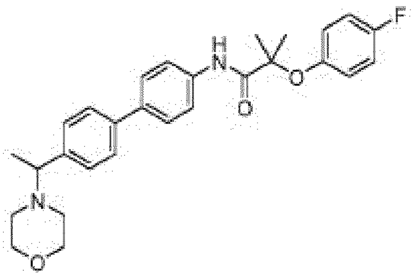 | (124) | 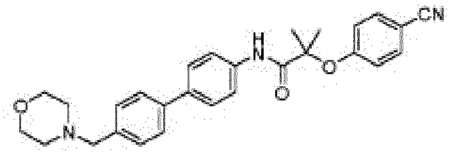 |
| (125) | 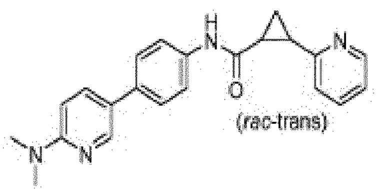 (*rac*-trans) | (126) | 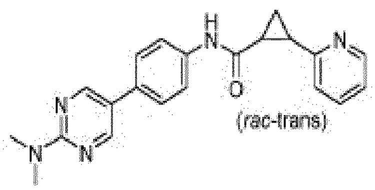 (*rac*-trans) |

(continued)

| | | | |
|---|---|---|---|
| (127) | 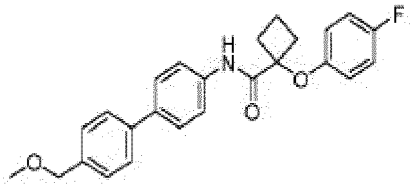 | (128) | 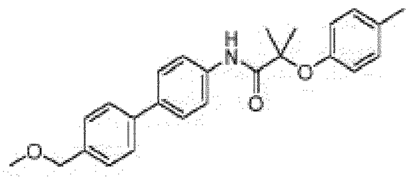 |
| (129) | 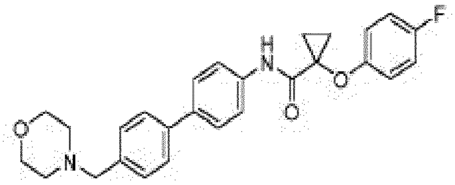 | (130) D2 | 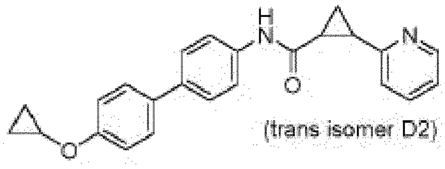 (trans isomer D2) |
| (131) | 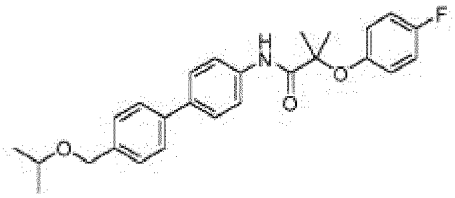 | (132) | 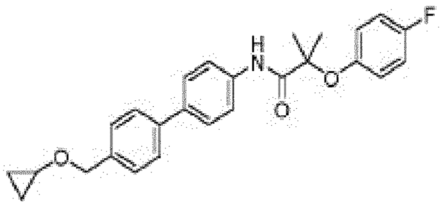 |
| (133) D1 | 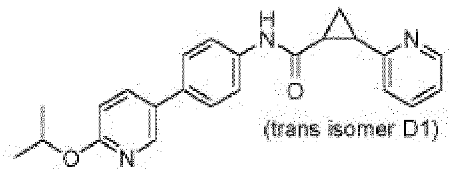 (trans isomer D1) | (134) D2 | 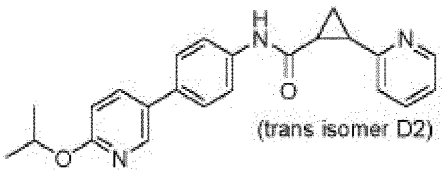 (trans isomer D2) |
| (135) | 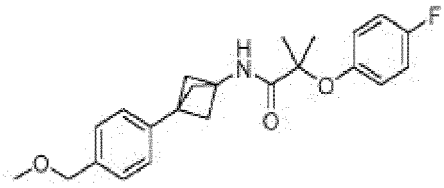 | (136) | 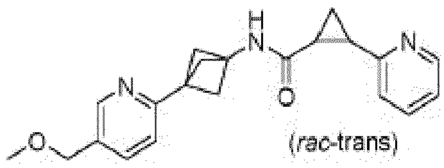 (*rac*-trans) |
| (137) | 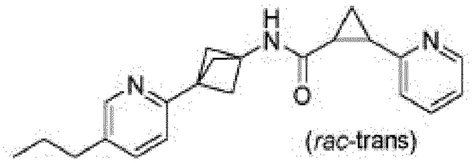 (*rac*-trans) | (138) | 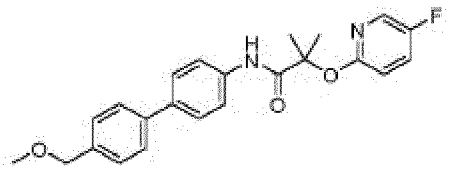 |
| (139) | 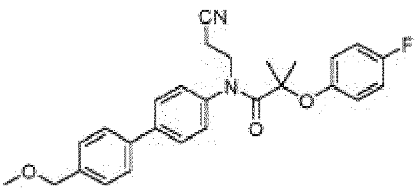 | (140) | 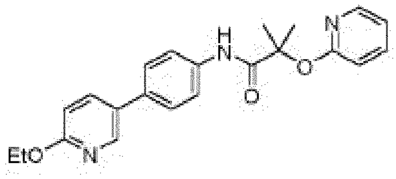 |
| (141) D1 | 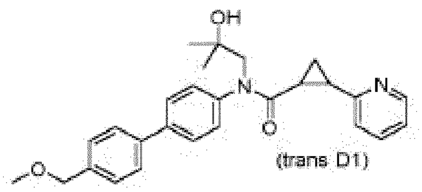 (trans D1) | (142) D2 | 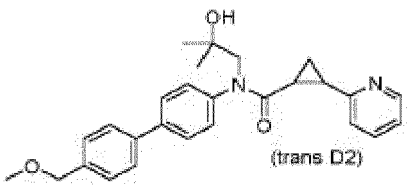 (trans D2) |

ou sel pharmaceutiquement acceptable de celui-ci.

**15.** Le composé de la revendication 1 ou 14, sachant que le composé est sélectionné parmi :

| | | | |
|---|---|---|---|
| (1) | 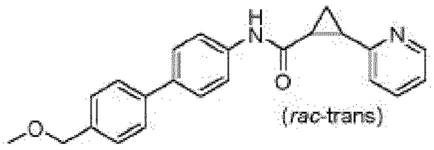 (*rac*-trans) | (2) | 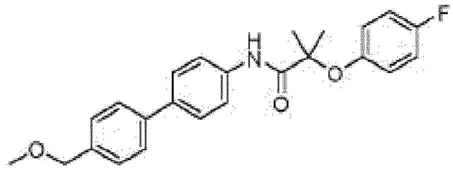 |
| (3) | 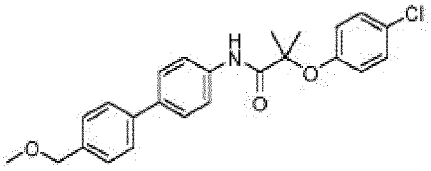 | (4) | 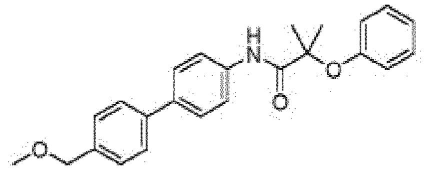 |
| (7) | 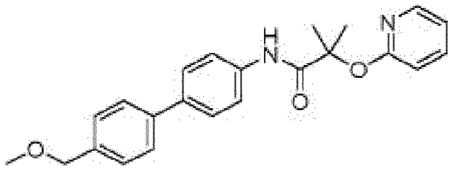 | (8) | 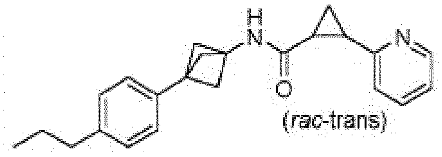 (*rac*-trans) |
| (9) | 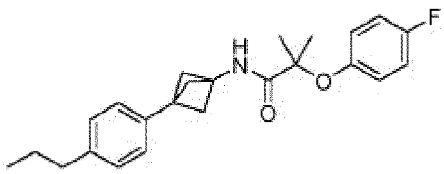 | (10) | 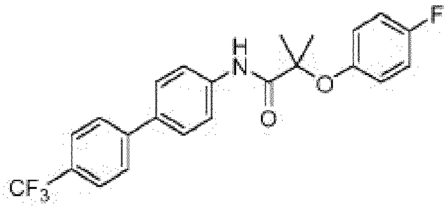 |
| (11) | 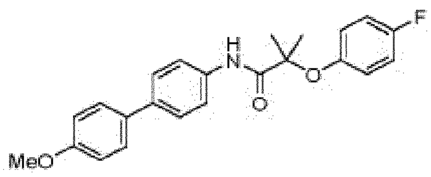 | (12) | 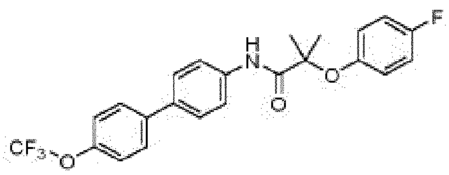 |
| (13) | 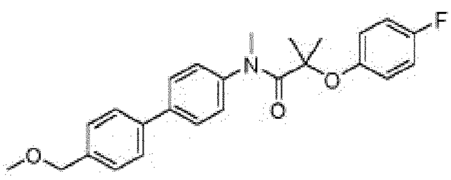 | (14) | 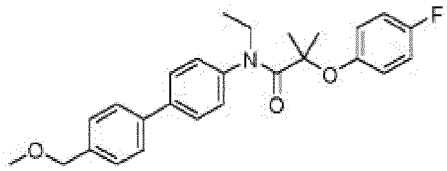 |
| (15) | 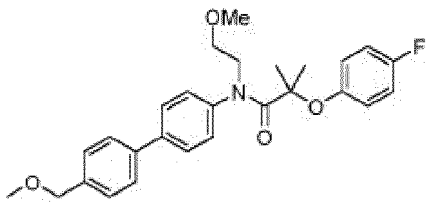 | (17) | 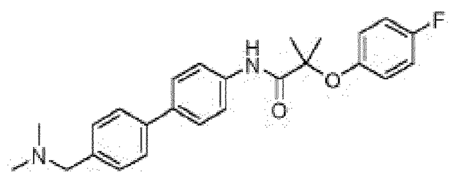 |
| (18) D1 | 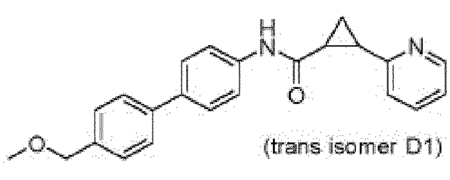 (trans isomer D1) | (22) | 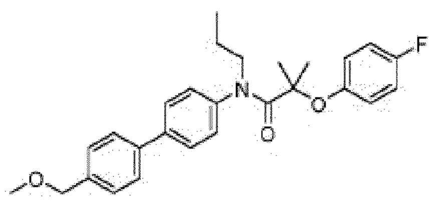 |

(continued)

| | | | |
|---|---|---|---|
| (25) | 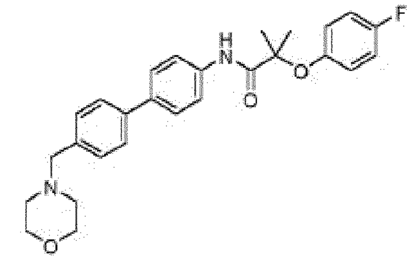 | (26) | 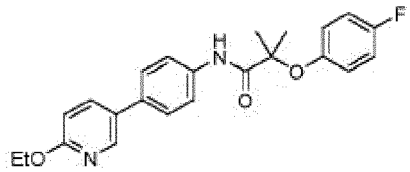 |
| (27) | 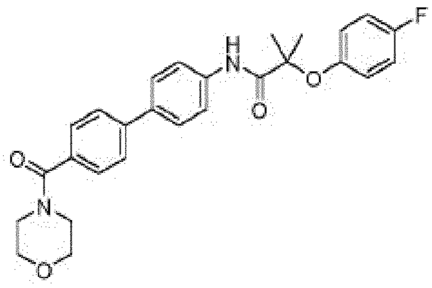 | (28) | 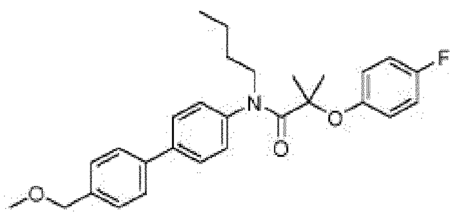 |
| (29) | 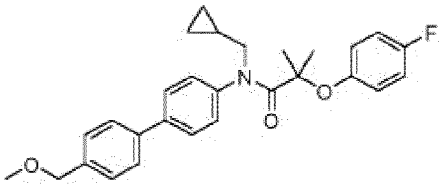 | (30) | 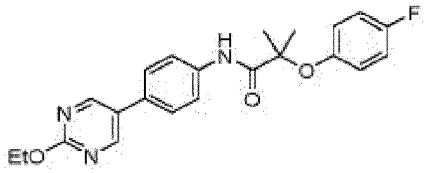 |
| (31) | 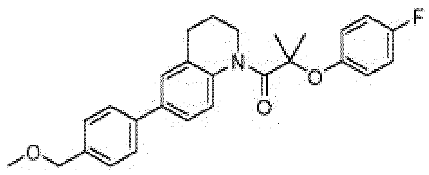 | (32) | 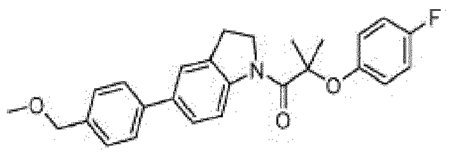 |
| (33) | 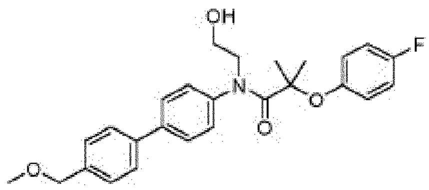 | (34) D1 | 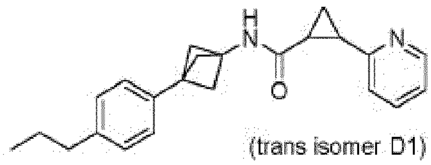 (trans isomer D1) |
| (35) D2 | 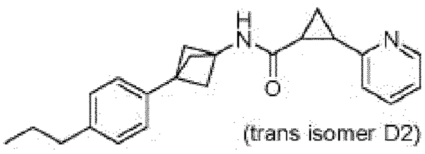 (trans isomer D2) | (36) | 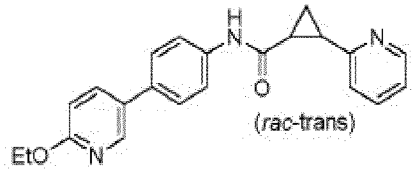 (*rac*-trans) |
| (37) | 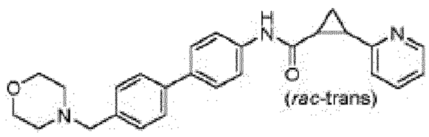 (*rac*-trans) | (39) | 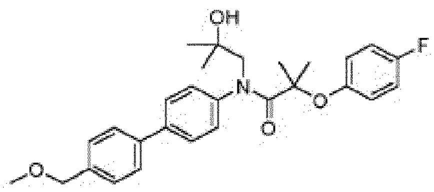 |

(continued)

| | | | |
|---|---|---|---|
| (40) | 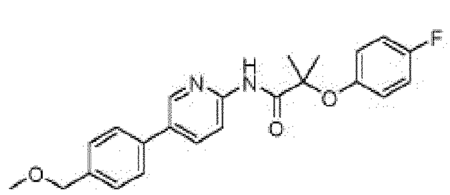 | (44) | 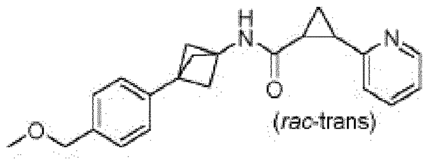 |
| (45) | 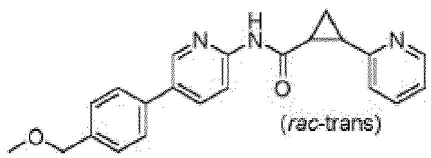 | (46) | 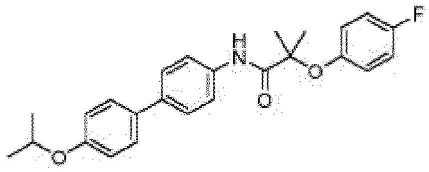 |
| (47) | 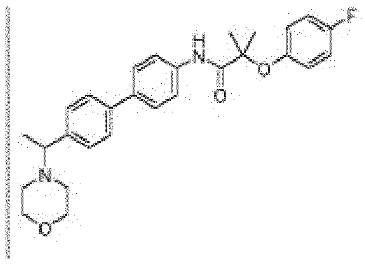 | (49) | 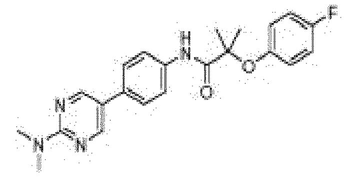 |
| (50) | 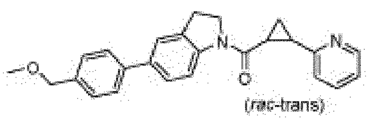 | (53) | 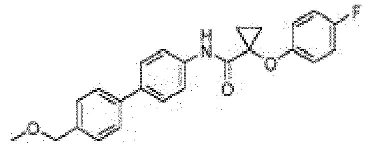 |
| (55)* | 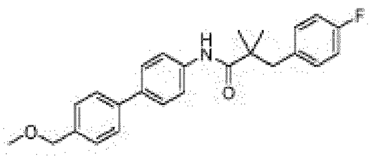 | (56)* | 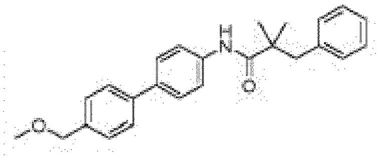 |
| (57) | 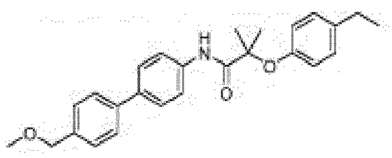 | (58) | 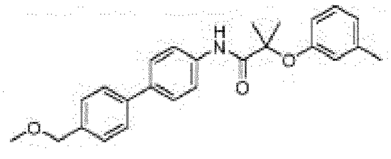 |
| (59) | 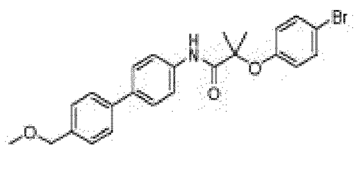 | (60) | 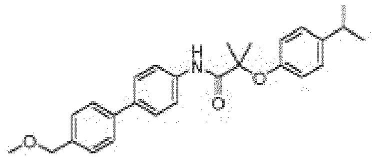 |
| (62) | 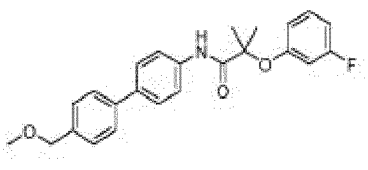 | (63) | 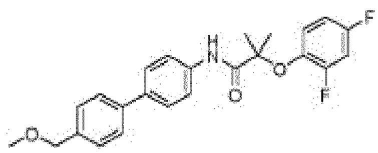 |

(continued)

| | | | |
|---|---|---|---|
| (64) | 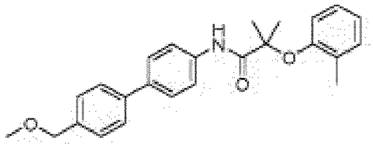 | | |
| (65) | 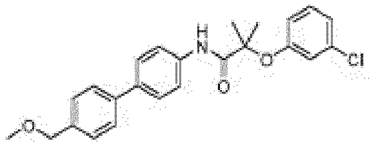 | (26) | 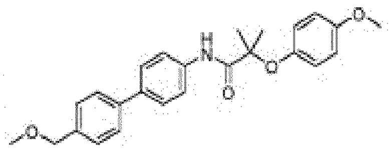 |
| (67) | 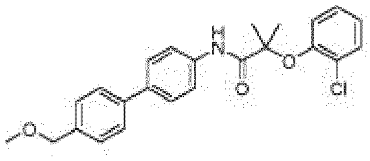 | (68) | 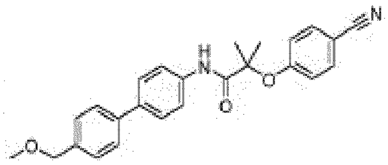 |
| (70) | 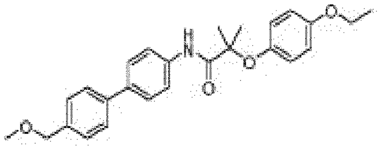 | (72) | 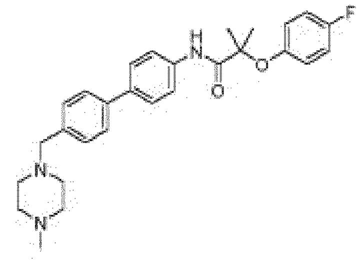 |
| (75) D1 | 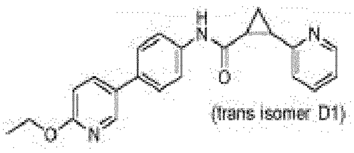 (trans isomer D1) | (76) D2 | 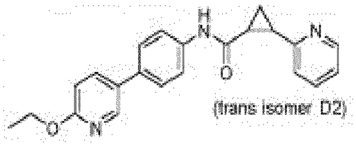 (trans isomer D2) |
| (77) D1 | 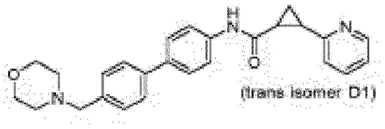 (trans isomer D1) | (78) D2 | 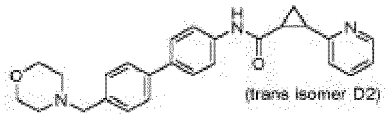 (trans isomer D2) |
| (19) | 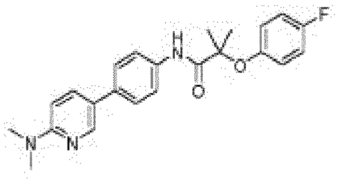 | (80) | 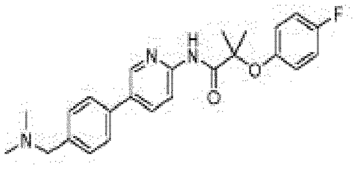 |
| (81) | 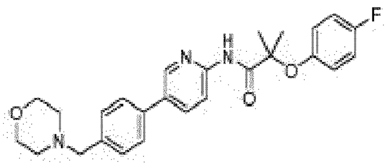 | (83) | 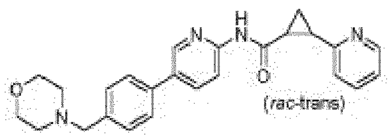 (*rac*-trans) |

(continued)

| | | | |
|---|---|---|---|
| (85) | 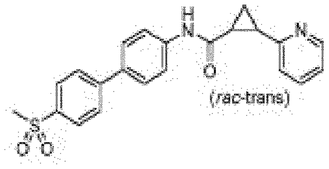 (*rac*-trans) | (26) | 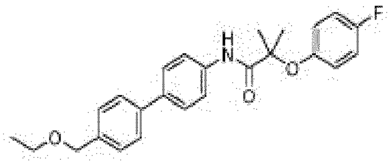 |
| (87) | 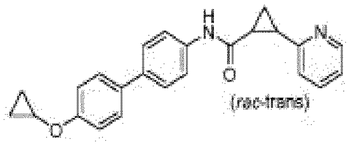 (*rac*-trans) | (88) | 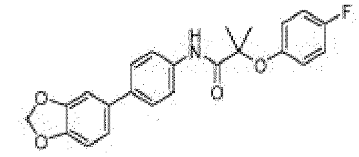 |
| (89) | 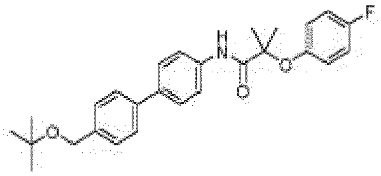 | (90) | 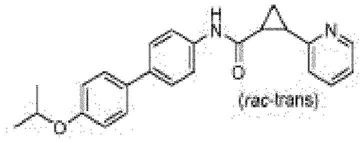 (*rac*-trans) |
| (91) | 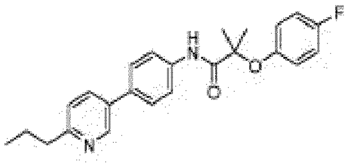 | (92) | 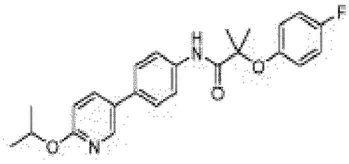 |
| (94) | 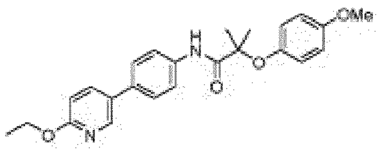 | (95) | 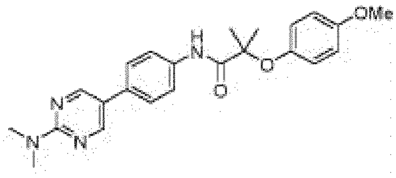 |
| (97) | 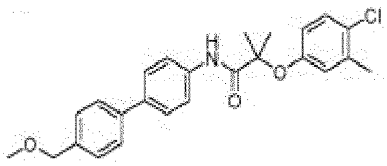 | (98) | 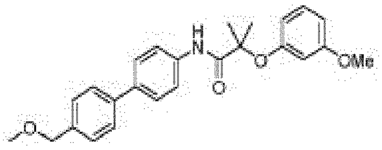 |
| (99) | 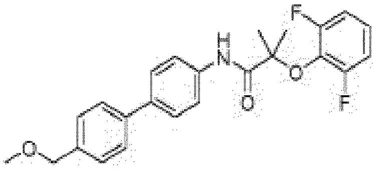 | (100) | 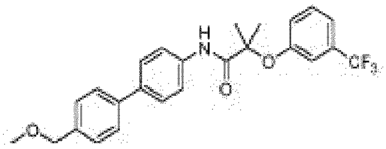 |
| (101) | 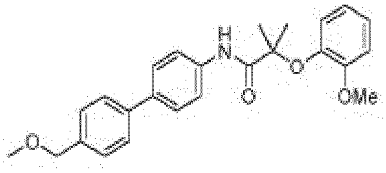 | (102) | 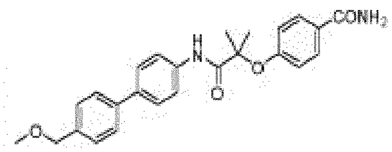 |

(continued)

| | | | |
|---|---|---|---|
| (103) | 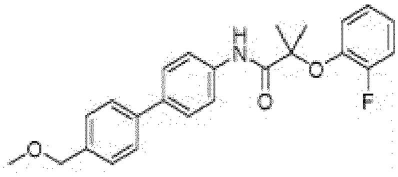 | (104) | 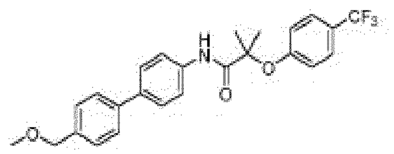 |
| (105) | 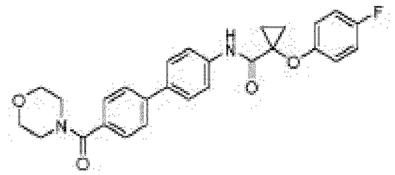 | (106) | 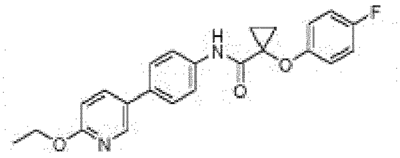 |
| (107) | 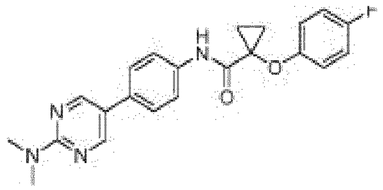 | (108) | 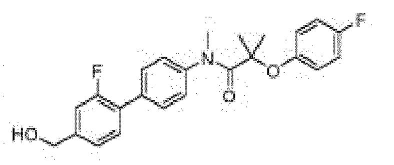 |
| (109) | 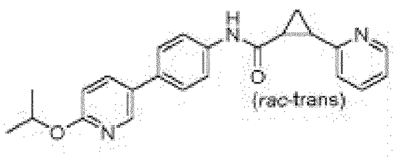 | (110) D1 | 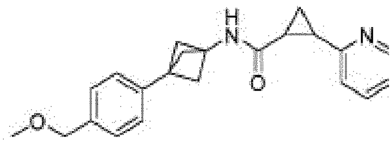 |
| (111) D2 | 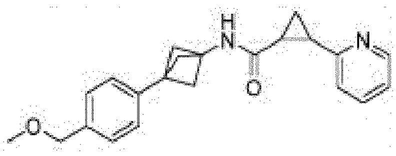 | | |
| (113) | 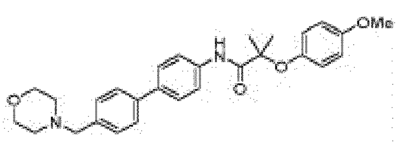 | (114) | 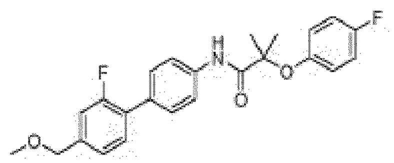 |
| (115) | 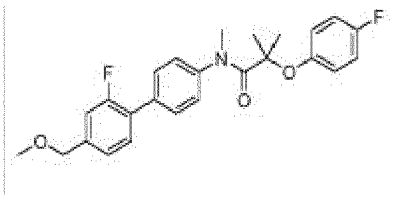 | (116) | 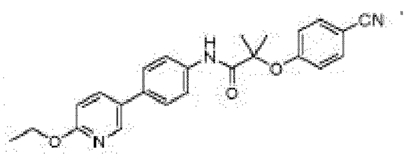 |
| (117) | 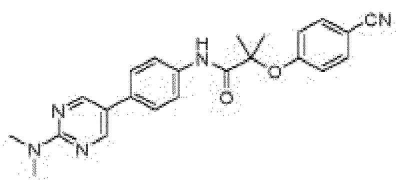 | | |

(continued)

| | | | |
|---|---|---|---|
| (119) | 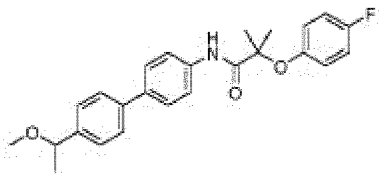 | (120) D1 | 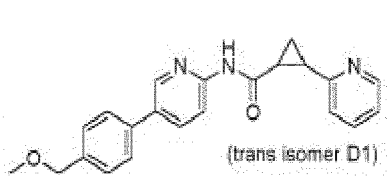 (trans isomer D1) |
| (121) D2 | 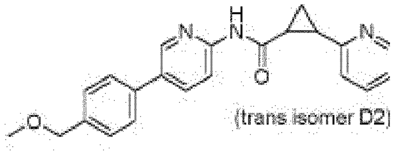 (trans isomer D2) | (122) E1 | 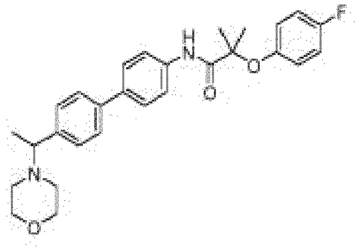 |
| (123) E2 | 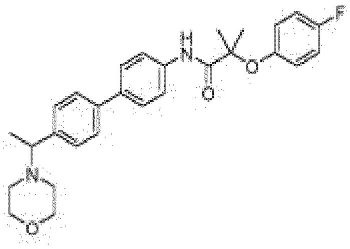 | (124) | 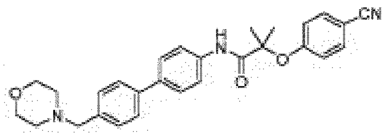 |
| (127) | 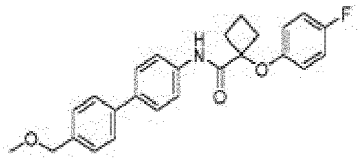 | (128) | 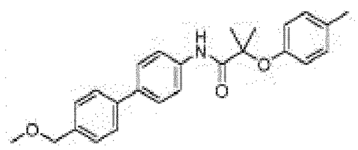 |
| (129) | 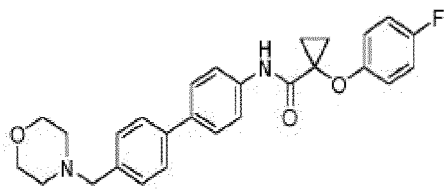 | (132) | 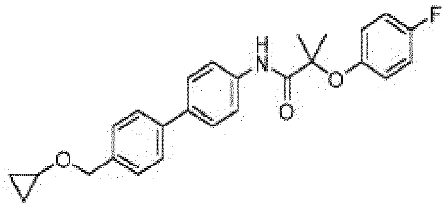 |
| (133) D1 | 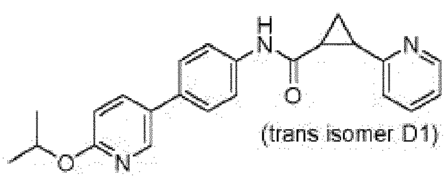 (trans isomer D1) | | |
| (135) | 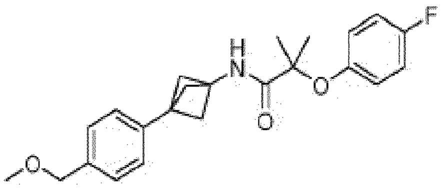 | (136) | 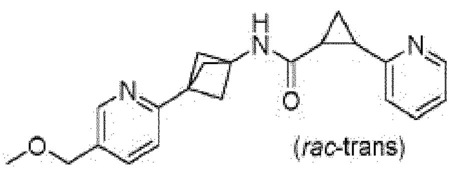 (*rac*-trans) |

(continued)

| | | | |
|---|---|---|---|
| (137) | 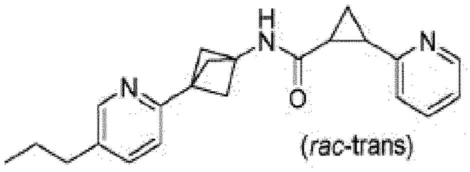 | (138) | 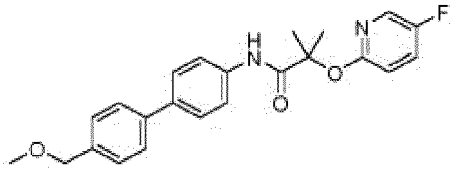 |
| (140) | 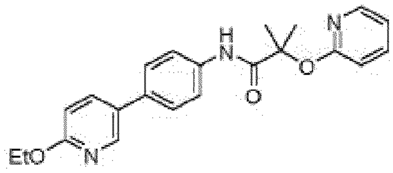 | | |

ou sel pharmaceutiquement acceptable de celui-ci.

**16.** Formulation pharmaceutique comprenant un composé de l'une quelconque des revendications 1 à 15, ou un sel pharmaceutiquement acceptable de celui-ci, et un excipient pharmaceutiquement acceptable.

**17.** Composé de l'une quelconque des revendications 1 à 15, ou sel pharmaceutiquement acceptable de celui-ci, destiné à être utilisé comme médicament ; ou destiné à être utilisé dans le traitement d'une maladie médiée par GPR88 ; ou destiné à être utilisé dans le traitement du syndrome de la Tourette, de la maladie de Huntington (HD), de l'addiction, de la maladie de Parkinson (PD), de la schizophrénie, et du trouble déficitaire de l'attention avec hyperactivité (ADHD), des mouvements choréiformes, du retard de la parole, des troubles d'apprentissage, de la dépression, des troubles du mouvement hypercinétiques **caractérisés par** la chorée et/ou la dystonie, de la psychose, des déficits cognitifs dans la schizophrénie, des troubles affectifs, du trouble bipolaire, de la maladie d'Alzheimer et des troubles des ganglions de la base.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011044212 A **[0013]**


### Non-patent literature cited in the description


- **BI et al.** *Bioorganic & Medicinal Chemistry Letters*, 2015, vol. 25, 1443-1447 **[0011]**
- **JIN et al.** *ACS Chem. Neurosci.*, 2014, vol. 5 (7), 576-587 **[0012]**
- **JIN et al.** *ACS Chem Neurosci.*, 19 October 2016, vol. 7 (10), 1418-1432 **[0012]**
- **JERRY MARCH**. Advanced Organic Chemistry. Wiley Interscience, 1992, 131-133 **[0178]**
- **M. E. AULTON**. Pharmaceuticals - The Science of Dosage Form Designs. Churchill Livingstone, 1988 **[0213]**
- **JIN, C et al.** *J. Med. Chem.*, 2018, vol. 61, 6748-6758 **[0314] [0323] [0324]**
- **GHATTAS et al.** *Mol. Cell. Biol.*, 1991, vol. 11, 5848-5859 **[0319]**
- **JANOWSKY, A. et al.** *Neurochem*, 1986, vol. 46, 1272-1276 **[0324]**